# EUROPEAN PATENT SPECIFICATION

(11) **EP 3 922 640 B1**
(45) Date of publication and mention of the grant of the patent: **08.11.2023**
(21) Application number: 20752192.3
(22) Date of filing: 06.02.2020
(51) Int. Cl.: C07K 14/435, G01N 33/68, G01N 33/569

(54) **METHOD FOR DIAGNOSING HUMAN T-CELL LEUKEMIA VIRUS TYPE 1 (HTLV-1) ASSOCIATED DISEASES**
VERFAHREN ZUR DIAGNOSE VON MIT MENSCHLICHEM T-ZELL-LEUKÄMIEVIRUS TYP 1 (HTLV-1) ASSOZIIERTEN KRANKHEITEN
MÉTHODE DE DIAGNOSTIC DE MALADIES ASSOCIÉES AU VIRUS DE TYPE 1 DE LA LEUCÉMIE À LYMPHOCYTES T HUMAINE (HTLV-1)

(30) Priority: 08.02.2019 JP 2019021539; 05.11.2019 JP 2019200955
(43) Date of publication of application: 15.12.2021
(73) Proprietor: NEC Corporation, 108-8001 Tokyo (JP)
(72) Inventor: YOSHIHARA, Yoshiko, Tokyo 108-8001 (JP); FUKUSHIMA, Takuya, Nakagami-gun, Okinawa 903-0125 (JP); MIYARA, Megumi, Nakagami-gun, Okinawa 903-0125 (JP)
(74) Representative: Vossius & Partner Patentanwälte Rechtsanwälte mbB
(86) International application number: PCT/JP2020/004458
(87) International publication number: WO 2020/162517

(56) References cited:
- WO-A1-2014/175375
- JP-A- 2005 147 798
- JP-A- 2005 147 798
- JP-A- 2005 533 524
- JP-A- 2013 083 670
- JP-A- 2014 059 298
- JP-A- 2014 059 299
- JP-A- 2014 163 769
- JP-A- 2016 114 360
- US-A1- 2002 132 370
- US-A1- 2010 086 948
- US-A1- 2017 242 043
- US-A1- 2018 188 267
- TANAKA YUETSU ET AL: "Association of high levels of plasma OX40 with acute adult T-cell leukemia", INTERNATIONAL JOURNAL OF HEMATOLOGY, ELSEVIER SCIENCE PUBLISHERS, NL, vol. 109, no. 3, 16 January 2019 (2019-01-16), pages 319-327, XP036710656, ISSN: 0925-5710, DOI: 10.1007/S12185-018-02580-Z [retrieved on 2019-01-16]
- TAKEMOTO, SHIGEKI et al.: "Plasma Soluble CD 30 as a Possible Marker of Adult T-cell Leukemia in HTLV-1 Carriers: a Nested Case-Control Study", Asian Pac. J. Cancer Prev., vol. 16, no. 18, 2015, pages 8253-8258, XP055730930,

## Description

### Technical Field

The present invention relates to a method for diagnosing human T-cell leukemia virus type 1 (HTLV-1) associated diseases, more specifically, a method for diagnosing adult T-cell leukemia (ATL) using a blood marker protein, i.e., coagulation factor Xa, and a use of the marker protein coagulation factor Xa for diagnosing ATL.

### Background Art

Human T-cell leukemia virus type 1 (HTLV-1) is a retrovirus principally infects CD4-positive T cells to cause adult T-cell leukemia (ATL). HTLV-1 also causes HTLV-1 associated myelopathy (HAM) and HTLV-1 uveitis (HU). Hereinafter ATL, HAM and HU will be collectively referred to as "HTLV-1 related diseases".

ATL is a malignant hematological tumor that invades various organs throughout the body and categorized into 4 disease forms: acute form, lymphoma-form, chronic form and smoldering form. The acute and lymphoma forms, which are also referred to "aggressive ATL", are hematopoietic malignancies with the poorest prognosis. Whereas, the chronic and smoldering forms, which are also referred to as "indolent ATL", mostly develop into blast crisis during the time course of the disease. The prognosis of these forms is poor.

As a treatment for ATL, a combination chemotherapy is known. However, even in a current reports, the median survival time of "aggressive ATL" still remains 13 months, which and not satisfactory. Patients with "Indolent ATL", in some cases, survive for a long time without treatment and are mostly monitored without treatment until the disease worsens due to blast crisis and the like.

Currently, a main infection route of HTLV-1 is considered as a mother-to-child transmission, particularly through mother milk. HTLV-1 related diseases are developed from individuals infected with HTLV-1 (HTLV-1 carriers) but most of the carriers are asymptomatic. Japan is only one country having a high prevalence of HTLV-1 among the developed countries. HTLV-1 carriers and HTLV-1 related diseases are often seen in the southwest of Japan including Kyushu and Okinawa districts.

In connection with the present invention, Patent Literature 1 discloses "a diagnostic agent for adult T-cell leukemia, containing a reagent that can detect the presence or absence of a transcript of TSLC1 gene or TSLC1 protein in cells or serum". The TSLC1 gene is considered as a gene specifically expressed in leukemia cells taken from ATL patients. Owing to the technique disclosed there, it is reported that T cell leukemia can be highly precisely diagnosed.

### Citation List

### Patent Literature

Patent Literature 1: Japanese Patent Laid-Open No. 2005-147798

### Non Patent Literature

Non Patent Literature 1: "Measurement of Cetuximab and Panitumumab-Unbound Serum EGFR Extracellular Domain Using an Assay Based on Slow Off-Rate Modified Aptamer (SOMAmer) Reagents", NJ Park et al., PLOS ONE, 2013, 8 (4): e61002.

### Summary of Invention

### Technical Problem

Compared to Kyushu and Okinawa districts where many experienced ATL specialist physicians and core hospitals are present, in metropolitan areas such as Kanto and Kinki districts, even though the real numbers of HTLV-1 carriers and ATL patients are equal to those in a high prevalence area of HTLV-1, the number of specialist physicians capable of diagnosing ATL is low at present.

In the circumstances, a main object of the present invention is to provide a technique that can realize a simple and accurate diagnosis of an HTLV-1 related disease, i.e., adult T-cell leukemia (ATL).

### Solution to Problem

The present invention is defined by the enclosed claims. The invention relates to a diagnostic method for adult T-cell leukemia (ATL), comprising the steps of: measuring an amount of a marker protein in a blood sample taken from a subject, wherein the marker protein is Coagulation Factor Xa; and if a measured value is a predetermined reference value or more, determining that when the subject is a carrier of human T-cell leukemia virus type 1 (HTLV-1) or an individual in remission of ATL, the subject suffers from, or is likely to develop, ATL. The present invention further relates to a use of the protein coagulation factor Xa for diagnosing adult T-cell leukemia (ATL).

### Advantageous Effects of Invention

The present invention provides a technique that can realize a simple and accurate diagnosis of adult T-cell leukemia (ATL).

### Brief Description of Drawings

[Figure 1] The figure is a graph showing measurement results of the amount of a protein in plasmas of HTLV-1 carriers and ATL patients.
[Figure 2] The figure is a graph showing measurement results of the amount of a protein in plasmas of HTLV-1 carriers and ATL patients.
[Figure 3] The figure is a graph showing measurement results of the amount of a protein in plasmas of HTLV-1 carriers and ATL patients.
[Figure 4] The figure is a graph showing measurement results of the amount of a protein in plasmas of HTLV-1 carriers and ATL patients.
[Figure 5] The figure is a graph showing measurement results of the amount of a protein in plasmas of HTLV-1 carriers and ATL patients.
[Figure 6] The figure is a graph showing measurement results of the amount of a protein in plasmas of HTLV-1 carriers and ATL patients.
[Figure 7] The figure is a graph showing measurement results of the amount of a protein in plasmas of HTLV-1 carriers and ATL patients.
[Figure 8] The figure is a graph showing measurement results of the amount of a protein in plasmas of HTLV-1 carriers and ATL patients.
[Figure 9] The figure is a graph showing measurement results of the amount of a protein in plasmas of HTLV-1 carriers and ATL patients.
[Figure 10] The figure is a graph showing measurement results of the amount of a protein in plasmas of HTLV-1 carriers and ATL patients.
[Figure 11] The figure is a graph showing measurement results of the amount of a protein in plasmas of HTLV-1 carriers and ATL patients.
[Figure 12] The figure is a graph showing measurement results of the amount of a protein in plasmas of HTLV-1 carriers and ATL patients.
[Figure 13] The figure is a graph showing measurement results of the amount of a protein in plasmas of HTLV-1 carriers and ATL patients.
[Figure 14] The figure is a graph showing measurement results of the amount of a protein in plasmas of HTLV-1 carriers and ATL patients.
[Figure 15] The figure is a graph showing measurement results of the amount of a protein in plasmas of HTLV-1 carriers and ATL patients.
[Figure 16] The figure is a graph showing measurement results of the amount of a protein in plasmas of HTLV-1 carriers and ATL patients.
[Figure 17] The figure is a graph showing measurement results of the amount of a protein in plasmas of HTLV-1 carriers and ATL patients.
[Figure 18] The figure is a graph showing measurement results of the amount of a protein in plasmas of HTLV-1 carriers and ATL patients.
[Figure 19] The figure is a graph showing measurement results of the amount of a protein in plasmas of HTLV-1 carriers and ATL patients.
[Figure 20] The figure is a graph showing measurement results of the amount of a protein in plasmas of HTLV-1 carriers and ATL patients.
[Figure 21] The figure is a graph showing measurement results of the amount of a protein in plasmas of HTLV-1 carriers and ATL patients.
[Figure 22] The figure is a graph showing measurement results of the amount of a protein in plasmas of HTLV-1 carriers and ATL patients.
[Figure 23] The figure is a graph showing measurement results of the amount of a protein in plasmas of HTLV-1 carriers and ATL patients.
[Figure 24] The figure is a graph showing measurement results of the amount of a protein in plasmas of HTLV-1 carriers and ATL patients.
[Figure 25] The figure is a graph showing measurement results of the amount of a protein in plasmas of HTLV-1 carriers and ATL patients.
[Figure 26] The figure is a graph showing measurement results of the amount of a protein in plasmas of HTLV-1 carriers and ATL patients.
[Figure 27] The figure is a graph showing measurement results of the amount of a protein in plasmas of HTLV-1 carriers and ATL patients.
[Figure 28] The figure is a graph showing measurement results of the amount of a protein in plasmas of HTLV-1 carriers and ATL patients.
[Figure 29] The figure is a graph showing measurement results of the amount of a protein in plasmas of HTLV-1 carriers and ATL patients.
[Figure 30] The figure is a graph showing measurement results of the amount of a protein in plasmas of HTLV-1 carriers and ATL patients.
[Figure 31] The figure is a graph showing measurement results of the amount of a protein in plasmas of HTLV-1 carriers and ATL patients.
[Figure 32] The figure is a graph showing measurement results of the amount of a protein in plasmas of HTLV-1 carriers and ATL patients.
[Figure 33] The figure is a graph showing measurement results of the amount of a protein in plasmas of HTLV-1 carriers and ATL patients.
[Figure 34] The figure is a graph showing measurement results of the amount of a protein in plasmas of HTLV-1 carriers and ATL patients.
[Figure 35] The figure is a graph showing measurement results of the amount of a protein in plasmas of HTLV-1 carriers and ATL patients.
[Figure 36] The figure is a graph showing measurement results of the amount of a protein in plasmas of HTLV-1 carriers and ATL patients.
[Figure 37] The figure is a graph showing measurement results of the amount of a protein in plasmas of HTLV-1 carriers and ATL patients.
[Figure 38] The figure is a graph showing measurement results of the amount of a protein in plasmas of HTLV-1 carriers and ATL patients.
[Figure 39] The figure is a graph showing measurement results of the amount of a protein in plasmas of HTLV-1 carriers and ATL patients.
[Figure 40] The figure is a graph showing measurement results of the amount of a protein in plasmas of HTLV-1 carriers and ATL patients.
[Figure 41] The figure is a graph showing measurement results of the amount of a protein in plasmas of HTLV-1 carriers and ATL patients.
[Figure 42] The figure is a graph showing measurement results of the amount of a protein in plasmas of HTLV-1 carriers and ATL patients.
[Figure 43] The figure is a graph showing measurement results of the amount of a protein in plasmas of HTLV-1 carriers and ATL patients.
[Figure 44] The figure is a graph showing measurement results of the amount of a protein in plasmas of HTLV-1 carriers and ATL patients.
[Figure 45] The figure is a graph showing measurement results of the amount of a protein in plasmas of HTLV-1 carriers and ATL patients.
[Figure 46] The figure is a graph showing measurement results of the amount of a protein in plasmas of HTLV-1 carriers and ATL patients.
[Figure 47] The figure is a graph showing measurement results of the amount of a protein in plasmas of HTLV-1 carriers and ATL patients.
[Figure 48] The figure is a graph showing measurement results of the amount of a protein in plasmas of HTLV-1 carriers and ATL patients.
[Figure 49] The figure is a graph showing measurement results of the amount of a protein in plasmas of HTLV-1 carriers and ATL patients.
[Figure 50] The figure is a graph showing measurement results of the amount of a protein in plasmas of HTLV-1 carriers and ATL patients.
[Figure 51] The figure is a graph showing measurement results of the amount of a protein in plasmas of HTLV-1 carriers and ATL patients.
[Figure 52] The figure is a graph showing measurement results of the amount of a protein in plasmas of HTLV-1 carriers and ATL patients.
[Figure 53] The figure is a graph showing measurement results of the amount of a protein in plasmas of HTLV-1 carriers and ATL patients.
[Figure 54] The figure is a graph showing measurement results of the amount of a protein in plasmas of HTLV-1 carriers and ATL patients.
[Figure 55] The figure is a graph showing measurement results of the amount of a protein in plasmas of HTLV-1 carriers and ATL patients.
[Figure 56] The figure is a graph showing measurement results of the amount of a protein in plasmas of HTLV-1 carriers and ATL patients.
[Figure 57] The figure is a graph showing measurement results of the amount of a protein in plasmas of HTLV-1 carriers and ATL patients.
[Figure 58] The figure is a graph showing measurement results of the amount of a protein in plasmas of HTLV-1 carriers and ATL patients.
[Figure 59] The figure is a graph showing measurement results of the amount of a protein in plasmas of HTLV-1 carriers and ATL patients.
[Figure 60] The figure is a graph showing measurement results of the amount of a protein in plasmas of HTLV-1 carriers and ATL patients.
[Figure 61] The figure is a graph showing measurement results of the amount of a protein in plasmas of HTLV-1 carriers and ATL patients.
[Figure 62] The figure is a graph showing measurement results of the amount of a protein in plasmas of HTLV-1 carriers and ATL patients.
[Figure 63] The figure is a graph showing measurement results of the amount of a protein in plasmas of HTLV-1 carriers and ATL patients.
[Figure 64] The figure is a graph showing measurement results of the amount of a protein in plasmas of HTLV-1 carriers and ATL patients.
[Figure 65] The figure is a graph showing measurement results of the amount of a protein in plasmas of HTLV-1 carriers and ATL patients.
[Figure 66] The figure is a graph showing measurement results of the amount of a protein in plasmas of HTLV-1 carriers and ATL patients.
[Figure 67] The figure is a graph showing measurement results of the amount of a protein in plasmas of HTLV-1 carriers and ATL patients.
[Figure 68] The figure is a graph showing measurement results of the amount of a protein in plasmas of HTLV-1 carriers and ATL patients.
[Figure 69] The figure is a graph showing measurement results of the amount of a protein in plasmas of HTLV-1 carriers and ATL patients.
[Figure 70] The figure is a graph showing measurement results of the amount of a protein in plasmas of HTLV-1 carriers and ATL patients.
[Figure 71] The figure is a graph showing measurement results of the amount of a protein in plasmas of HTLV-1 carriers and ATL patients.
[Figure 72] The figure is a graph showing measurement results of the amount of a protein in plasmas of HTLV-1 carriers and ATL patients.
[Figure 73] The figure is a graph showing measurement results of the amount of a protein in plasmas of HTLV-1 carriers and ATL patients.
[Figure 74] The figure is a graph showing measurement results of the amount of a protein in plasmas of HTLV-1 carriers and ATL patients.
[Figure 75] The figure is a graph showing measurement results of the amount of a protein in plasmas of HTLV-1 carriers and ATL patients.
[Figure 76] The figure is a graph showing measurement results of the amount of a protein in plasmas of HTLV-1 carriers and ATL patients.
[Figure 77] The figure is a graph showing measurement results of the amount of a protein in plasmas of HTLV-1 carriers and ATL patients.
[Figure 78] The figure is a graph showing measurement results of the amount of a protein in plasmas of HTLV-1 carriers and ATL patients.
[Figure 79] The figure is a graph showing measurement results of the amount of a protein in plasmas of HTLV-1 carriers and ATL patients.
[Figure 80] The figure is a graph showing measurement results of the amount of a protein in plasmas of HTLV-1 carriers and ATL patients.
[Figure 81] The figure is a graph showing measurement results of the amount of a protein in plasmas of HTLV-1 carriers and ATL patients.
[Figure 82] The figure is a graph showing measurement results of the amount of a protein in plasmas of HTLV-1 carriers and ATL patients.
[Figure 83] The figure is a graph showing measurement results of the amount of a protein in plasmas of HTLV-1 carriers and ATL patients.
[Figure 84] The figure is a graph showing measurement results of the amount of a protein in plasmas of HTLV-1 carriers and ATL patients.
[Figure 85] The figure is a graph showing measurement results of the amount of a protein in plasmas of HTLV-1 carriers and ATL patients.
[Figure 86] The figure is a graph showing measurement results of the amount of a protein in plasmas of HTLV-1 carriers and ATL patients.
[Figure 87] The figure is a graph showing measurement results of the amount of a protein in plasmas of HTLV-1 carriers and ATL patients.
[Figure 88] The figure is a graph showing measurement results of the amount of a protein in plasmas of HTLV-1 carriers and ATL patients.
[Figure 89] The figure is a graph showing measurement results of the amount of a protein in plasmas of HTLV-1 carriers and ATL patients.
[Figure 90] The figure is a graph showing measurement results of the amount of a protein in plasmas of HTLV-1 carriers and ATL patients.
[Figure 91] The figure is a graph showing measurement results of the amount of a protein in plasmas of HTLV-1 carriers and ATL patients.
[Figure 92] The figure is a graph showing measurement results of the amount of a protein in plasmas of HTLV-1 carriers and ATL patients.
[Figure 93] The figure is a graph showing measurement results of the amount of a protein in plasmas of HTLV-1 carriers and ATL patients.
[Figure 94] The figure is a graph showing measurement results of the amount of a protein in plasmas of HTLV-1 carriers and ATL patients.
[Figure 95] The figure is a graph showing measurement results of the amount of a protein in plasmas of HTLV-1 carriers and ATL patients.
[Figure 96] The figure is a graph showing measurement results of the amount of a protein in plasmas of HTLV-1 carriers and ATL patients.
[Figure 97] The figure is a graph showing measurement results of the amount of a protein in plasmas of HTLV-1 carriers and ATL patients.
[Figure 98] The figure is a graph showing measurement results of the amount of a protein in plasmas of HTLV-1 carriers and ATL patients.
[Figure 99] The figure is a graph showing measurement results of the amount of a protein in plasmas of HTLV-1 carriers and ATL patients.
[Figure 100] The figure is a graph showing measurement results of the amount of a protein in plasmas of HTLV-1 carriers and ATL patients.
[Figure 101] The figure is a graph showing measurement results of the amount of a protein in plasmas of HTLV-1 carriers and ATL patients.
[Figure 102] The figure is a graph showing measurement results of the amount of a protein in plasmas of HTLV-1 carriers and ATL patients.
[Figure 103] The figure is a graph showing measurement results of the amount of a protein in plasmas of HTLV-1 carriers and ATL patients.
[Figure 104] The figure is a graph showing measurement results of the amount of a protein in plasmas of HTLV-1 carriers and ATL patients.
[Figure 105] The figure is a graph showing measurement results of the amount of a protein in plasmas of HTLV-1 carriers and ATL patients.
[Figure 106] The figure is a graph showing measurement results of the amount of a protein in plasmas of HTLV-1 carriers and ATL patients.
[Figure 107] The figure is a graph showing measurement results of the amount of a protein in plasmas of ATL patients and ATL patients in remission.
[Figure 108] The figure is a graph showing measurement results of the amount of a protein in plasmas of ATL patients and ATL patients in remission.
[Figure 109] The figure is a graph showing measurement results of the amount of a protein in plasmas of ATL patients and ATL patients in remission.
[Figure 110] The figure is a graph showing measurement results of the amount of a protein in plasmas of ATL patients and ATL patients in remission.
[Figure 111] The figure is a graph showing measurement results of the amount of a protein in plasmas of ATL patients and ATL patients in remission.
[Figure 112] The figure is a graph showing measurement results of the amount of a protein in plasmas of ATL patients and ATL patients in remission.
[Figure 113] The figure is a graph showing measurement results of the amount of a protein in plasmas of ATL patients and ATL patients in remission.
[Figure 114] The figure is a graph showing measurement results of the amount of a protein in plasmas of ATL patients and ATL patients in remission.
[Figure 115] The figure is a graph showing measurement results of the amount of a protein in plasmas of ATL patients and ATL patients in remission.
[Figure 116] The figure is a graph showing measurement results of the amount of a protein in plasmas of ATL patients and ATL patients in remission.
[Figure 117] The figure is a graph showing measurement results of the amount of a protein in plasmas of ATL patients and ATL patients in remission.
[Figure 118] The figure is a graph showing measurement results of the amount of a protein in plasmas of ATL patients and ATL patients in remission.
[Figure 119] The figure is a graph showing measurement results of the amount of a protein in plasmas of ATL patients and ATL patients in remission.
[Figure 120] The figure is a graph showing measurement results of the amount of a protein in plasmas of ATL patients and ATL patients in remission.
[Figure 121] The figure is a graph showing measurement results of the amount of a protein in plasmas of ATL patients and ATL patients in remission.
[Figure 122] The figure is a graph showing measurement results of the amount of a protein in plasmas of ATL patients and ATL patients in remission.
[Figure 123] The figure is a graph showing measurement results of the amount of a protein in plasmas of ATL patients and ATL patients in remission.
[Figure 124] The figure is a graph showing measurement results of the amount of a protein in plasmas of ATL patients and ATL patients in remission.
[Figure 125] The figure is a graph showing measurement results of the amount of a protein in plasmas of ATL patients and ATL patients in remission.
[Figure 126] The figure is a graph showing measurement results of the amount of a protein in plasmas of ATL patients and ATL patients in remission.
[Figure 127] The figure is a graph showing measurement results of the amount of a protein in plasmas of ATL patients and ATL patients in remission.
[Figure 128] The figure is a graph showing measurement results of the amount of a protein in plasmas of ATL patients and ATL patients in remission.
[Figure 129] The figure is a graph showing measurement results of the amount of a protein in plasmas of ATL patients and ATL patients in remission.
[Figure 130] The figure is a graph showing measurement results of the amount of a protein in plasmas of ATL patients and ATL patients in remission.
[Figure 131] The figure is a graph showing measurement results of the amount of a protein in plasmas of ATL patients and ATL patients in remission.
[Figure 132] The figure is a graph showing measurement results of the amount of a protein in plasmas of ATL patients and ATL patients in remission.
[Figure 133] The figure is a graph showing measurement results of the amount of a protein in plasmas of ATL patients and ATL patients in remission.
[Figure 134] The figure is a graph showing measurement results of the amount of a protein in plasmas of ATL patients and ATL patients in remission.
[Figure 135] The figure is a graph showing measurement results of the amount of a protein in plasmas of ATL patients and ATL patients in remission.
[Figure 136] The figure is a graph showing measurement results of the amount of a protein in plasmas of ATL patients and ATL patients in remission.
[Figure 137] The figure is a graph showing measurement results of the amount of a protein in plasmas of ATL patients and ATL patients in remission.
[Figure 138] The figure is a graph showing measurement results of the amount of a protein in plasmas of ATL patients and ATL patients in remission.
[Figure 139] The figure is a graph showing measurement results of the amount of a protein in plasmas of ATL patients and ATL patients in remission.
[Figure 140] The figure is a graph showing measurement results of the amount of a protein in plasmas of ATL patients and ATL patients in remission.
[Figure 141] The figure is a graph showing measurement results of the amount of a protein in plasmas of ATL patients and ATL patients in remission.
[Figure 142] The figure is a graph showing measurement results of the amount of a protein in plasmas of ATL patients and ATL patients in remission.
[Figure 143] The figure is a graph showing measurement results of the amount of a protein in plasmas of ATL patients and ATL patients in remission.
[Figure 144] The figure is a graph showing measurement results of the amount of a protein in plasmas of ATL patients and ATL patients in remission.
[Figure 145] The figure is a graph showing measurement results of the amount of a protein in plasmas of ATL patients and ATL patients in remission.
[Figure 146] The figure is a graph showing measurement results of the amount of a protein in plasmas of ATL patients and ATL patients in remission.
[Figure 147] The figure is a graph showing measurement results of the amount of a protein in plasmas of ATL patients and ATL patients in remission.
[Figure 148] The figure is a graph showing measurement results of the amount of a protein in plasmas of ATL patients and ATL patients in remission.
[Figure 149] The figure is a graph showing measurement results of the amount of a protein in plasmas of ATL patients and ATL patients in remission.
[Figure 150] The figure is a graph showing measurement results of the amount of a protein in plasmas of ATL patients and ATL patients in remission.
[Figure 151] The figure is a graph showing measurement results of the amount of a protein in plasmas of ATL patients and ATL patients in remission.
[Figure 152] The figure is a graph showing measurement results of the amount of a protein in plasmas of ATL patients and ATL patients in remission.
[Figure 153] The figure is a graph showing measurement results of the amount of a protein in plasmas of ATL patients and ATL patients in remission.
[Figure 154] The figure is a graph showing measurement results of the amount of a protein in plasmas of ATL patients and ATL patients in remission.
[Figure 155] The figure is a graph showing measurement results of the amount of a protein in plasmas of ATL patients and ATL patients in remission.
[Figure 156] The figure is a graph showing measurement results of the amount of a protein in plasmas of ATL patients and ATL patients in remission.
[Figure 157] The figure is a graph showing measurement results of the amount of a protein in plasmas of ATL patients and ATL patients in remission.
[Figure 158] The figure is a graph showing measurement results of the amount of a protein in plasmas of ATL patients and ATL patients in remission.
[Figure 159] The figure is a graph showing measurement results of the amount of a protein in plasmas of ATL patients and ATL patients in remission.
[Figure 160] The figure is a graph showing measurement results of the amount of a protein in plasmas of ATL patients and ATL patients in remission.
[Figure 161] The figure is a graph showing measurement results of the amount of a protein in plasmas of ATL patients and ATL patients in remission.
[Figure 162] The figure is a graph showing measurement results of the amount of a protein in plasmas of ATL patients and ATL patients in remission.
[Figure 163] The figure is a graph showing measurement results of the amount of a protein in plasmas of ATL patients and ATL patients in remission.
[Figure 164] The figure is a graph showing measurement results of the amount of a protein in plasmas of ATL patients and ATL patients in remission.
[Figure 165] The figure is a graph showing measurement results of the amount of a protein in plasmas of ATL patients and ATL patients in remission.
[Figure 166] The figure is a graph showing measurement results of the amount of a protein in plasmas of ATL patients and ATL patients in remission.
[Figure 167] The figure is a graph showing measurement results of the amount of a protein in plasmas of ATL patients and ATL patients in remission.
[Figure 168] The figure is a graph showing measurement results of the amount of a protein in plasmas of ATL patients and ATL patients in remission.
[Figure 169] The figure is a graph showing measurement results of the amount of a protein in plasmas of ATL patients and ATL patients in remission.
[Figure 170] The figure is a graph showing measurement results of the amount of a protein in plasmas of ATL patients and ATL patients in remission.
[Figure 171] The figure is a graph showing measurement results of the amount of a protein in plasmas of ATL patients and ATL patients in remission.
[Figure 172] The figure is a graph showing measurement results of the amount of a protein in plasmas of ATL patients and ATL patients in remission.
[Figure 173] The figure is a graph showing measurement results of the amount of a protein in plasmas of ATL patients and ATL patients in remission.
[Figure 174] The figure is a graph showing measurement results of the amount of a protein in plasmas of ATL patients and ATL patients in remission.
[Figure 175] The figure is a graph showing measurement results of the amount of a protein in plasmas of ATL patients and ATL patients in remission.
[Figure 176] The figure is a graph showing measurement results of the amount of a protein in plasmas of ATL patients and ATL patients in remission.
[Figure 177] The figure is a graph showing measurement results of the amount of a protein in plasmas of ATL patients and ATL patients in remission.
[Figure 178] The figure is a graph showing measurement results of the amount of a protein in plasmas of ATL patients and ATL patients in remission.
[Figure 179] The figure is a graph showing measurement results of the amount of a protein in plasmas of ATL patients and ATL patients in remission.
[Figure 180] The figure is a graph showing measurement results of the amount of a protein in plasmas of ATL patients and ATL patients in remission.
[Figure 181] The figure is a graph showing measurement results of the amount of a protein in plasmas of ATL patients and ATL patients in remission.
[Figure 182] The figure is a graph showing measurement results of the amount of a protein in plasmas of ATL patients and ATL patients in remission.
[Figure 183] The figure is a graph showing measurement results of the amount of a protein in plasmas of ATL patients and ATL patients in remission.
[Figure 184] The figure is a graph showing measurement results of the amount of a protein in plasmas of ATL patients and ATL patients in remission.
[Figure 185] The figure is a graph showing measurement results of the amount of a protein in plasmas of ATL patients and ATL patients in remission.
[Figure 186] The figure is a graph showing measurement results of the amount of a protein in plasmas of ATL patients and ATL patients in remission.
[Figure 187] The figure is a graph showing measurement results of the amount of a protein in plasmas of ATL patients and ATL patients in remission.
[Figure 188] The figure is a graph showing measurement results of the amount of a protein in plasmas of ATL patients and ATL patients in remission.
[Figure 189] The figure is a graph showing measurement results of the amount of a protein in plasmas of ATL patients and ATL patients in remission.
[Figure 190] The figure is a graph showing measurement results of the amount of a protein in plasmas of ATL patients and ATL patients in remission.
[Figure 191] The figure is a graph showing measurement results of the amount of a protein in plasmas of ATL patients and ATL patients in remission.
[Figure 192] The figure is a graph showing measurement results of the amount of a protein in plasmas of ATL patients and ATL patients in remission.
[Figure 193] The figure is a graph showing measurement results of the amount of a protein in plasmas of ATL patients and ATL patients in remission.
[Figure 194] The figure is a graph showing measurement results of the amount of a protein in plasmas of ATL patients and ATL patients in remission.
[Figure 195] The figure is a graph showing measurement results of the amount of a protein in plasmas of ATL patients and ATL patients in remission.
[Figure 196] The figure is a graph showing measurement results of the amount of a protein in plasmas of ATL patients and ATL patients in remission.
[Figure 197] The figure is a graph showing measurement results of the amount of a protein in plasmas of ATL patients and ATL patients in remission.
[Figure 198] The figure is a graph showing measurement results of the amount of a protein in plasmas of ATL patients and ATL patients in remission.
[Figure 199] The figure is a graph showing measurement results of the amount of a protein in plasmas of ATL patients and ATL patients in remission.
[Figure 200] The figure is a graph showing measurement results of the amount of a protein in plasmas of ATL patients and ATL patients in remission.
[Figure 201] The figure is a graph showing measurement results of the amount of a protein in plasmas of ATL patients and ATL patients in remission.
[Figure 202] The figure is a graph showing measurement results of the amount of a protein in plasmas of ATL patients and ATL patients in remission.
[Figure 203] The figure is a graph showing measurement results of the amount of a protein in plasmas of ATL patients and ATL patients in remission.
[Figure 204] The figure is a graph showing measurement results of the amount of a protein in plasmas of ATL patients and ATL patients in remission.
[Figure 205] The figure is a graph showing measurement results of the amount of a protein in plasmas of ATL patients and ATL patients in remission.
[Figure 206] The figure is a graph showing measurement results of the amount of a protein in plasmas of HTLV-1 carriers, ATL patients and ATL patients in remission.
[Figure 207] The figure is a graph showing measurement results of the amount of a protein in plasmas of HTLV-1 carriers, ATL patients and ATL patients in remission.
[Figure 208] The figure is a graph showing measurement results of the amount of a protein in plasmas of HTLV-1 carriers, ATL patients and ATL patients in remission.
[Figure 209] The figure is a graph showing measurement results of the amount of a protein in plasmas of HTLV-1 carriers, ATL patients and ATL patients in remission.
[Figure 210] The figure is a graph showing measurement results of the amount of a protein in plasmas of HTLV-1 carriers, ATL patients and ATL patients in remission.
[Figure 211] The figure is a graph showing measurement results of the amount of a protein in plasmas of HTLV-1 carriers, ATL patients and ATL patients in remission.
[Figure 212] The figure is a graph showing measurement results of the amount of a protein in plasmas of HTLV-1 carriers, ATL patients and ATL patients in remission.
[Figure 213] The figure is a graph showing measurement results of the amount of a protein in plasmas of HTLV-1 carriers, ATL patients and ATL patients in remission.
[Figure 214] The figure is a graph showing measurement results of the amount of a protein in plasmas of HTLV-1 carriers, ATL patients and ATL patients in remission.
[Figure 215] The figure is a graph showing measurement results of the amount of a protein in plasmas of HTLV-1 carriers, ATL patients and ATL patients in remission.
[Figure 216] The figure is a graph showing measurement results of the amount of a protein in plasmas of HTLV-1 carriers, ATL patients and ATL patients in remission.
[Figure 217] The figure is a graph showing measurement results of the amount of a protein in plasmas of HTLV-1 carriers, ATL patients and ATL patients in remission.
[Figure 218] The figure is a graph showing measurement results of the amount of a protein in plasmas of HTLV-1 carriers, ATL patients and ATL patients in remission.
[Figure 219] The figure is a graph showing measurement results of the amount of a protein in plasmas of HTLV-1 carriers, ATL patients and ATL patients in remission.
[Figure 220] The figure is a graph showing measurement results of the amount of a protein in plasmas of HTLV-1 carriers, ATL patients and ATL patients in remission.
[Figure 221] The figure is a graph showing measurement results of the amount of a protein in plasmas of HTLV-1 carriers, ATL patients and ATL patients in remission.
[Figure 222] The figure is a graph showing measurement results of the amount of a protein in plasmas of HTLV-1 carriers, ATL patients and ATL patients in remission.
[Figure 223] The figure is a graph showing measurement results of the amount of a protein in plasmas of HTLV-1 carriers, ATL patients and ATL patients in remission.
[Figure 224] The figure is a graph showing measurement results of the amount of a protein in plasmas of HTLV-1 carriers, ATL patients and ATL patients in remission.
[Figure 225] The figure is a graph showing measurement results of the amount of a protein in plasmas of HTLV-1 carriers, ATL patients and ATL patients in remission.
[Figure 226] The figure is a graph showing measurement results of the amount of a protein in plasmas of HTLV-1 carriers, ATL patients and ATL patients in remission.
[Figure 227] The figure is a graph showing measurement results of the amount of a protein in plasmas of HTLV-1 carriers, ATL patients and ATL patients in remission.
[Figure 228] The figure is a graph showing measurement results of the amount of a protein in plasmas of HTLV-1 carriers, ATL patients and ATL patients in remission.
[Figure 229] The figure is a graph showing measurement results of the amount of a protein in plasmas of HTLV-1 carriers, ATL patients and ATL patients in remission.
[Figure 230] The figure is a graph showing measurement results of the amount of a protein in plasmas of HTLV-1 carriers, ATL patients and ATL patients in remission.

### Description of Embodiments

Now, the present invention will be described.

### 1. Method for diagnosing HTLV-1 related disease

The method for diagnosing an HTLV-1 related disease, i.e., adult T-cell leukemia (ATL), according to the present invention comprises the following steps (1) and (2) :
(1) measuring an amount of a marker protein, i.e., Coagulation Factor Xa, in a blood sample taken from a subject (measurement step); and
(2) if a measured value is a predetermined reference value or more, determining that the subject has an HTLV-1 related disease, i.e., ATL, or has a high possibility of developing the disease (onset diagnosis) in the case where the subject is not definitely diagnosed with the HTLV-1 related disease, i.e., ATL, and, as described but not claimed herein, determining that the subject is in remission of an HTLV-1 related disease or has a high possibility of achieving a remission of the disease (remission diagnosis) in the case where the subject is definitely diagnosed with the HTLV-1 related disease (determination step).

In the present invention, the "onset" includes not only a first onset but also recurrence after remission, and the "remission" includes not only first remission but also re-remission after recurrence. Accordingly, step (2) can be defined as follows:
(2) if a measured value is a predetermined reference value or more, determining that the subject has an HTLV-1 related disease, i.e., ATL or has a high possibility of developing the disease in the case where the subject is an HTLV-1 carrier or an individual in remission of an HTLV-1 related disease, i.e., ATL (a person having a history with HTLV-1, i.e., ATL), and, as described but not claimed herein, determining that the subject is in remission of an HTLV-1 related disease or has a possibility of achieving a remission of the disease, in the case where the subject is a patient with an HTLV-1 related disease.

### [Measurement step (Step (1))]

As described in Example, the present inventors have identified the protein defined in the claims and listed as protein No. 1 of Table 1 (i.e., Coagulation Factor Xa). Besides the claimed protein, further proteins not encompassed by the claims are listed in Table 1 as the proteins (hereinafter referred to as "marker proteins") that significantly change in amount in ATL patient's blood samples compared to HTLV-1 carriers' blood samples. If the amounts of these marker proteins in blood samples taken from subjects are used as references, it is possible to identify that the subjects have an HTLV-1 related disease, i.e., ATL, or have a high possibility of developing the disease.

When onset diagnosis as defined in the claims is made, the subject is preferably an HTLV-1 carrier or an individual in remission of an HTLV-1 related disease, i.e., ATL. The HTLV-1 carrier can be selected in accordance with a known method in the technical field.

For example, the HTLV-1 carrier can be selected by detecting the presence or absence of an anti-HTLV-1 antibody in blood, for example, by PA (gelatin particle aggregation) method, CLEIA (chemiluminescent enzyme immunoassay) method or western blot.

The HTLV-1 carrier can be selected, for example, by detecting the presence or absence of insertion of HTLV-1 in the genome by southern blot.

In addition, the present inventors have identified the proteins listed in Table 2 as the marker proteins that significantly change in amount in blood samples of individuals in remission of ATL compared to blood samples of ATL patients. If the amounts of these marker proteins in blood samples taken from subjects are used as references, it is possible to identify that subjects are in remission of an HTLV-1 related disease or have a high possibility of achieving a remission of the disease.

When remission diagnosis is made, the subject is preferably a patient with an HTLV-1 related disease and more preferably a patient diagnosed to have an HTLV-1 related disease by the diagnostic method described herein.

The HTLV-1 related disease is ATL.

The blood sample taken from a subject may be whole blood, serum or plasma.

When onset diagnosis as defined in the claims is made, the marker protein to be measured is defined as being represented by the protein No. 1 listed in Table 1, i.e., coagulation factor Xa.

A blood coagulation factor X (UniProt ID: P00742) represented by No. 2 of Table 1 is activated to produce a blood coagulation factor Xa (UniProt ID: P00742) represented by No. 1. It is reported that factor Xa is involved in inflammation, wound healing and immunity through activation of PAR (protease-activated receptor) 1 and PAR2. As far as the present inventors know, it has not been reported that blood coagulation factors X and Xa are involved in HTLV-1 related diseases. Note that, the same UniProt ID is given to blood coagulation factor X and blood coagulation factor Xa.

As described herein, when remission diagnosis is made, the marker protein to be measured is defined as at least one selected from the proteins listed in Table 2.

The marker protein is preferably at least one selected from the proteins represented by Nos. 1 to 633 listed in Table 2, more preferably at least one selected from the proteins represented by Nos. 1 to 440.

As the marker protein to be measured, 2 or more, preferably 3 or more, more preferably 4 or more, and further preferably 5 or more proteins are used in combination. The larger the number of marker proteins, the better the determination accuracy. For the reason, 6 or more proteins may be used.

The marker protein may be a full-length protein or a fragment thereof.

The "full-length protein" refers to a protein having a naturally occurring amino acid sequence, which is the same as expressed *in vivo,* or a mutant thereof. The fragment thereof may have a part of the amino acid sequence of a full-length protein, having a length of, for example, 5 to 10, 11 to 20, 21 to 30, 31 to 40, or 41 to 50 amino acids. The amino acid length may be 51 or more.

The amount of a marker protein in a blood sample can be measured by a method known in the technical field and using a detection reagent such as an antibody or a fragment thereof (e.g., F(ab), F(ab)2, F(ab)' and Fv fragment), a nucleic acid or an aptamer (nucleic acid aptamer or peptide aptamer) that specifically binds to the protein.

The antibody may be a monoclonal or a polyclonal antibody and may belong to any one of the classes of IgG, IgM, IgA, IgD and IgE.

The methods for preparing these detection reagents are known in the technical field. Proper detection reagents are commercially available.

The detection reagent is preferably tagged with a label detectable by an optical, magnetic or electrical means.

The detection reagent is preferably labeled with a fluorescent substance having an excitation wavelength and emission wavelength suitably detected by a commercially available fluorescence analyzer. Examples of the fluorescence substance include phycoerythrin (PE), fluorescein isothiocyanate (FITC), rhodamine (RH), Texas Red (TX), Cy3, Hoechst 33258, and 4',6-diamizino-2-phenyl indole (DAPI).

When a fluorescently labeled detection reagent is used, first, the detection reagent is allowed to bind to a solid phase. Then, a blood sample is brought into contact with the solid phase, reacted and washed, as needed. Fluorescence emitted from the fluorescent label of a marker protein held on the solid phase via the detection reagent, is detected. Based on the intensity thereof, the amount of protein can be determined.

The amount of the marker protein in a blood sample can be measured by, for example, LC/MS (liquid chromatography/mass spectrometry).

The amount of the marker protein can be measured by using an entrusted protein measurement service, for example, SOMAscan, provided by a company, SomaLogic.

Described herein but not claimed is a kit for diagnosing an HTLV-1 related disease which can contain not only a detection reagent as mentioned above but also various reagents for use in the measurement methods mentioned herein.

The amount of marker protein can be measured once or a plurality of times at intervals with respect to the same subject.

### [Determination step (Step (2))]

In this step, the measured value obtained in the measurement step (step 1) is compared to a predetermined reference value. If, as defined in the claims, the measured value is the reference value or more, it is determined that the subject has the HTLV-1 related disease, i.e., ATL or has a high possibility of developing the disease (onset diagnosis), in the case where the subject is not definitely diagnosed with an HTLV-1 related disease (HTLV-1 carrier or an individual in remission of an HTLV-1 related disease), and, as described but not claimed herein, it may be determined that the subject is in remission of the HTLV-1 related disease or has a high possibility of achieving a remission of the disease (remission diagnosis) in the case where the subject is definitely diagnosed with an HTLV-1 related disease (a patient with an HTLV-1 related disease).

When onset diagnosis as defined in the claims is made, an average, median or mode of measured values obtained in blood samples of, for example, an HTLV-1 carrier group or a healthy subject group (not HTLV-1 carrier), can be employed as the reference value.

With respect to the proteins represented by Nos. 1, 2, 4, 5, 7, 9, 10, 11, 16, 17, 19, 20, 21, 22, 25, 28, 31, 32, 33, 37, 39, 41, 42, 43, 44, 46, 47, 48, 49, 52, 56, 57, 58, 59, 60, 61, 62, 63, 64, 65, 66, 67, 68, 70, 72, 73, 74, 75, 76, 77, 79, 80, 81, 82, 88, 89, 90, 91, 92, 93, 94, 95, 96, 97, 98, 99, 100, 101, 102, 103, 104, 106, 108, 109, 112, 113, 115, 118, 119, 120, 121, 123, 124, 125, 128, 129, 130, 131, 133, 134, 135, 139, 141, 142, 143, 144, 149, 154, 155, 156, 157, 159, 161, 164, 165, 167, 168, 171, 172, 173, 174, 175, 176, 180, 181, 182, 183, 184, 185, 187, 189, 192, 195, 196, 198, 199, 200, 201, 203, 205, 206, 207, 208, 209, 211, 214, 215, 219, 220, 221, 222, 223, 224, 225, 226, 228, 230, 231, 232, 236, 237, 238, 240, 241, 242, 243, 244, 246, 247, 248, 249, 250, 251, 252, 253, 254, 255, 256, 259, 260, 263, 264, 267, 269, 271, 274, 279, 280, 283, 285, 288, 289, 290, 291, 293, 294, 295, 296, 298, 299, 300, 301, 302, 303, 305, 306, 307, 309, 310, 311, 312, 313, 315, 316, 317, 318, 321, 322, 323, 326, 327, 329, 330, 331, 332, 333, 334, 338, 339, 340, 341, 342, 343, 345, 346, 347, 348, 350, 352, 354, 355, 356, 357, 358, 359, 364, 365, 366, 367, 371, 373, 374, 375, 376, 378, 379, 382, 383, 384, 385, 386, 387, 388, 389, 390, 393, 394, 395, 396, 397, 398, 399, 400, 401, 405, 406, 407, 409, 411, 412, 416, 418, 420, 423, 425, 426, 427, 430, 431, 432, 433, 434, 435, 437, 438, 439, 440, 441, 443, 445, 446, 447, 450, 451, 452, 453, 454, 455, 456, 457, 460, 461, 463, 465, 466, 469, 470, 471, 472, 473, 474, 475, 477, 478, 480, 481, 482, 483, 484, 486, 487, 489, 492, 493, 494, 495, 498, 499, 500, 502, 503, 504, 505, 506, 507, 509, 510, 511, 512, 513, 514, 515, 516, 518, 523, 524, 529, 531, 532, 534, 537, 538, 540, 542, 543, 544, 546, 547, 548, 549, 551, 552, 554, 555, 556, 558, 560, 561, 565, 566, 568, 572, 573, 574, 575, 576, 579, 580, 583, 584, 585, 586, 587, 588, 589, 590, 591, 593, 594, 596, 597, 599, 600, 601, 602, 603, 605, 606, 607, 609, 610, 612, 613, 614, 615, 616, 617, 618, 619, 620, 622, 624, 627, 628, 629 and 631 of Table 1 in a patient group, a significant increase was observed compared to a carrier group.

With respect to the marker protein that has a significantly increased amount in these ATL patients, if the amount of the marker protein is increased to the reference value or more, preferably higher than the reference value by 1-5%, more preferably 6-10%, further preferably 11-25%, and most preferably 26-50%, it is determined that the subject suffers from, or is likely to develop, the HTLV-1 related disease, i.e., ATL. As the amount is significantly higher than the reference value, the determination accuracy improves. Because of this, if desired, a value higher than the reference value by 51% or more, 100% or more, 150% or more, or 200% or more may be used as an indicator.

With respect to the proteins represented by Nos. 3, 6, 8, 12, 13, 14, 15, 18, 23, 24, 26, 27, 29, 30, 34, 35, 36, 38, 40, 45, 50, 51, 53, 54, 55, 69, 71, 78, 83, 84, 85, 86, 87, 105, 107, 110, 111, 114, 116, 117, 122, 126, 127, 132, 136, 137, 138, 140, 145, 146, 147, 148, 150, 151, 152, 153, 158, 160, 162, 163, 166, 169, 170, 177, 178, 179, 186, 188, 190, 191, 193, 194, 197, 202, 204, 210, 212, 213, 216, 217, 218, 227, 229, 233, 234, 235, 239, 245, 257, 258, 261, 262, 265, 266, 268, 270, 272, 273, 275, 276, 277, 278, 281, 282, 284, 286, 287, 292, 297, 304, 308, 314, 319, 320, 324, 325, 328, 335, 336, 337, 344, 349, 351, 353, 360, 361, 362, 363, 368, 369, 370, 372, 377, 380, 381, 391, 392, 402, 403, 404, 408, 410, 413, 414, 415, 417, 419, 421, 422, 424, 428, 429, 436, 442, 444 448, 449, 458, 459, 462, 464, 467, 468, 476, 479, 485, 488, 490, 491, 496, 497, 501, 508, 517, 519, 520, 521, 522, 525, 526, 527, 528, 530, 533, 535, 536, 539, 541, 545, 550, 553, 557, 559, 562, 563, 564, 567, 569, 570, 571, 577, 578, 581, 582, 592, 595, 598, 604, 608, 611, 621, 623, 625, 626 and 630 in a patient group, a significant decrease was observed compared to a carrier group.

With respect to the marker protein that has a significantly decreased amount in these ATL patients, if the amount of the marker protein is decreased to the reference value or less, preferably lower than the reference value by 1-5%, more preferably 6-10%, further preferably 11-25%, and most preferably 26-50%, it is determined that the subject suffers from, or is likely to develop, the HTLV-1 related disease. As the amount is significantly lower than the reference value, the determination accuracy improves. Because of this, if desired, a value lower than the reference value by 51% or more, 100% or more, 150% or more, or 200% or more may be used as an indicator.

When the amount of a marker protein is measured a plurality of times at time intervals with respect to the same subject, the determination accuracy can be improved by taking a time-dependent change in the marker-protein amount into consideration.

More specifically, with respect to the marker protein that has a significantly increased amount in plasma of ATL patients compared to that in plasma of HTLV-1 carriers, if a time-dependent increase of the marker-protein in amount (for example, an increase from a value below the reference value to a value beyond the reference value) is observed, it is determined that the subject suffers from, or is more likely to develop, the HTLV-1 related disease, i.e., ATL. If the amount sharply increases with time, the subject can be diagnosed to develop a blast crisis.

With respect to the marker protein that has a significantly decreased amount in plasma of ATL patients compared to that in plasma of HTLV-1 carriers, if a time-dependent decrease of the marker-protein in amount (for example, and a decrease from a value beyond the reference value to a value below the reference value) is observed, it is determined that the subject suffers from, or is more likely to develop, the HTLV-1 related disease. If the amount sharply decreases with time, the subject can be diagnosed to develop a blast crisis.

When remission diagnosis, described but not claimed herein, is made, an average, median or mode of the measured values obtained in blood samples of, for example, an HTLV-1 related disease group, can be employed as the reference value.

With respect to the proteins represented by Nos. 468, 595, 607, 642, 661, 668, 739, 759, 760, 774, 775, 779, 781 and 786 of Table 2, a significant increase was observed in individuals in remission compared to a patient group.

With respect to the marker protein that has a significantly increased amount in these individuals in remission of ATL, if the amount of the marker protein is increased to the reference value or more, preferably is higher than the reference value by 1-5%, more preferably 6-10%, further preferably 11-25%, and most preferably 26-50%, it is determined that the subject is in a remission, or is likely to be in remission, of the HTLV-1 related disease. As the amount is significantly higher than the reference value, the determination accuracy improves. Because of this, if desired, a value higher than the reference value by 51% or more, 100% or more, 150% or more, or 200% or more may be used as an indicator.

With respect to the proteins represented by Nos. 1 to 467, 469 to 594, 596 to 606, 608 to 641, 643 to 660, 662 to 667, 669 to 738, 740 to 758, 761 to 773, 776 to 778, 780, 782 to 785, 786 to 790 of Table 2, a significant decrease was observed in individuals in remission compared to a patient group.

With respect to the marker protein that has a significantly decreased amount in these individuals in remission with ATL, if the amount of the marker protein is decreased to the reference value or less, preferably lower than the reference value by 1-5%, more preferably 6-10%, further preferably 11-25%, and most preferably 26-50%, it is determined that the subject is in remission, or is likely to be in remission, of the HTLV-1 related disease. As the amount is significantly lower than the reference value, the determination accuracy improves. Because of this, if desired, a value lower than the reference value by 51% or more, 100% or more, 150% or more, or 200% or more may be used as an indicator.

When the amount of marker protein is measured a plurality of times at time intervals with respect to the same subject, the determination accuracy can be improved by taking a time-dependent change in the marker-protein amount into consideration.

More specifically, with respect to the marker protein that has a significantly increased amount in plasma of an individual in remission of ATL compared to that in plasma of ATL patients, if a time-dependent increase in the marker-protein amount (for example, an increase from a value below the reference value to a value beyond the reference value) is observed, it is determined that the subject is in a remission, or is more likely to be in remission, of the HTLV-1 related disease. If the subject received a treatment for an HTLV-1 related disease during this period, a significant decrease in the marker protein indicates the effectiveness of the treatment.

With respect to the marker protein that has a significantly decreased amount in plasma of an individual in remission of ATL compared to that in plasma of ATL patients, if a time-dependent decrease in the marker-protein amount (for example, and a decrease from a value beyond the reference value to a value below the reference value) is observed, it is determined that the subject is in remission, or is more likely to be in remission, of the HTLV-1 related disease. If the subject received a treatment for an HTLV-1 related disease during this period, a significant decrease in the marker protein indicates the effectiveness of the treatment.

A statistical analysis method or machine learning can be used for determination.

Particularly, discriminant analysis using training data is preferably used. When two groups (for example, the HTLV-1 carrier group and the ATL patient group of the present invention) are discriminated, linear discriminant analysis is mostly sufficient.

However, linear discriminant analysis is limited in application because it is based on the assumption that the population variances of individual groups are equal. Accordingly, when discriminant analysis is carried out using more complicated data, non-linear discriminant analysis or discriminant analysis using machine learning is desirably used. Examples of the non-linear discriminant analysis include discriminant analysis based on Mahalanobis' generalized distance. Examples of the discrimination analysis using machine learning include k-nearest neighbor algorithm, Naive Bayes classifier, decision tree, neural network, support vector machine, and ensemble learning such as a bagging method, a boosting method and a random forest method. Such a method has been developed in the field of pattern recognition, and findings in the pattern recognition can be applied to the present invention.

In addition, even if an analysis method using no training data, such as principal component analysis, hierarchical clustering, non-hierarchical clustering, and self-organizing map, is employed, discrimination can be made by performing analysis in combination with the subject's data on status of an HTLV-1 related disease previously known.

### Example

### [Test example 1: Analysis of plasma protein of ATL patient]

The amounts of proteins in plasmas of HTLV-1 carriers (40 carriers), ATL patients (40 patients) and individuals having a remission of ATL (5 patients in remission) were measured and compared.

The plasmas of HTLV-1 carriers (hereinafter referred to as a "carrier group"), ATL patients (hereinafter referred to as a "patient group") and individuals having a remission of ATL (hereinafter referred to as "remission group") were provided by the Okinawa Bio Information Bank (ATL/HTLV-1 biobank) of Ryukyu University.

Using an entrusted protein measurement service, "SOMAscan" of a company, SomaLogic, the amounts of 1305 types of proteins were exhaustively measured. SOMAscan is a system that simultaneously quantifies more than 1000 types of proteins by use of aptamers SOMAmer (registered trademark) immobilized to a chip (see, Non Patent Literature 1). The outline of the procedure is as follows.

The plasma was diluted and incubated with beads, which were coated with streptavidin (SA) and having an SOMAmer reagent mixture previously immobilized thereto. The beads were washed to remove proteins non-specifically bound. The protein specifically bound to the SOMAmer reagent was labeled by use of an NHS-biotin reagent. The biotin-labeled protein-SOMAmer reagent complex and an unbound SOMAmer reagent were released from the beads by cutting a photocleaving linker with ultraviolet irradiation.

The released biotin-labeled protein-SOMAmer reagent complex and unbound SOMAmer reagent were incubated with second streptavidin coated beads. In this manner, the biotin-labeled protein-SOMAmer reagent complex was allowed to bind to the second streptavidin coated beads. The SOMAmer reagent unbound was removed by washing the beads. The SOMAmer reagent was released from the second streptavidin coated beads having the biotin-labeled protein-SOMAmer reagent complex bound thereto, in denaturation conditions.

The SOMAmer reagent was hybridized with a DNA microarray and fluorescence from cyanine 3 in the SOMAmer reagent was detected. The detected fluorescence intensity value was normalized and corrected. In this manner, the amount of a protein was determined as a relative fluorescence unit (RFU).

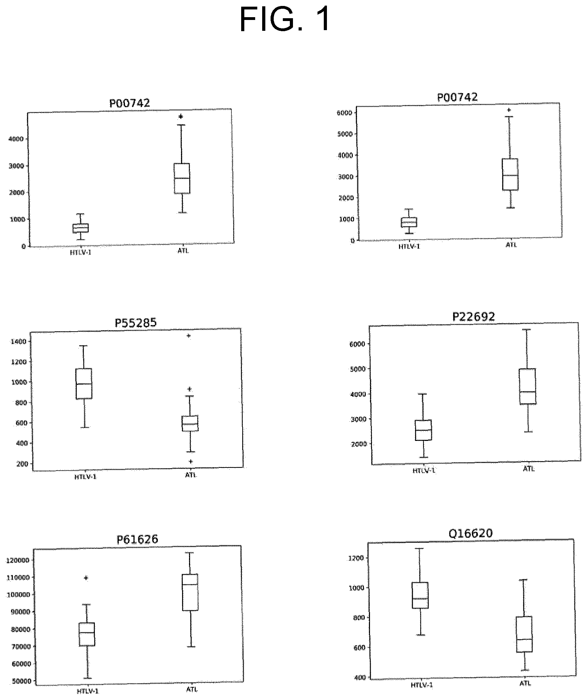

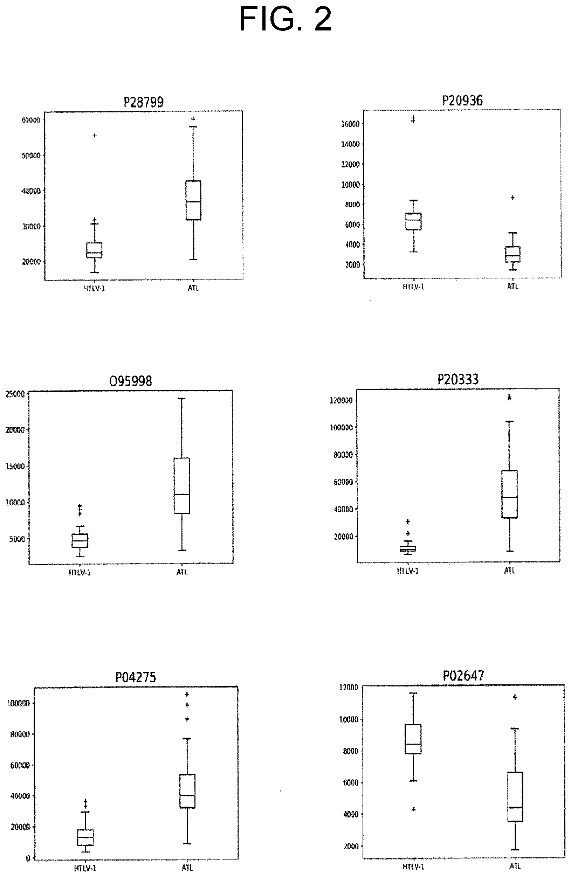

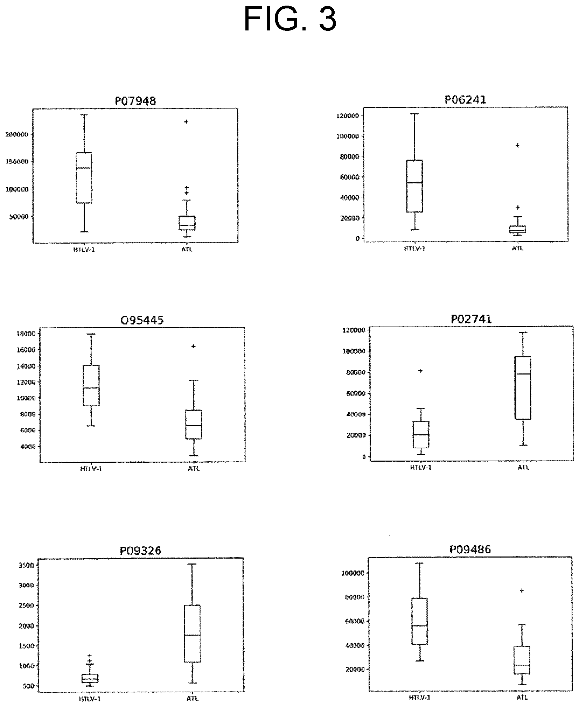

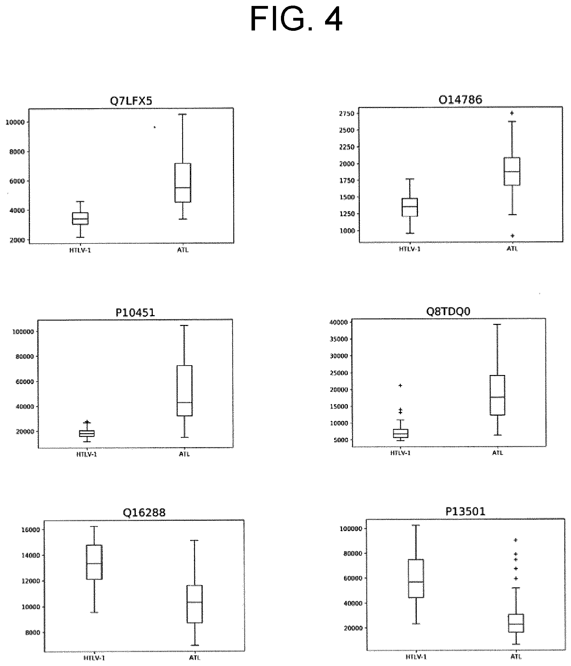

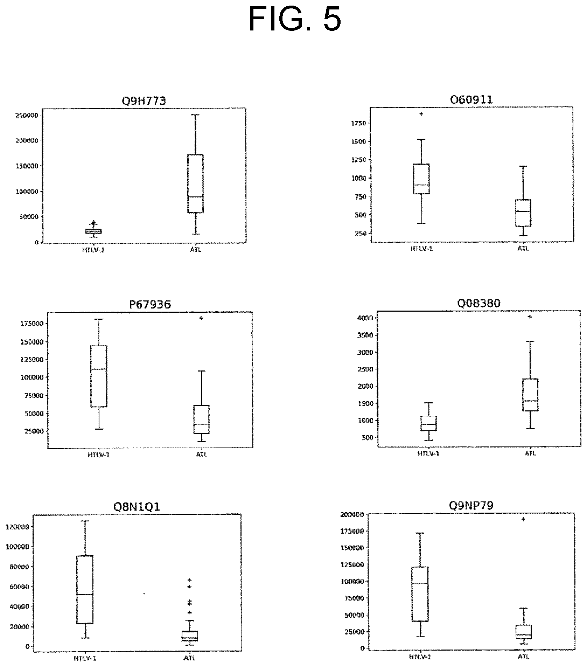

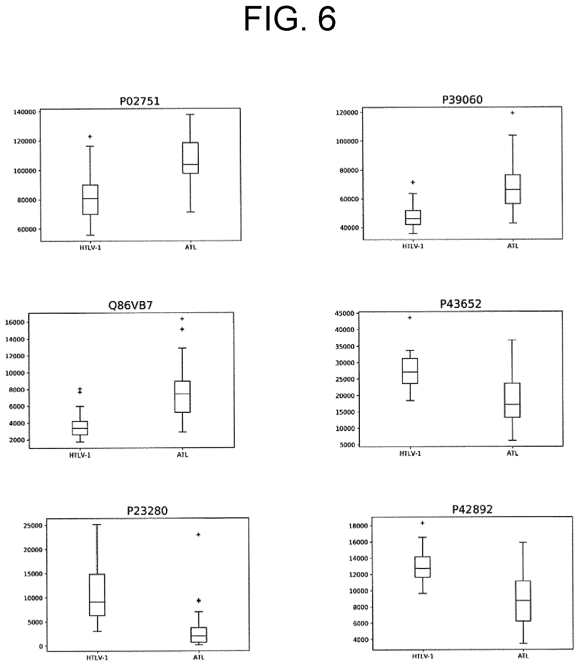

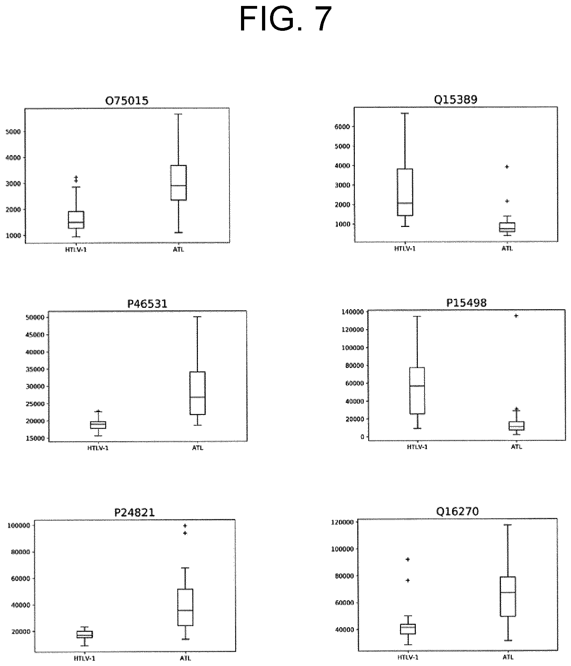

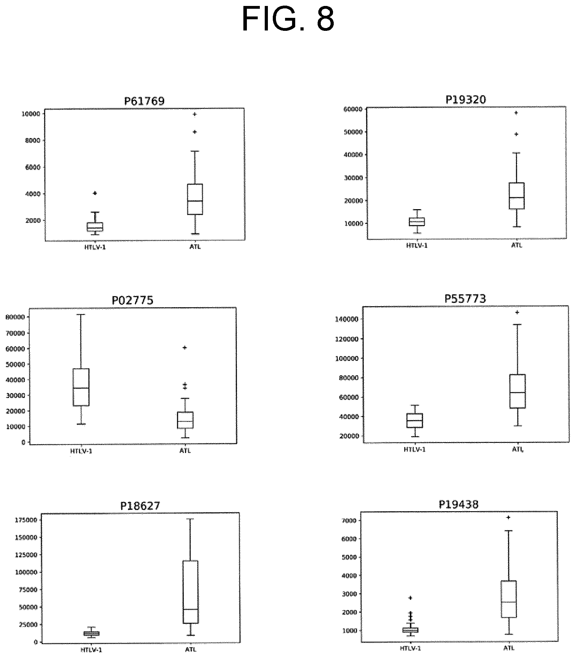

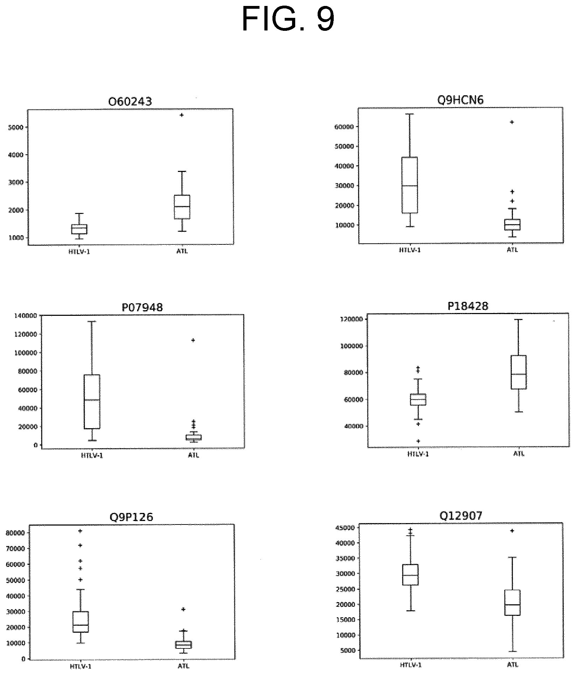

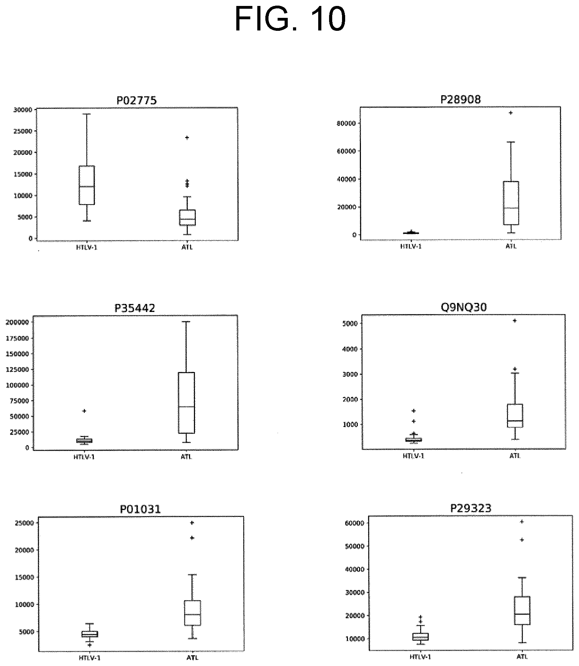

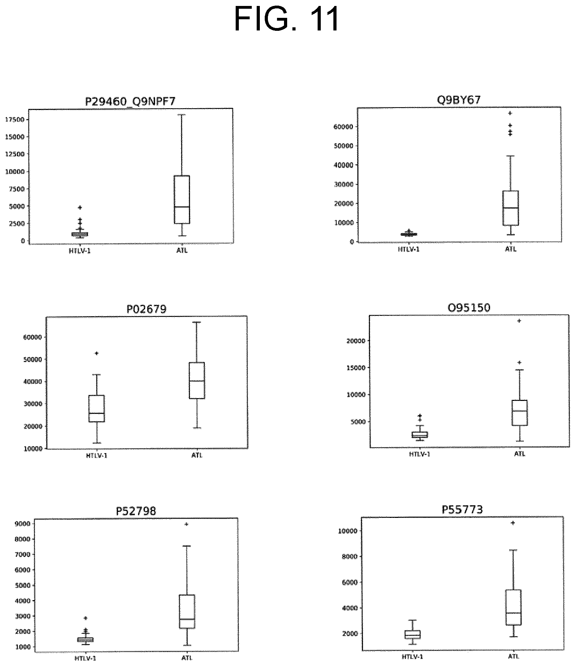

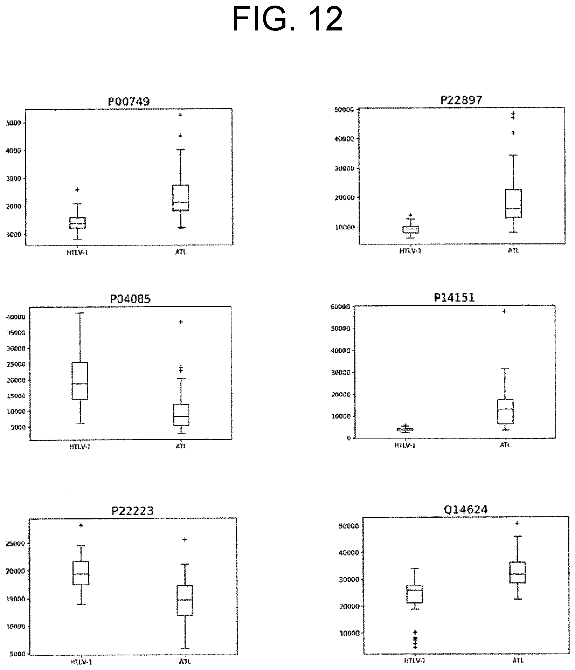

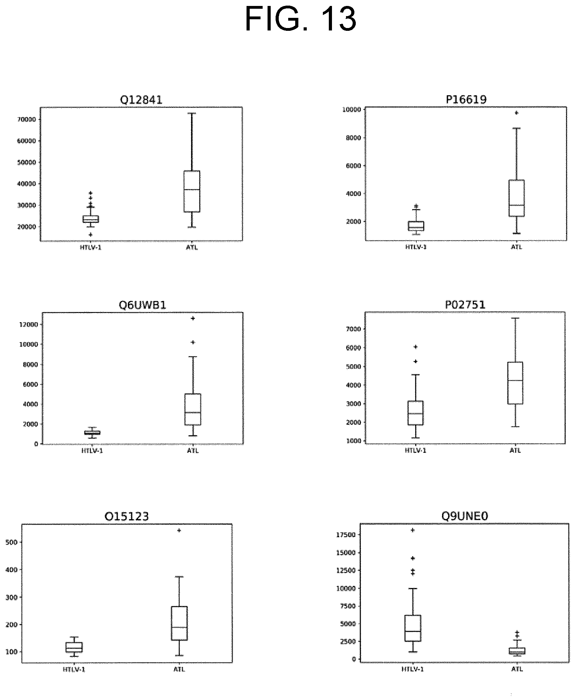

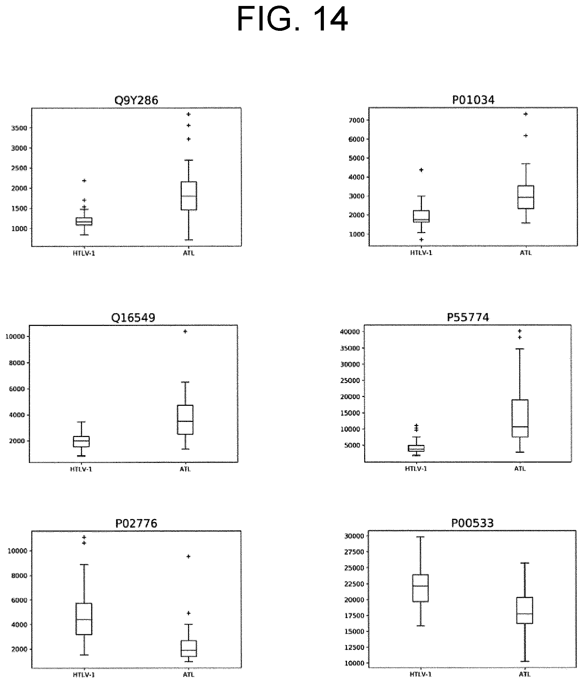

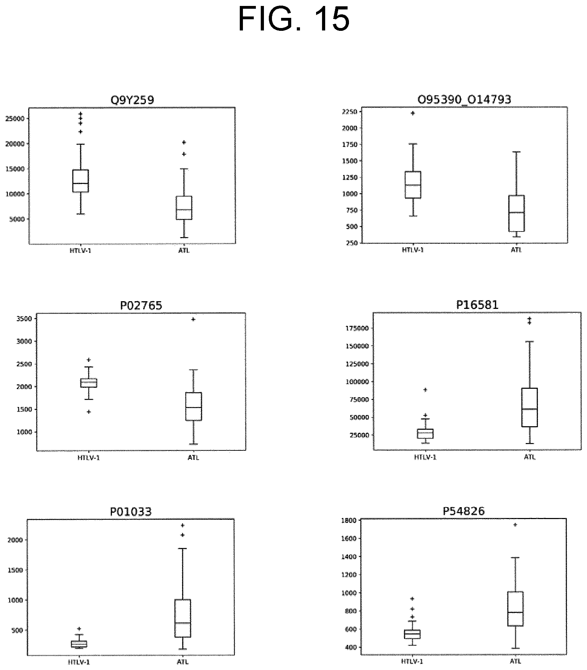

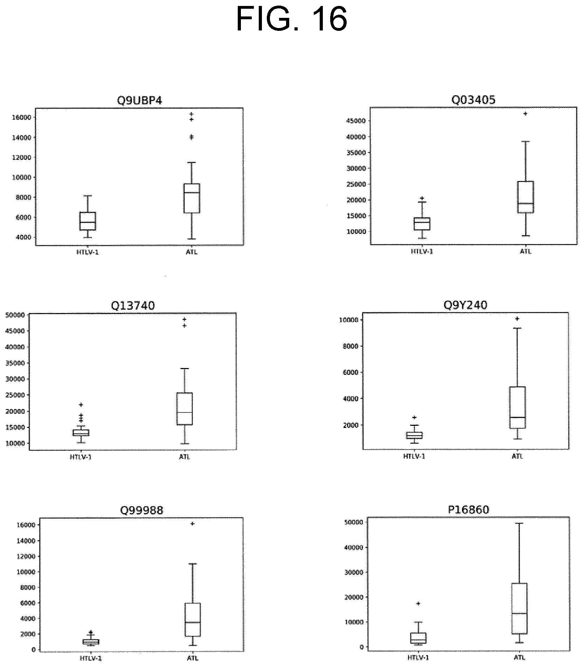

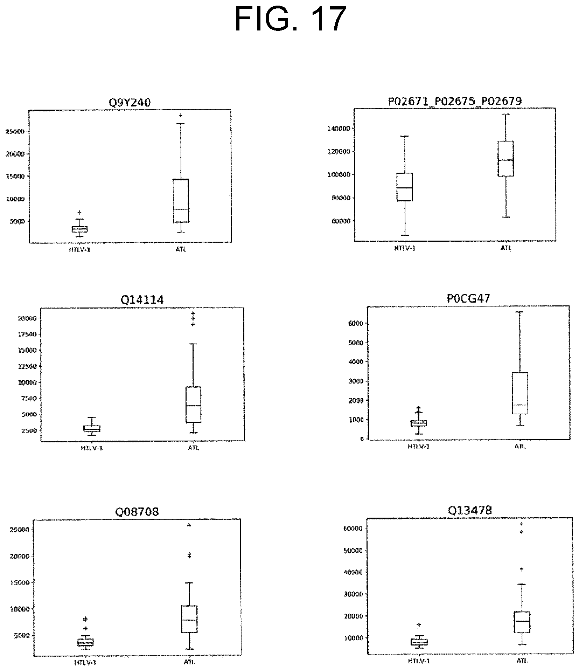

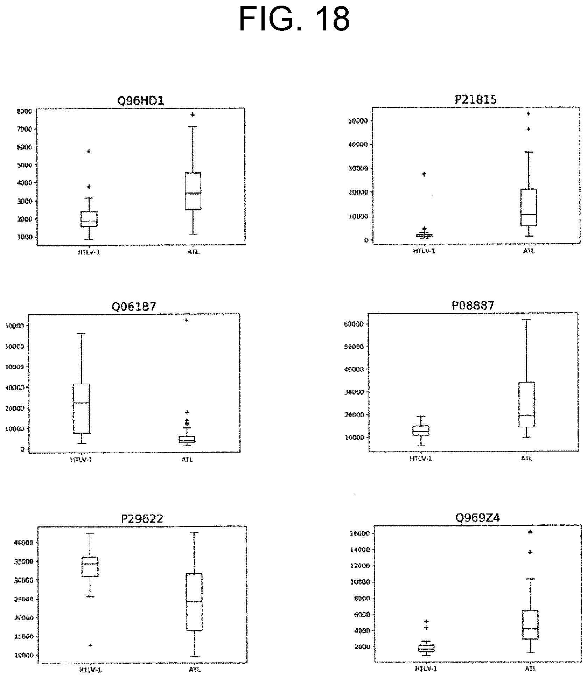

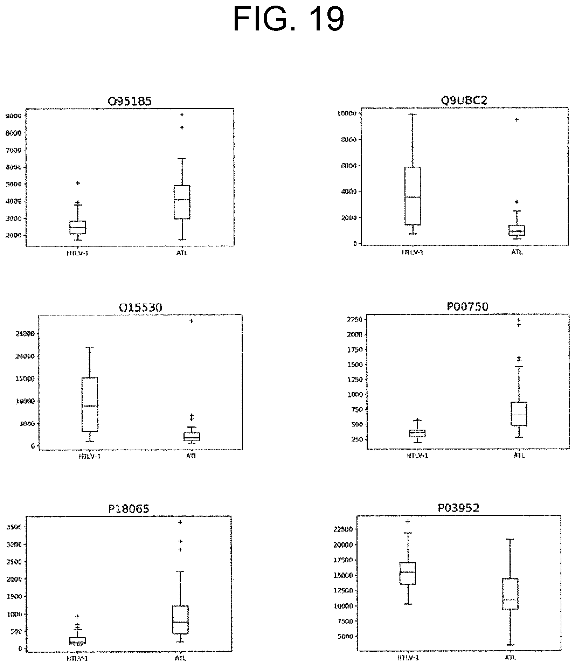

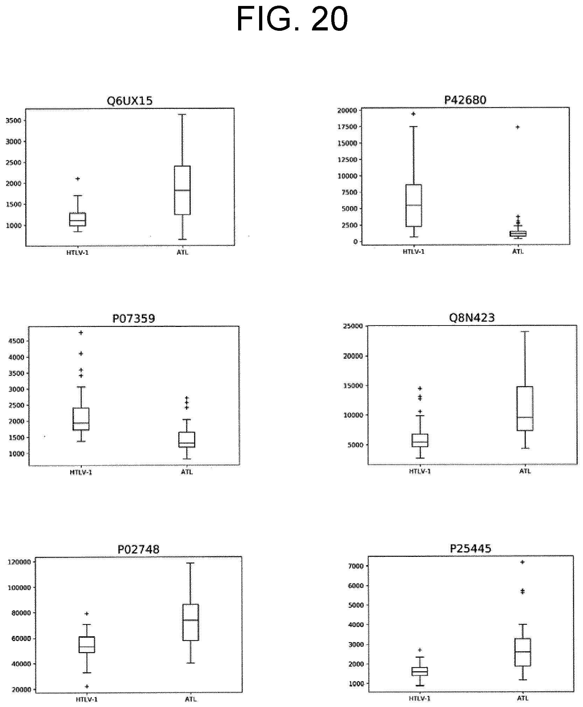

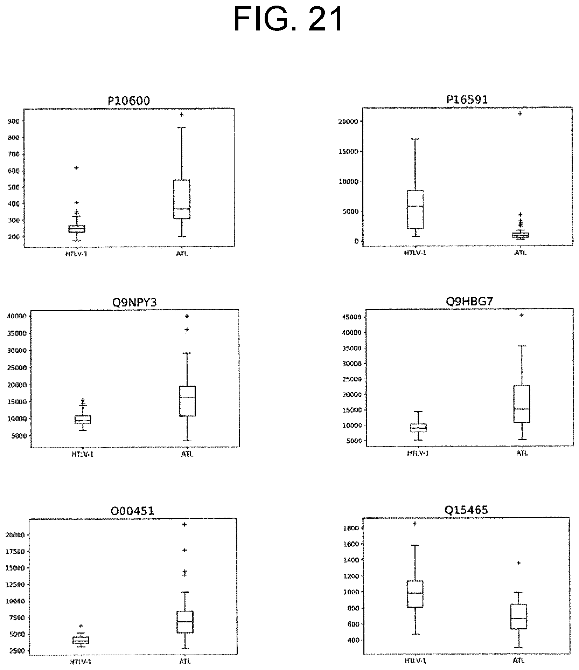

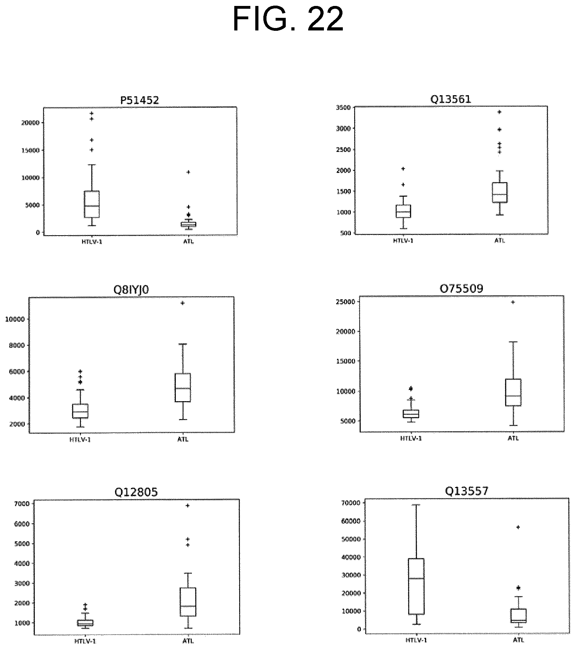

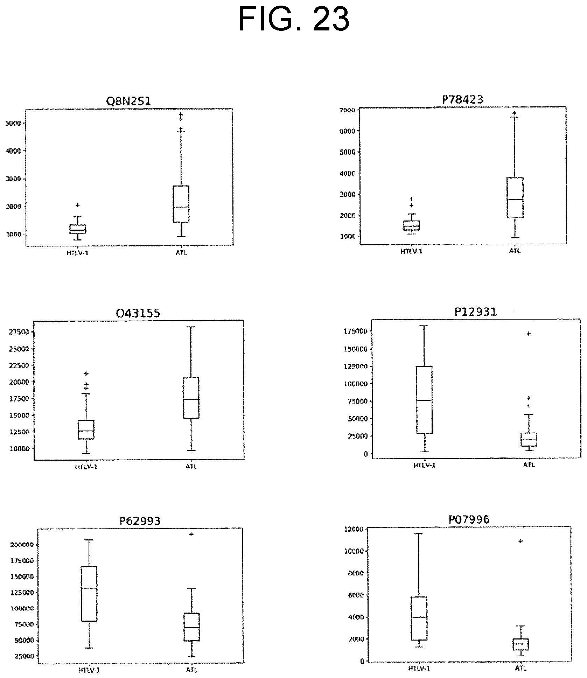

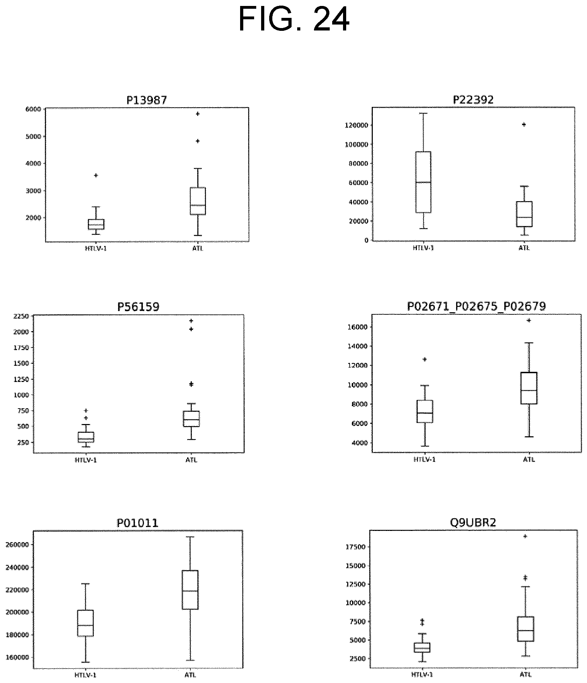

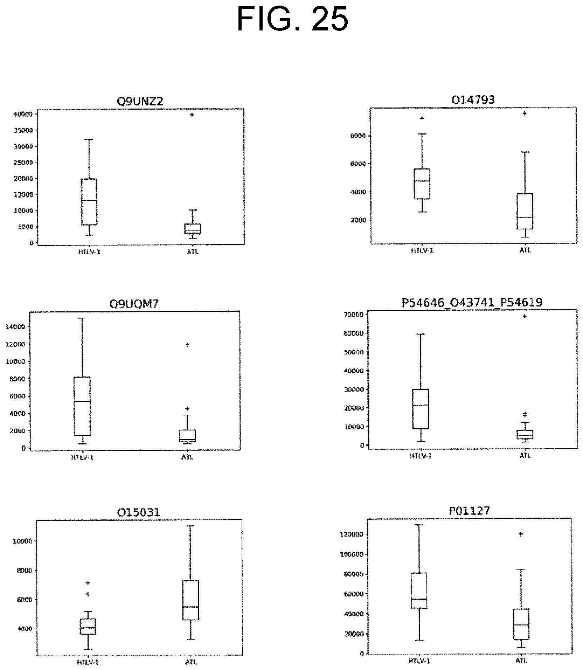

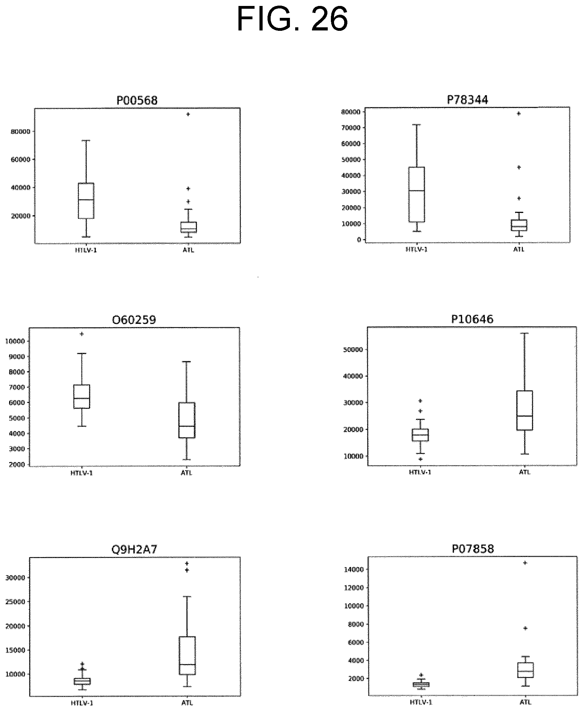

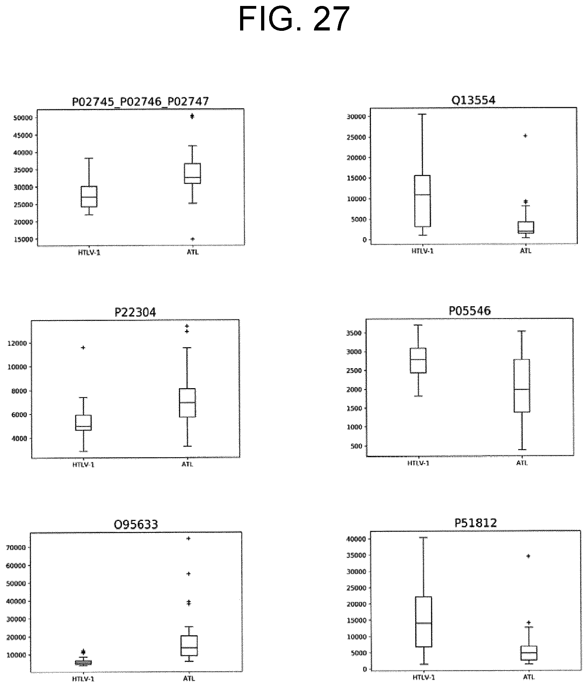

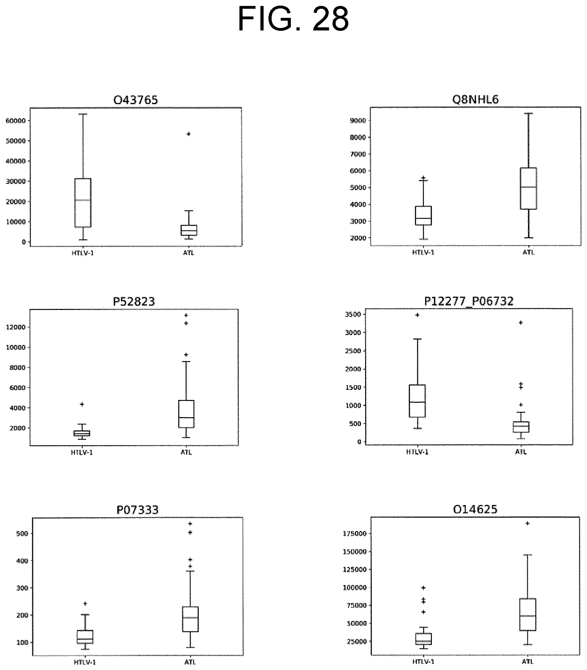

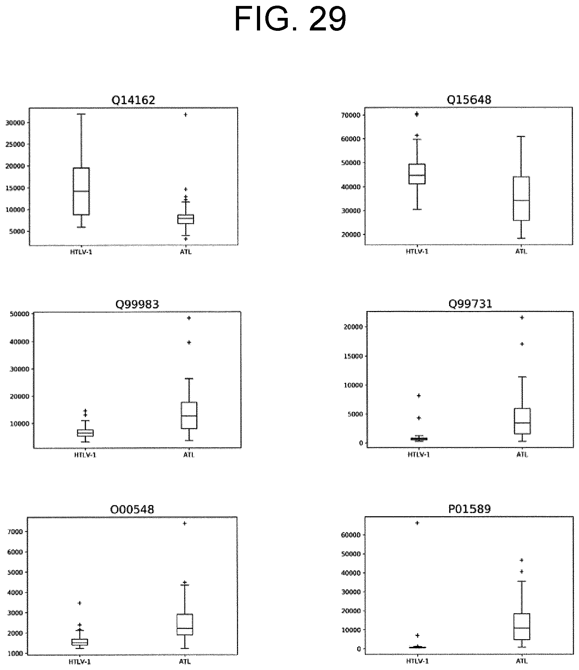

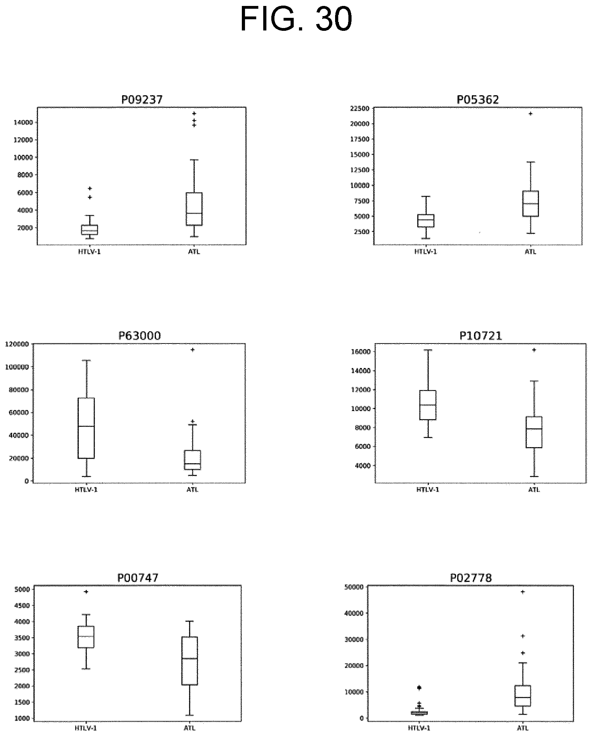

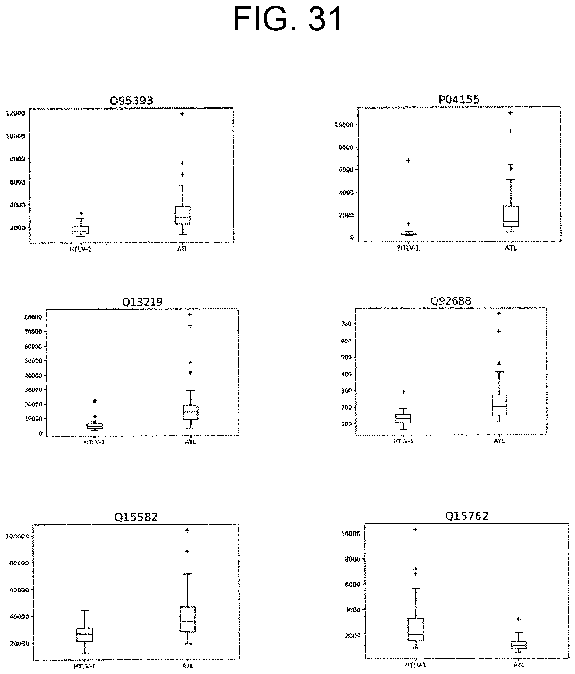

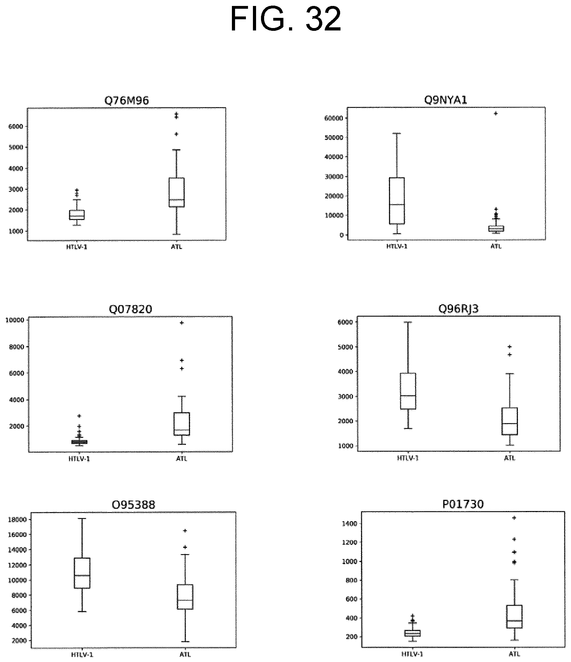

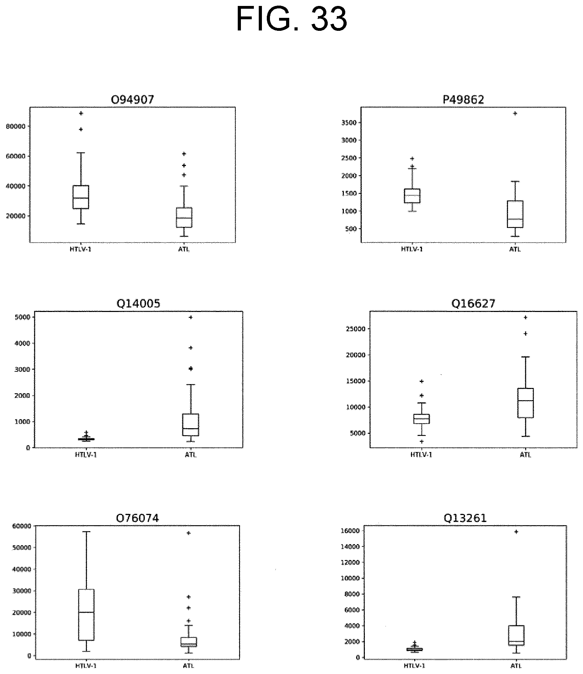

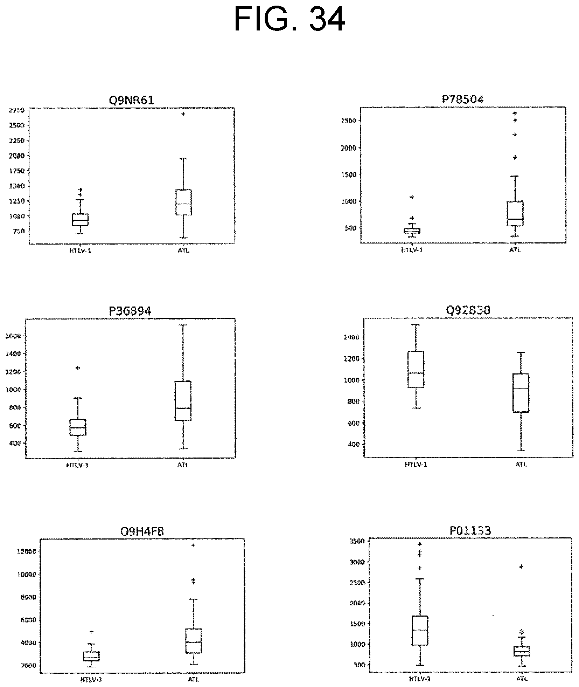

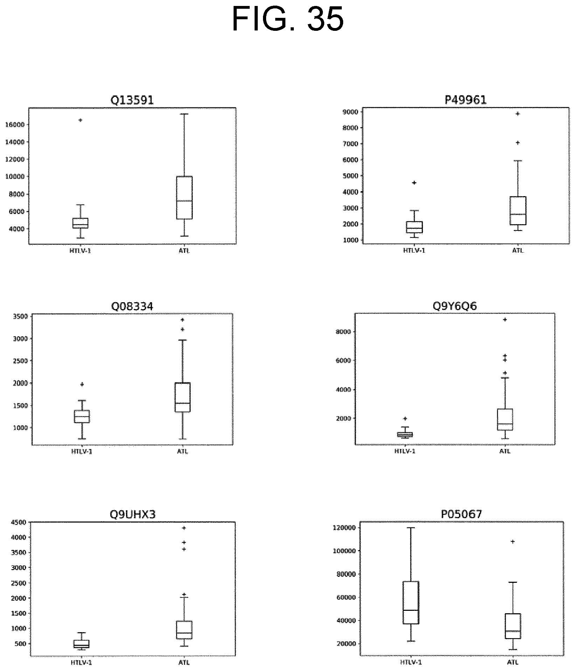

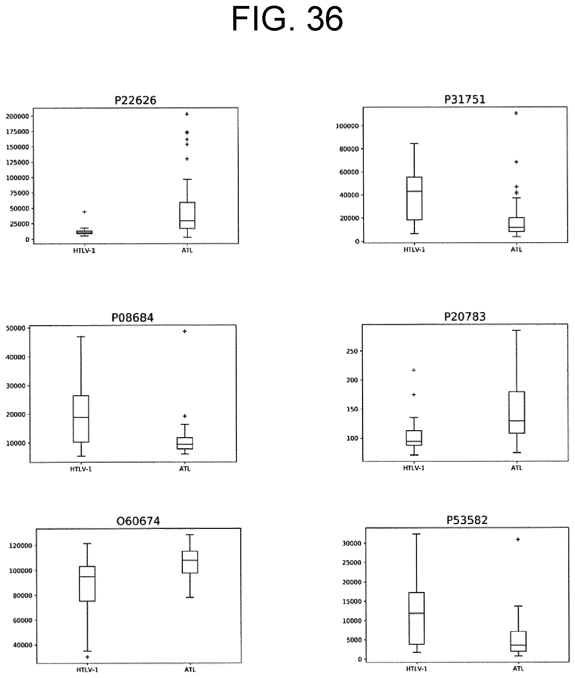

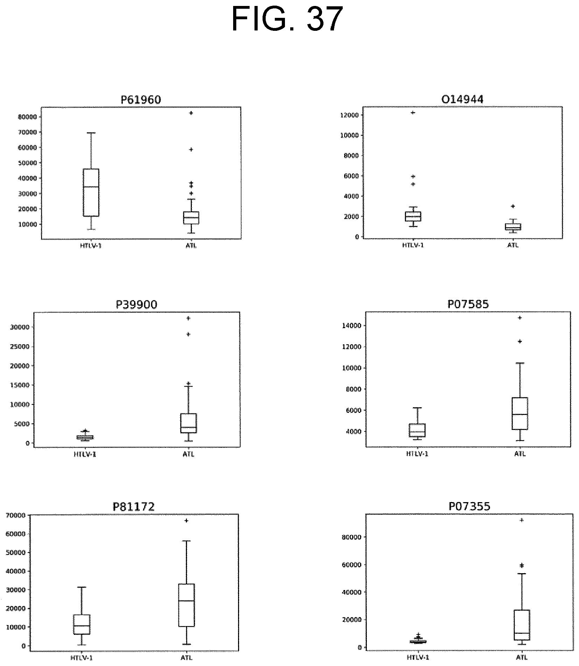

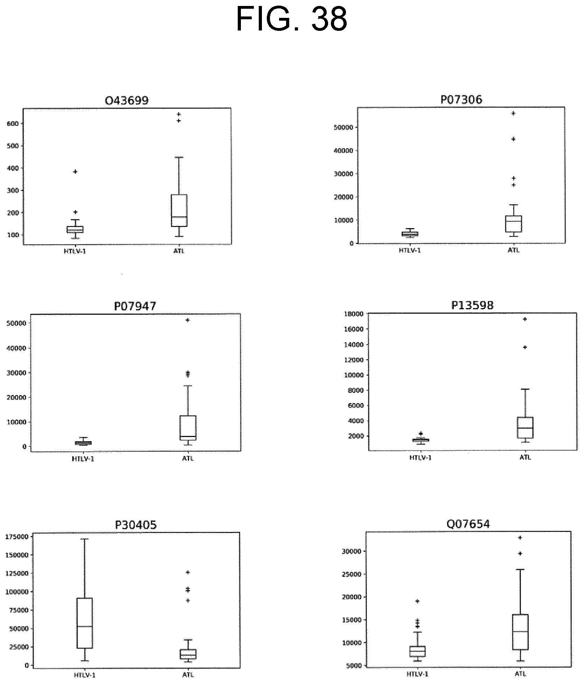

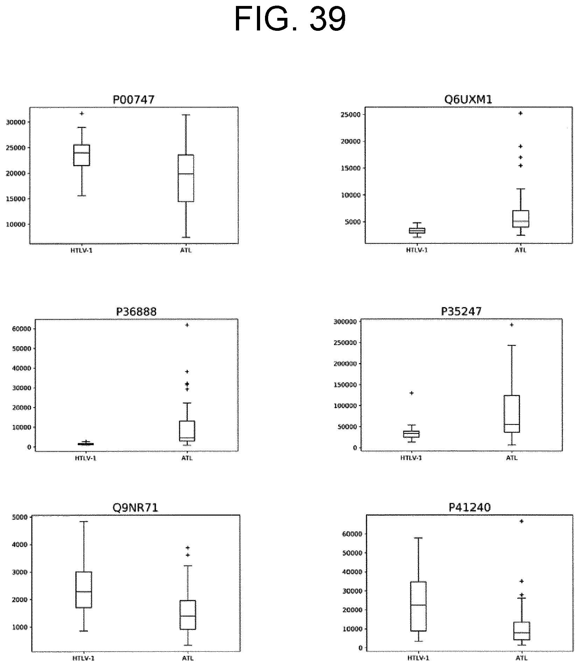

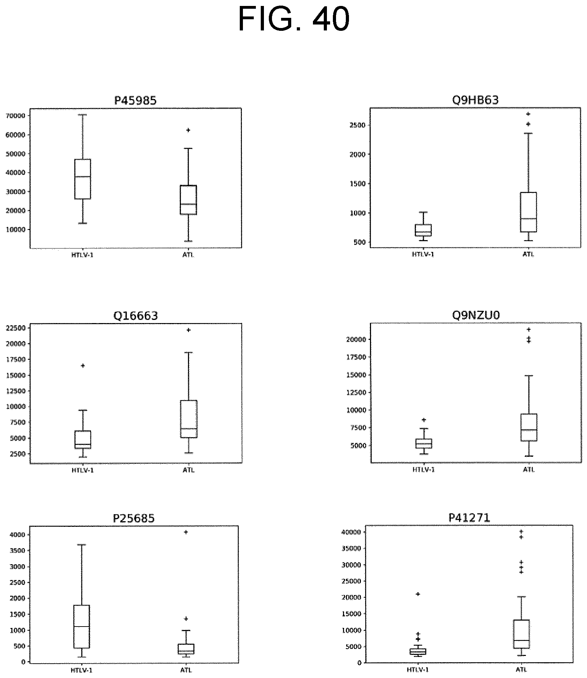

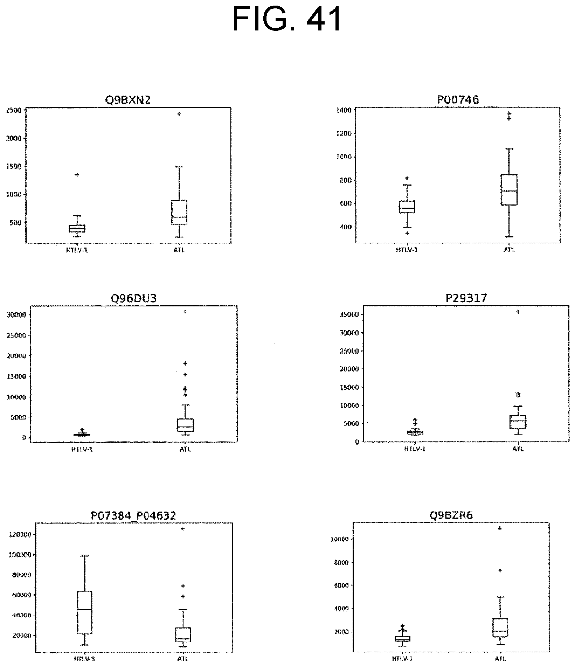

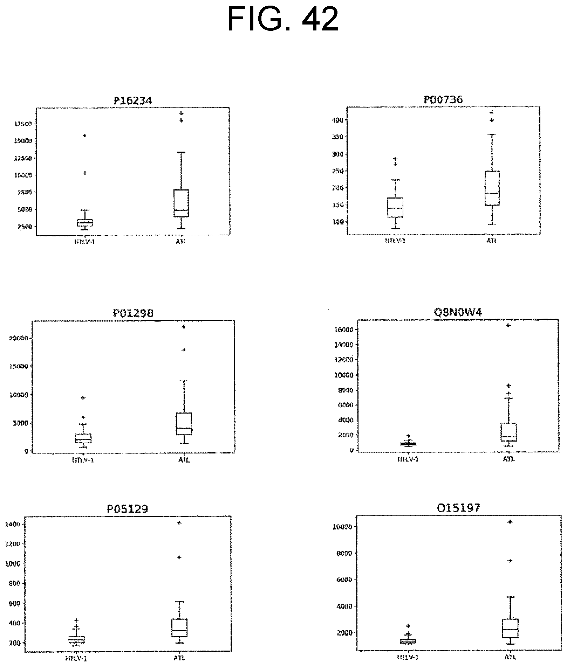

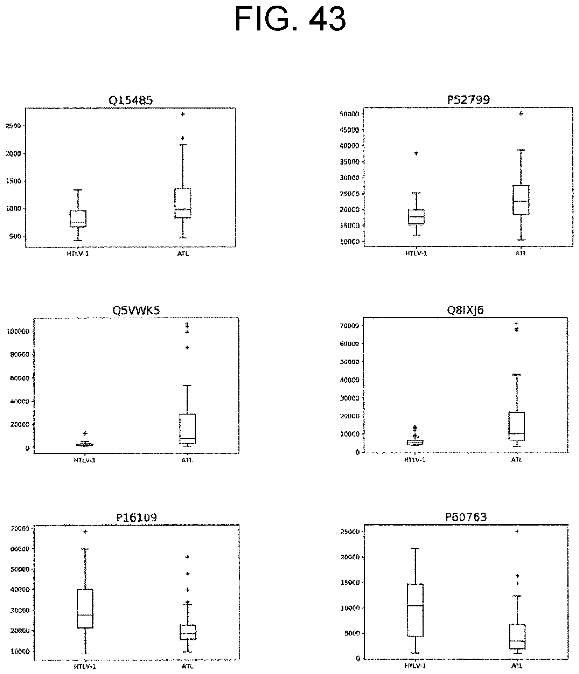

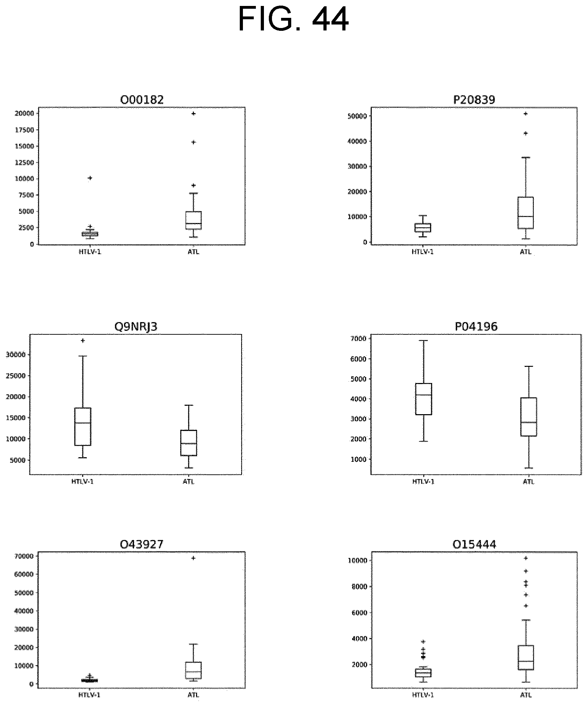

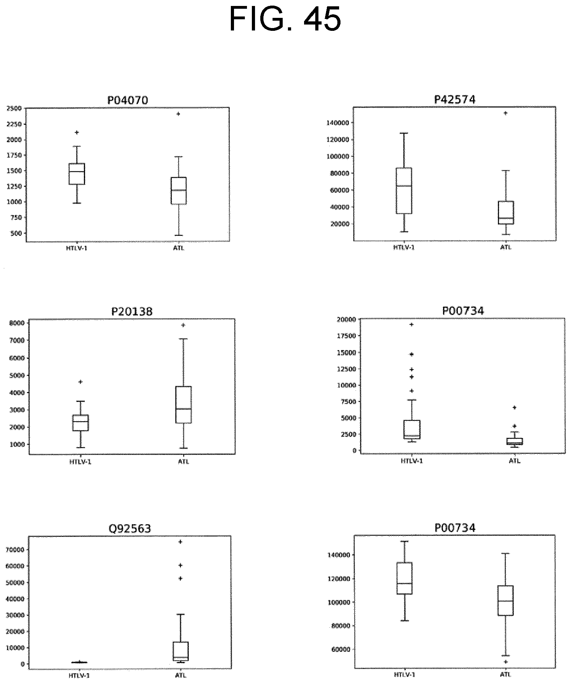

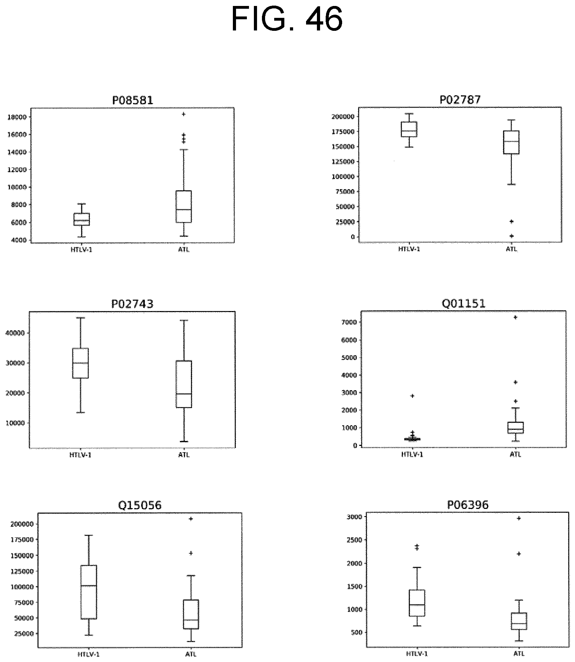

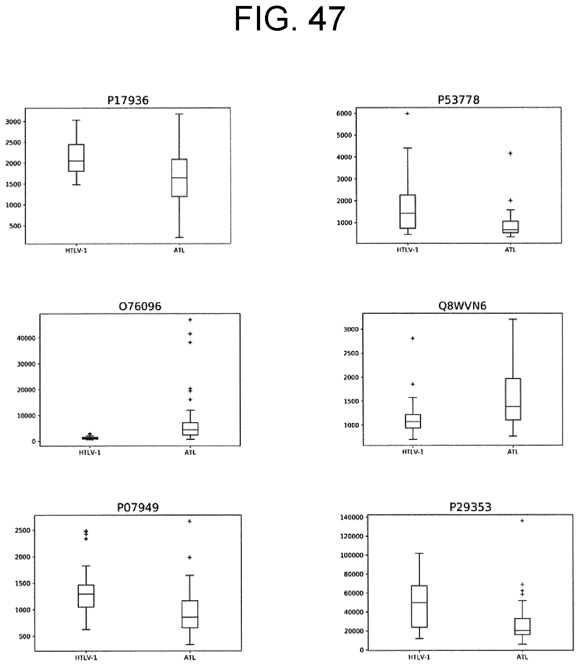

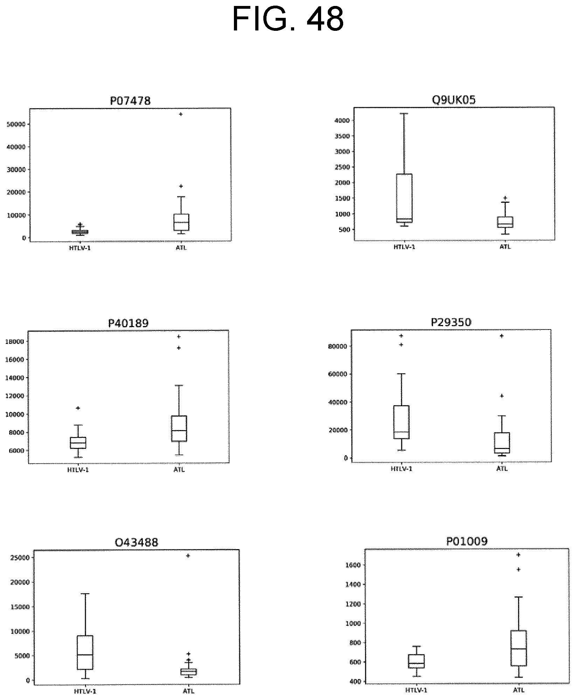

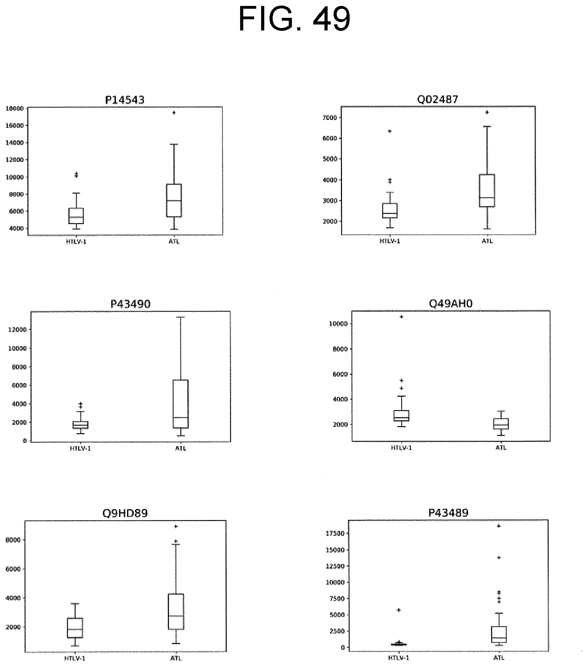

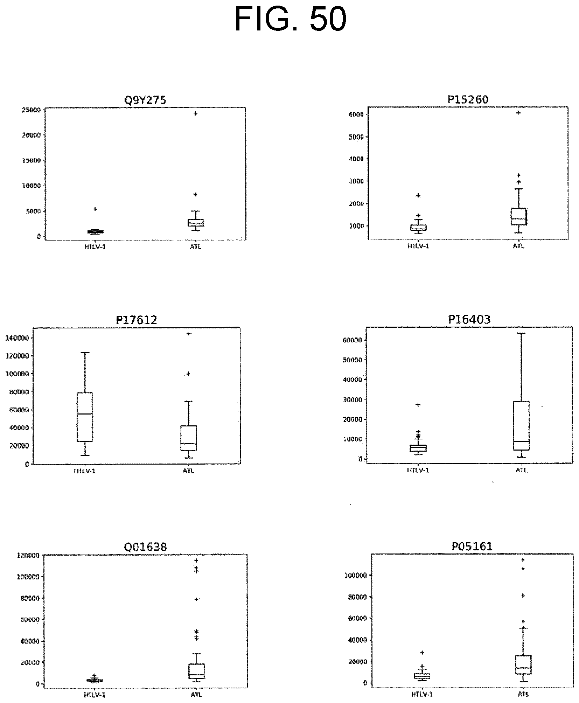

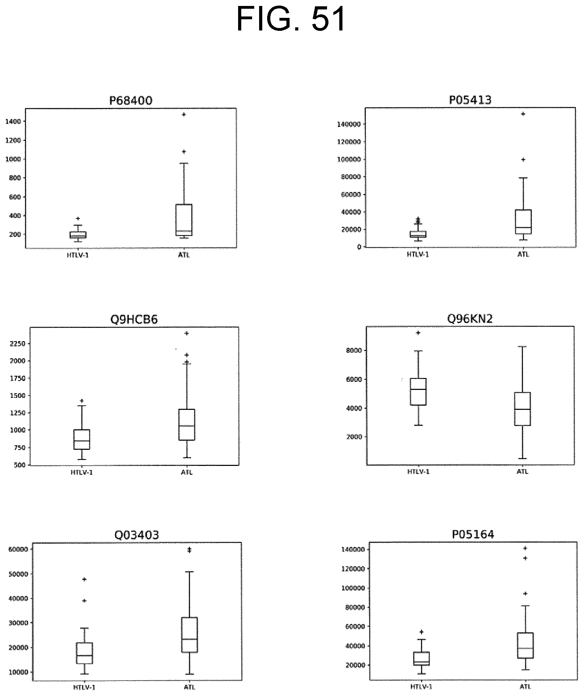

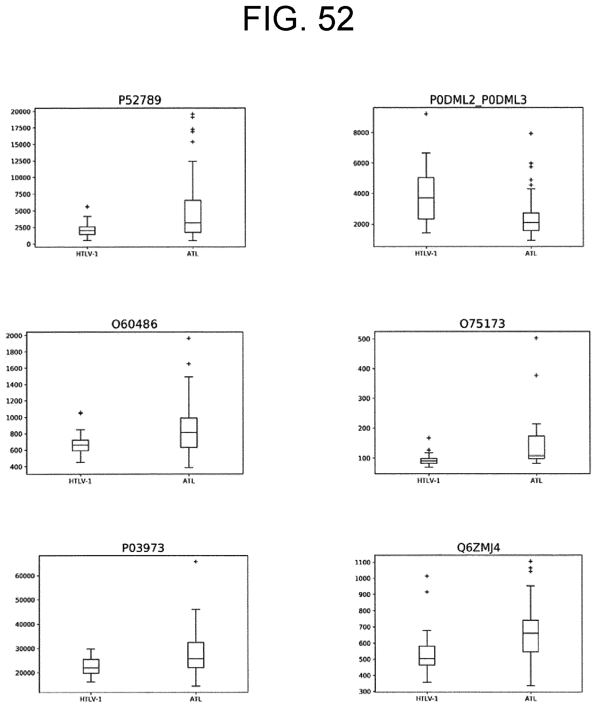

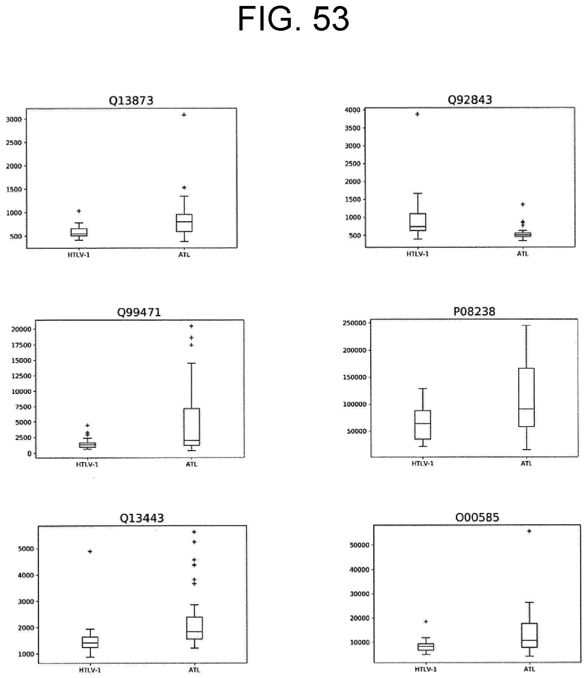

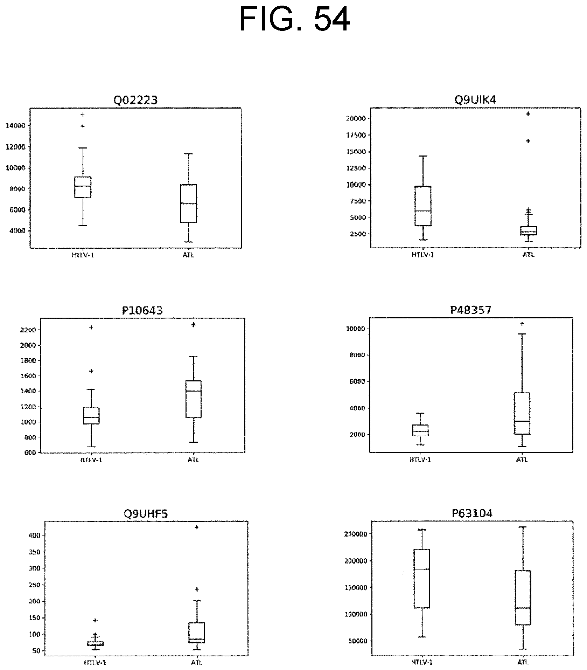

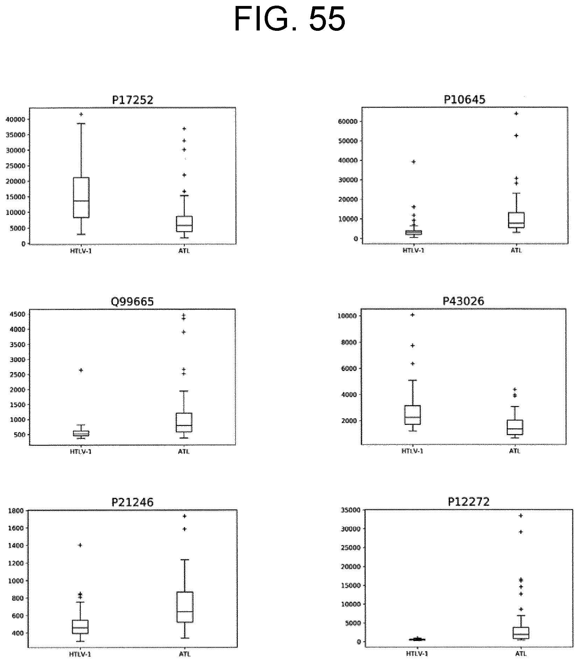

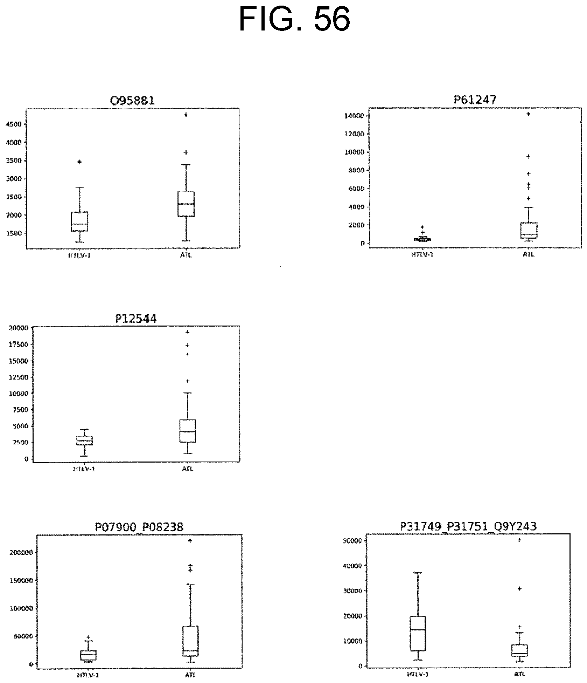

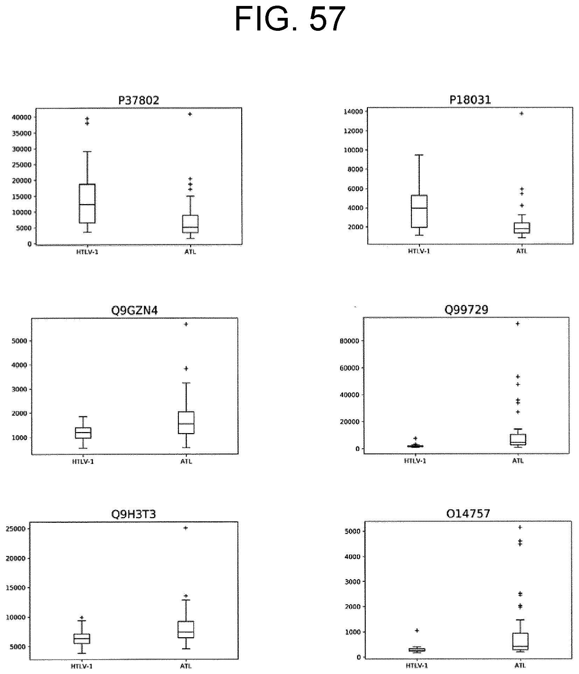

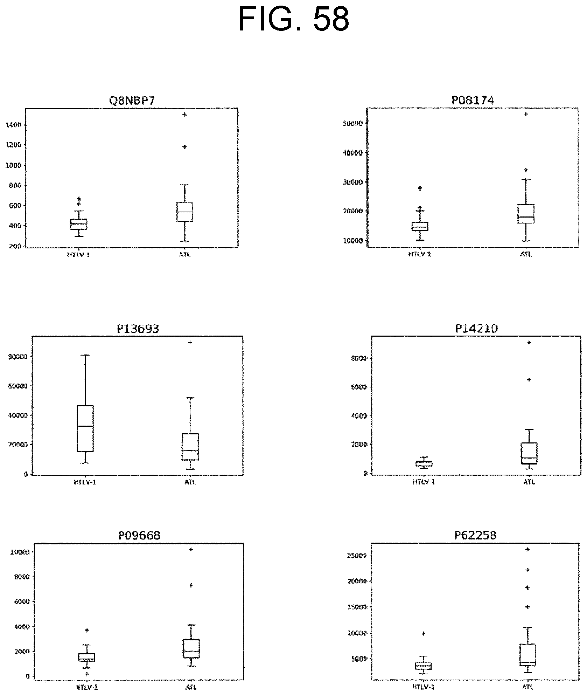

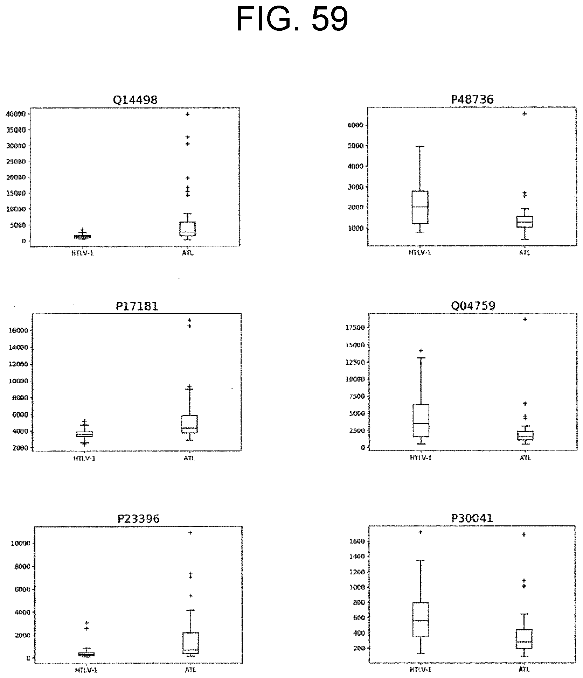

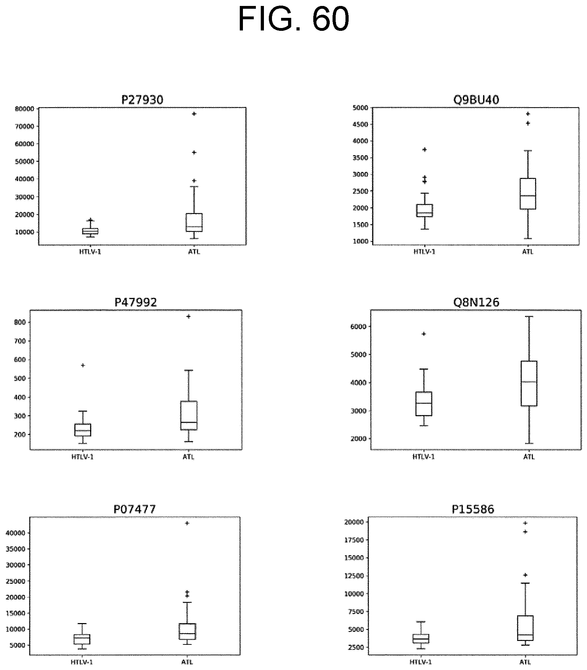

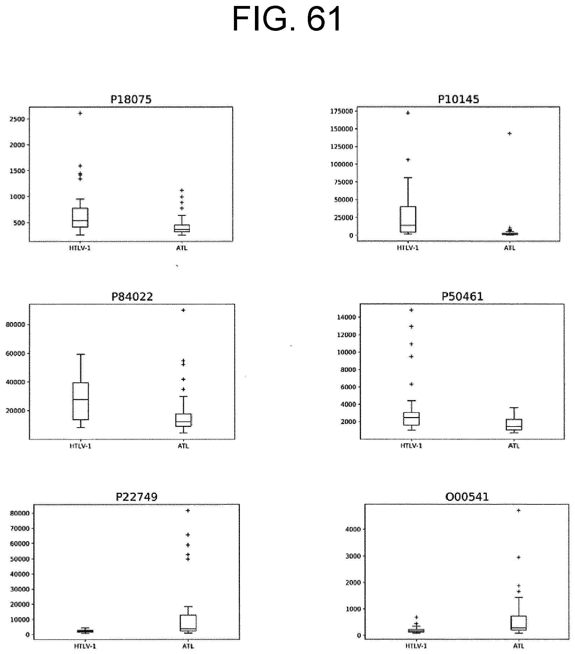

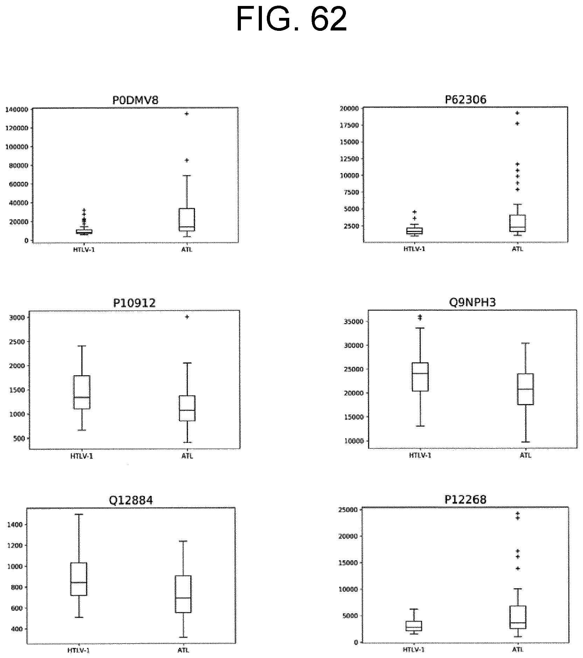

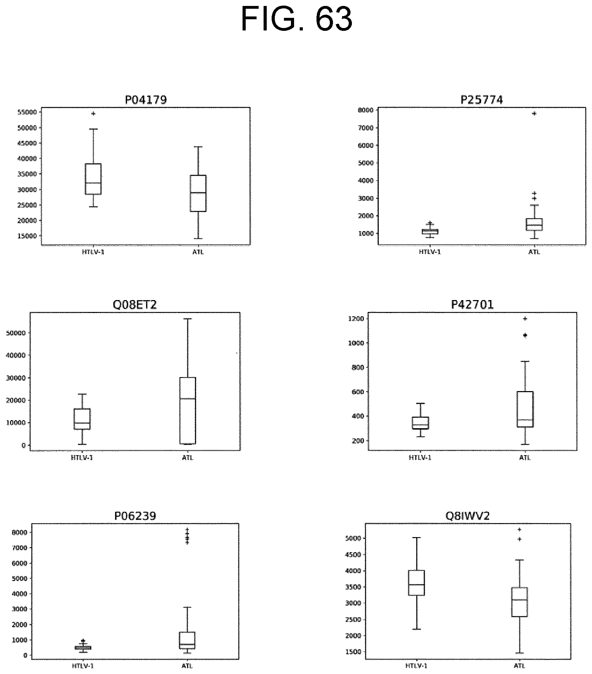

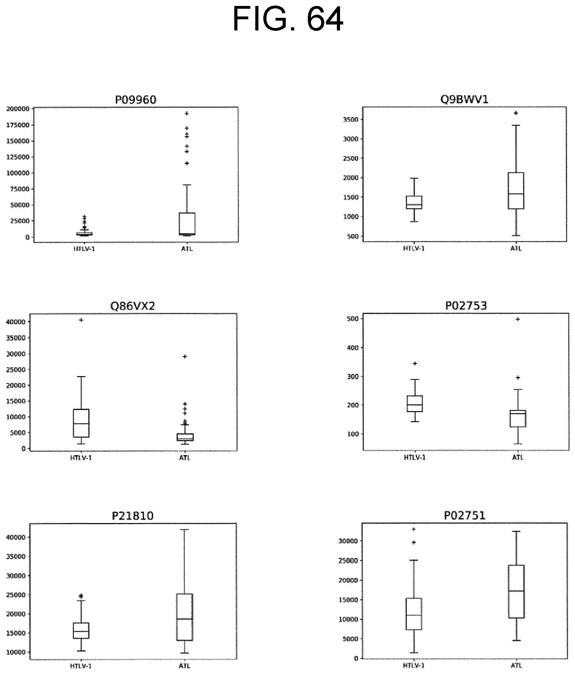

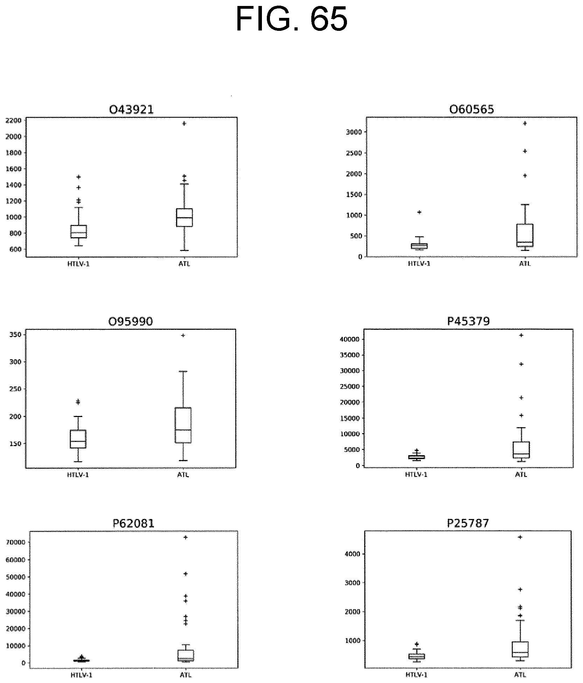

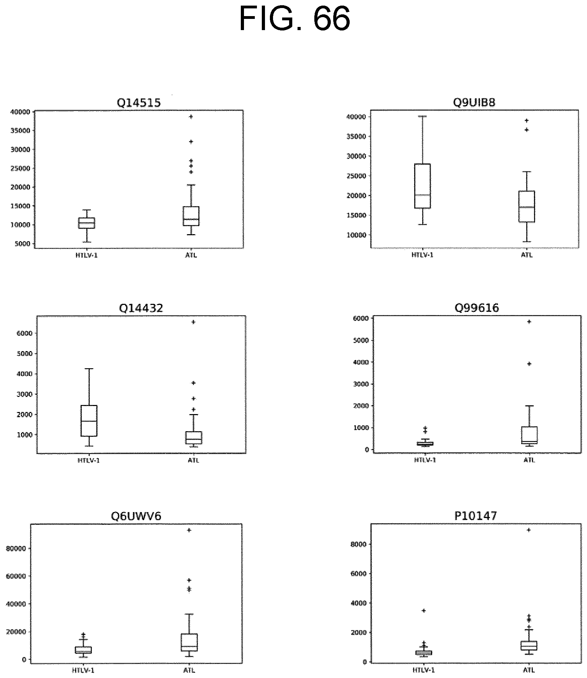

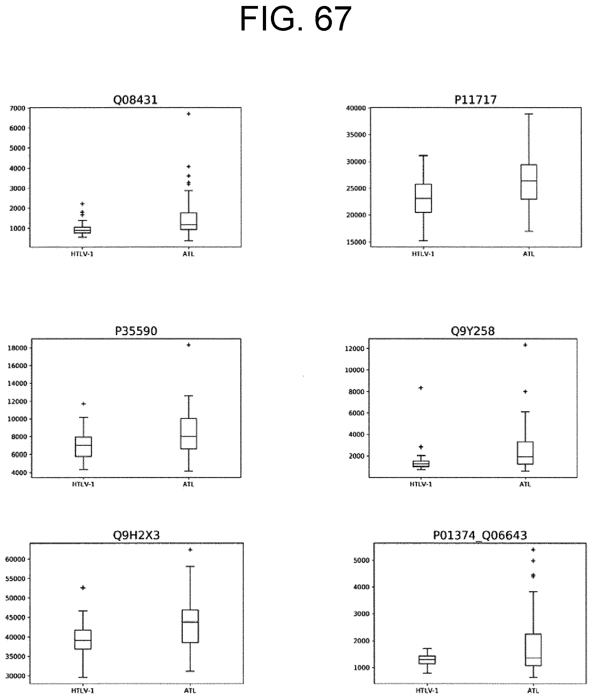

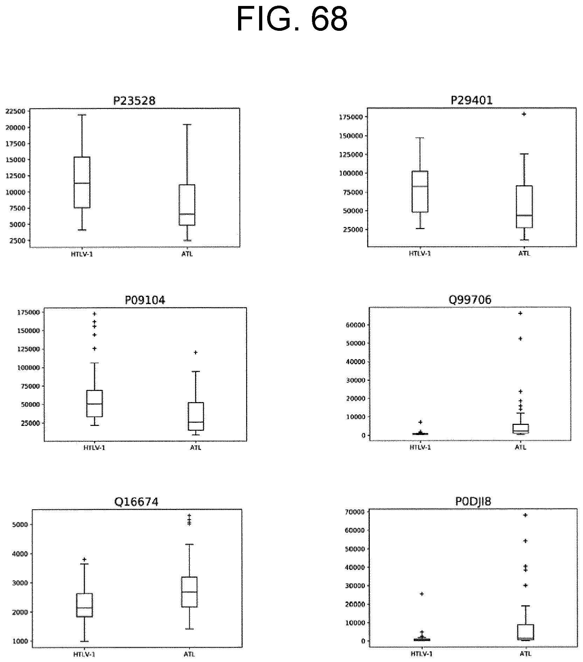

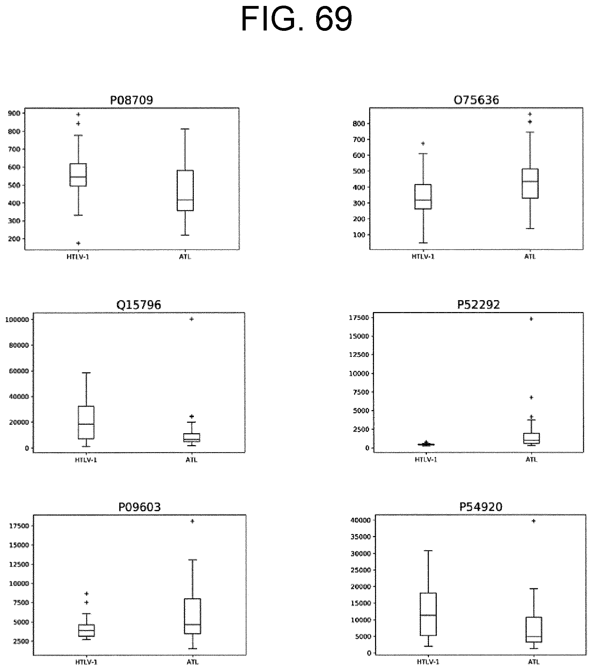

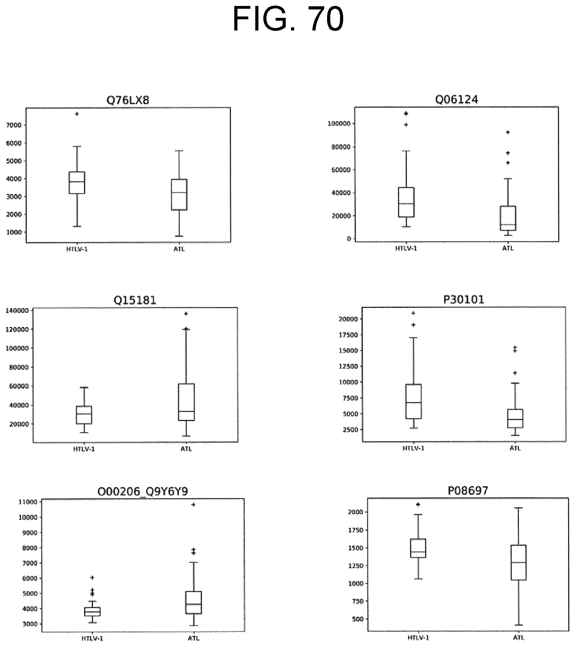

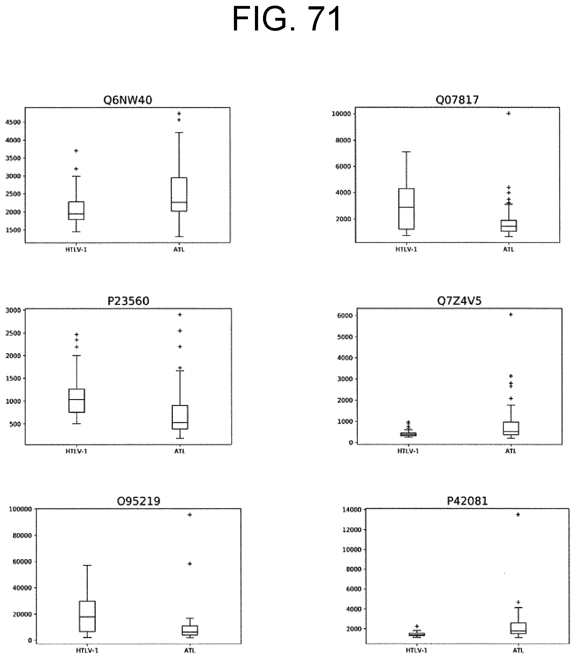

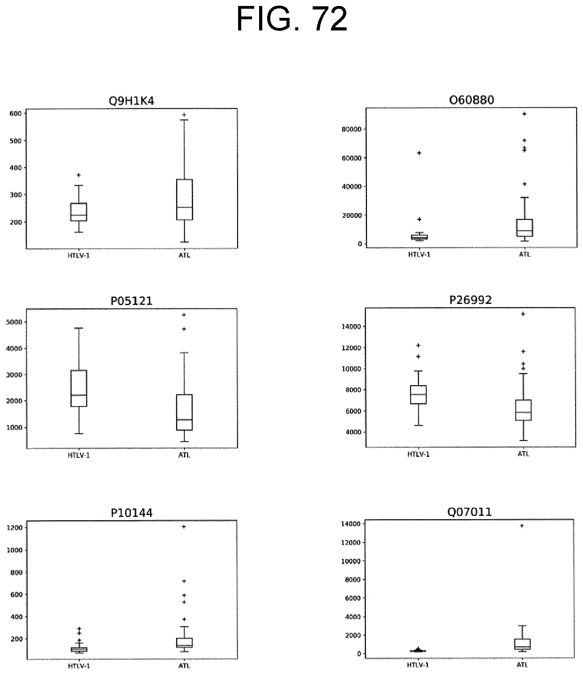

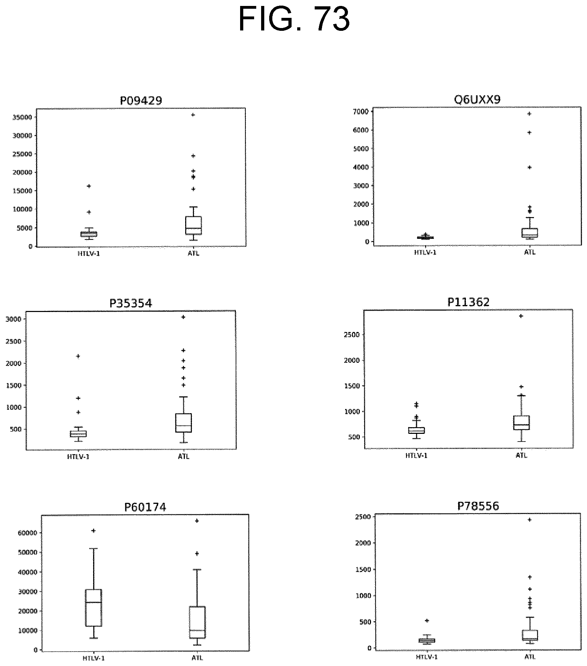

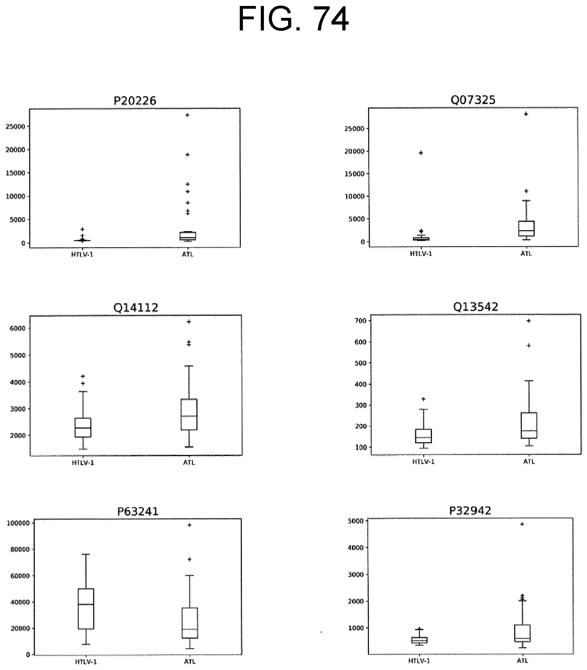

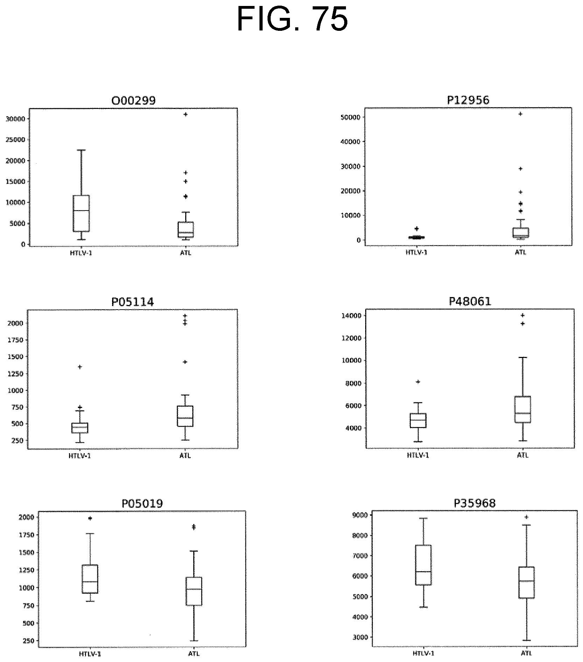

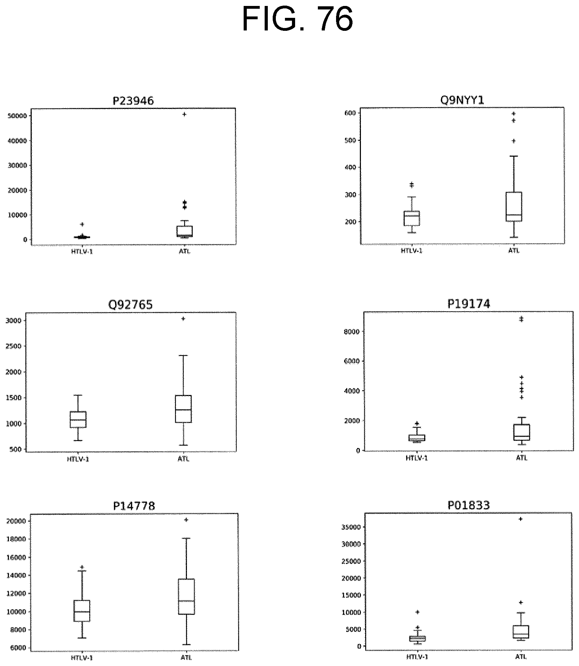

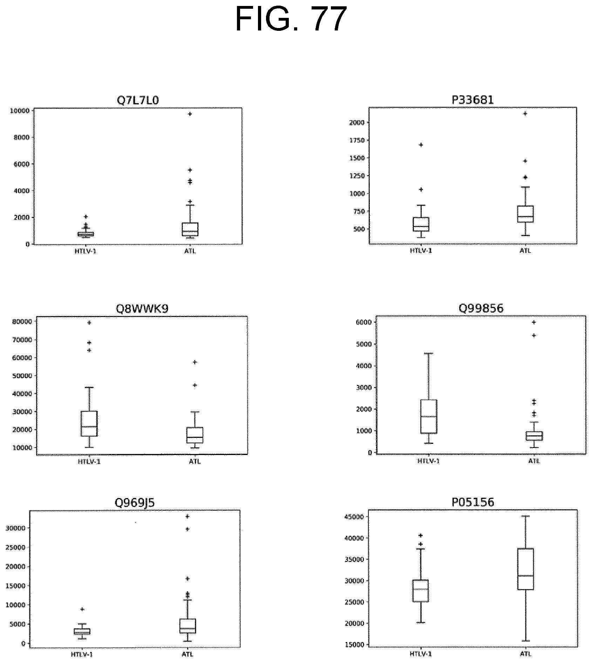

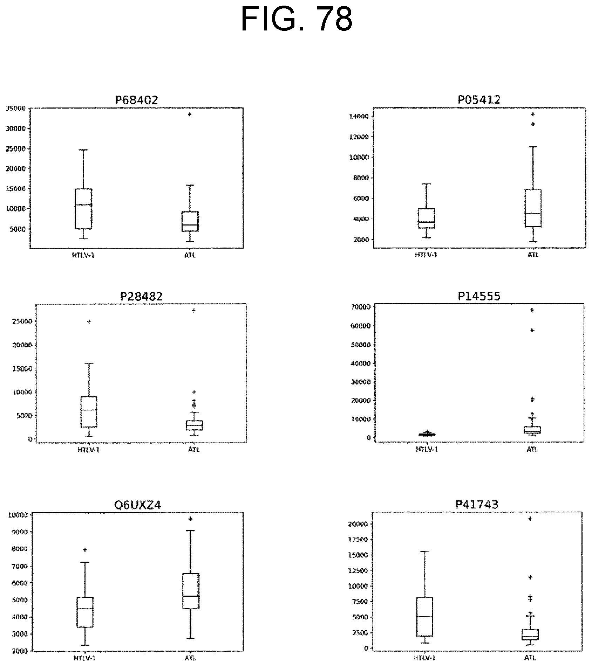

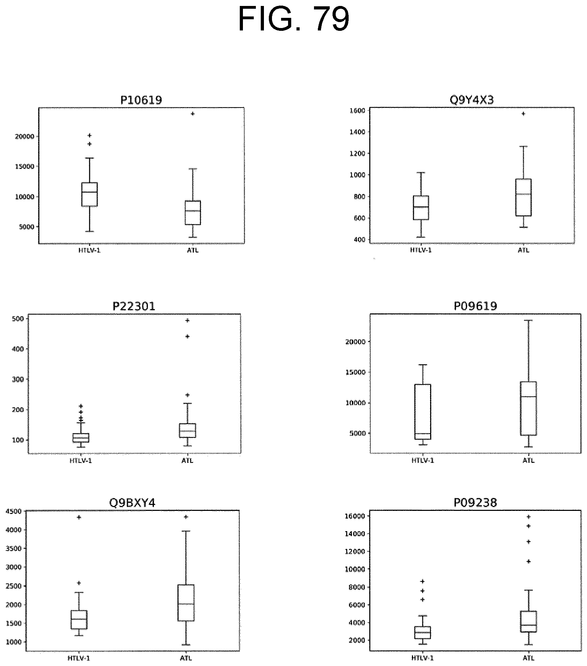

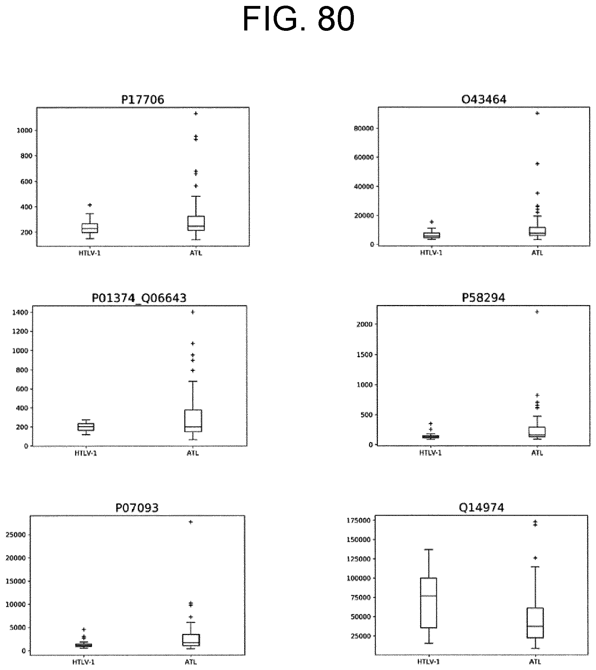

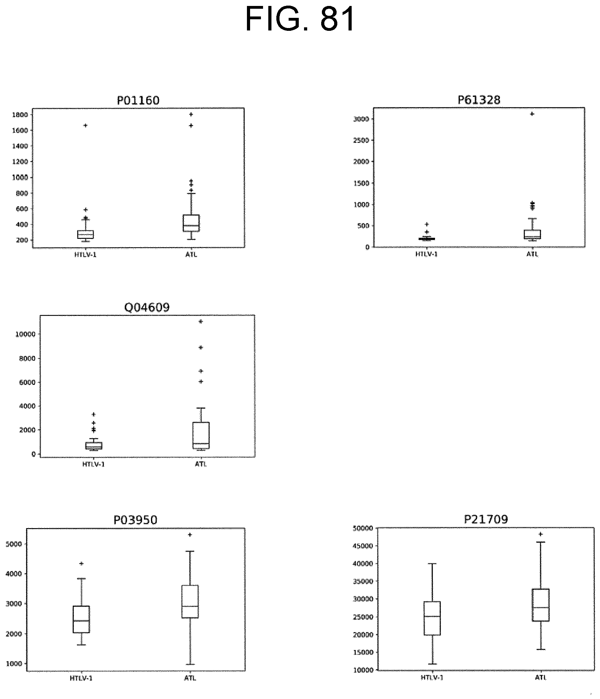

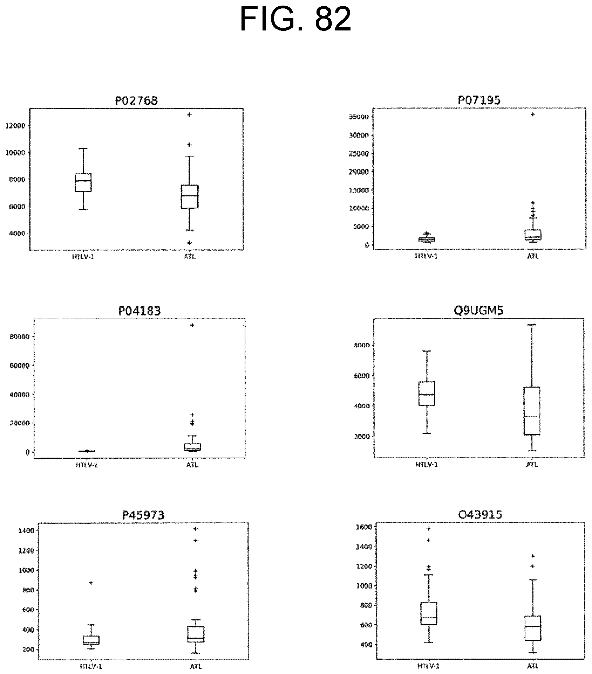

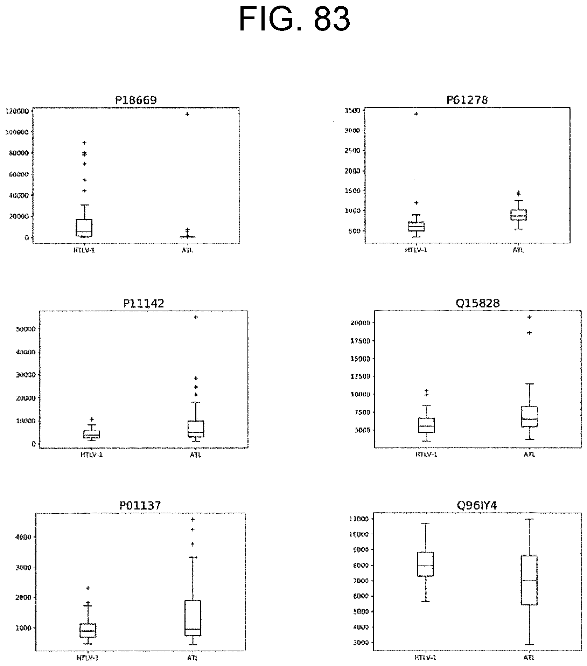

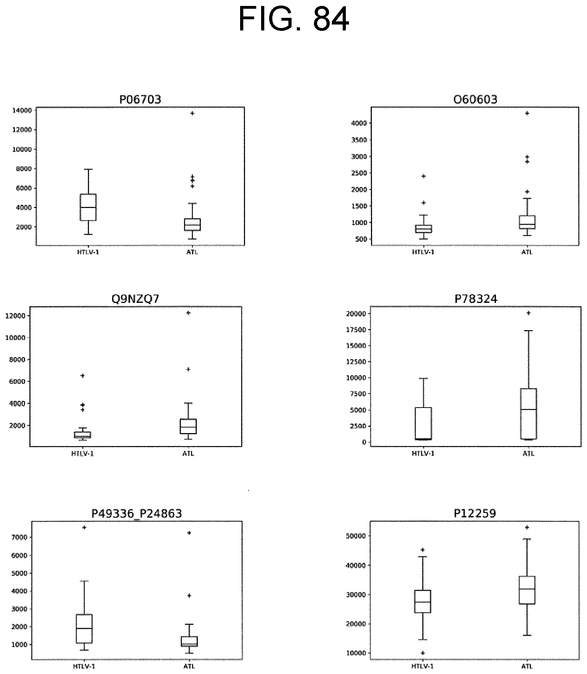

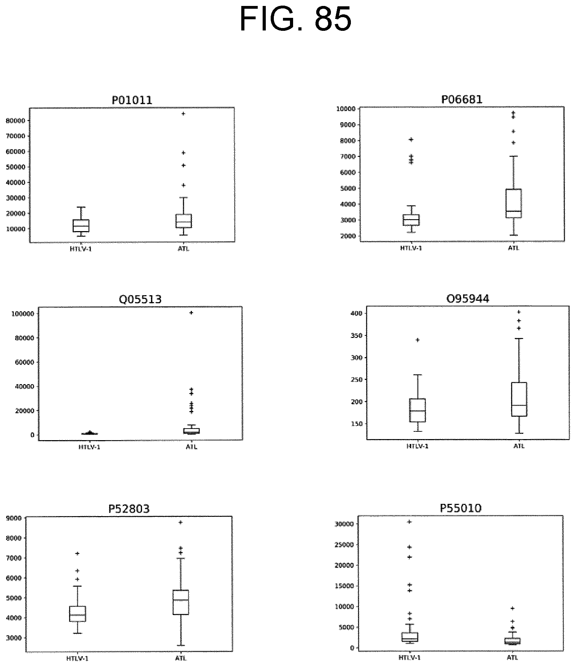

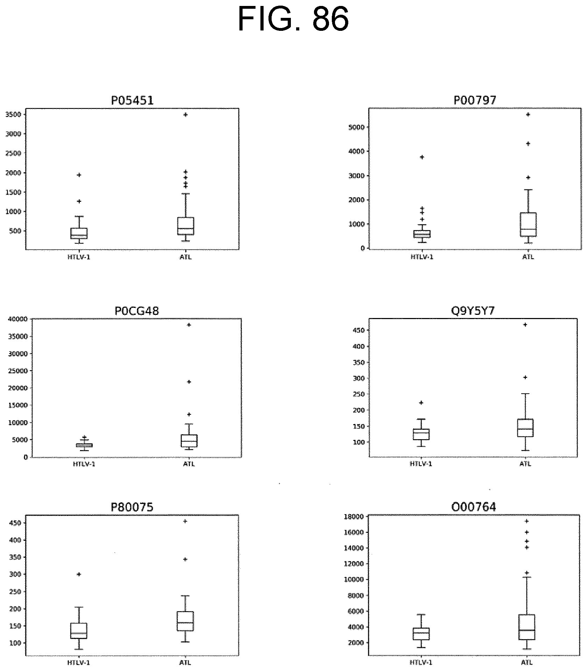

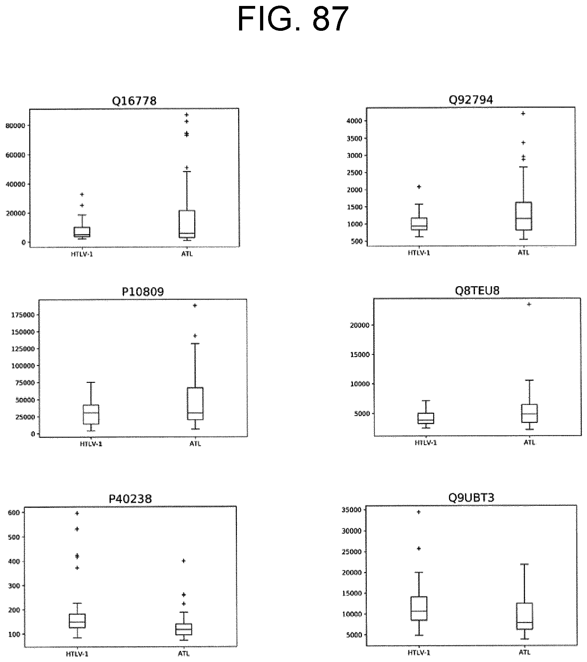

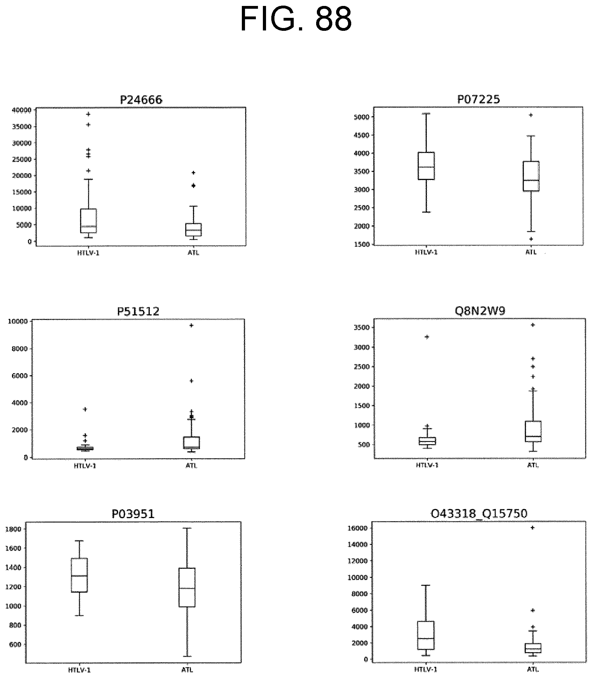

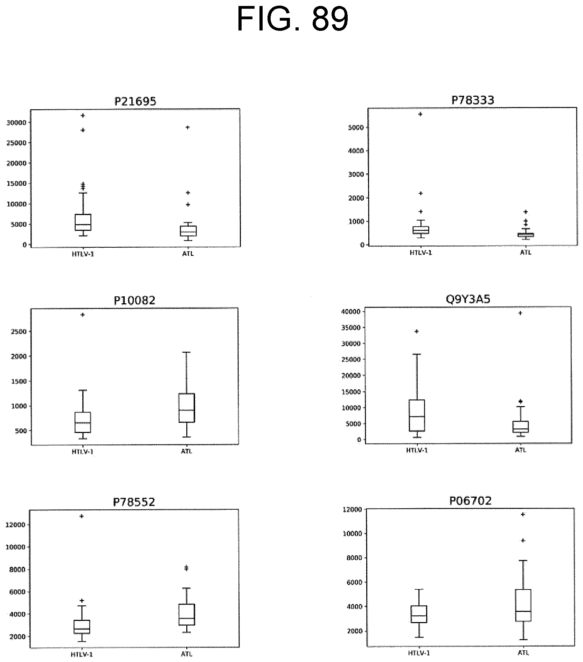

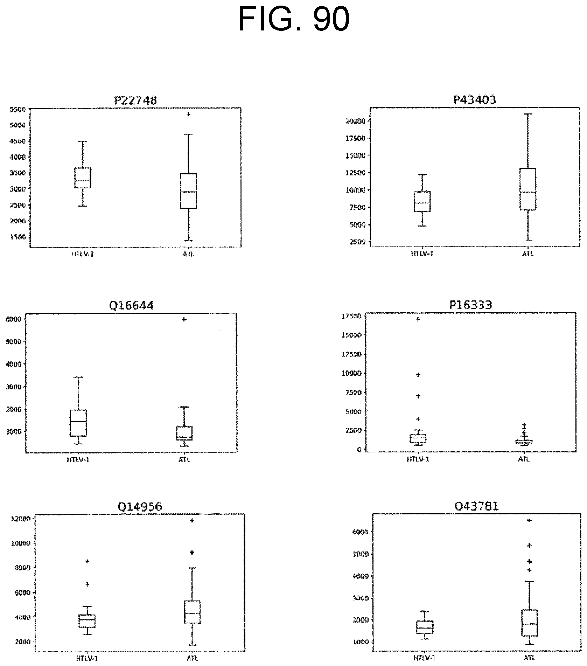

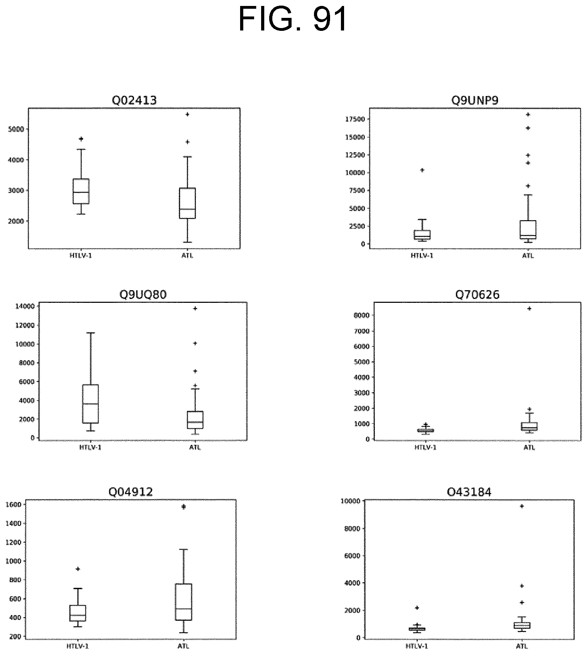

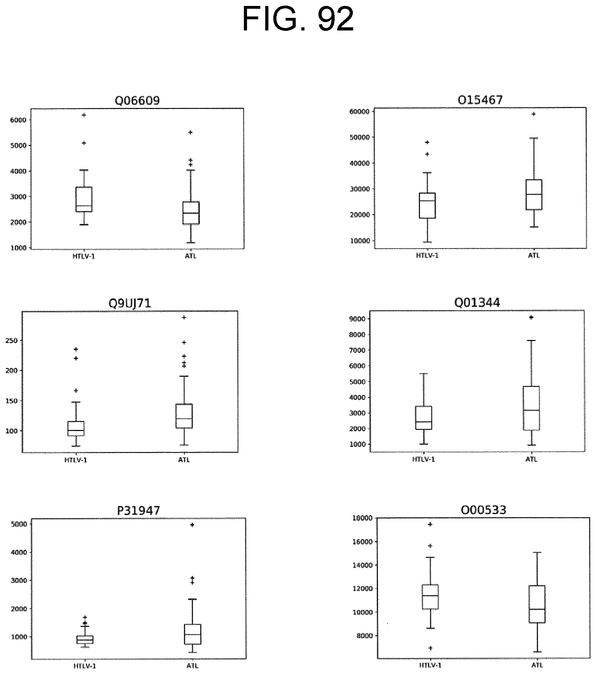

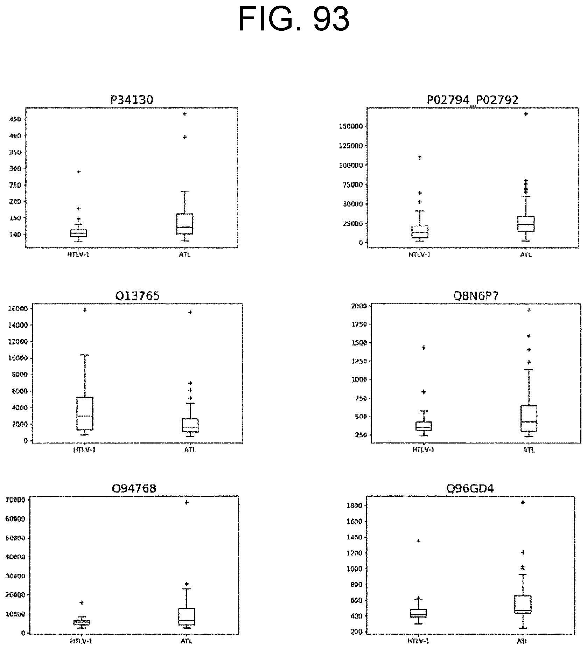

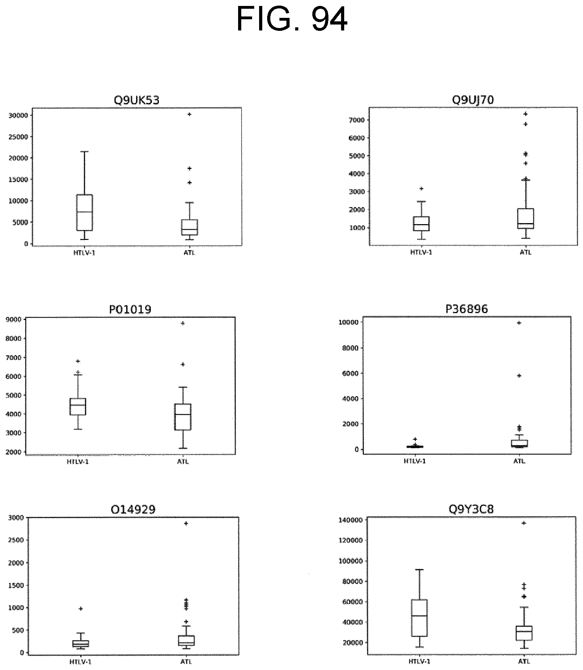

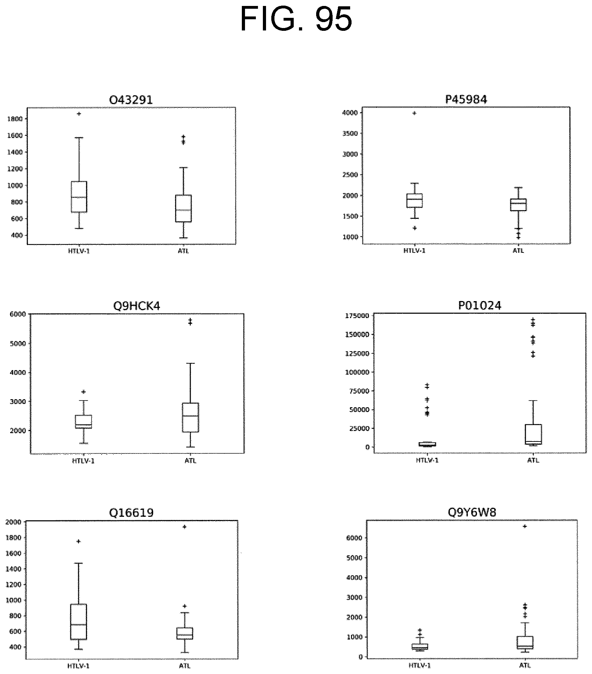

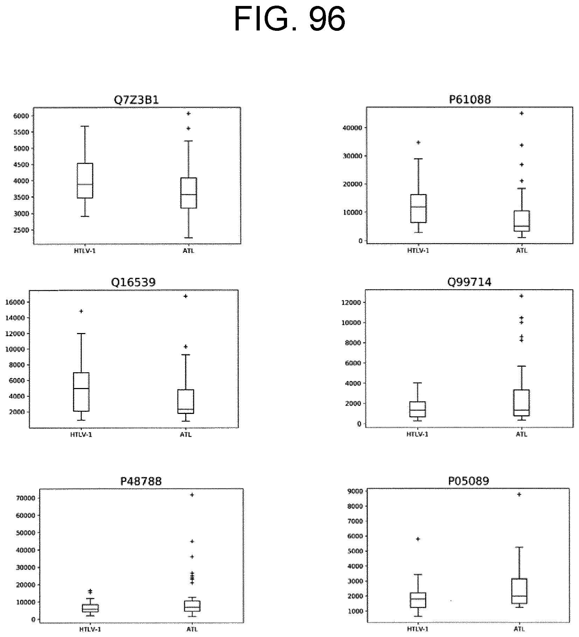

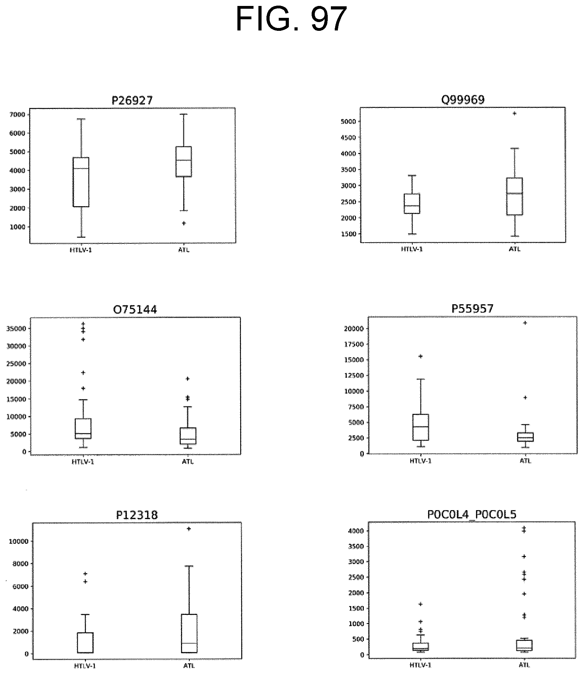

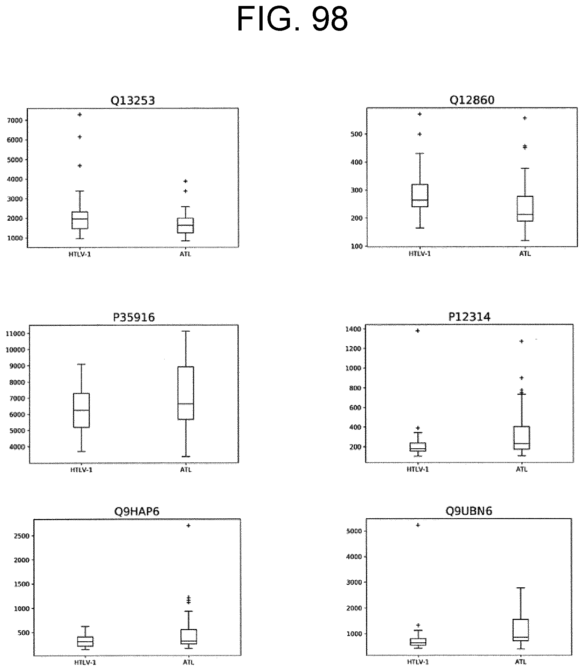

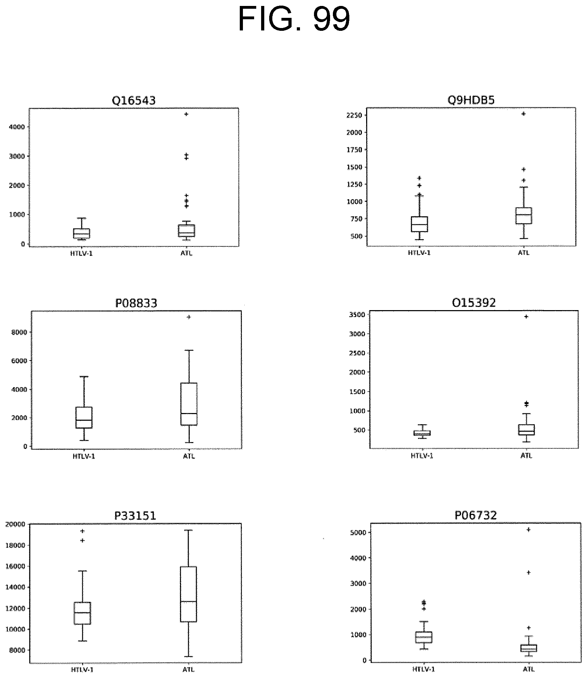

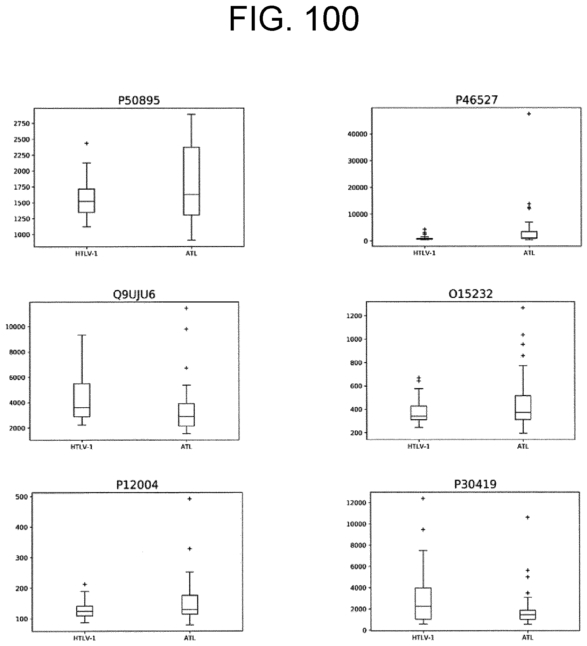

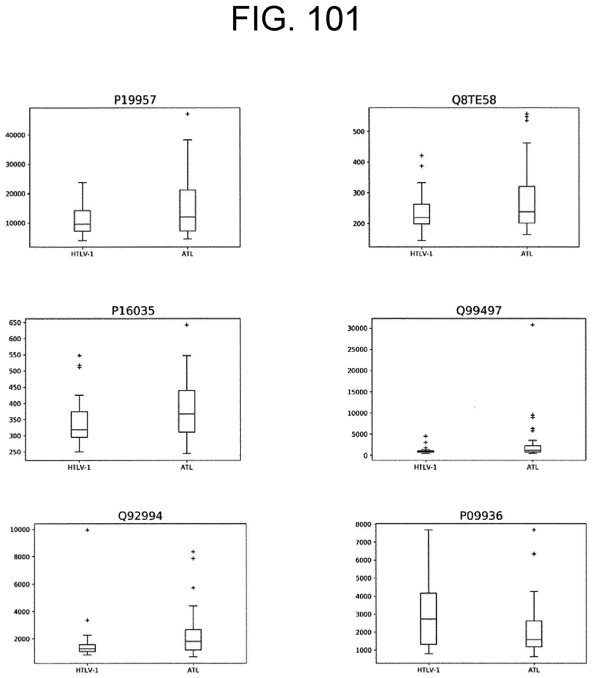

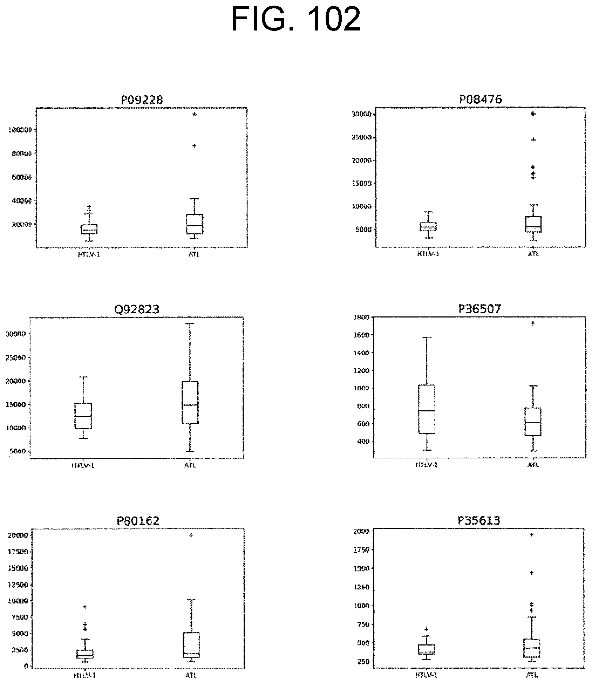

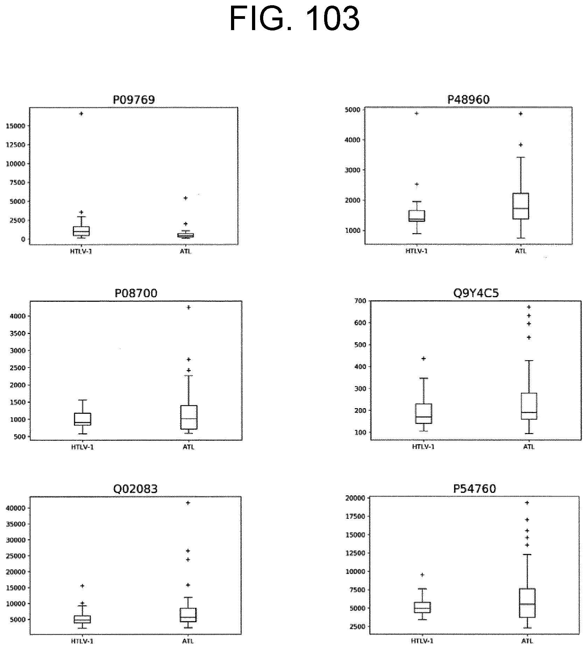

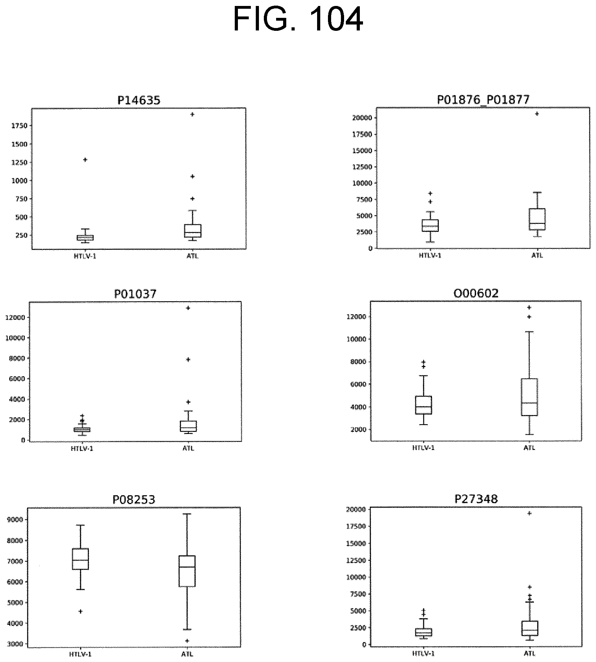

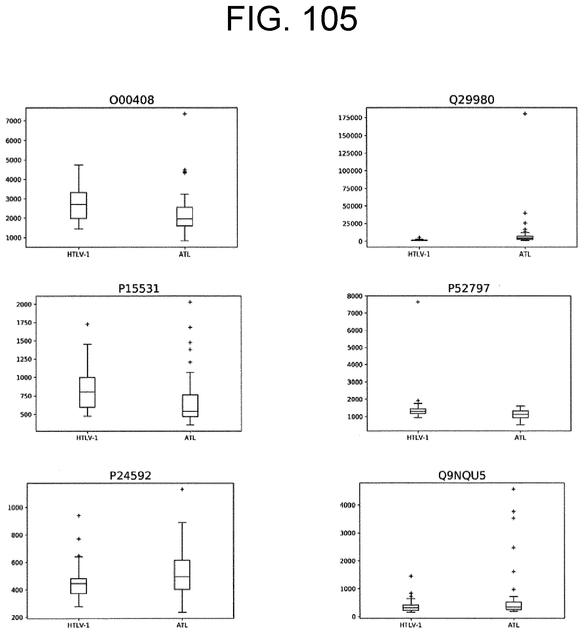

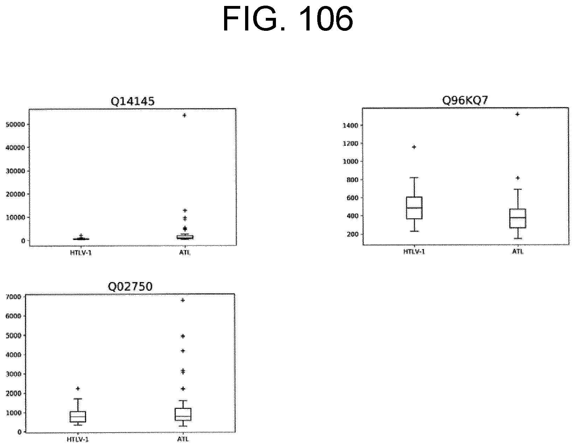

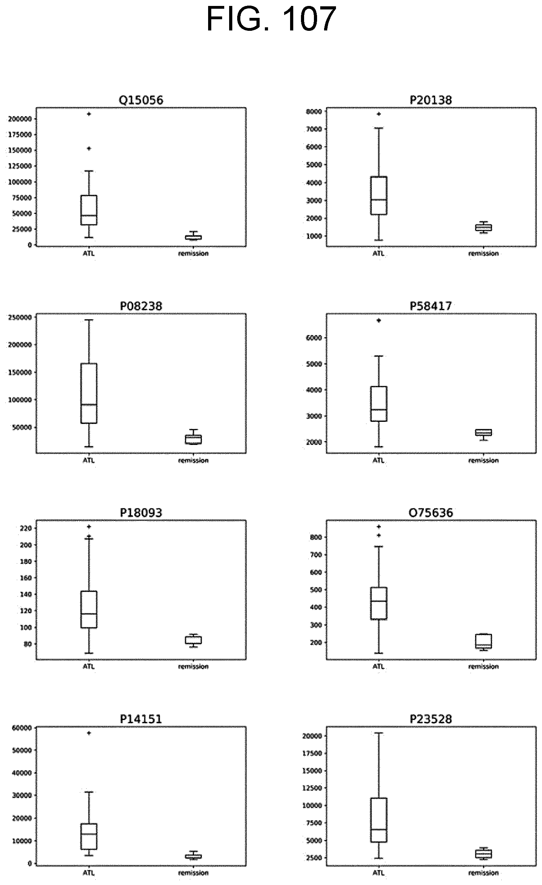

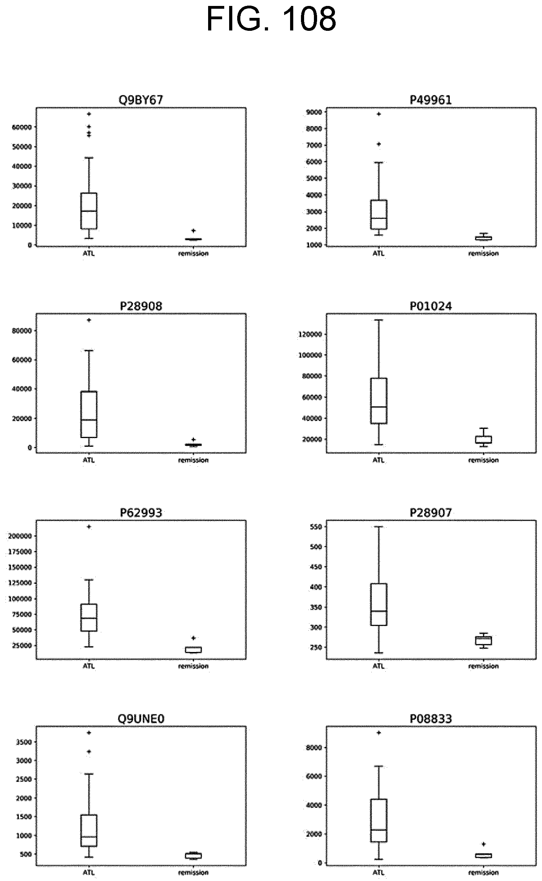

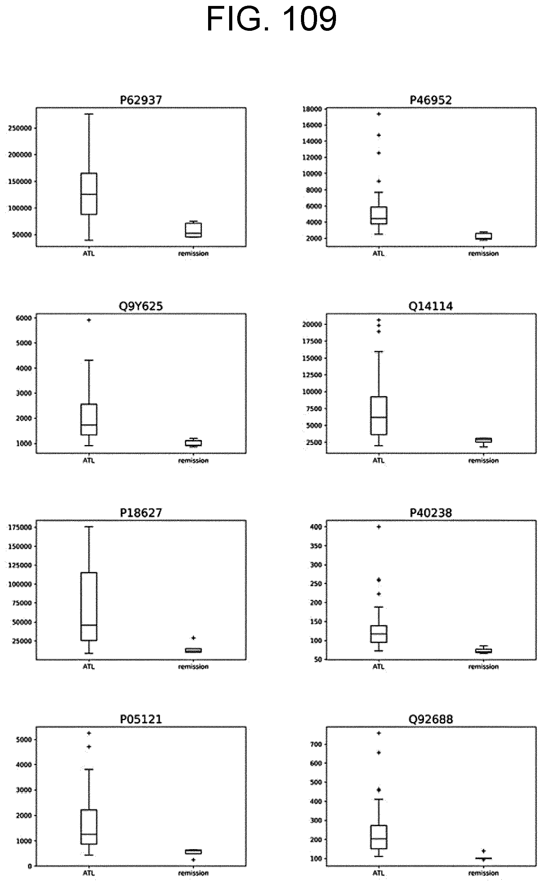

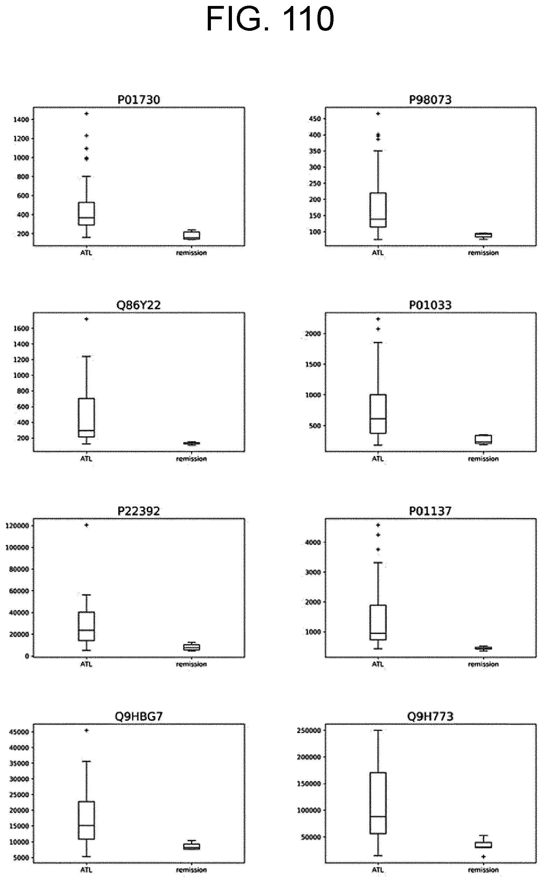

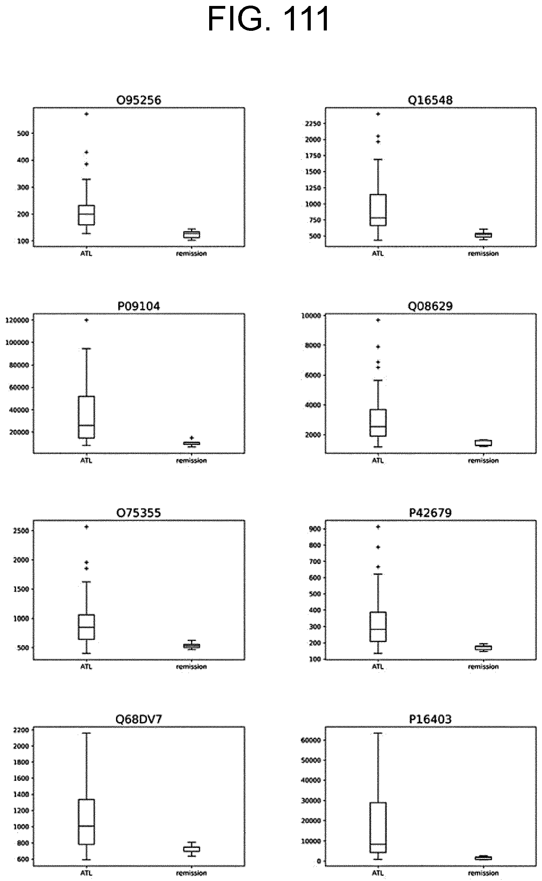

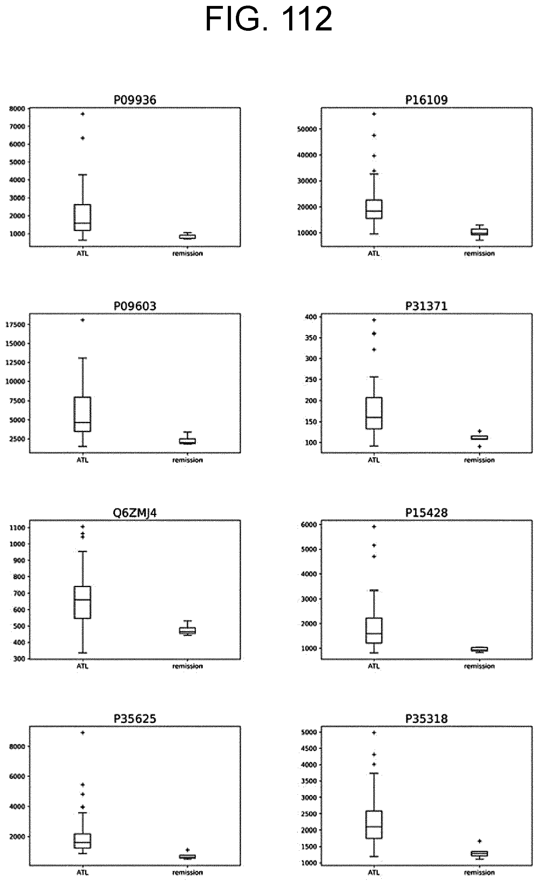

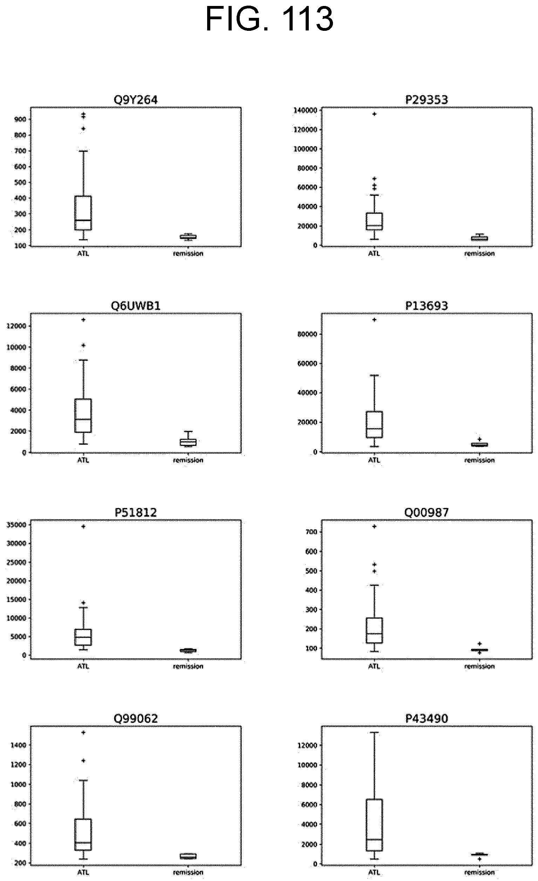

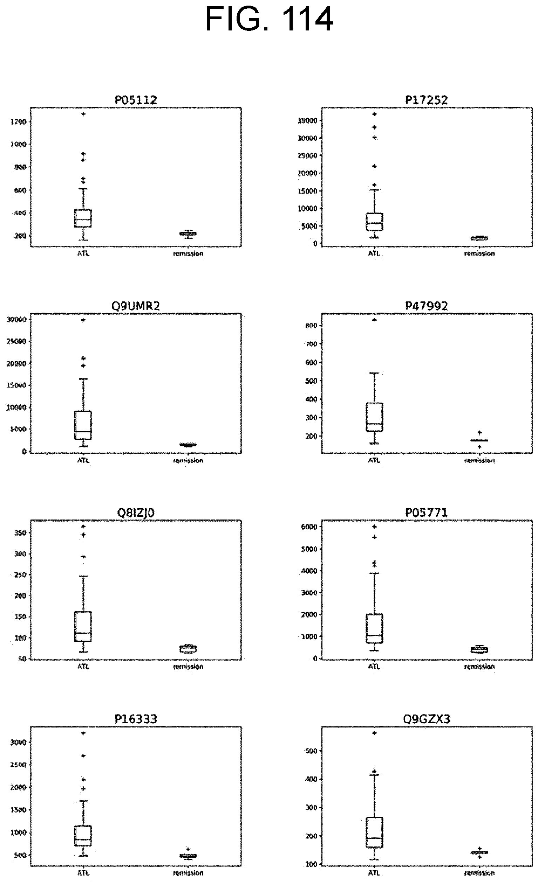

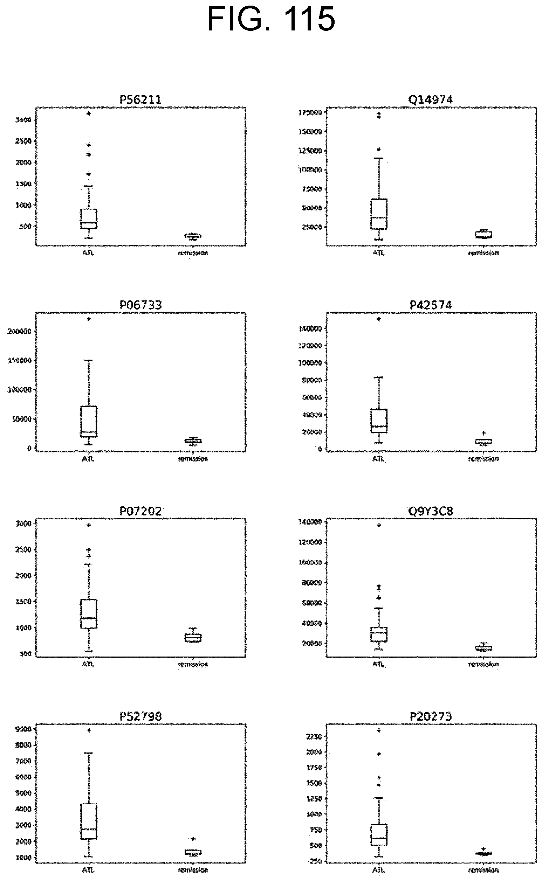

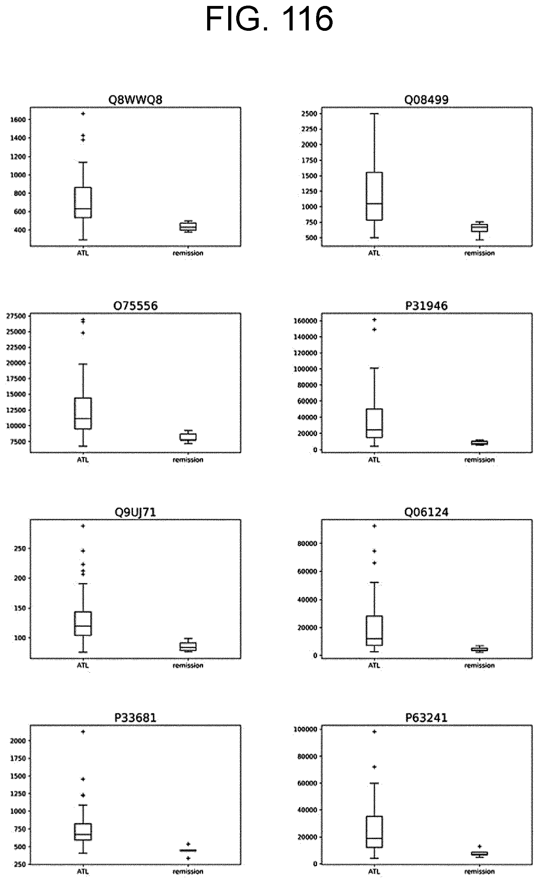

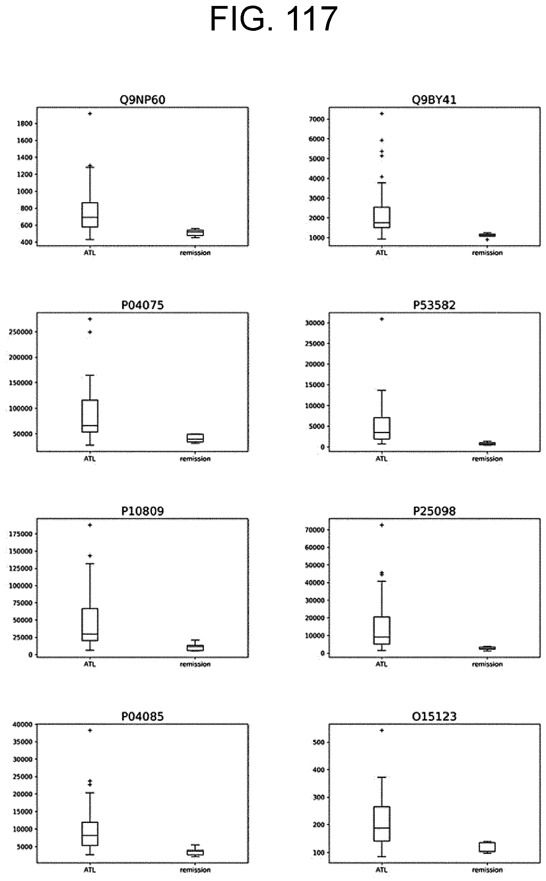

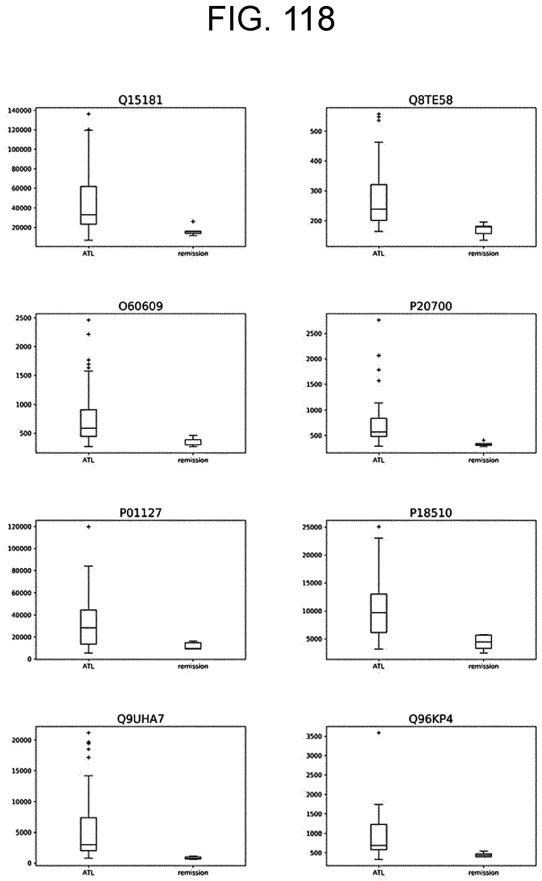

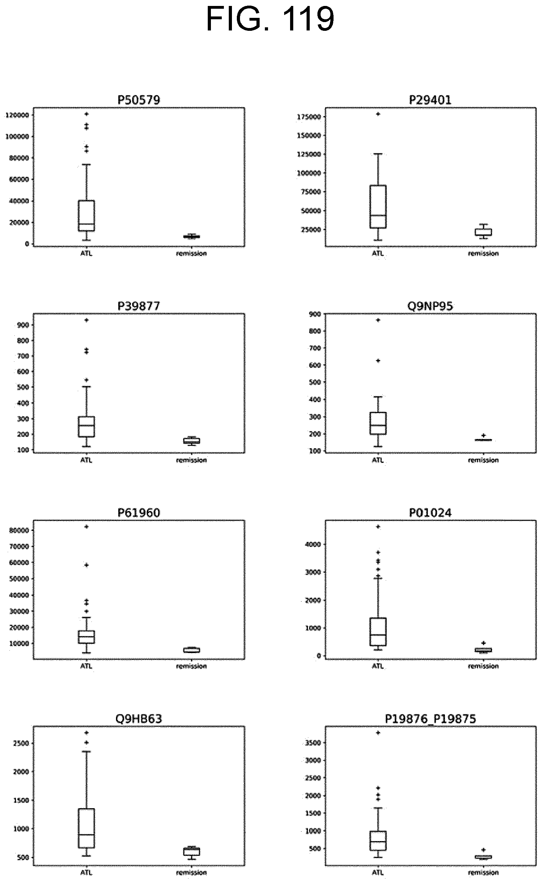

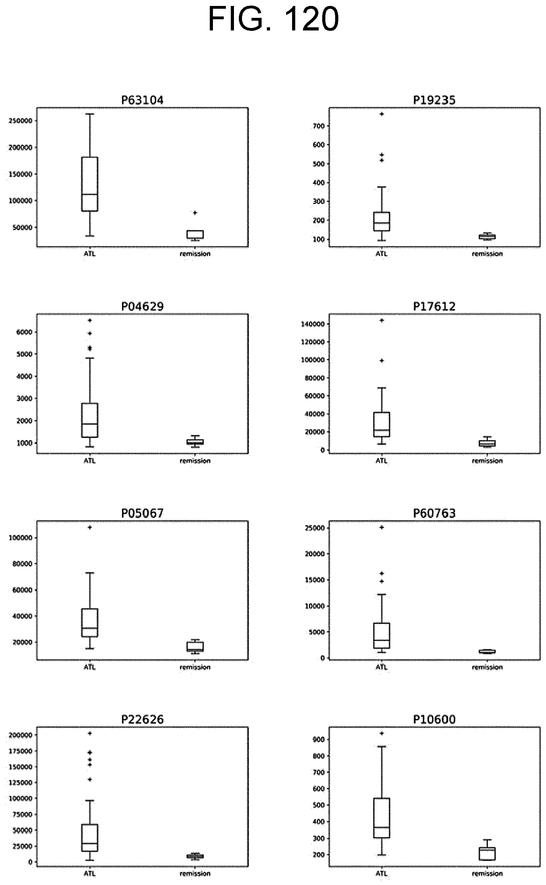

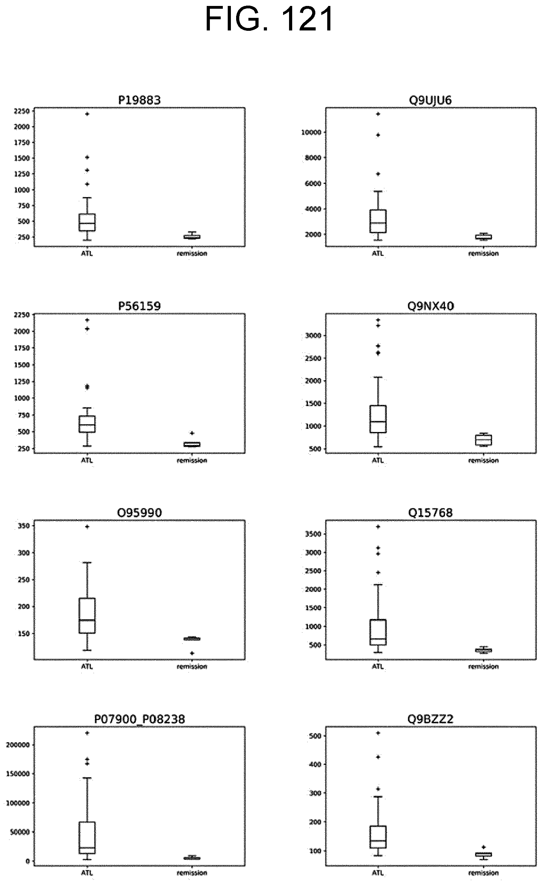

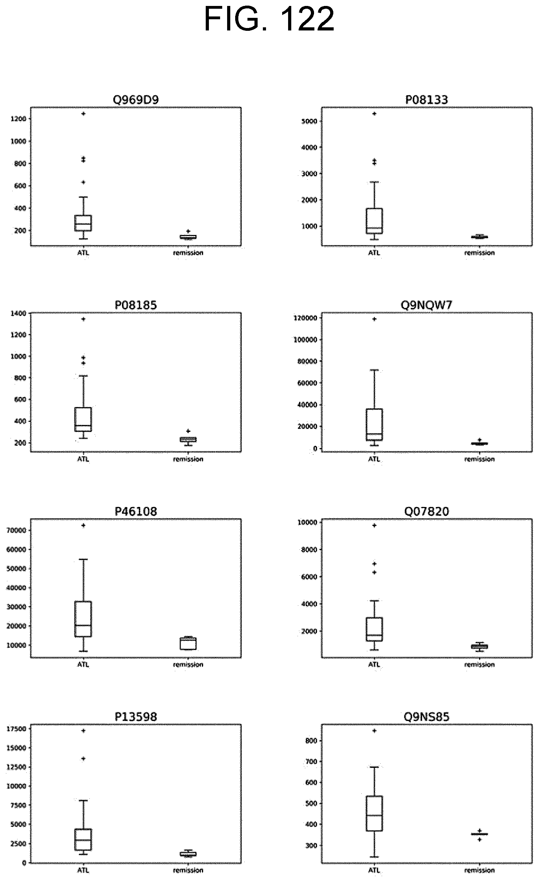

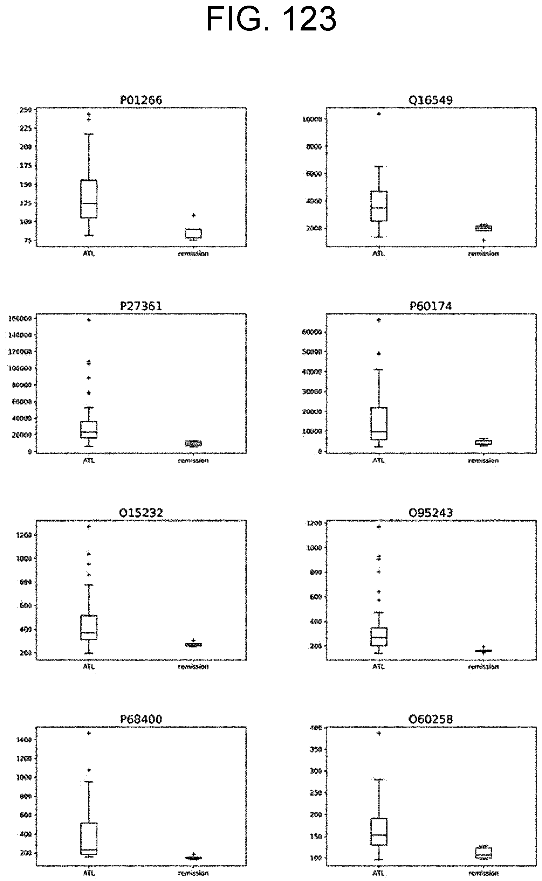

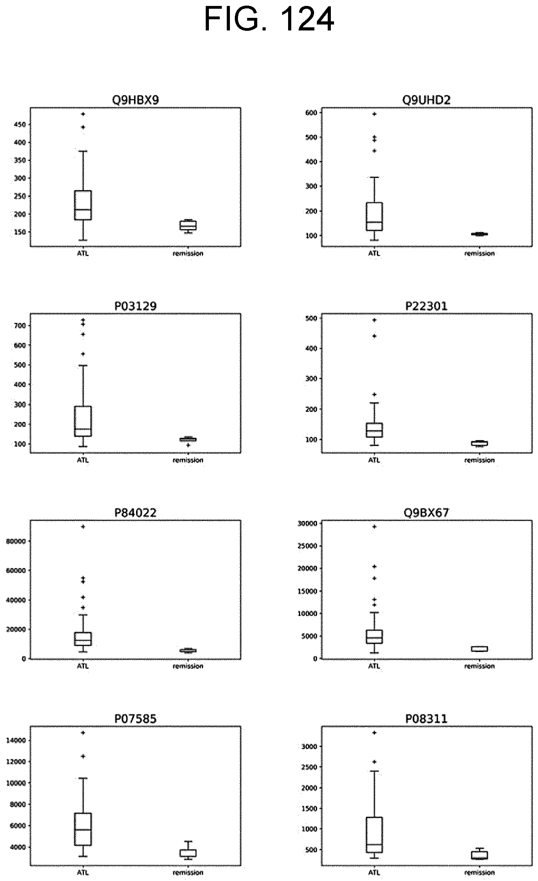

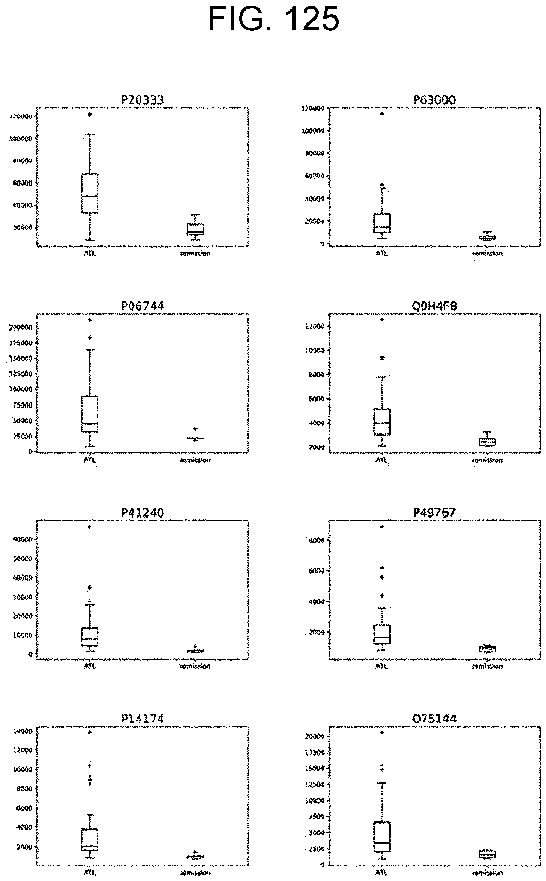

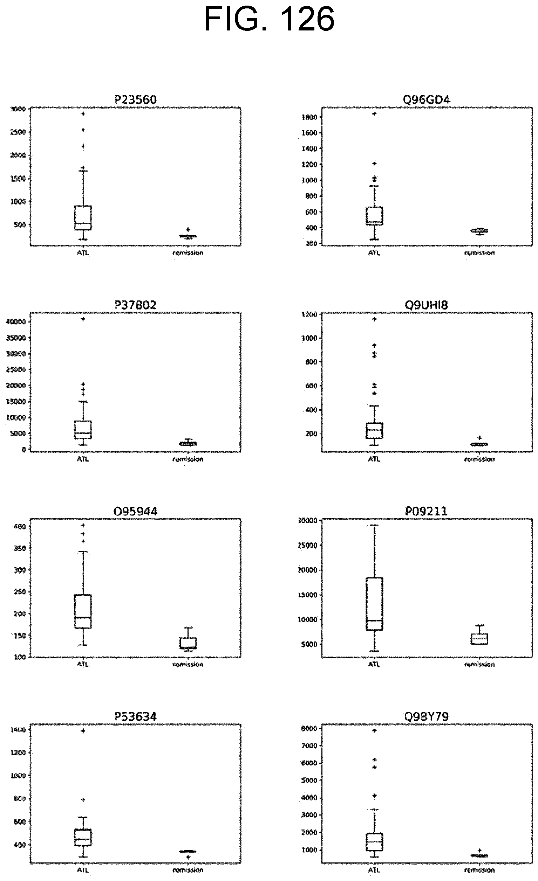

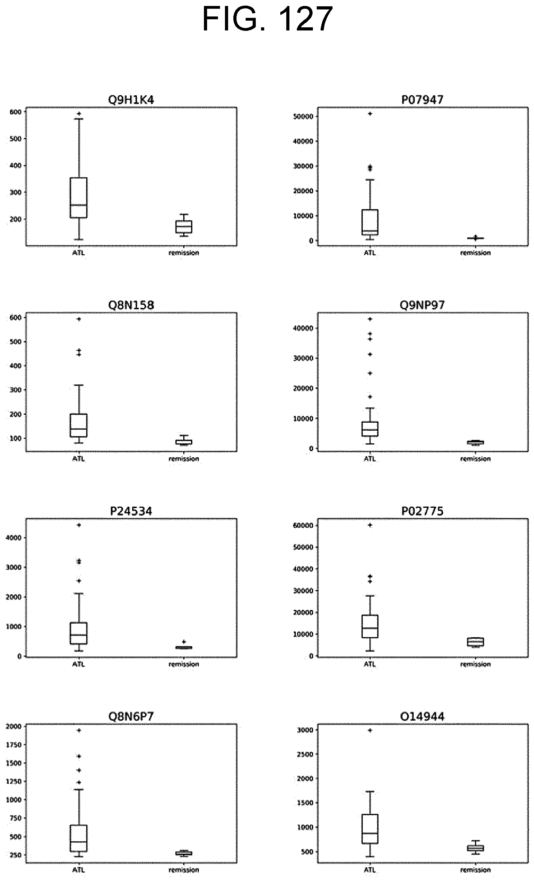

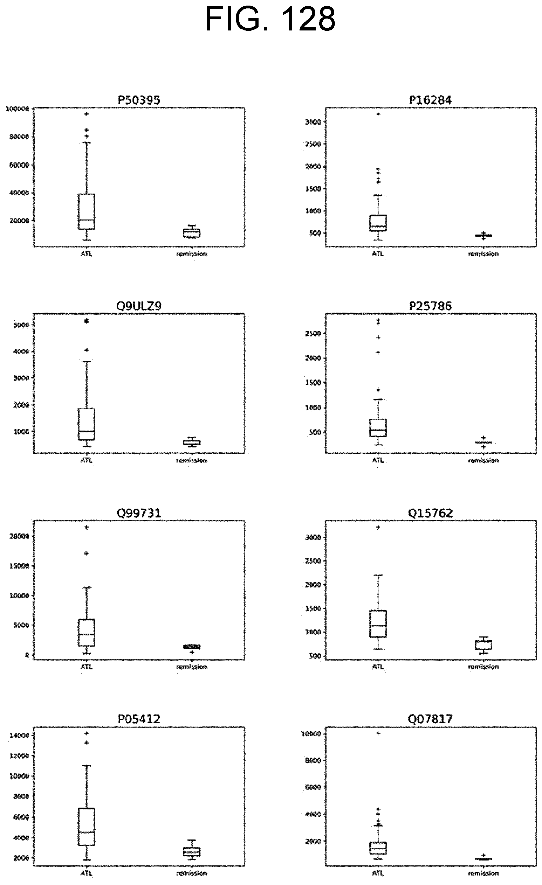

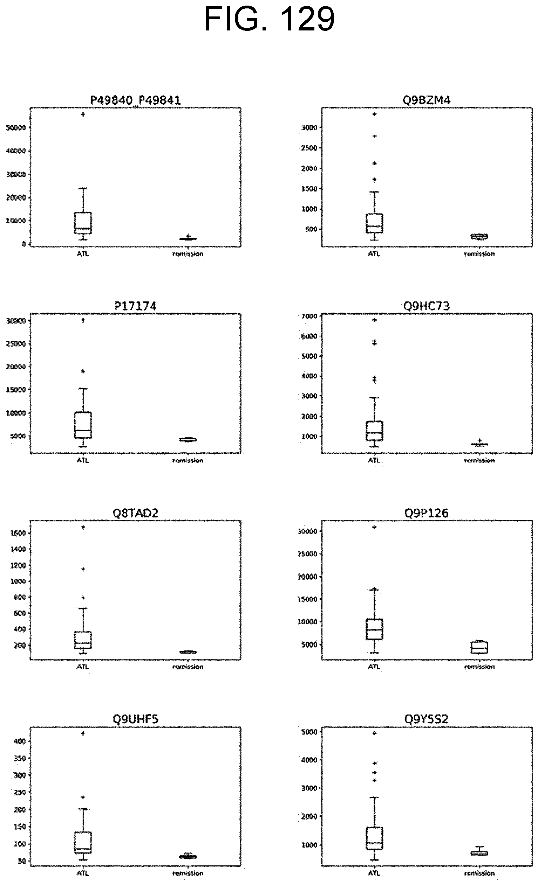

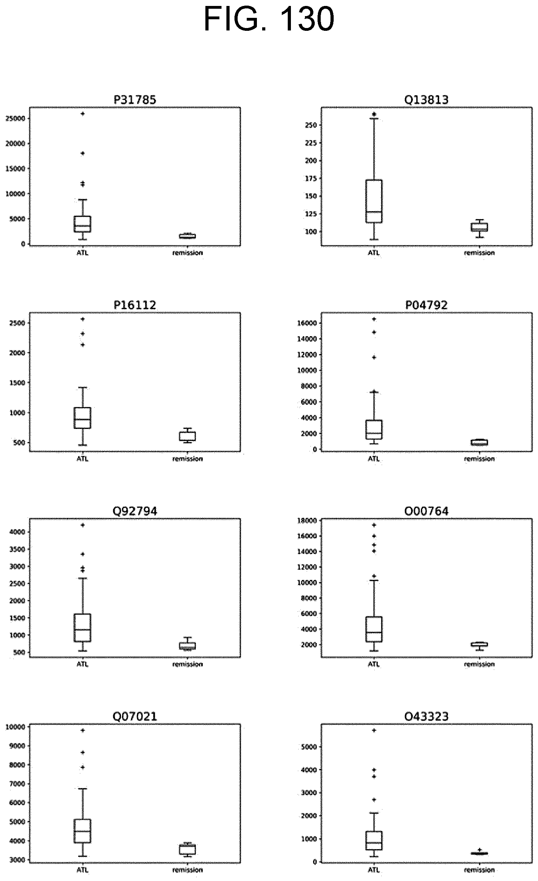

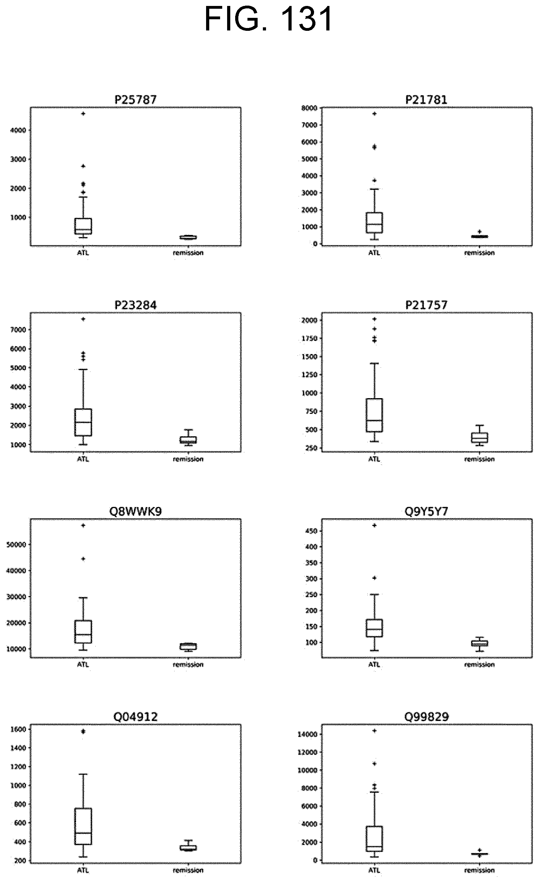

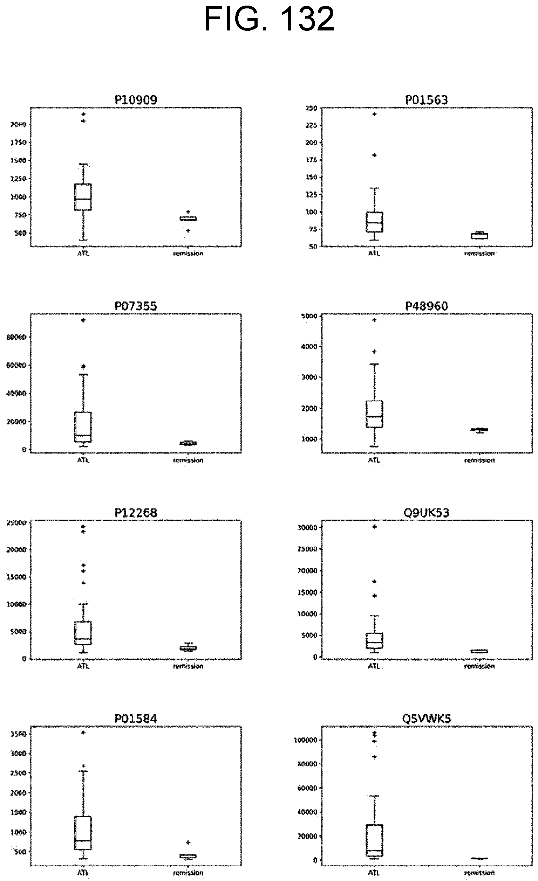

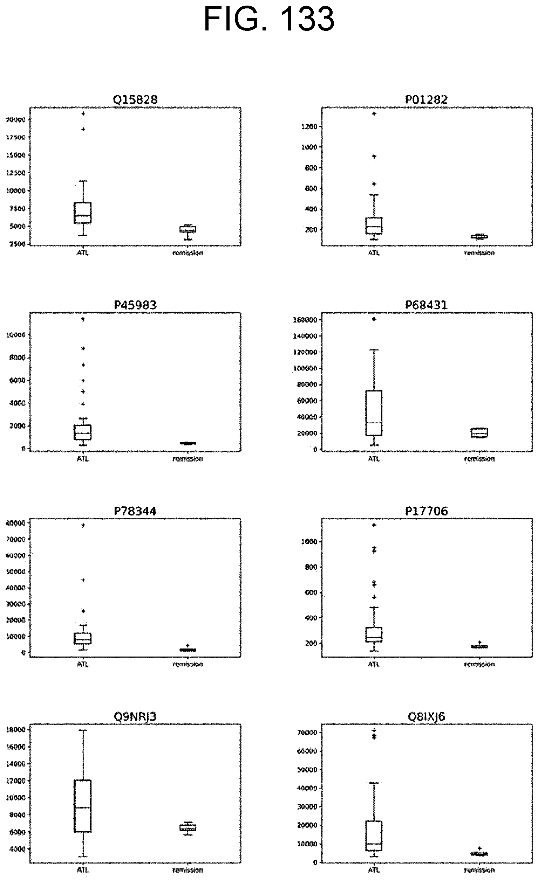

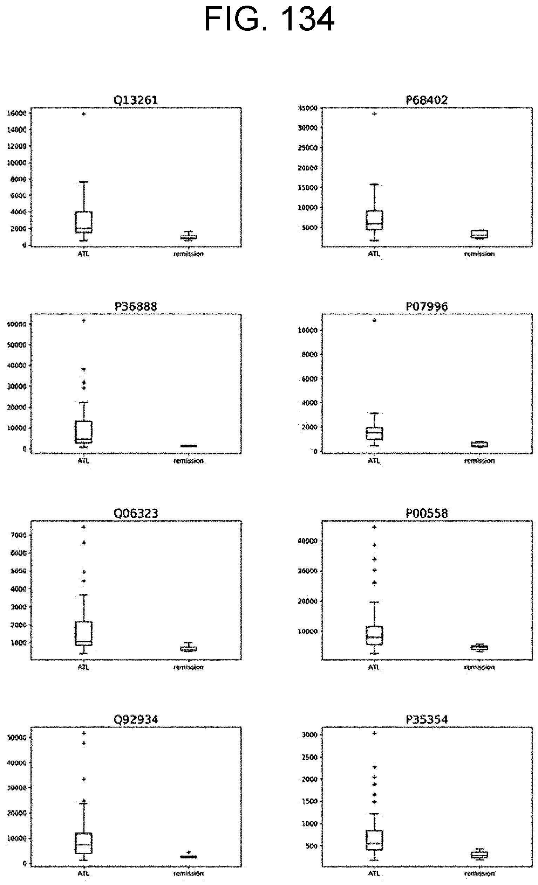

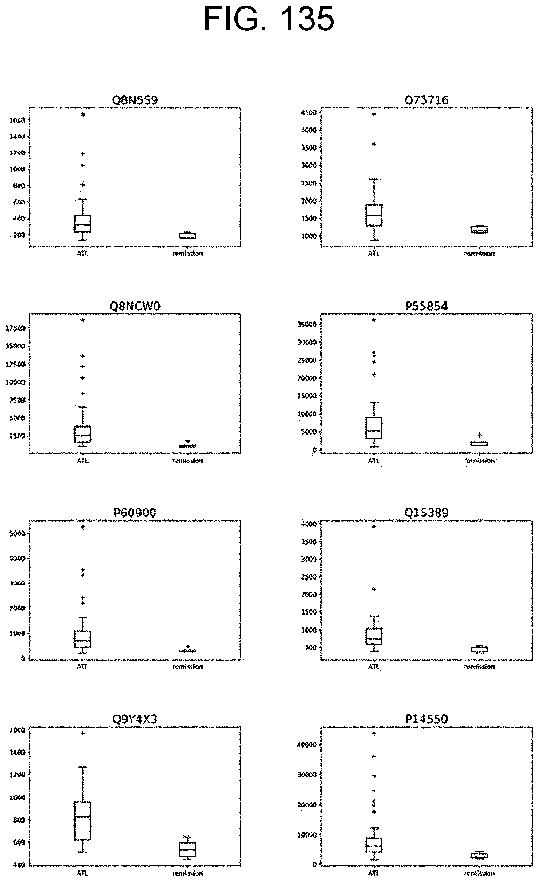

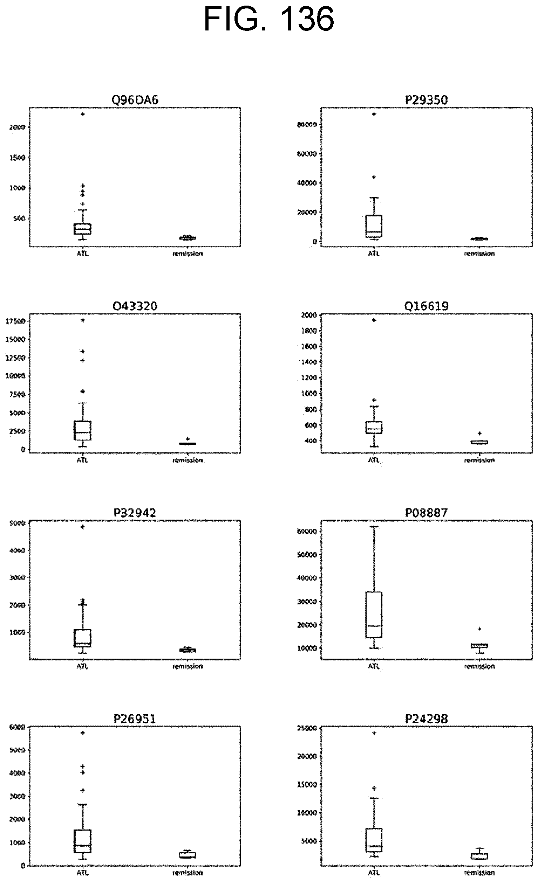

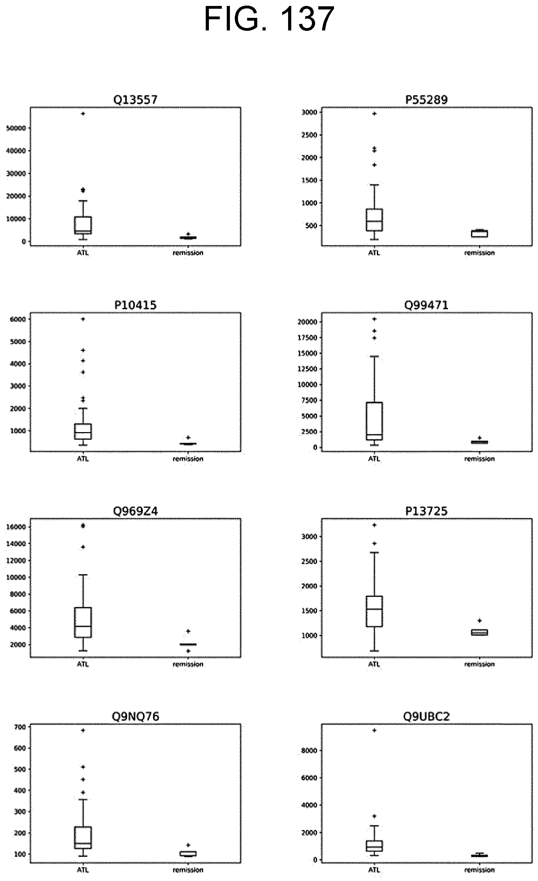

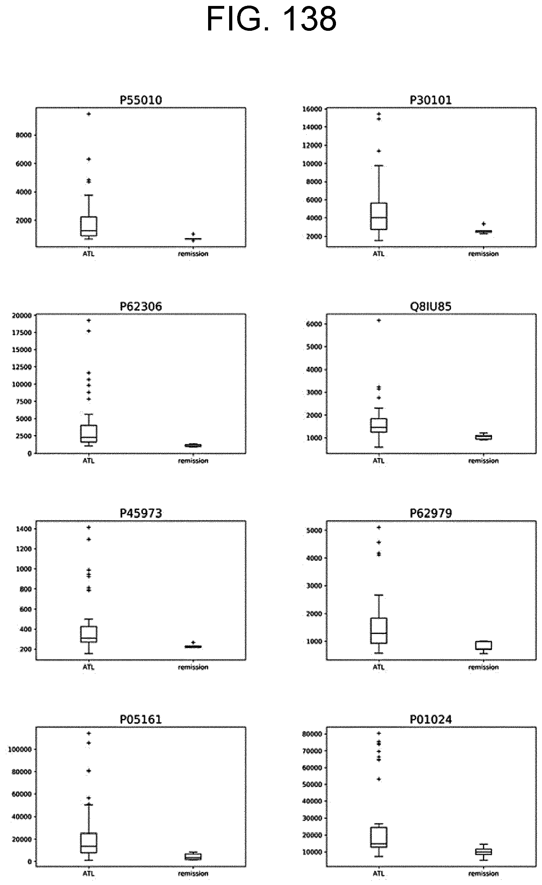

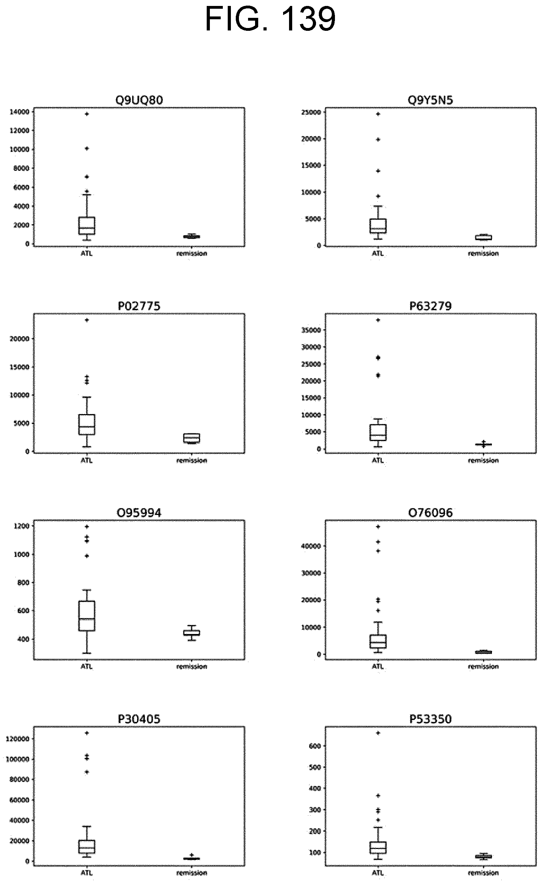

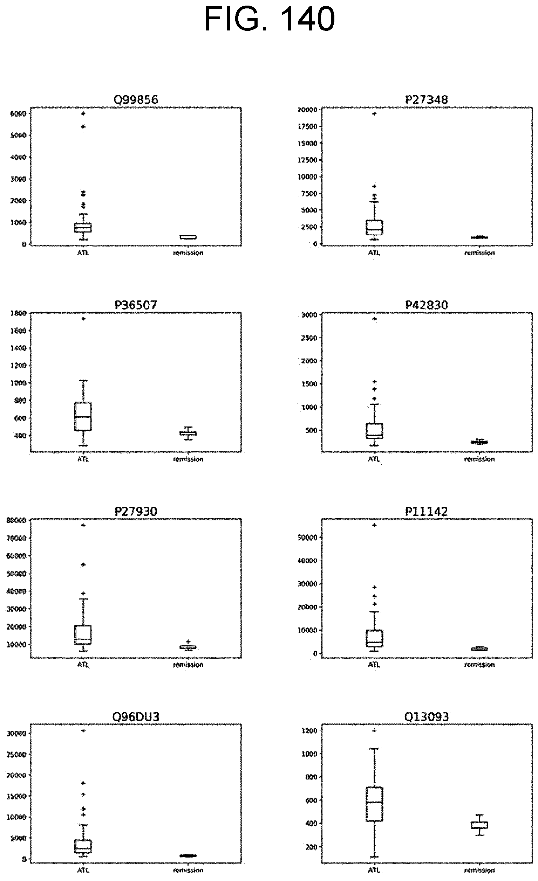

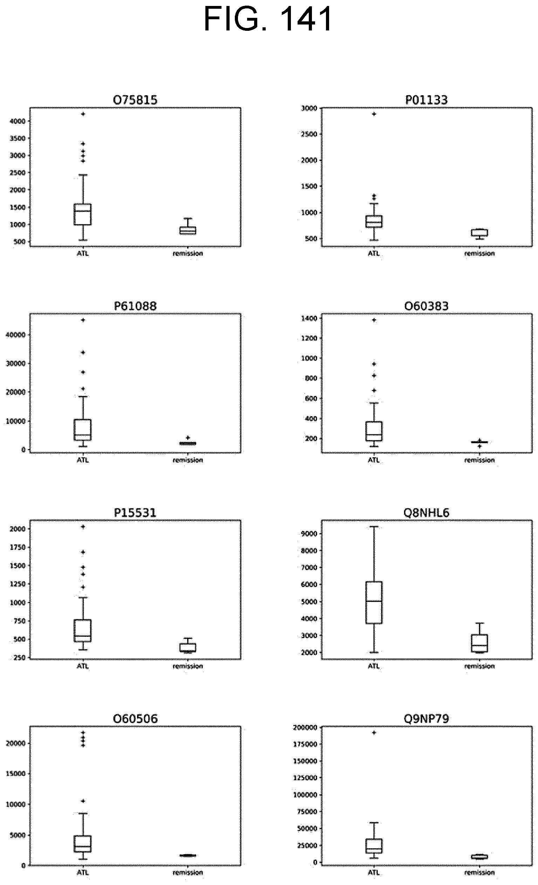

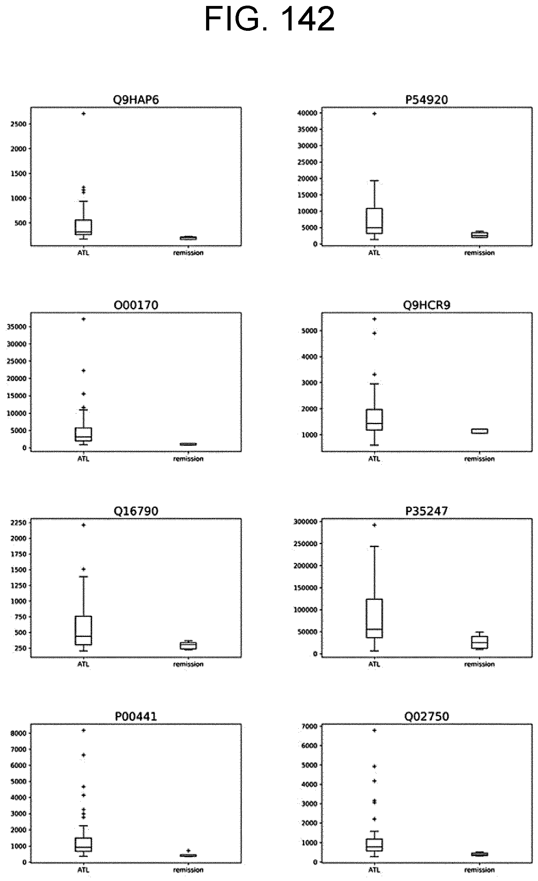

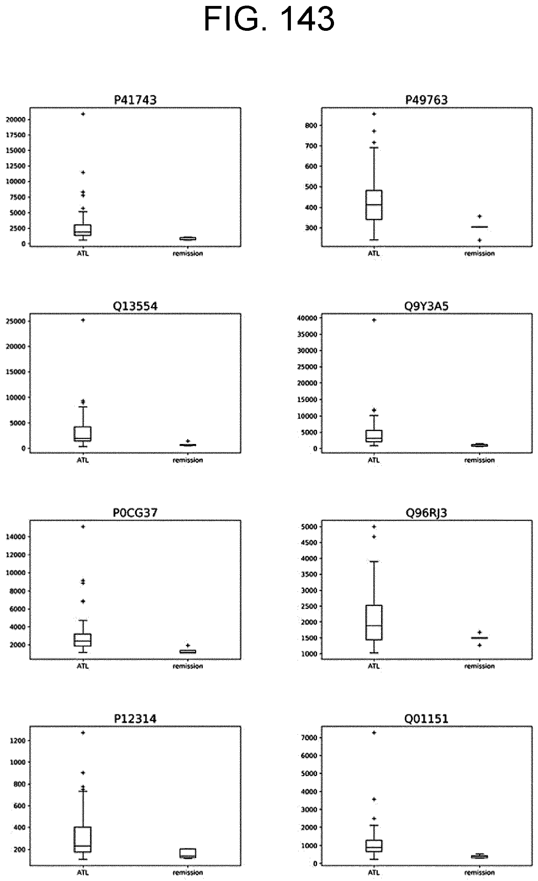

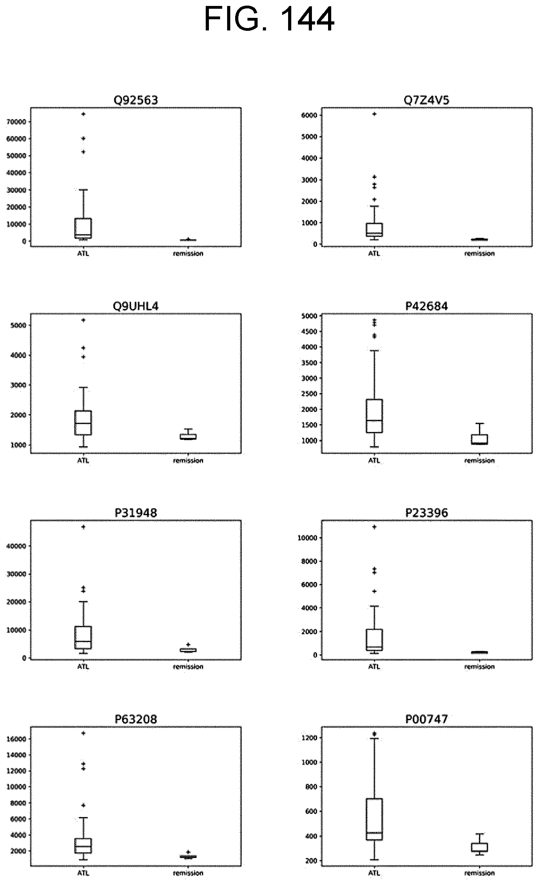

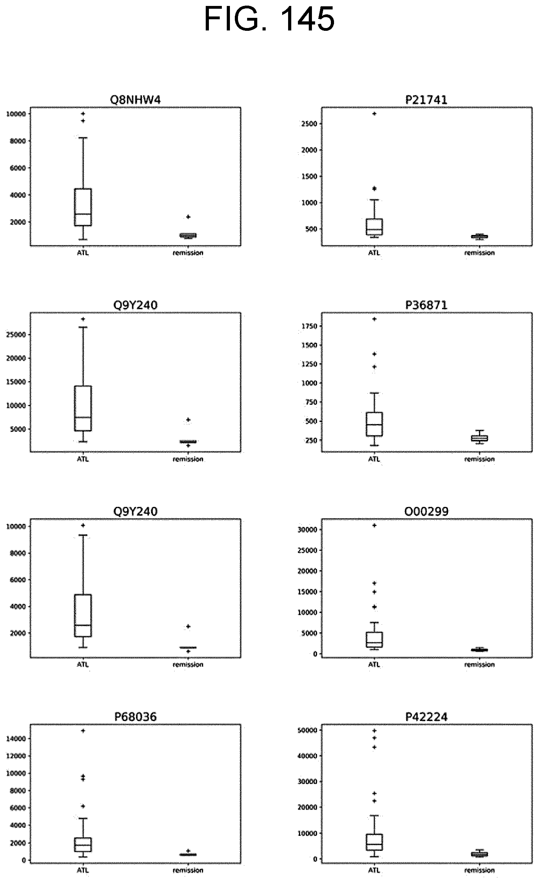

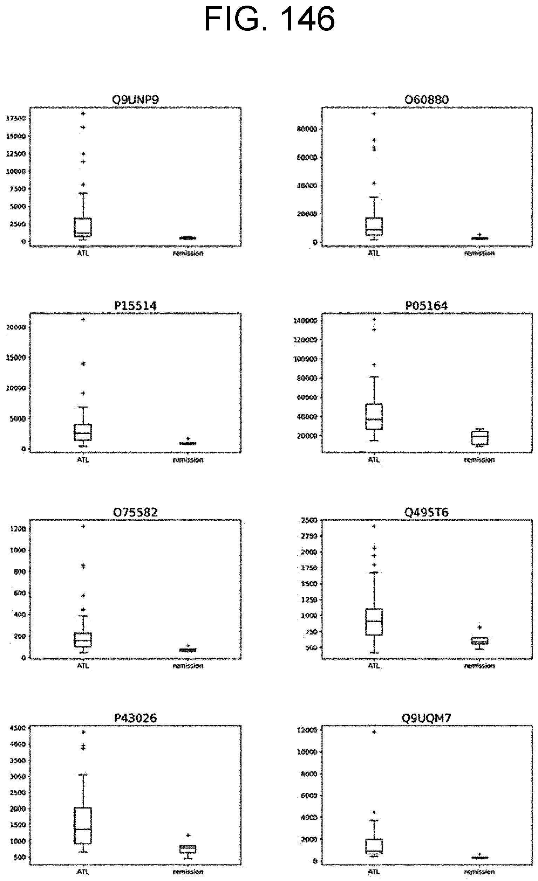

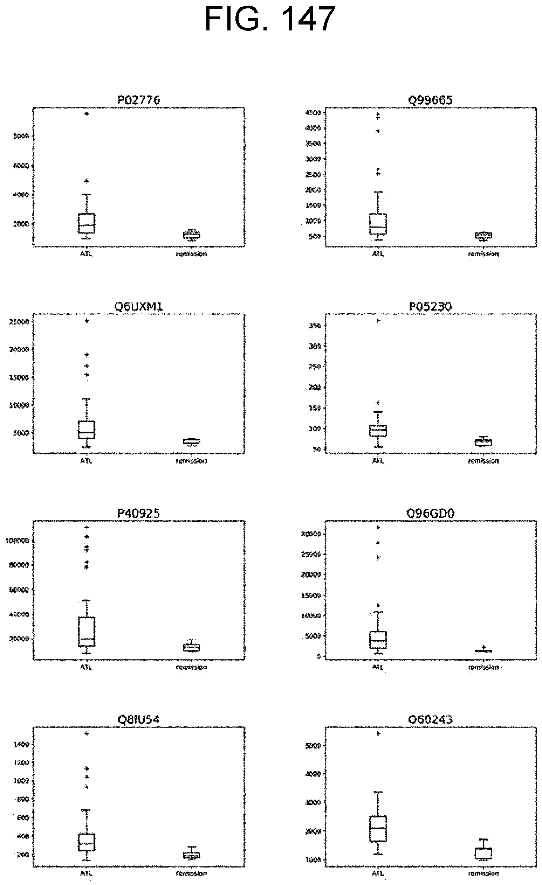

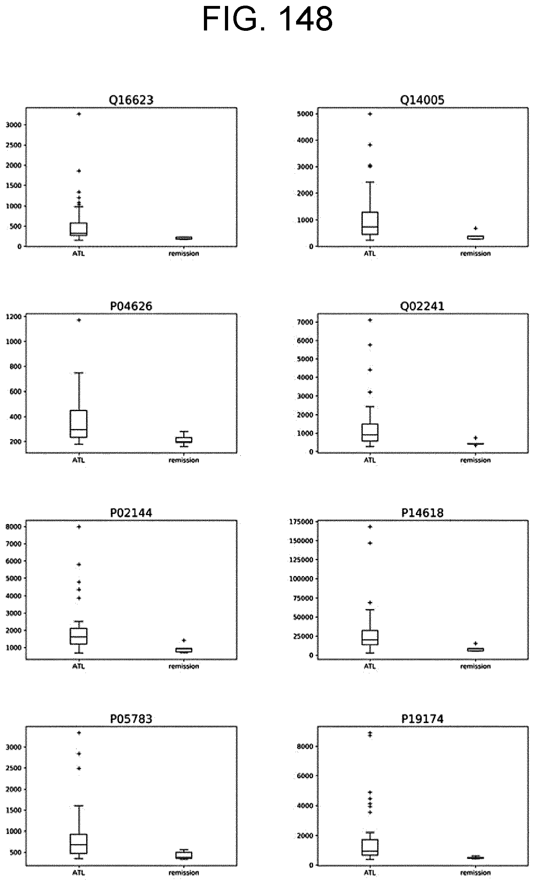

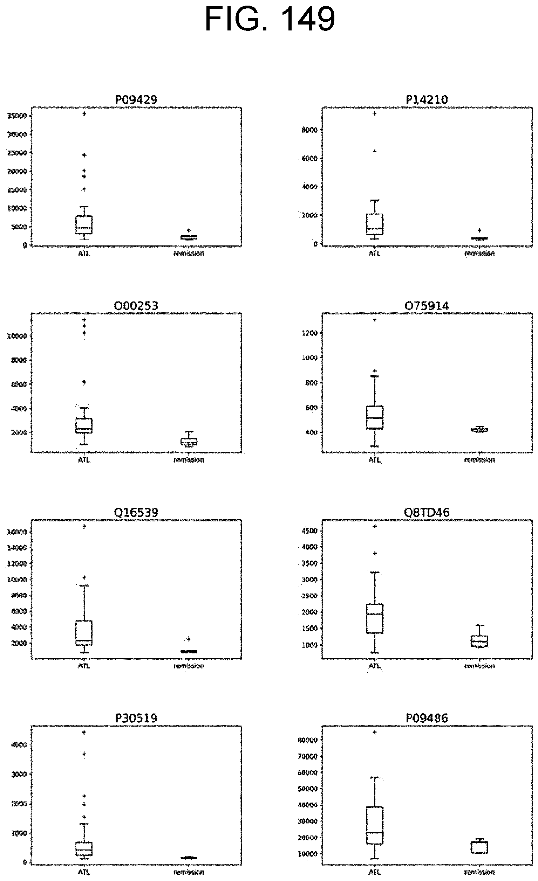

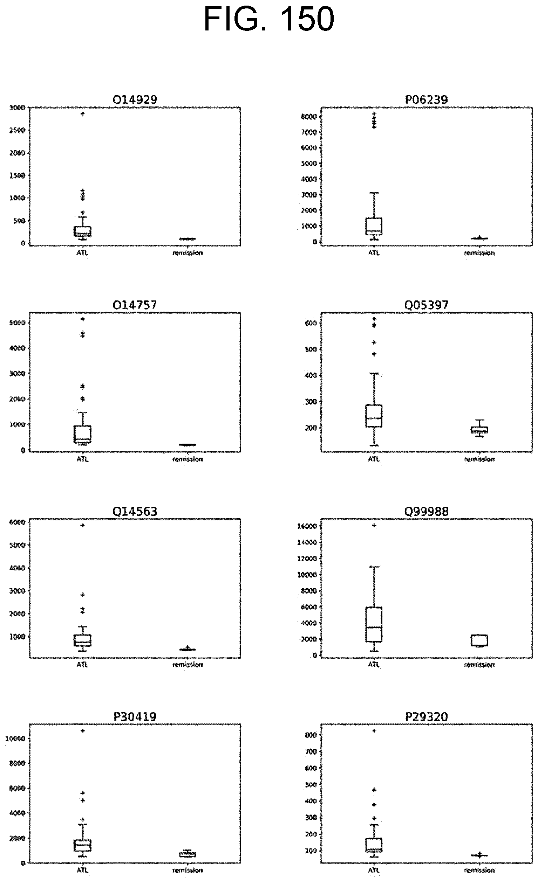

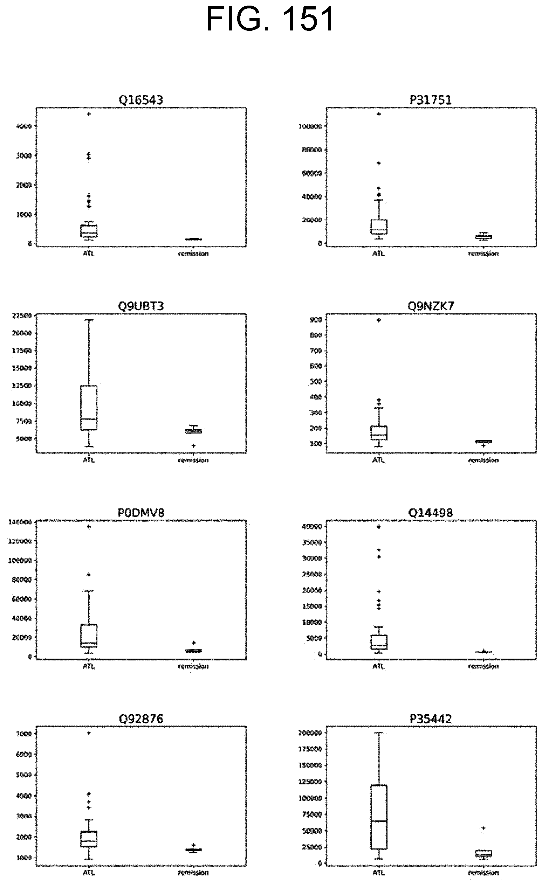

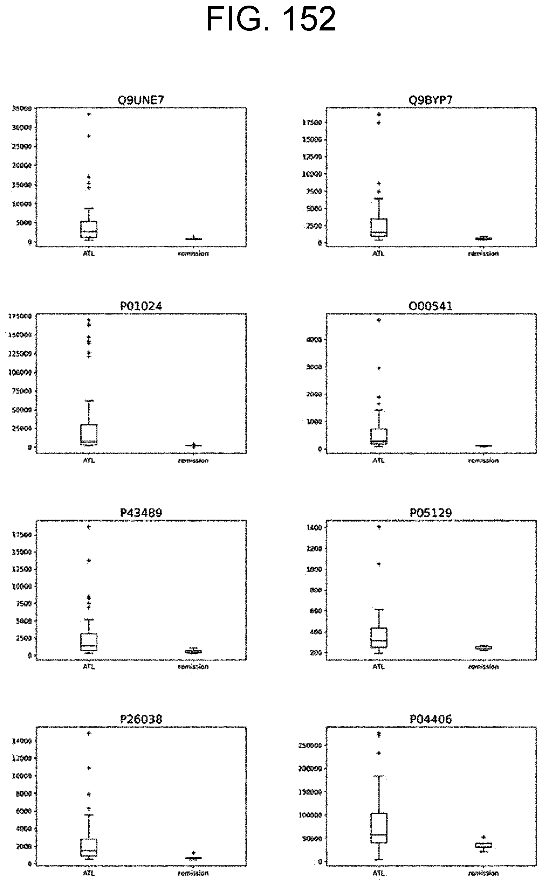

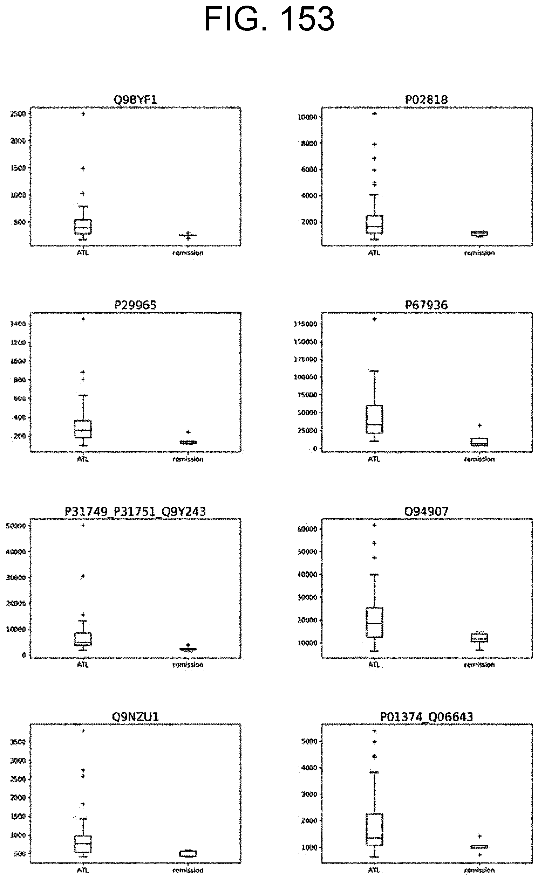

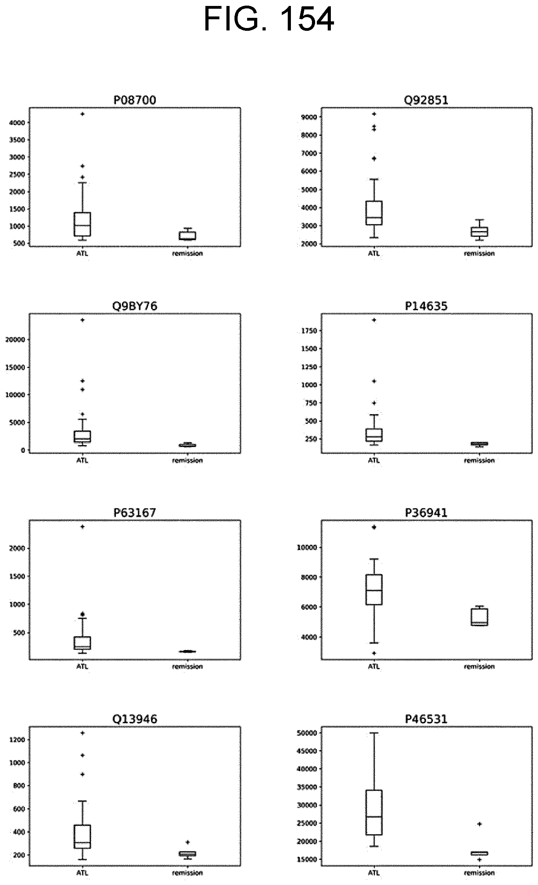

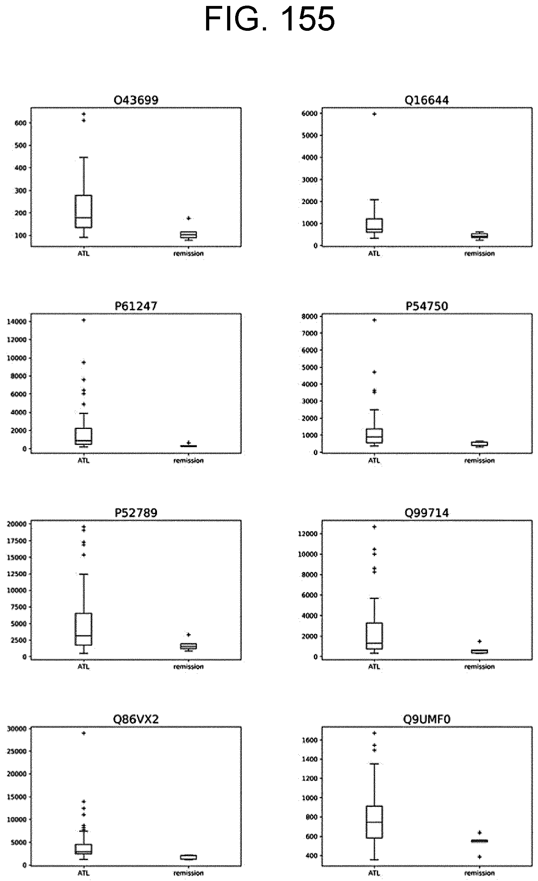

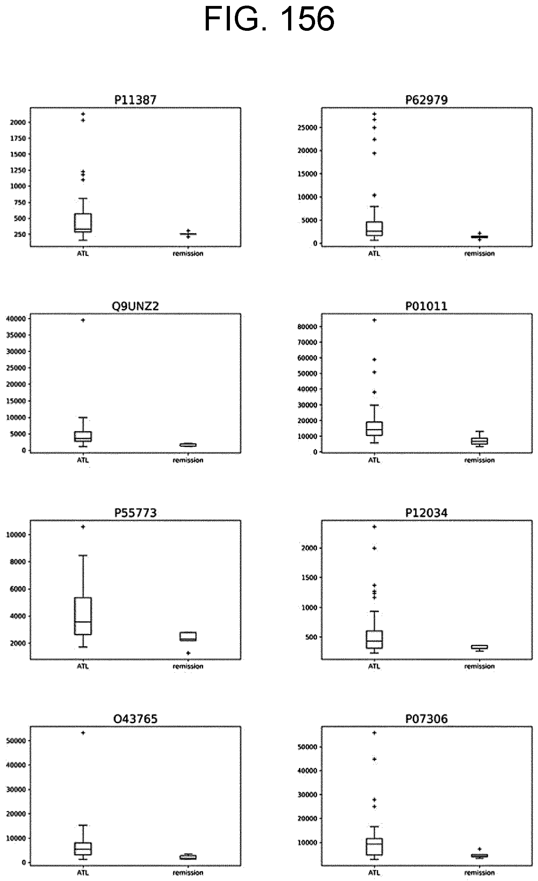

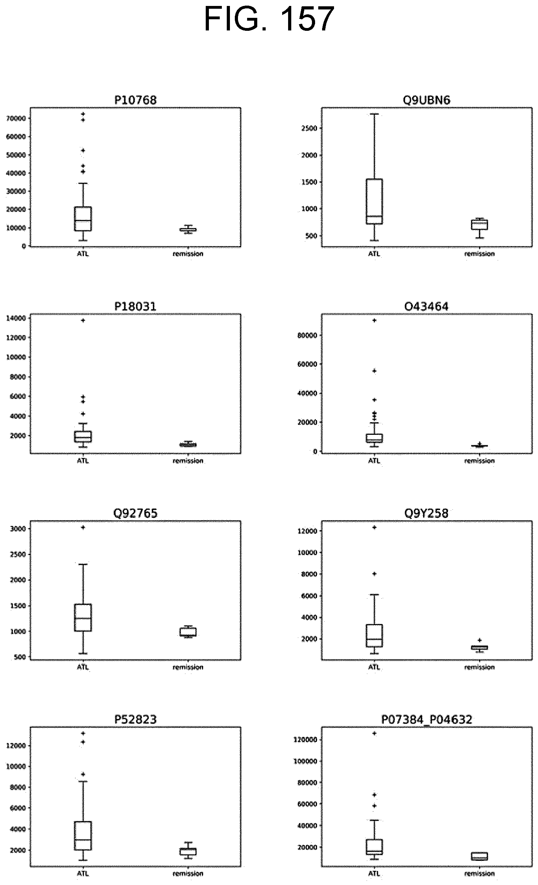

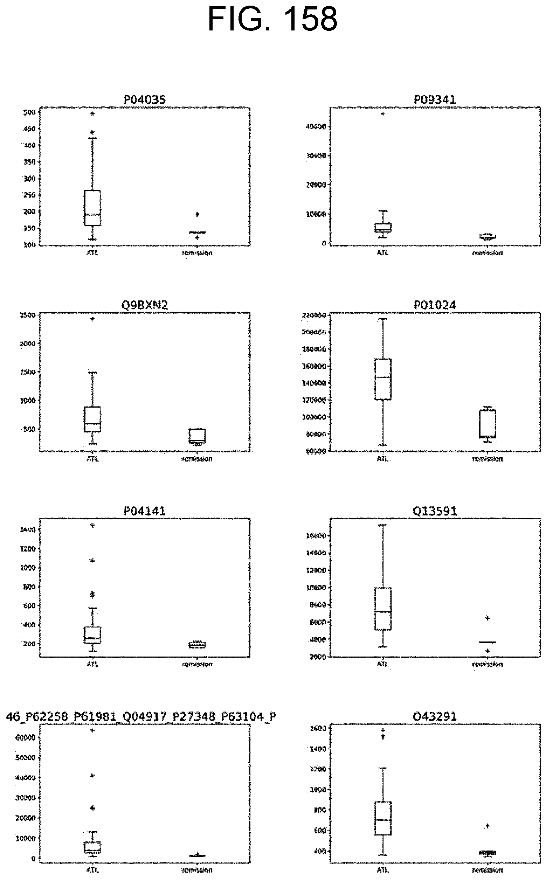

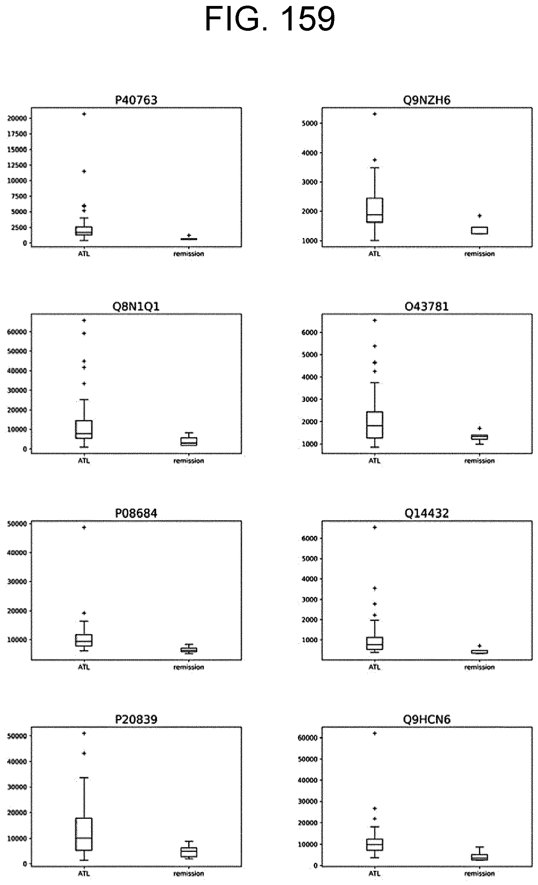

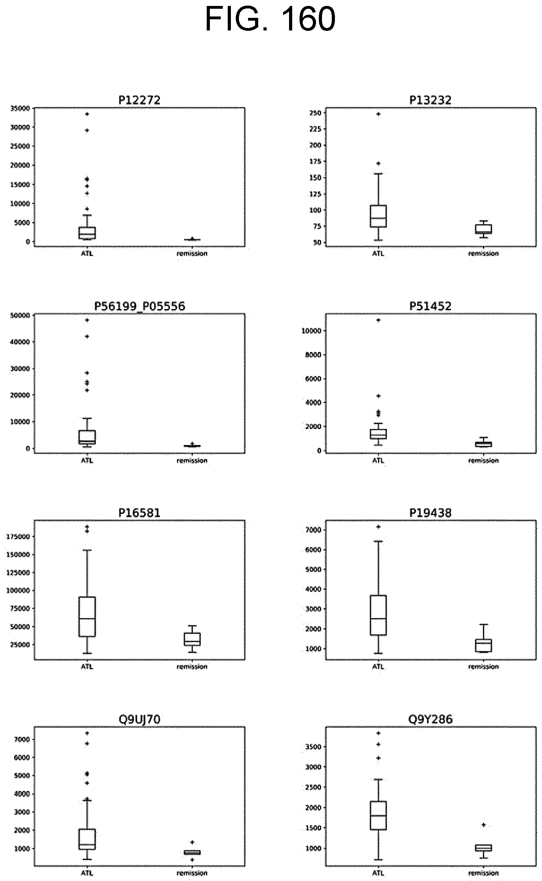

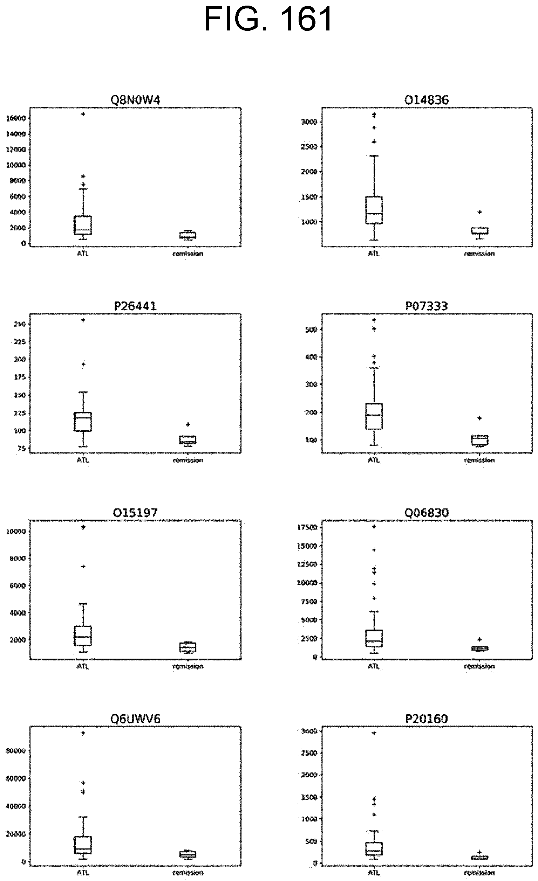

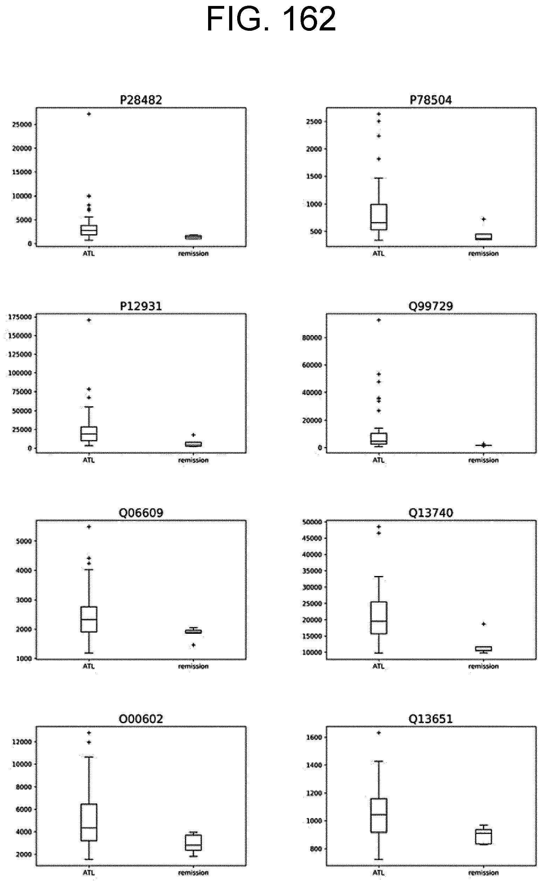

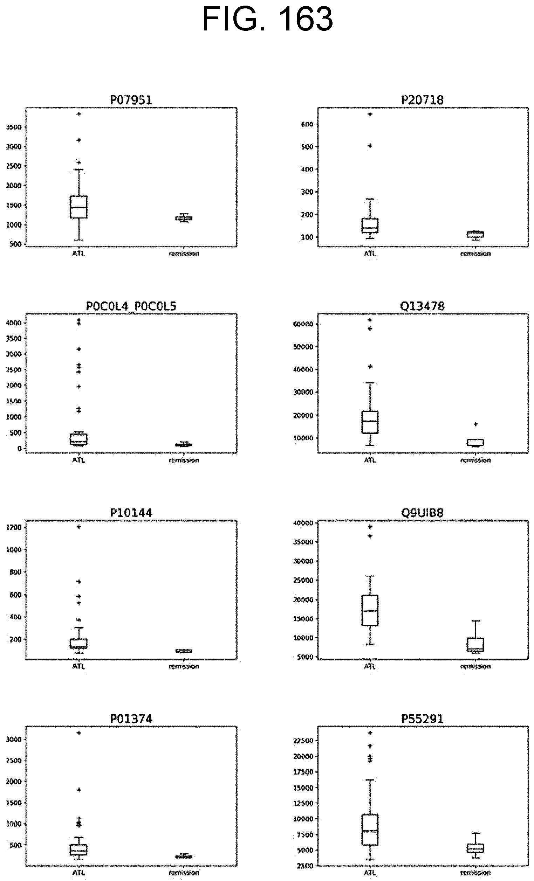

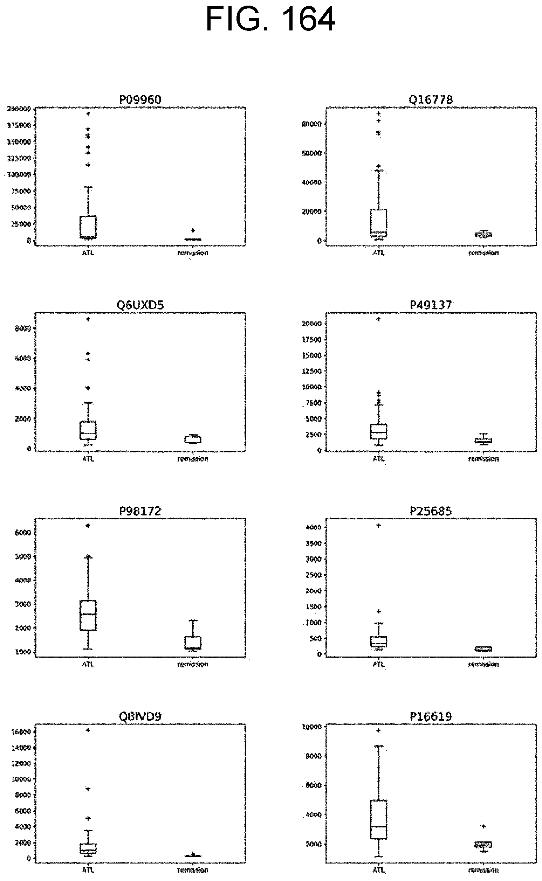

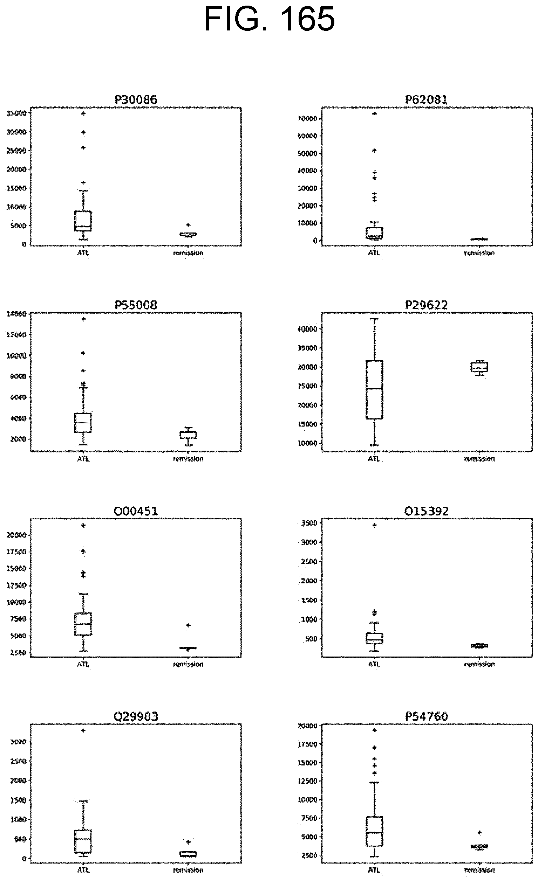

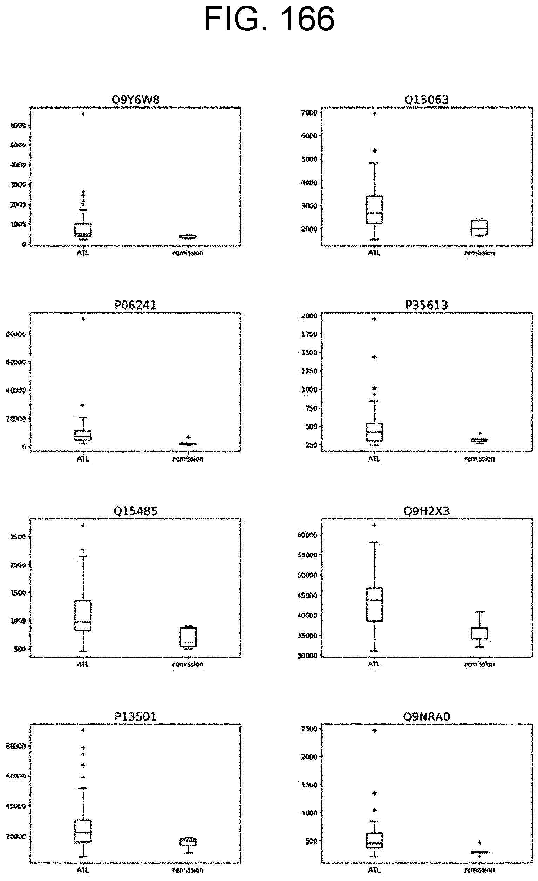

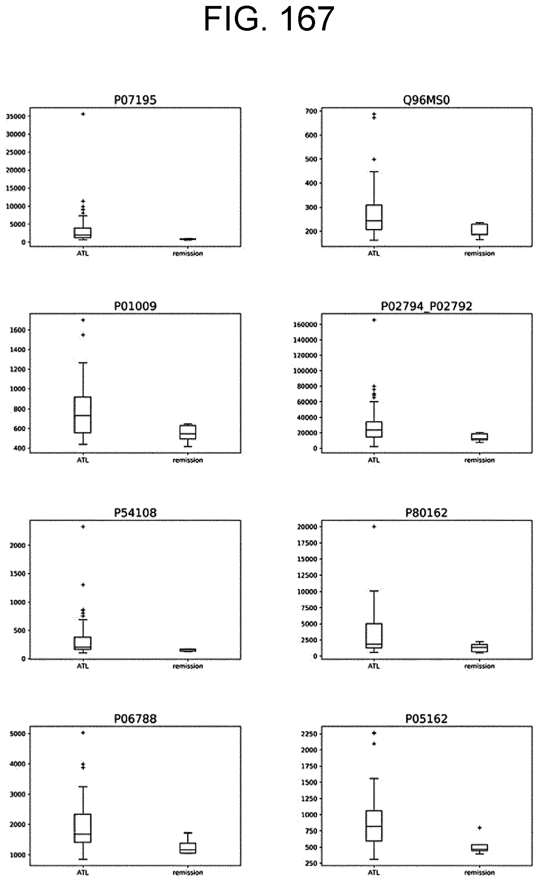

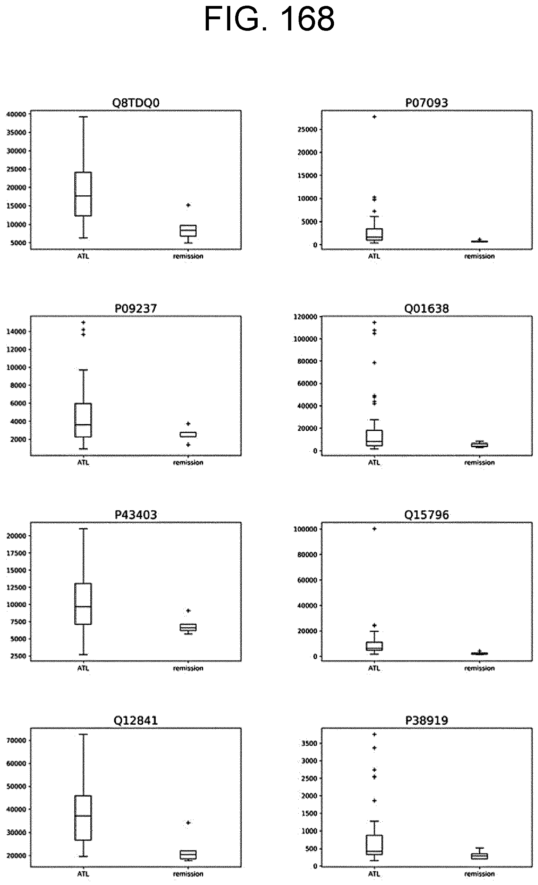

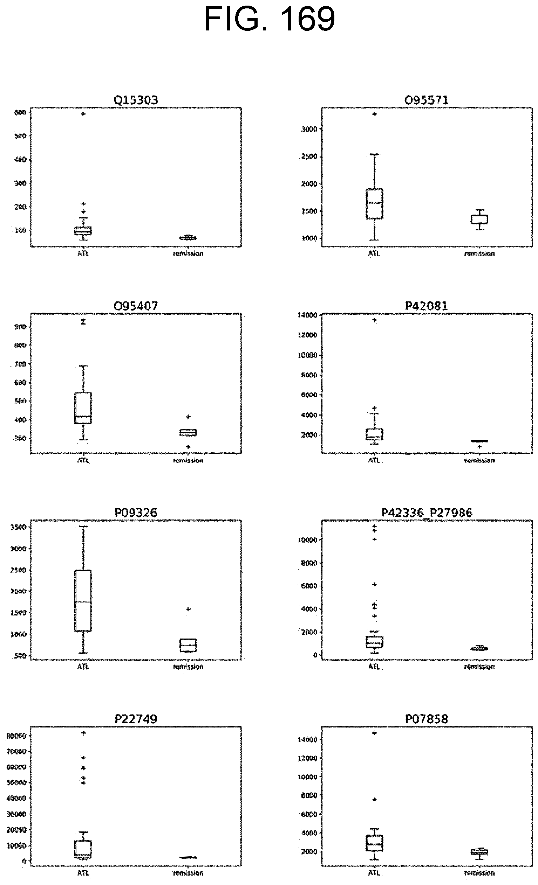

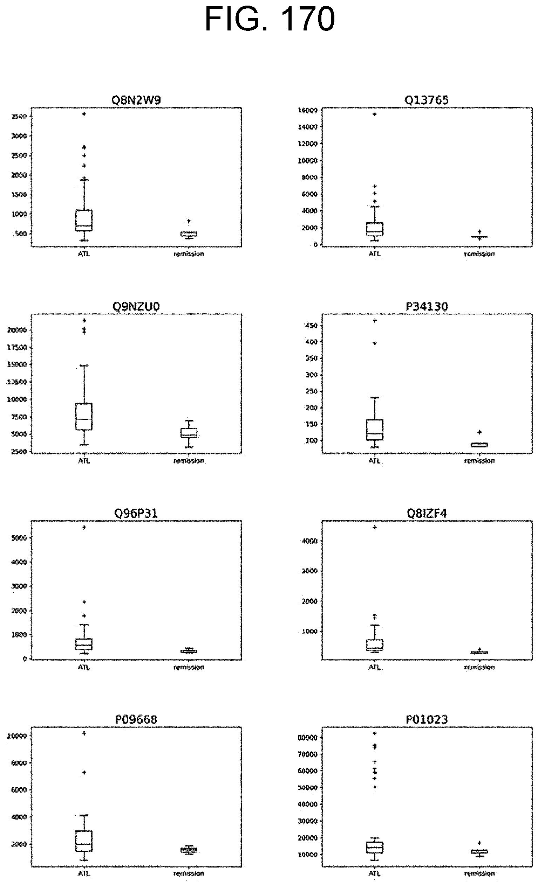

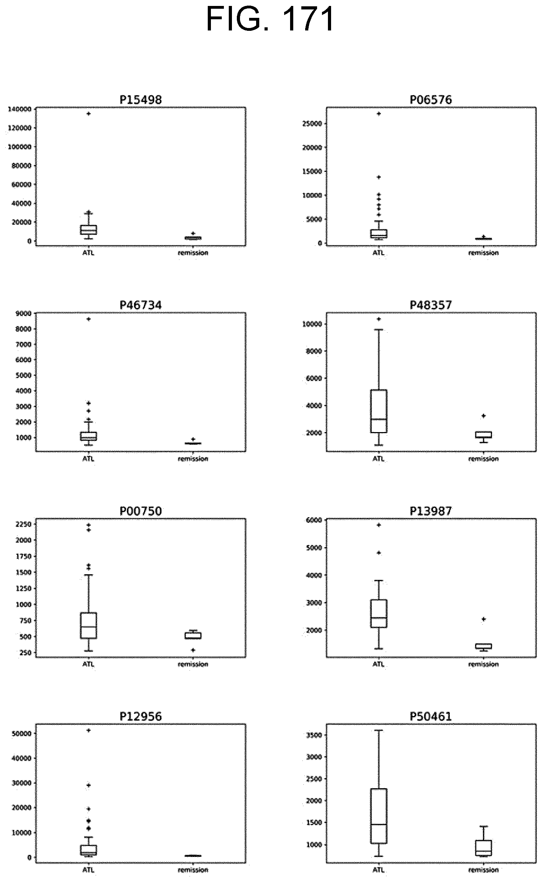

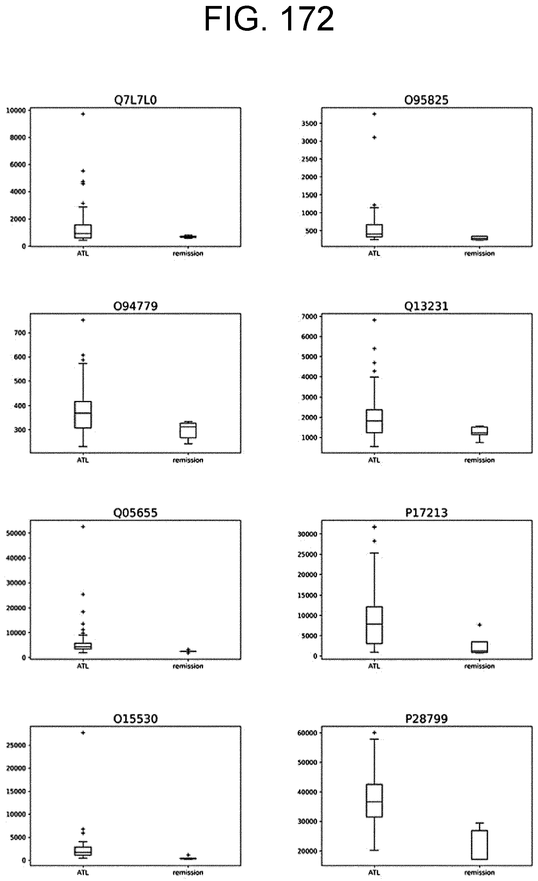

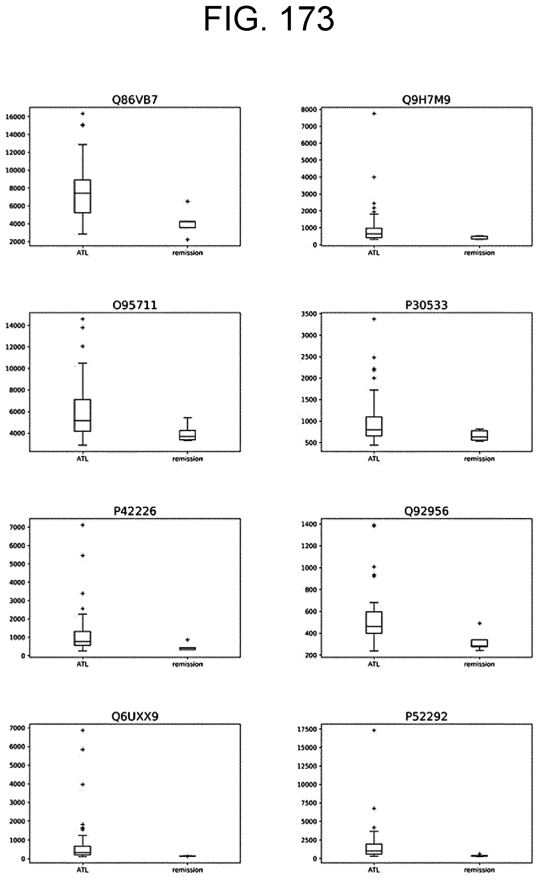

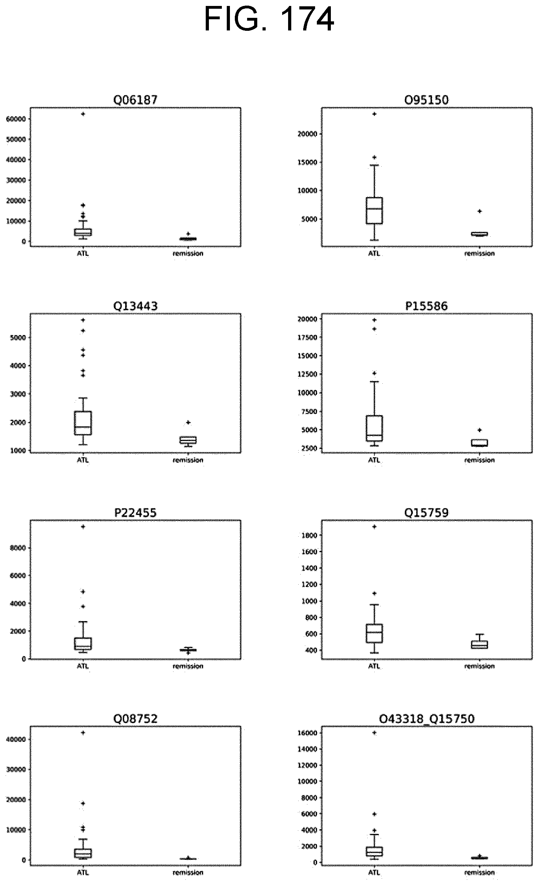

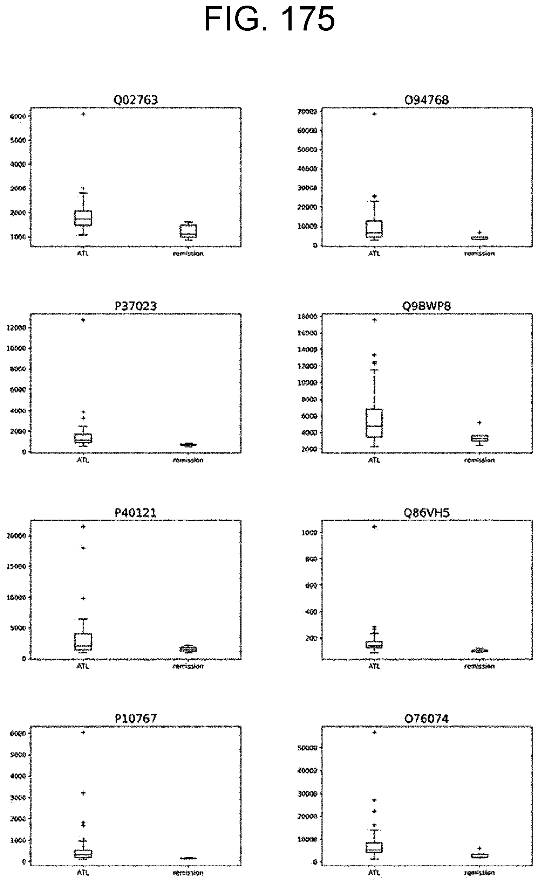

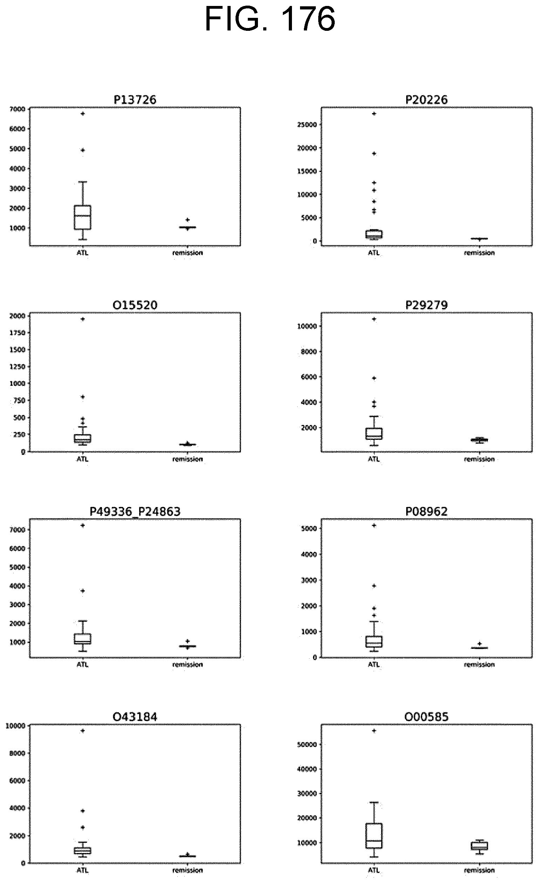

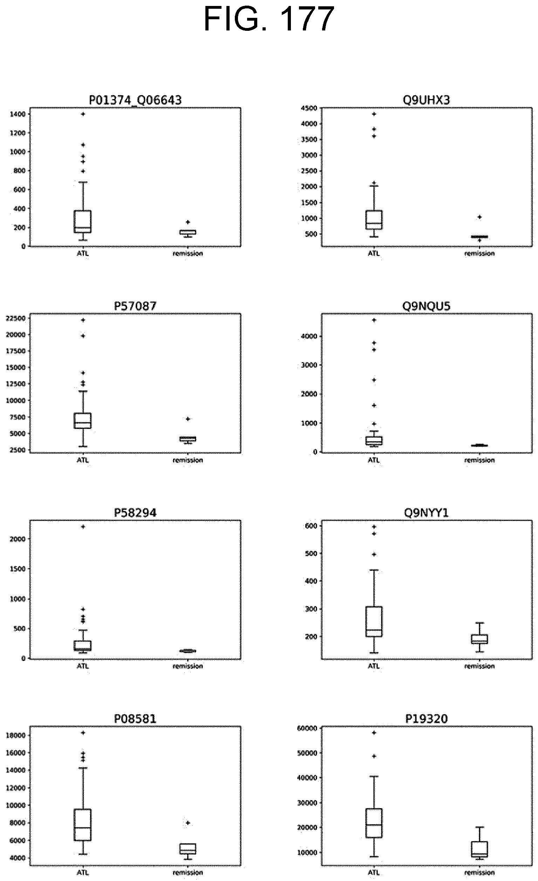

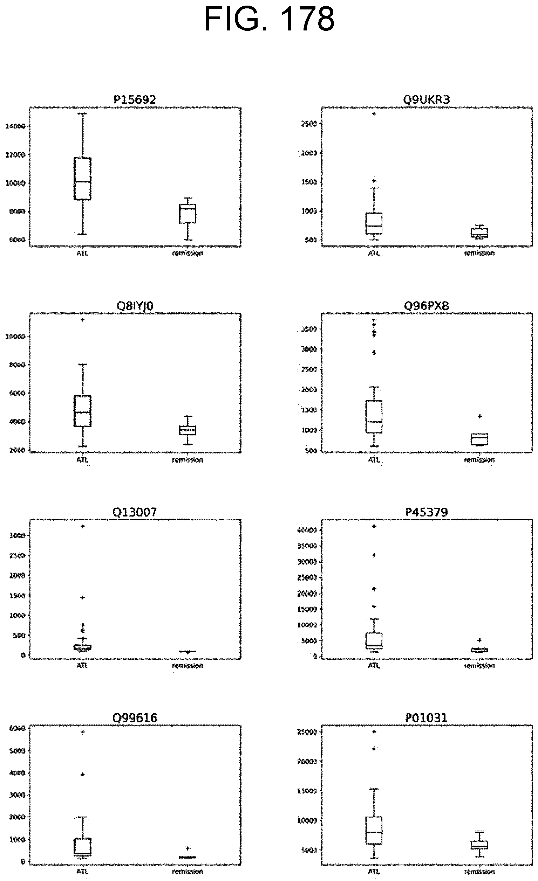

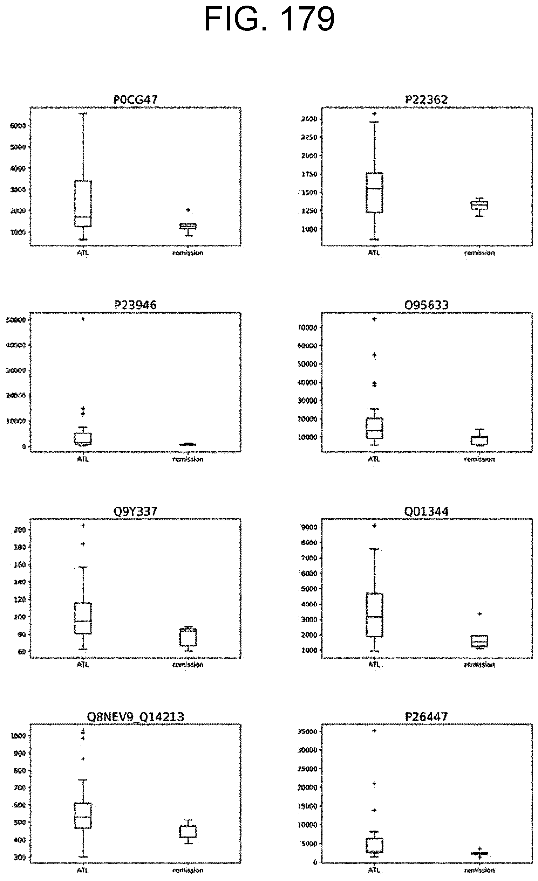

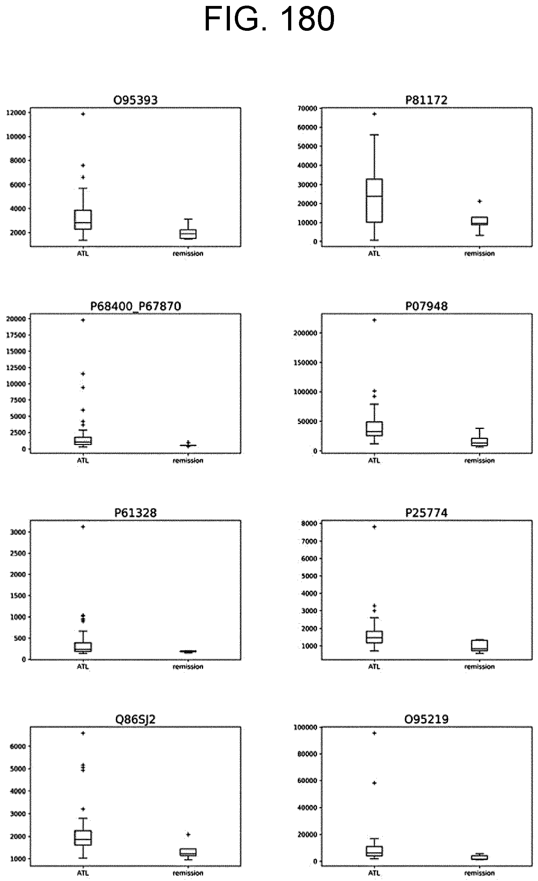

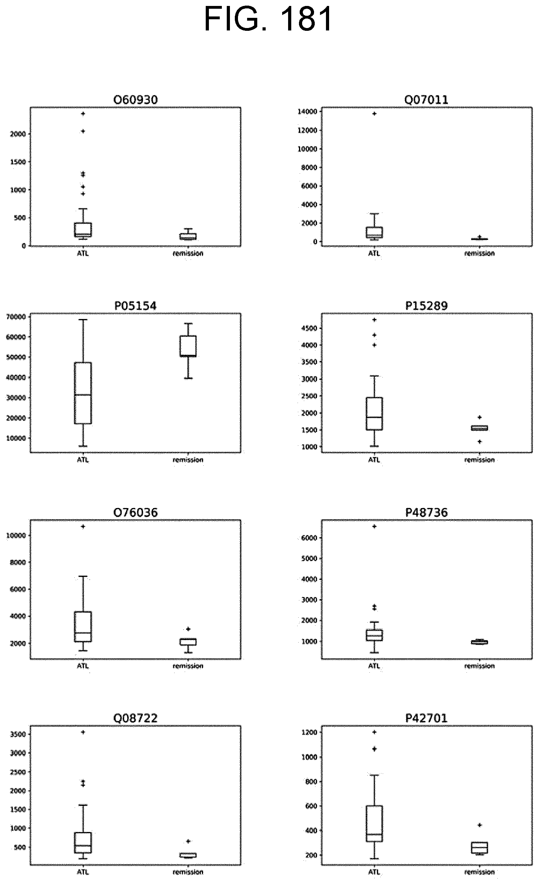

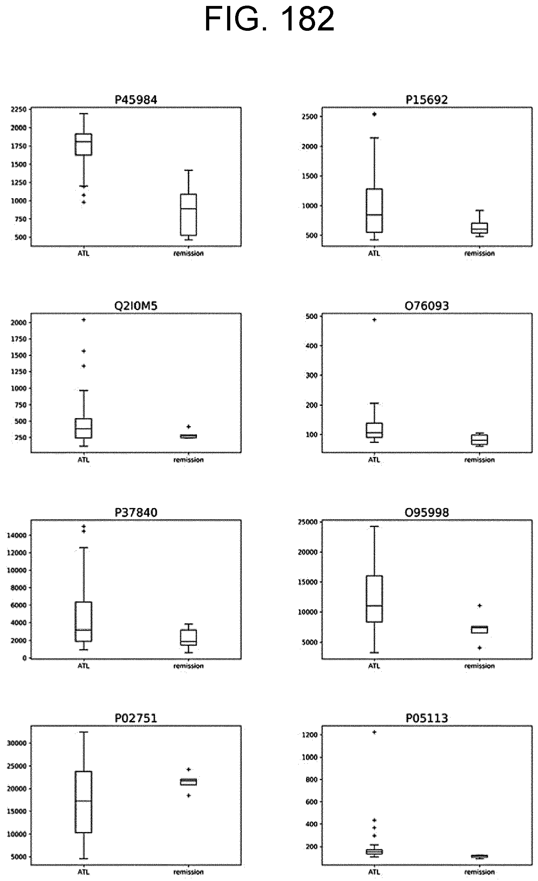

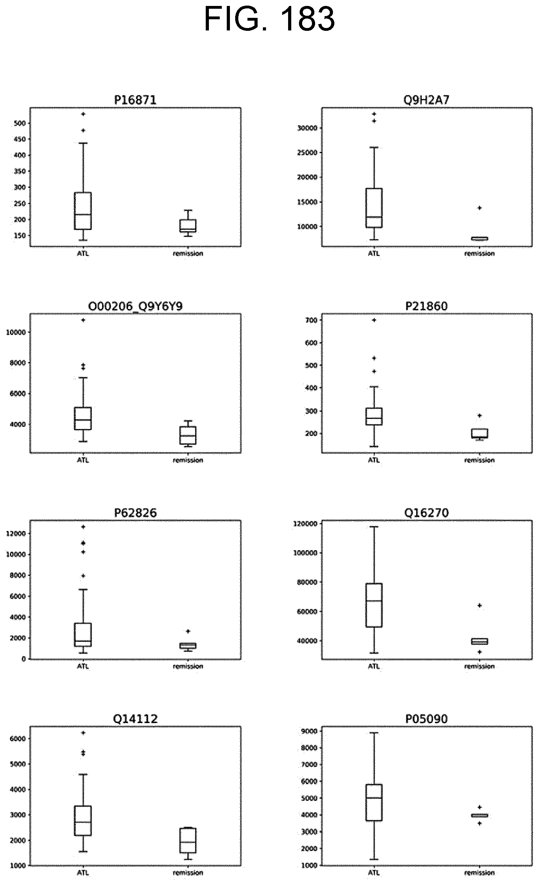

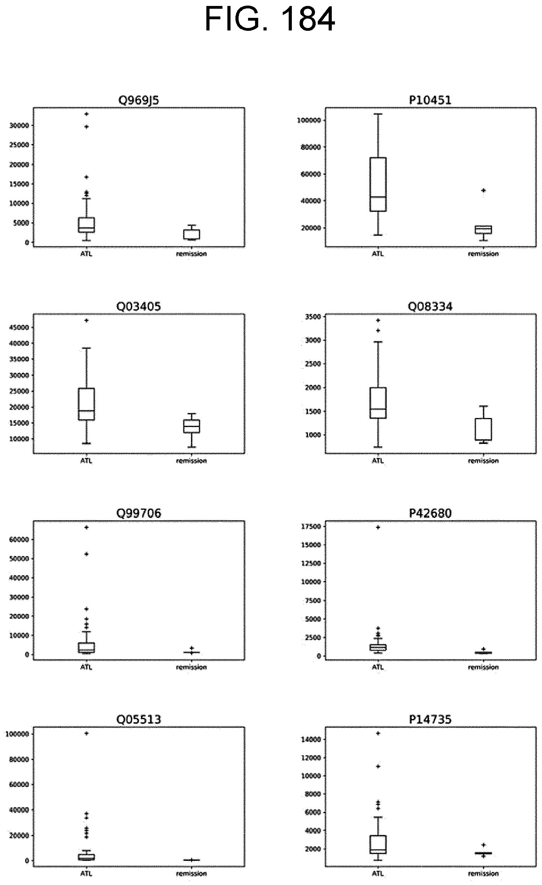

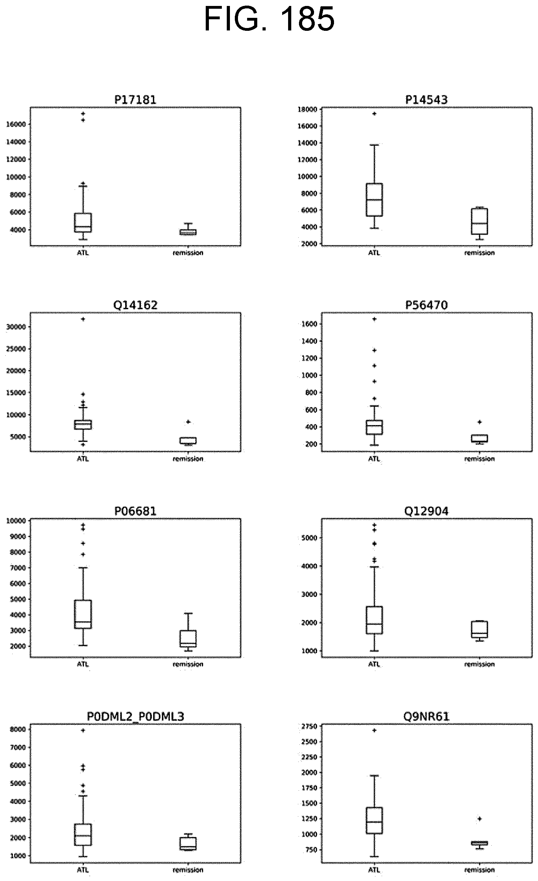

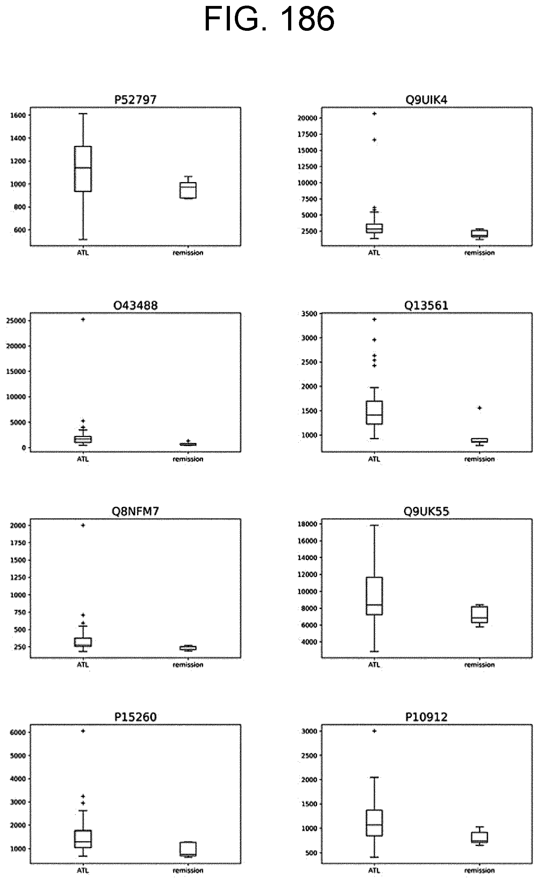

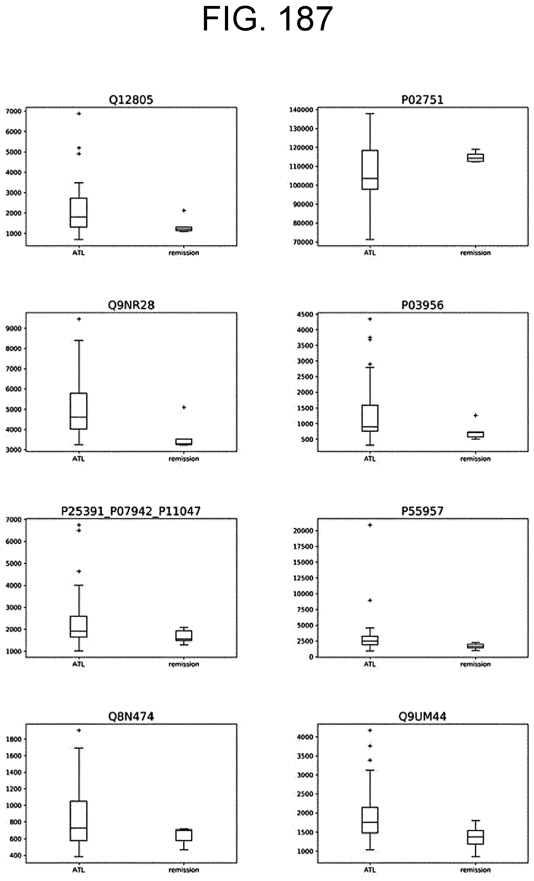

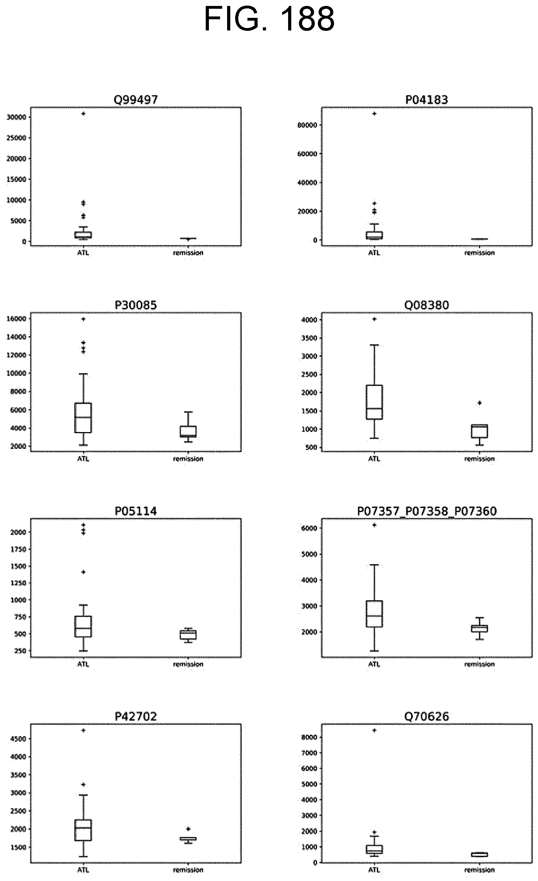

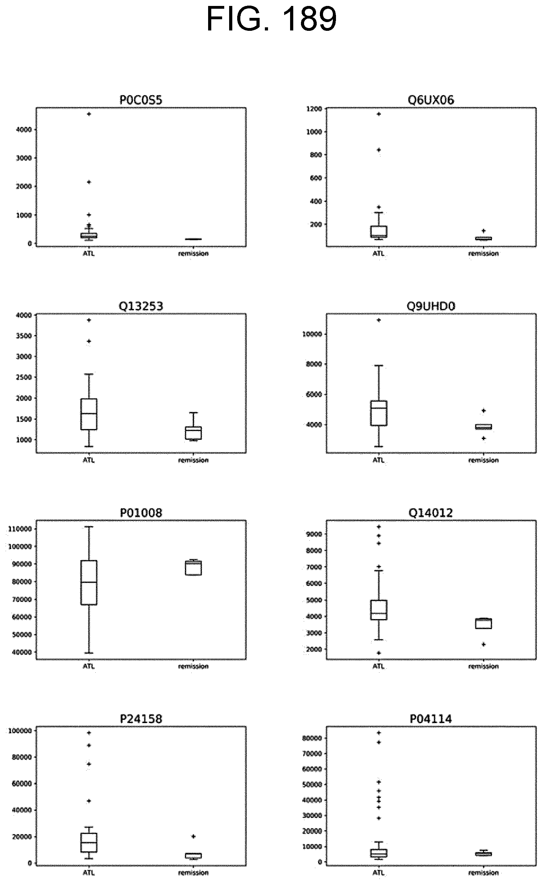

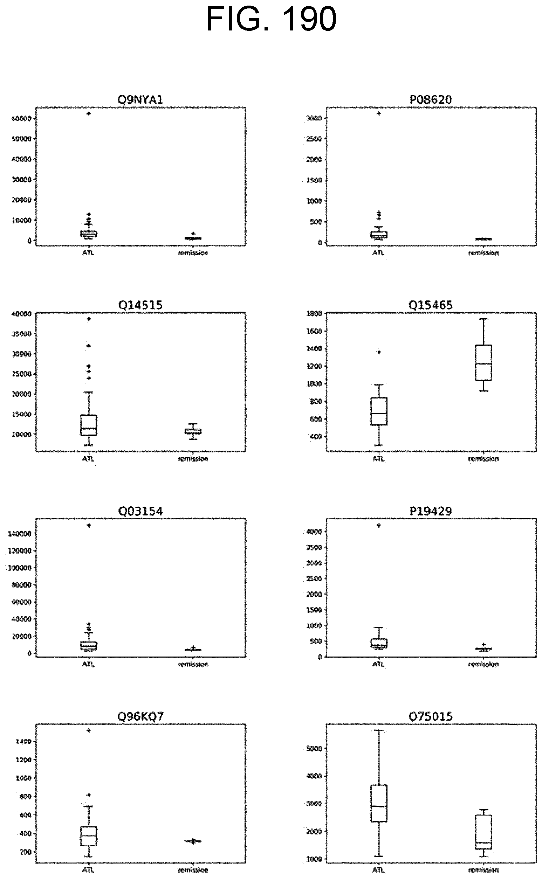

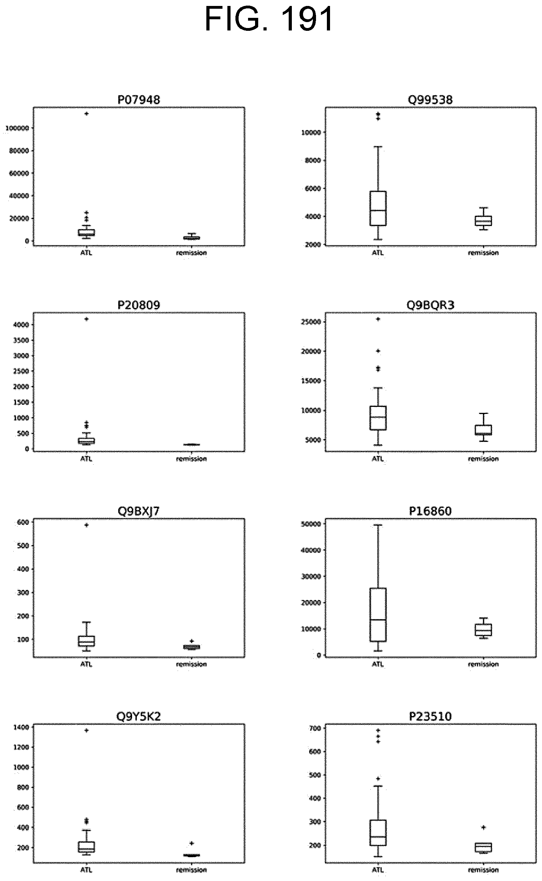

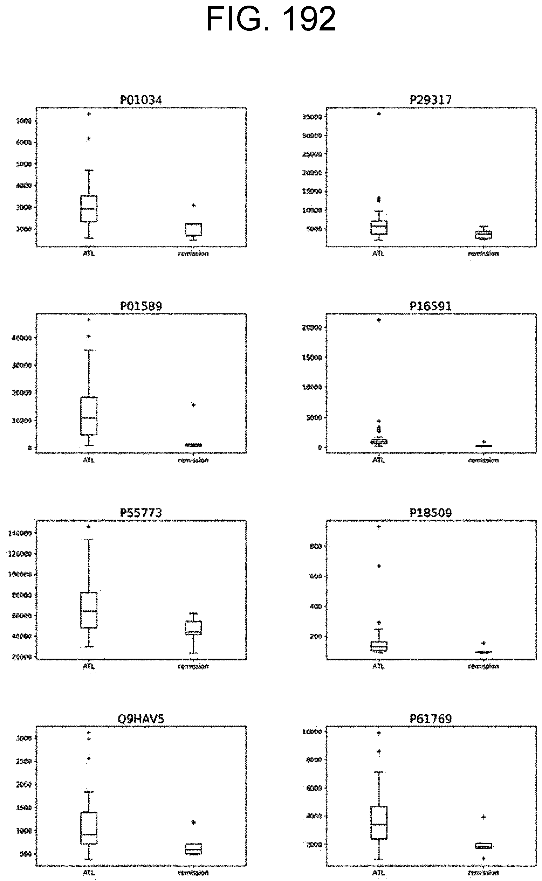

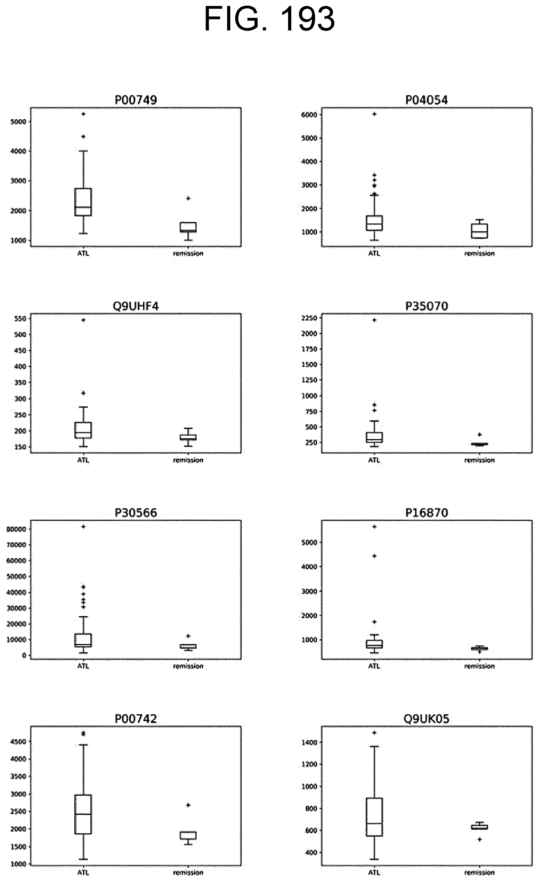

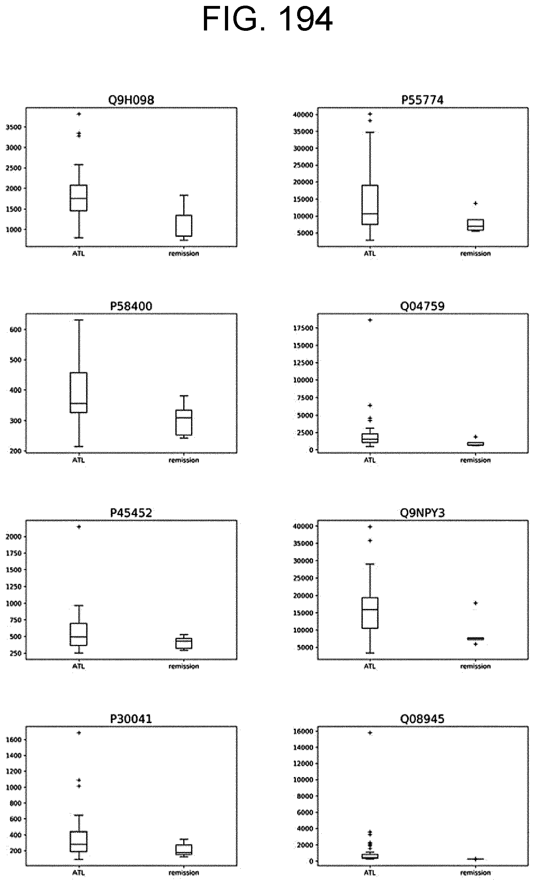

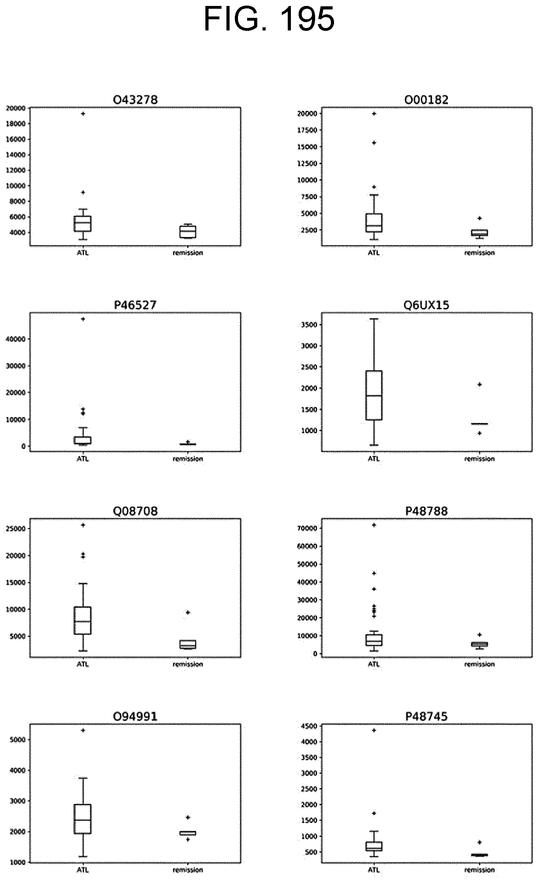

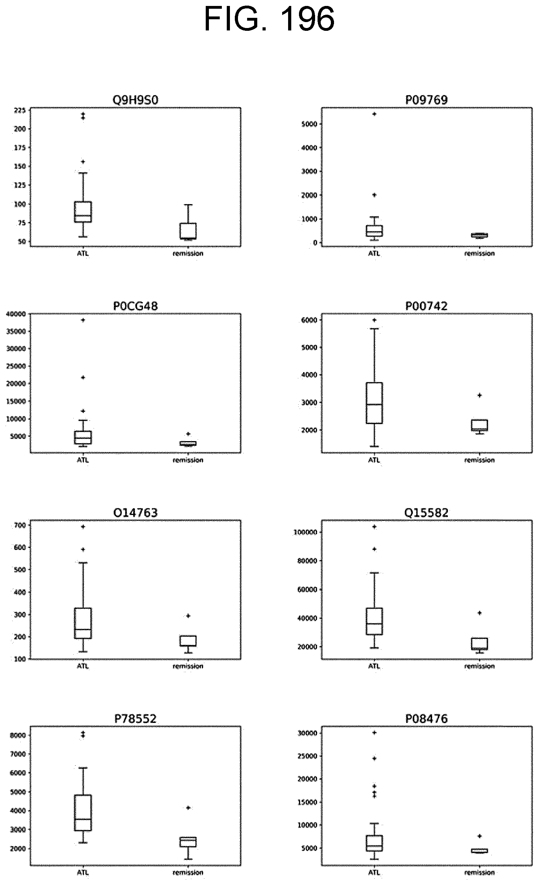

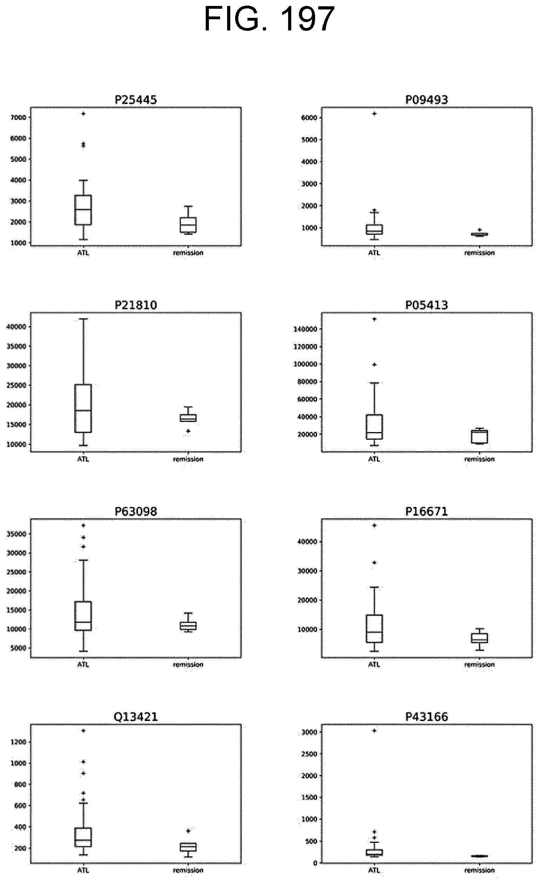

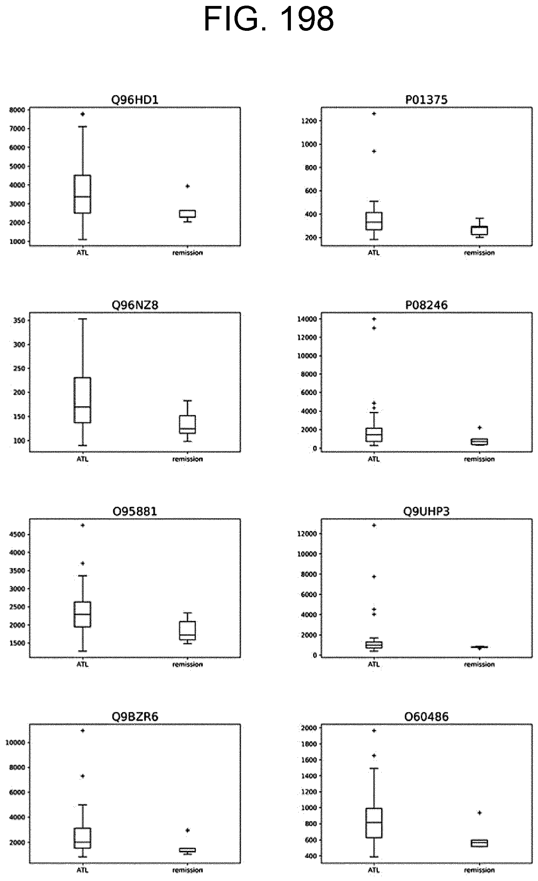

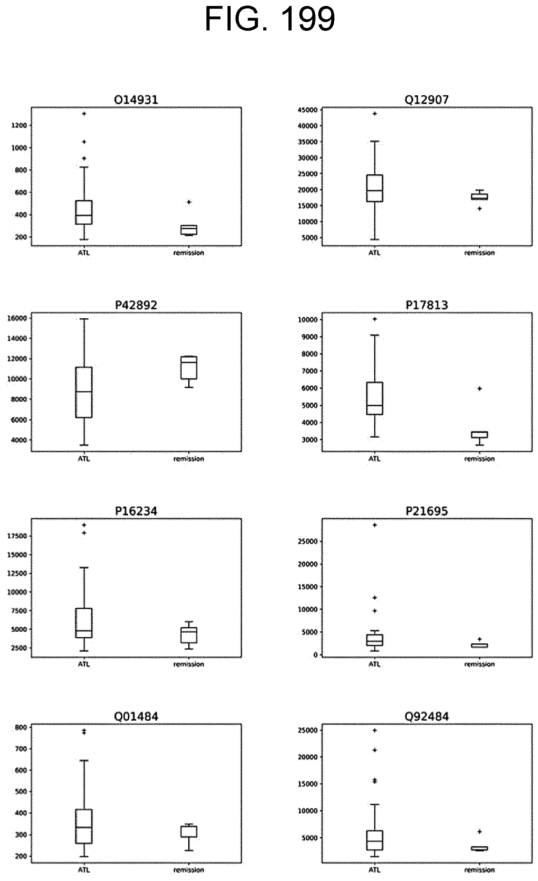

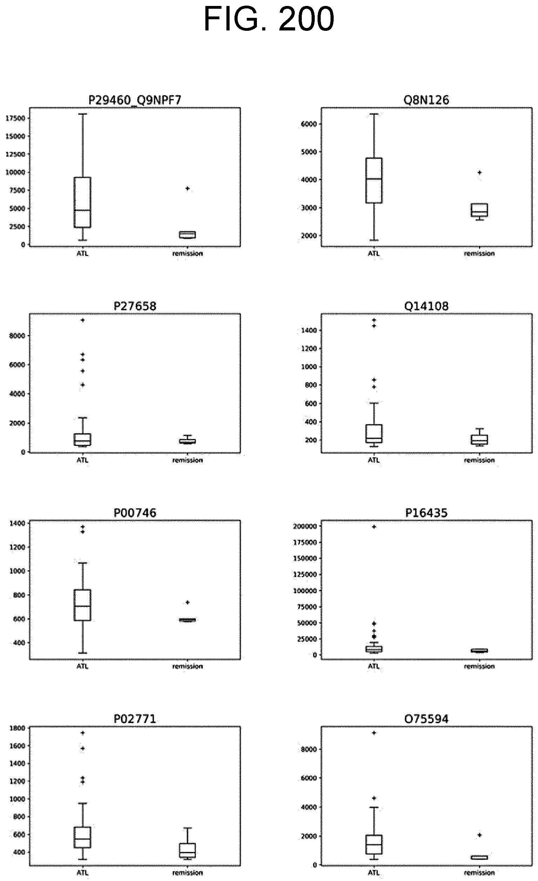

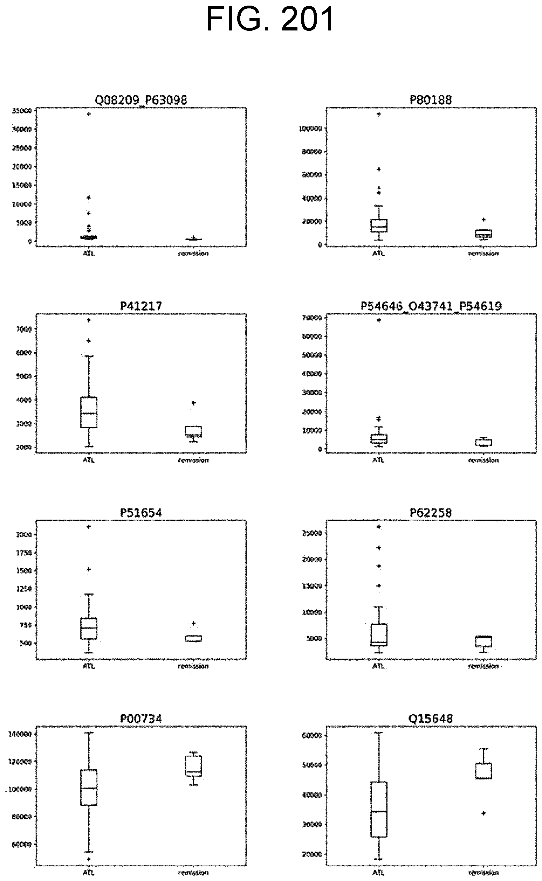

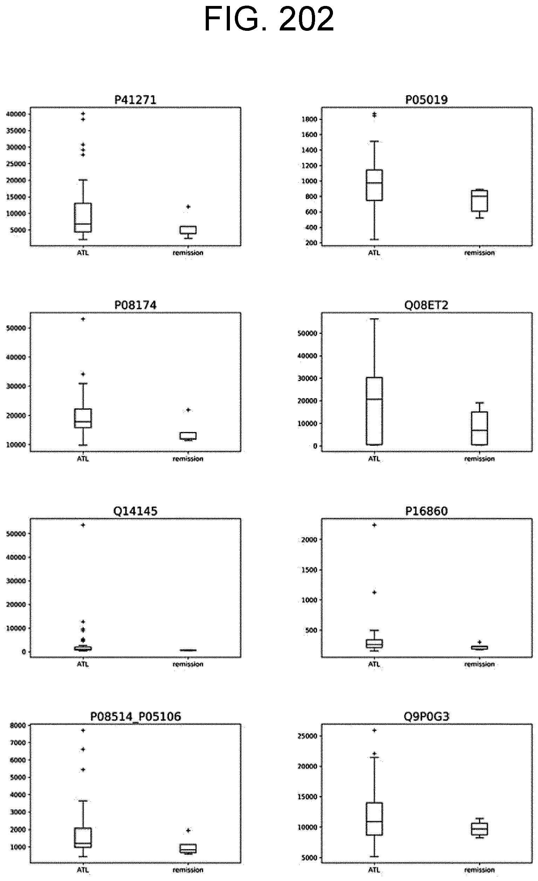

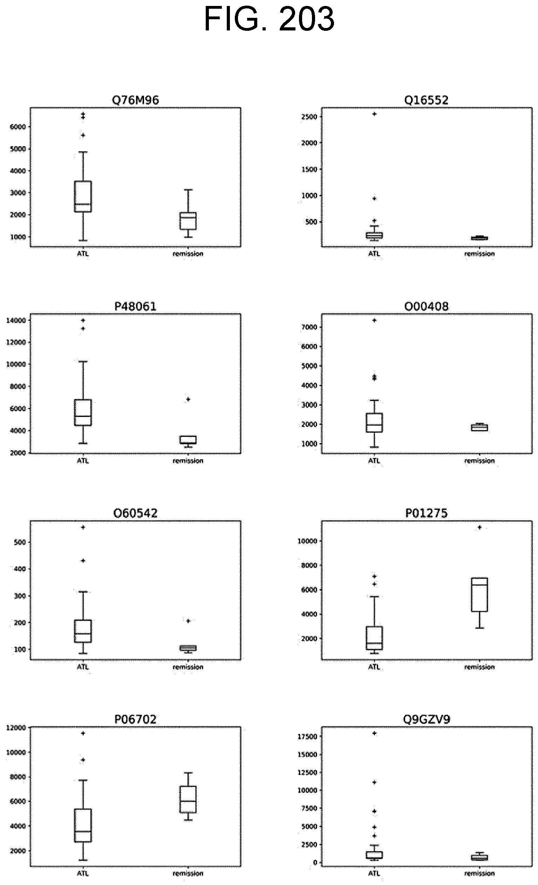

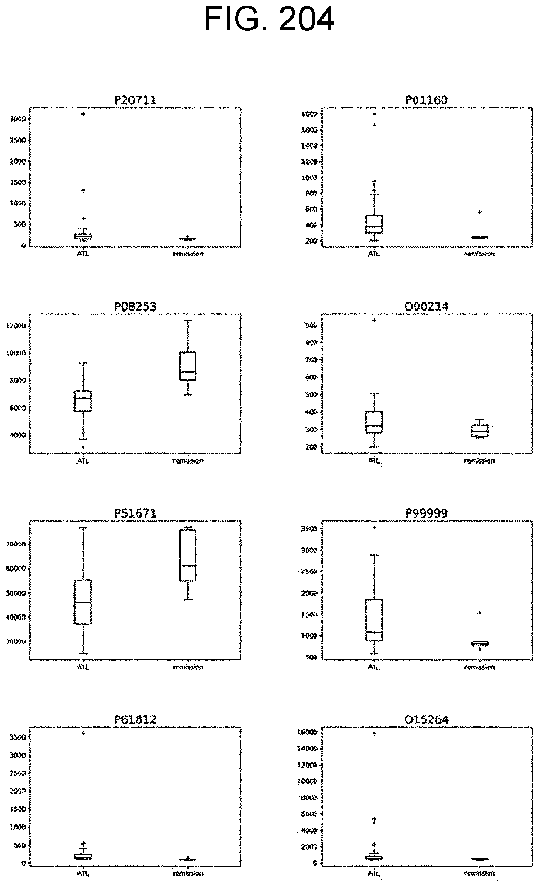

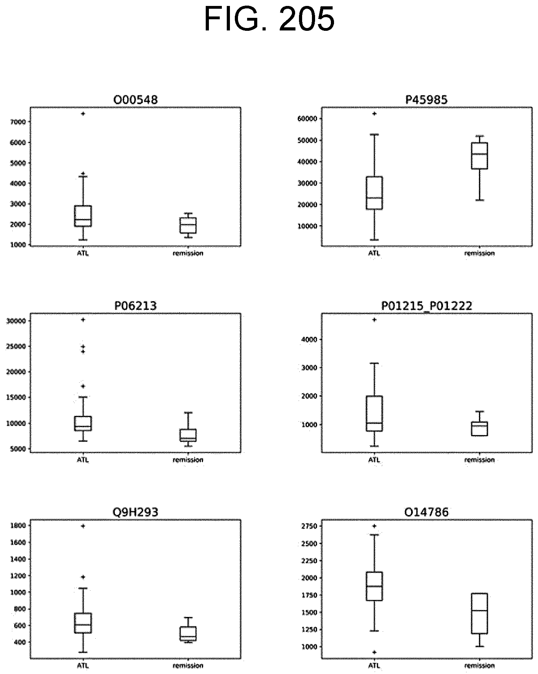

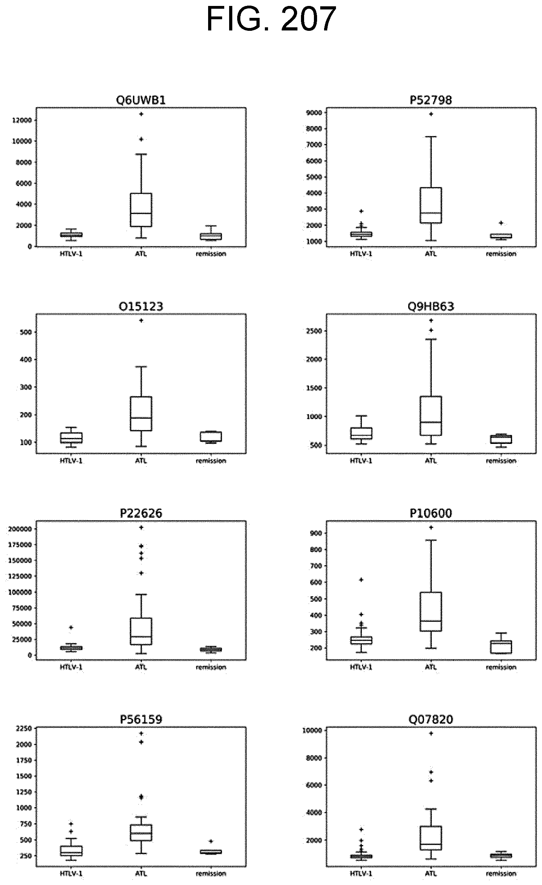

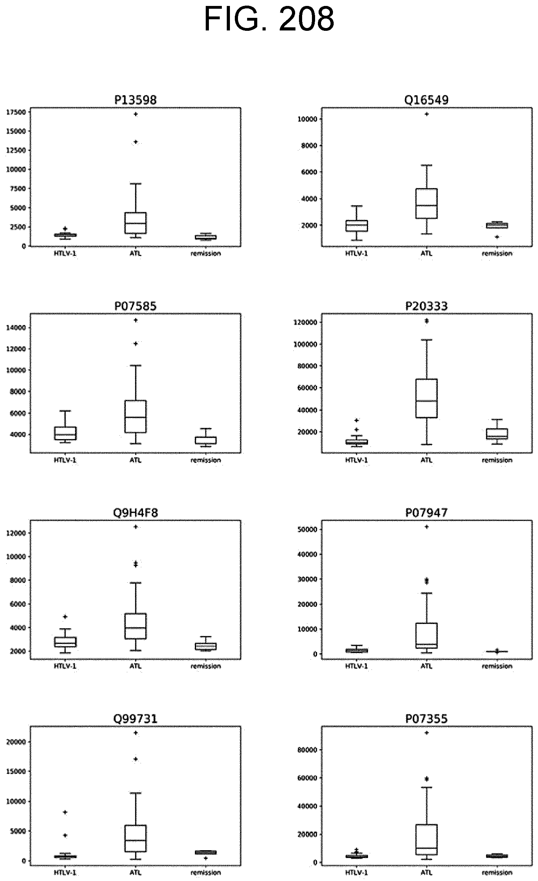

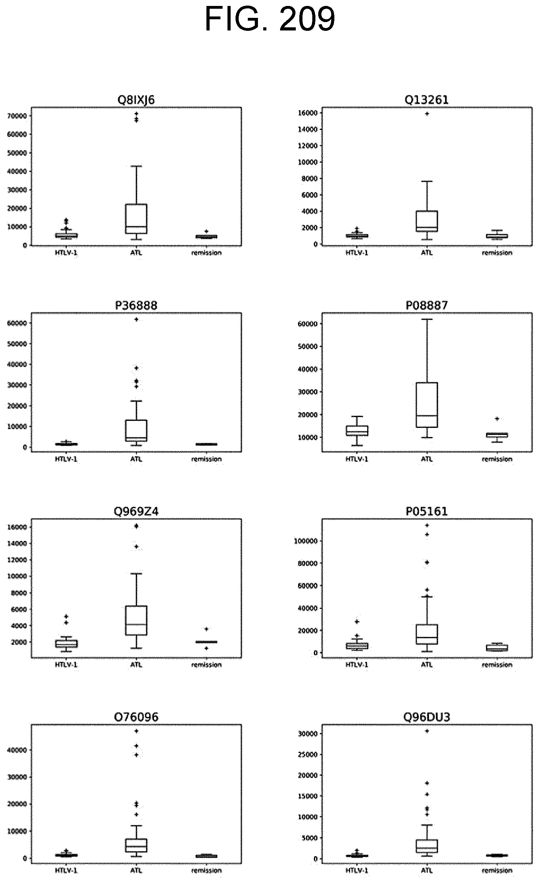

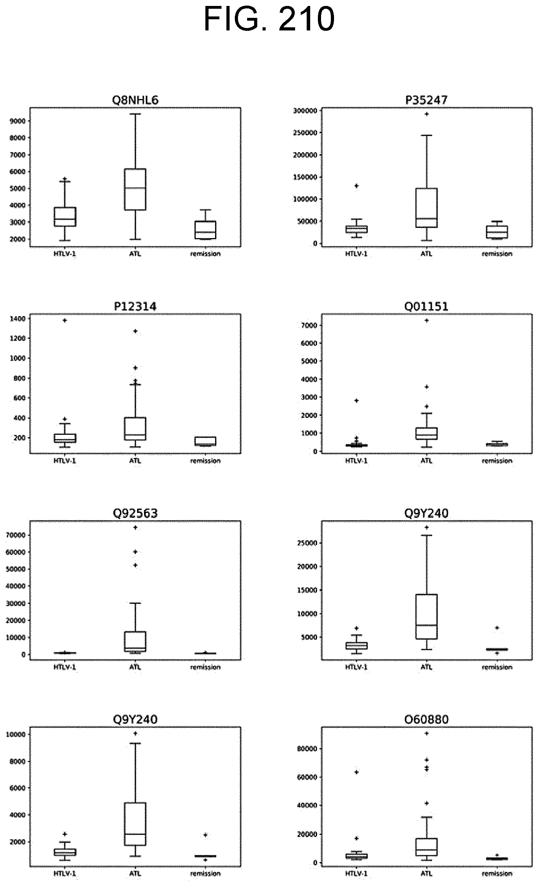

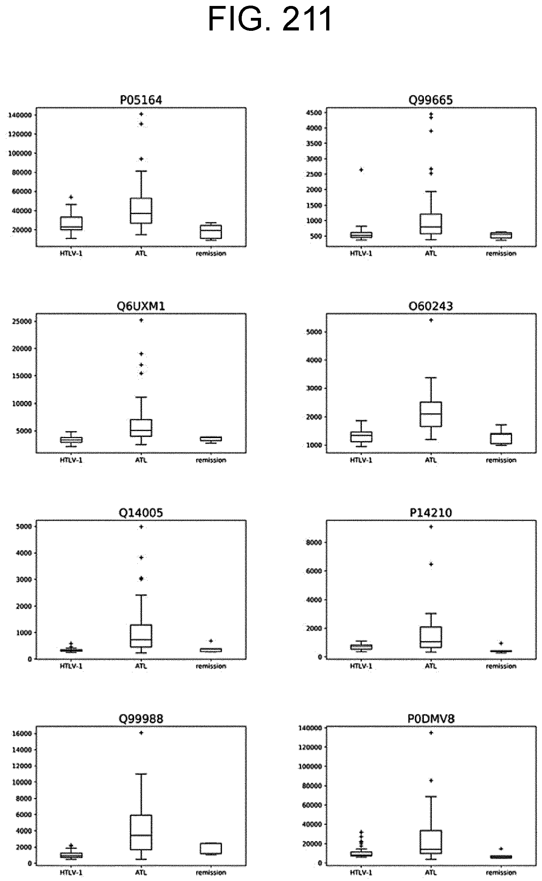

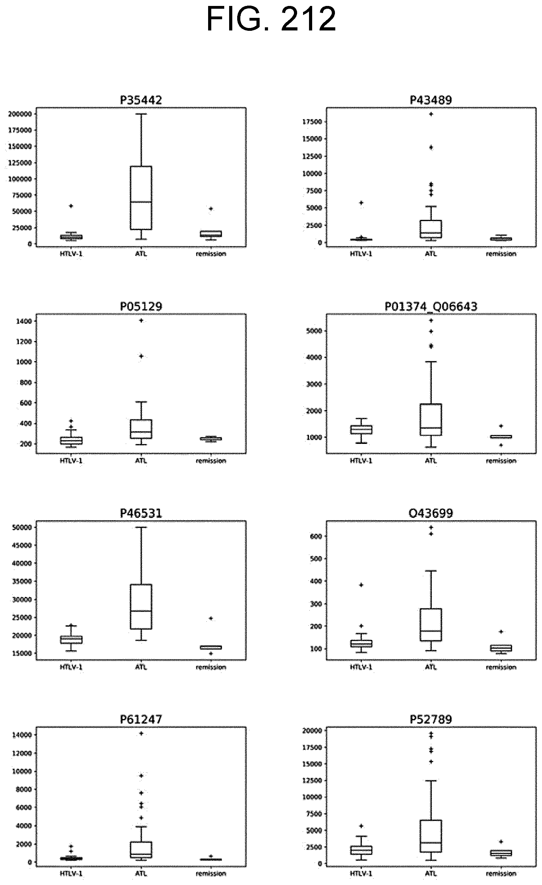

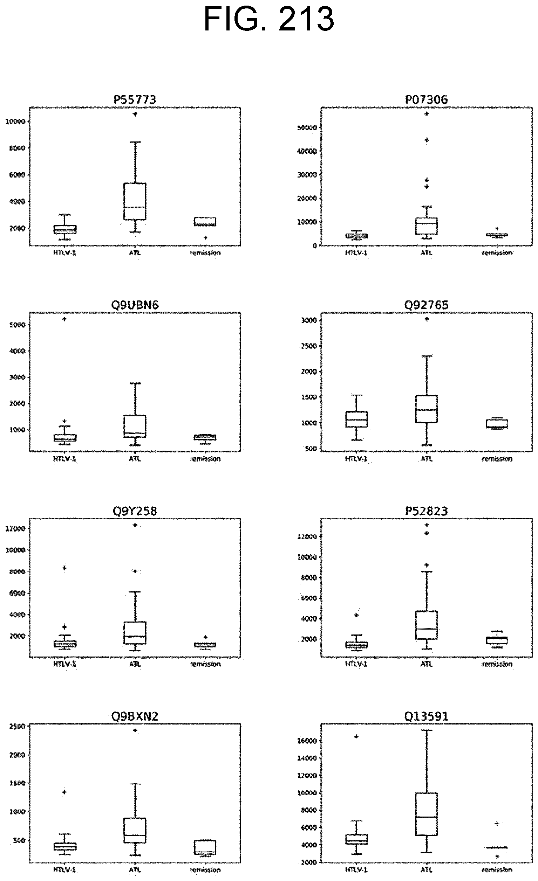

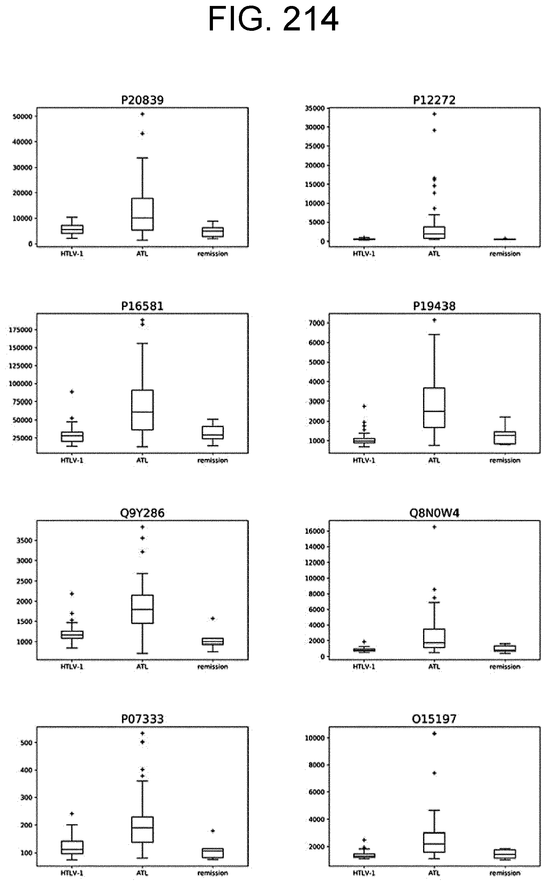

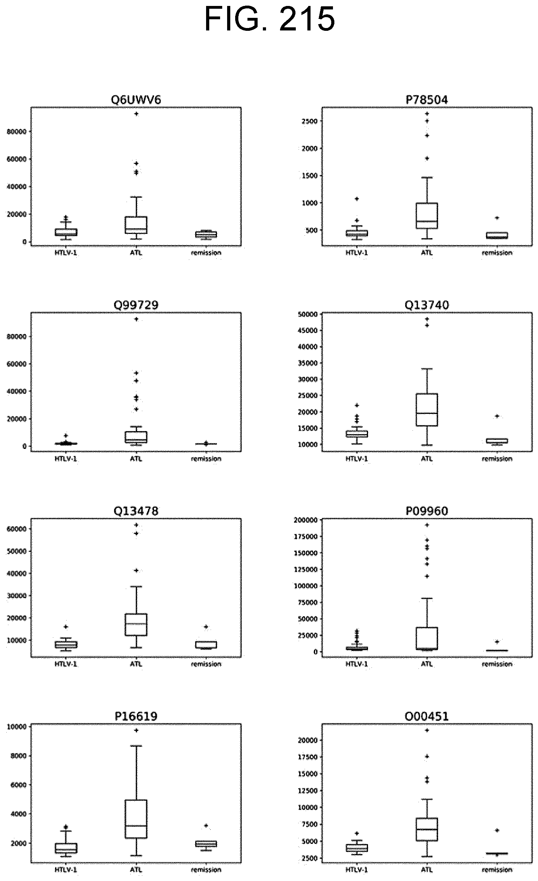

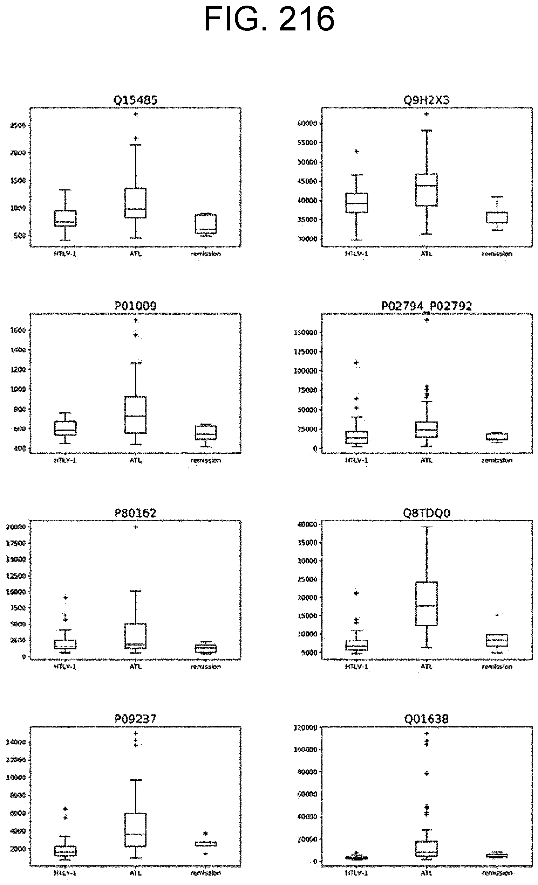

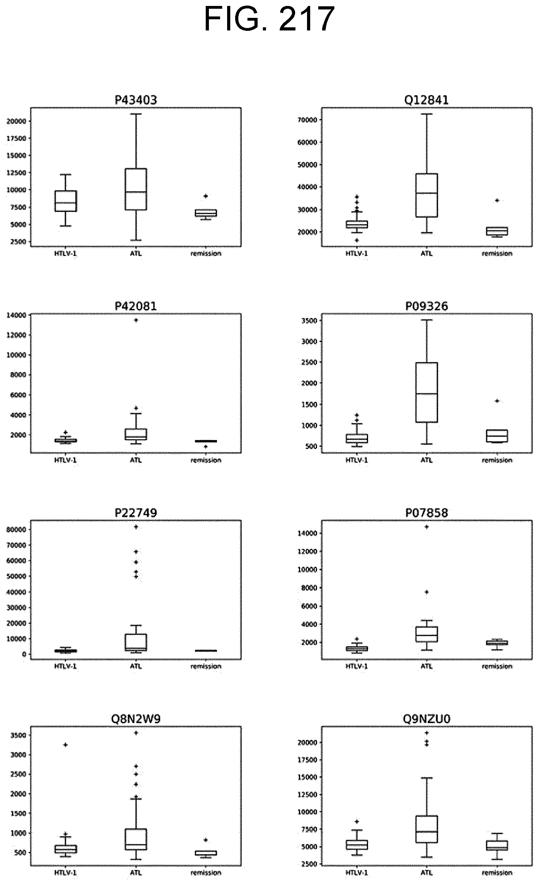

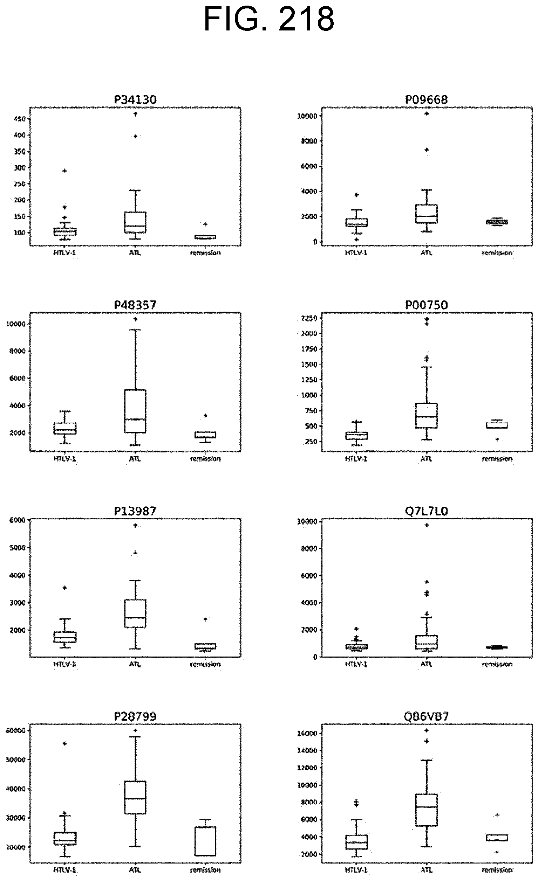

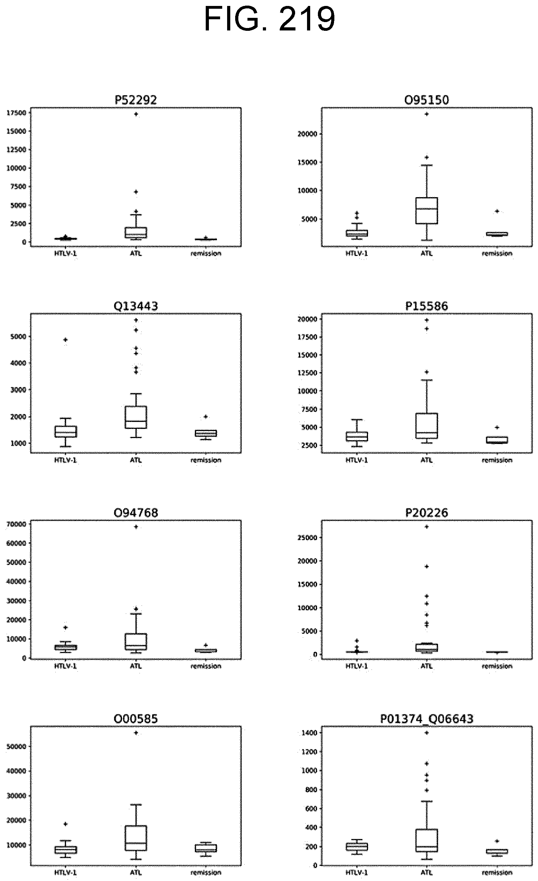

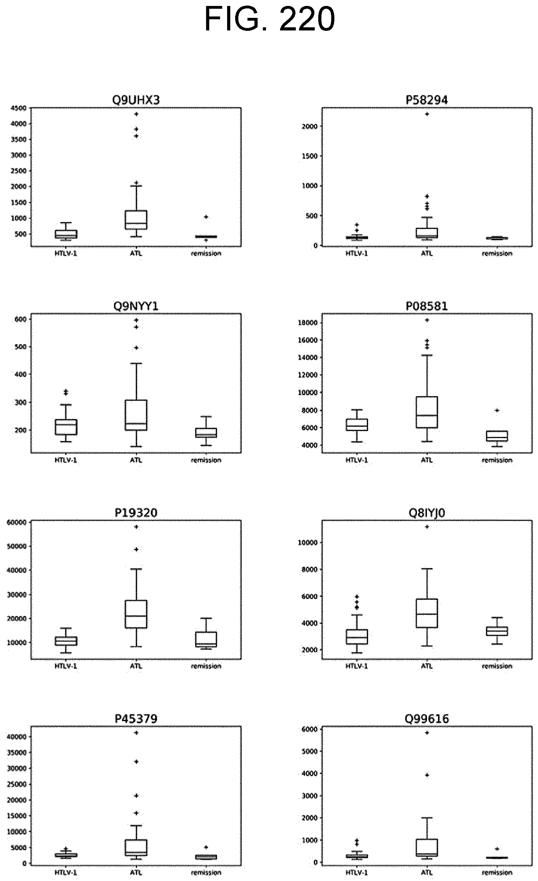

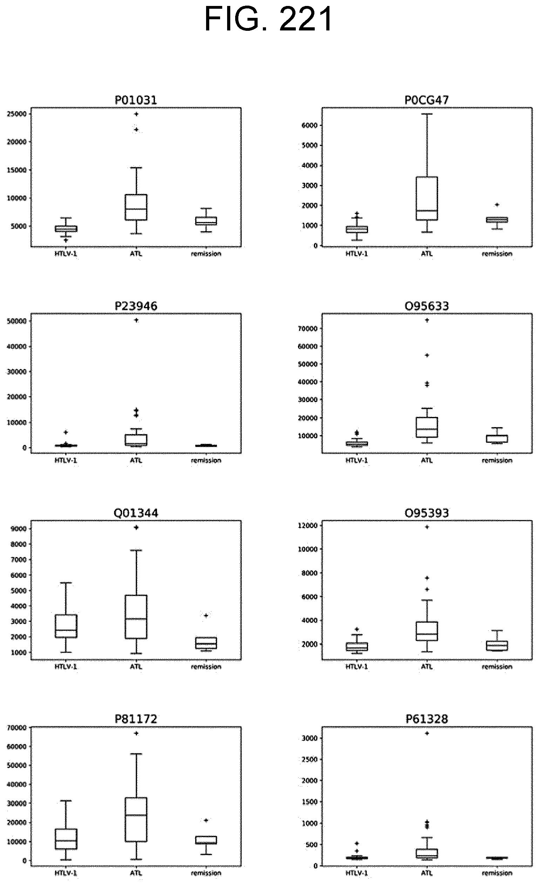

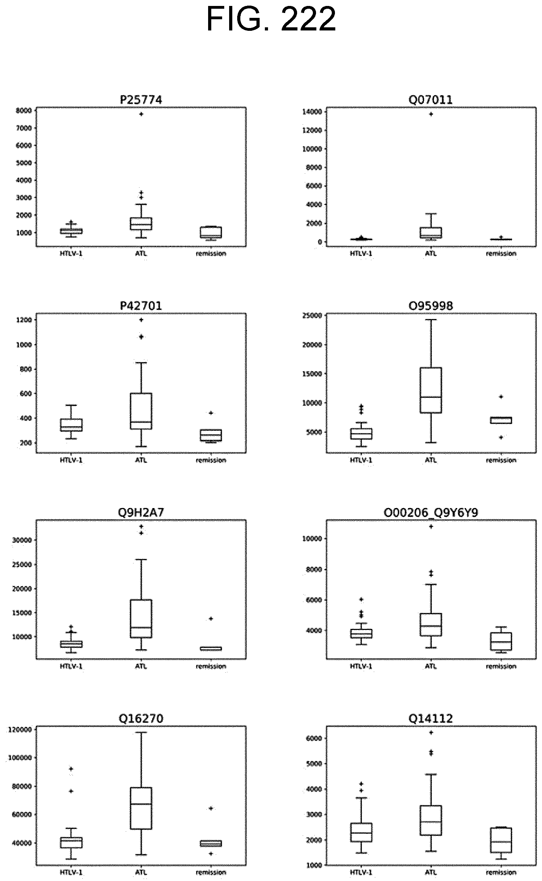

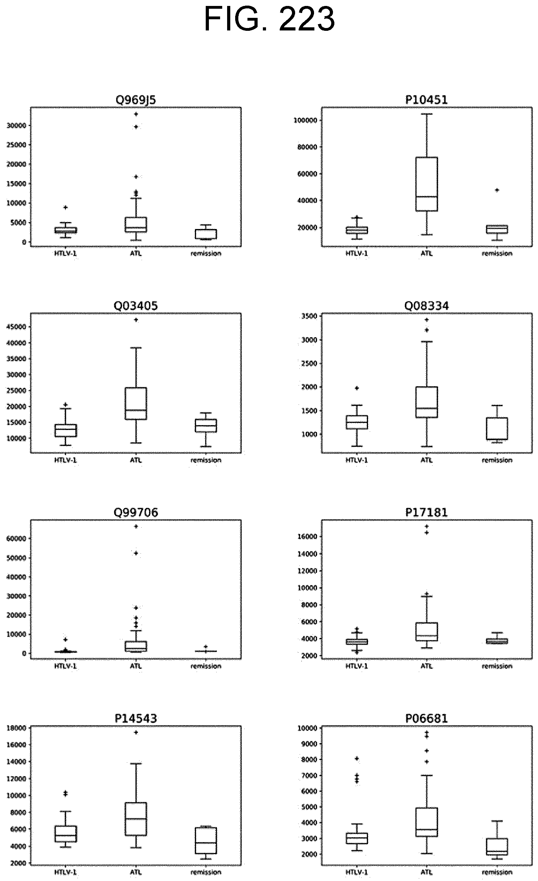

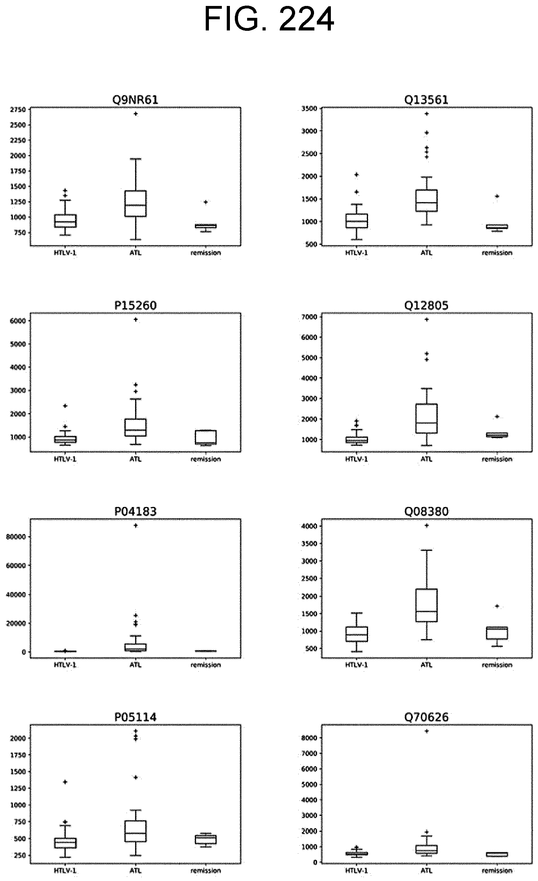

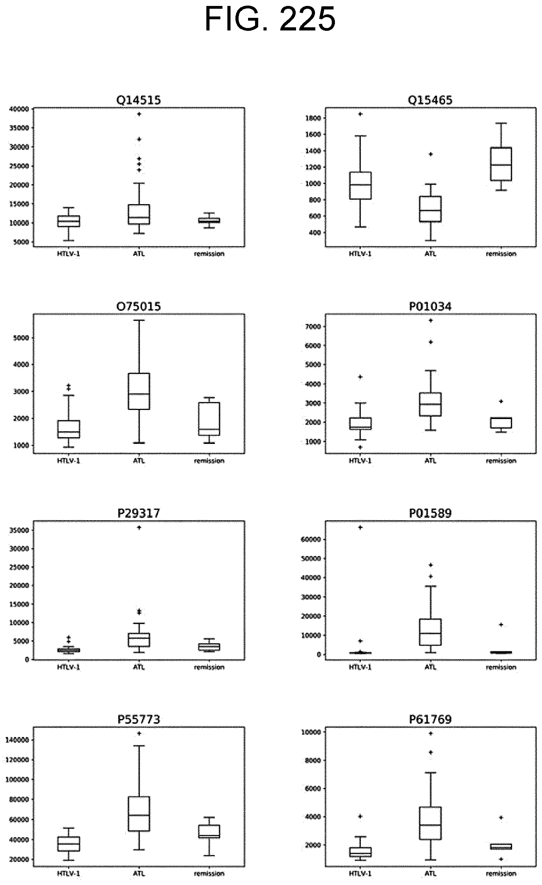

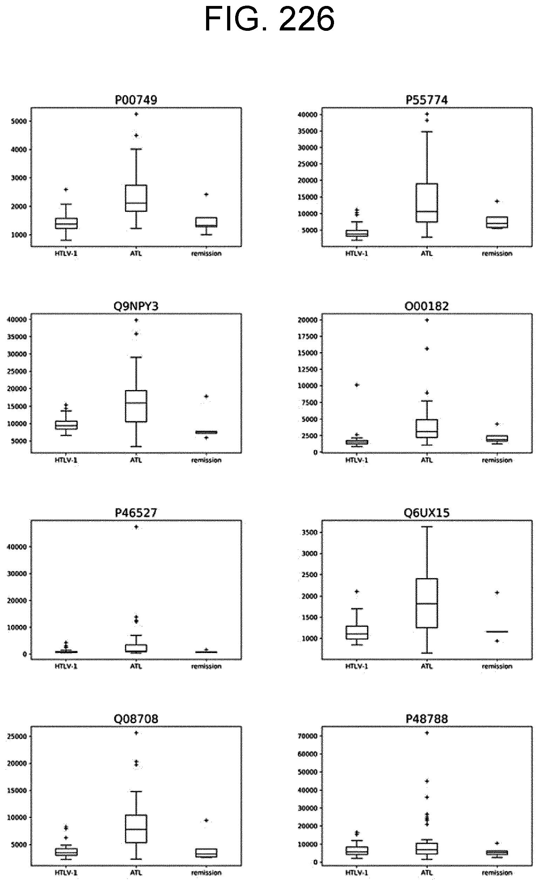

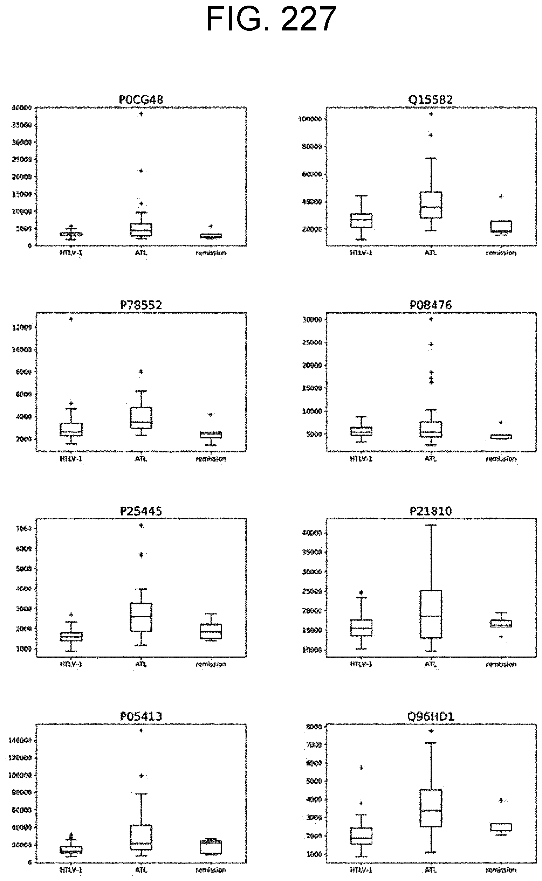

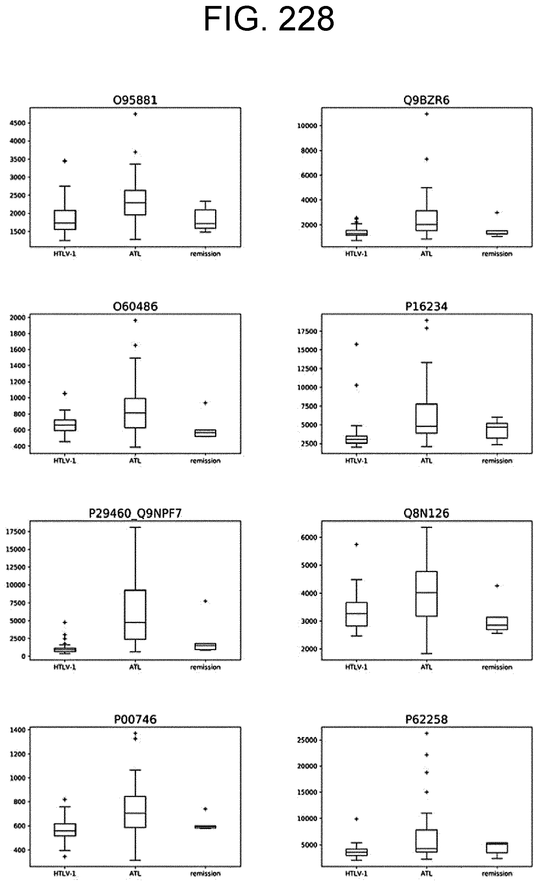

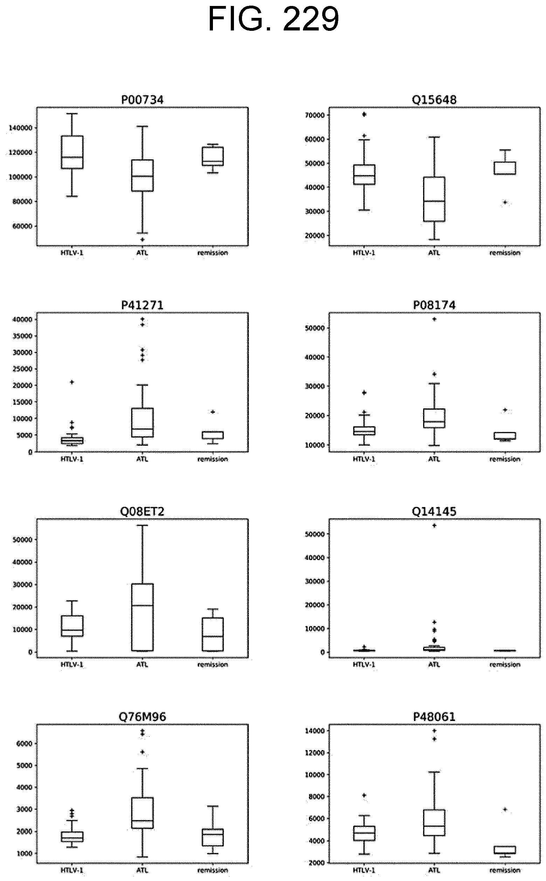

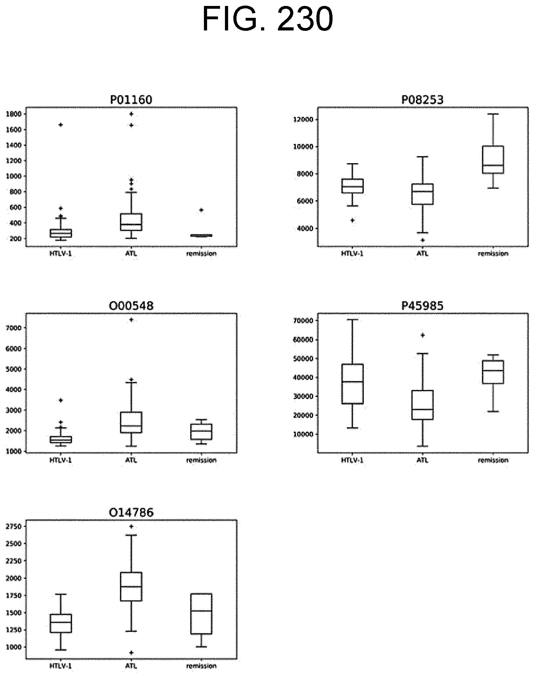

The results are shown in Tables 1 and 2 and Figures 1 to 230. At the top of each graph in figures, UniProt ID of a protein is shown. In the case of a protein having 2 or more UniProt IDs, all of them were described next to each other.

**[Table 1]**

| Protein No. | Name of protein | UniProt ID | p value | Average value in carrier group | Average value in patient group | Median value in carrier group | Median value in patient group |
|---|---|---|---|---|---|---|---|
| 1 | Coagulation_Factor_Xa | P00742 | 1.82E-16 | 618 | 2602 | 642 | 2419 |
| 2 | Coagulation_Factor_X | P00742 | 4.13E-16 | 786 | 3122 | 786 | 2921 |
| 3 | Cadherin-6 | P55285 | 9.32E-14 | 975 | 582 | 969 | 557 |
| 4 | IGFBP-4 | P22692 | 2.90E-12 | 2583 | 4181 | 2505 | 3968 |
| 5 | Lysozyme | P61626 | 5.65E-12 | 77282 | 100043 | 77310 | 104047 |
| 6 | TrkB | Q16620 | 1.70E-11 | 938 | 667 | 919 | 636 |
| 7 | GRN | P28799 | 2.73E-11 | 24063 | 37334 | 22295 | 36614 |
| 8 | RASA1 | P20936 | 2.77E-11 | 6719 | 3018 | 6406 | 2789 |
| 9 | IL-18_BPa | 095998 | 5.03E-11 | 4965 | 12067 | 4667 | 10985 |
| 10 | TNF_sR-II | P20333 | 6.82E-11 | 11129 | 51875 | 9993 | 47932 |
| 11 | vWF | P04275 | 8.25E-11 | 13800 | 44646 | 12941 | 39515 |
| 12 | Apo_A-I | P02647 | 8.79E-11 | 8527 | 5094 | 8392 | 4356 |
| 13 | LYNB | P07948 | 9.20E-11 | 127208 | 43072 | 137770 | 32330 |
| 14 | FYN | P06241 | 9.30E-11 | 54557 | 11344 | 54431 | 7392 |
| 15 | ApoM | 095445 | 2.24E-10 | 11500 | 6732 | 11225 | 6490 |
| 16 | CRP | P02741 | 2.29E-10 | 21940 | 67638 | 20273 | 77904 |
| 17 | CD48 | P09326 | 4.59E-10 | 713 | 1844 | 667 | 1745 |
| 18 | ON | P09486 | 5.02E-10 | 60842 | 27995 | 56194 | 22916 |
| 19 | ST4S6 | Q7LFX5 | 5.03E-10 | 3408 | 5879 | 3403 | 5497 |
| 20 | NRP1 | 014786 | 6.68E-10 | 1352 | 1875 | 1355 | 1874 |
| 21 | Osteopontin | P10451 | 8.02E-10 | 18375 | 49729 | 17983 | 42709 |
| 22 | TIMD3 | Q8TDQ0 | 8.09E-10 | 7572 | 18301 | 6742 | 17638 |
| 23 | TrkC | Q16288 | 9.69E-10 | 13276 | 10256 | 13325 | 10311 |
| 24 | RANTES | P13501 | 1.33E-09 | 59815 | 27994 | 56711 | 22737 |
| 25 | XTP3A | Q9H773 | 1.62E-09 | 21818 | 111104 | 21283 | 87965 |
| 26 | Cathepsin_V | 060911 | 1.95E-09 | 984 | 565 | 905 | 542 |
| 27 | Tropomyosin_4 | P67936 | 2.93E-09 | 106535 | 45669 | 111221 | 32924 |
| 28 | LG3BP | Q08380 | 3.04E-D9 | 886 | 1790 | 889 | 1559 |
| 29 | Carbonic_anhydrase_XIII | QSN1Q1 | 3.13E-09 | 56423 | 13975 | 51630 | 7746 |
| 30 | DRG-1 | Q9NP79 | 3.74E-09 | 87913 | 27691 | 96133 | 19676 |
| 31 | FN1.4 | P02751 | 3.88E-09 | 80946 | 106550 | 80780 | 103495 |
| 32 | Endostatin | P39060 | 3.95E-09 | 47505 | 67971 | 46224 | 66189 |
| 33 | sCD163 | Q86VB7 | 4.94E-09 | 3606 | 7606 | 3347 | 7419 |
| 34 | Afarnin | P43652 | 6.76E-09 | 27398 | 18435 | 27105 | 17161 |
| 35 | Carbonic_anhydrase_6 | P23280 | 6.76E-09 | 10695 | 3217 | 9093 | 1994 |
| 36 | Endothelin-converting_enzyme_1 | P42892 | 6.84E-09 | 13022 | 9062 | 12730 | 8752 |
| 37 | FCG3B | 075015 | 7.39E-09 | 1683 | 3109 | 1487 | 2892 |
| 38 | Angiopoietin-1 | Q15389 | 7.55E-09 | 2572 | 893 | 2059 | 737 |
| 39 | Notch_1 | P46531 | 8.01E-09 | 18994 | 28986 | 19047 | 26793 |
| 40 | VAV | P15498 | 8.41E-09 | 56013 | 15041 | 56625 | 10974 |
| 41 | Tenascin | P24821 | 8.87E-09 | 17280 | 40264 | 17028 | 35605 |
| 42 | IGFBP-7 | Q16270 | 9.52E-09 | 42549 | 66232 | 41460 | 67309 |
| 43 | b2-Microglobulin | P61769 | 1.42E-08 | 1573 | 3797 | 1397 | 3400 |
| 44 | VCAM-1 | P19320 | 1.44E-08 | 10748 | 22797 | 10571 | 21005 |
| 45 | CTAP-III | P02775 | 1.47E-08 | 36483 | 15674 | 34495 | 12758 |
| 46 | Ck-b-8-1 | P55773 | 1.66E-08 | 35464 | 68059 | 35629 | 64163 |
| 47 | LAG-3 | P18627 | 2.31E-08 | 12158 | 64829 | 11852 | 45759 |
| 48 | TNF_sR-I | P19438 | 2.37E-08 | 1080 | 2831 | 979 | 2512 |
| 49 | H6ST1 | 060243 | 2.37E-08 | 1310 | 2209 | 1334 | 2094 |
| 50 | GPVI | Q9HCN6 | 2.39E-08 | 31556 | 11840 | 29755 | 9825 |
| 51 | LYN | P07948 | 2.46E-08 | 51489 | 10289 | 48708 | 6029 |
| 52 | LBP | P18428 | 2.61E-08 | 59667 | 81928 | 59882 | 78280 |
| 53 | CLC1B | Q9P126 | 2.68E-08 | 27317 | 9118 | 21196 | 8172 |
| 54 | Lectin_mannose-binding_2 | Q12907 | 0.000000028 | 30224 | 20793 | 29326 | 19690 |
| 55 | NAP-2 | P02775 | 3.34E-08 | 12943 | 5529 | 12075 | 4331 |
| 56 | CD30 | P28908 | 3.49E-08 | 1083 | 23615 | 973 | 18909 |
| 57 | TSP2 | P35442 | 3.51E-08 | 11490 | 76766 | 10152 | 64305 |
| 58 | Endocan | Q9NQ30 | 4.27E-08 | 424 | 1436 | 359 | 1130 |
| 59 | C5a | P01031 | 4.58E-08 | 4412 | 9090 | 4403 | 8015 |
| 6D | EPHB2 | P29323 | 5.39E-08 | 11152 | 22502 | 10454 | 20457 |
| 61 | 0014_IL-23 | P29460(Q9NPF7) | 5.67E-08 | 1075 | 6496 | 897 | 4751 |
| 62 | Nectin-like_protein_2 | Q9BY67 | 5.69E-08 | 3652 | 21267 | 3550 | 17315 |
| 63 | Fibrinogen._g-chain_dirner | P02679 | 5.95E-08 | 27645 | 40703 | 25578 | 39872 |
| 64 | TNFSF15 | 095150 | 7.99E-08 | 2628 | 7169 | 2375 | 6800 |
| 65 | Ephrin-A4 | P52798 | 8.16E-08 | 1488 | 3341 | 1428 | 2746 |
| 66 | MPIF-1 | P55773 | 8.67E-08 | 1915 | 4013 | 1852 | 3557 |
| 67 | uPA | P00749 | 9.17E-08 | 1415 | 2358 | 1379 | 2115 |
| 68 | Macrophage_mannose_receptor | P22897 | 9.21E-08 | 9257 | 19419 | 9291 | 16031 |
| 69 | PDGF-AA | P04085 | 0.000000119 | 19962 | 10151 | 18810 | 8192 |
| 70 | sL-Selectin | P14151 | 0.000000129 | 3782 | 14521 | 3647 | 13032 |
| 71 | P-Cadherin | P22223 | 0.000000129 | 19504 | 14559 | 19406 | 14719 |
| 72 | ITI,_heavy_chain_H4 | Q14624 | 0.000000133 | 22620 | 32685 | 25828 | 31788 |
| 73 | FSTL1 | Q12841 | 0.000000139 | 23887 | 38451 | 23163 | 37095 |
| 74 | LD78-beta | P16619 | 0.000000159 | 1718 | 3755 | 1553 | 3165 |
| 75 | TCCR | Q6UWB1 | 0.000000171 | 1099 | 3906 | 1052 | 3121 |
| 76 | FN1.3 | P02751 | 0.000000207 | 2623 | 4181 | 2458 | 4239 |
| 77 | Angiopoietin-2 | 015123 | 0.000000211 | 115 | 208 | 113 | 188 |
| 78 | EDAR | Q9UNE0 | 0.000000213 | 5314 | 1231 | 3925 | 961 |
| 79 | Siglec-7 | Q9Y286 | 0.000000214 | 1200 | 1877 | 1164 | 1797 |
| 80 | Cystatin_C | P01034 | 0.000000239 | 1903 | 3119 | 1747 | 2928 |
| 81 | PCSK7 | Q16549 | 0.000000276 | 1956 | 3683 | 2002 | 3483 |
| 82 | PARC | P55774 | 0.000000305 | 4439 | 13696 | 3764 | 10627 |
| 83 | PF-4 | P02776 | 0.00000034 | 4803 | 2358 | 4402 | 1895 |
| 84 | ERBB1 | P00533 | 0.000000354 | 22119 | 17996 | 22153 | 17735 |
| 85 | CHKB | Q9Y259 | 0.000000355 | 13395 | 7714 | 12087 | 6768 |
| 86 | 0230_GDF-11_8 | 095390(014793) | 0.000000358 | 1148 | 728 | 1127 | 710 |
| 87 | a2-HS-Glycoprotein | P02765 | 0.000000375 | 2066 | 1572 | 2094 | 1530 |
| 88 | sE-Selectin | P16581 | 0.000000404 | 28999 | 70072 | 27988 | 61266 |
| 89 | TlMP-1 | P01033 | 0.000000412 | 278 | 771 | 264 | 614 |
| 90 | GAS1 | P54826 | 0.00000042 | 562 | 839 | 546 | 783 |
| 91 | DKK3 | Q9UBP4 | 0.000000421 | 5627 | 8437 | 5455 | 8433 |
| 92 | suPAR | Q03405 | 0.000000426 | 12523 | 20841 | 12802 | 18794 |
| 93 | ALCAM | Q13740 | 0.000000434 | 13507 | 21624 | 12927 | 19555 |
| 94 | SCGF-beta | Q9Y240 | 0.000000454 | 1260 | 3514 | 1179 | 2575 |
| 95 | MIC-1 | Q99988 | 0.000000462 | 1036 | 4137 | 921 | 3431 |
| 96 | N-terminal_pro-BNP | P16860 | 0.000000418 | 3859 | 15980 | 2737 | 13394 |
| 97 | SCGF-alpha | Q9Y240 | 0.000000489 | 3337 | 9954 | 3194 | 7496 |
| 98 | 0247_Fibrinogen | P02671 (P02675/P02679) | 0.000000498 | 89214 | 112850 | 88422 | 111585 |
| 99 | LRP8 | Q14114 | 0.000000504 | 2727 | 7609 | 2661 | 6194 |
| 100 | PolyUbiquitin_K48 | POCG47 | 0.000000515 | 790 | 2336 | 805 | 1722 |
| 101 | CLM6 | Q08708 | 0.000000521 | 3772 | 8624 | 3457 | 7724 |
| 102 | IL-18_Ra | Q13478 | 0.000000559 | 8087 | 19642 | 7764 | 17346 |
| 103 | CREL1 | Q96HD1 | 0.000000601 | 2065 | 3737 | 1869 | 3379 |
| 104 | BSP | P21815 | 0.000000605 | 2616 | 14676 | 1901 | 10492 |
| 105 | BTK | Q06187 | 0.000000608 | 22528 | 6791 | 22435 | 3854 |
| 106 | IL-6_sRa | P08887 | 0.000000608 | 12607 | 24838 | 12423 | 19547 |
| 107 | Kallistatin | P29622 | 0.000000667 | 33264 | 24304 | 34318 | 24164 |
| 108 | RELT | Q969Z4 | 0.000000668 | 1856 | 5248 | 1661 | 4144 |
| 109 | UNC5H3 | 095185 | 0.000000678 | 2621 | 4170 | 2454 | 4061 |
| 110 | EP15R | Q9UBC2 | 0.000000734 | 3792 | 1311 | 3532 | 913 |
| 111 | PDPK1 | 015530 | 0.000000736 | 9421 | 2712 | 8877 | 1724 |
| 112 | tPA | P00750 | 0.000000807 | 355 | 779 | 358 | 650 |
| 113 | IGFBP-2 | P18065 | 0.000000955 | 258 | 1008 | 180 | 749 |
| 114 | Prekallikrein | P03952 | 0.00000101 | 15683 | 11596 | 15452 | 10900 |
| 115 | Layilin | Q6UX15 | 0.00000108 | 1180 | 1960 | 1108 | 1819 |
| 116 | TEC | P42680 | 0.0000012 | 6526 | 1693 | 5491 | 1133 |
| 117 | GP1BA | P07359 | 0.00000127 | 2196 | 1448 | 1935 | 1311 |
| 118 | ILT-4 | Q8N423 | 0.00000129 | 6144 | 11225 | 5422 | 9503 |
| 119 | C9 | P02748 | 0.00000133 | 53767 | 73262 | 53308 | 73789 |
| 120 | Fas_soluble | P25445 | 0.00000146 | 1596 | 2753 | 1581 | 2594 |
| 121 | TGF-b3 | P10600 | 0.00000148 | 259 | 434 | 247 | 365 |
| 122 | PER | P16591 | 0.00000153 | 6334 | 1673 | 5794 | 932 |
| 123 | C1QR1 | Q9NPY3 | 0.00000154 | 9800 | 16555 | 9378 | 15923 |
| 124 | LY9 | Q9HBG7 | 0.00000154 | 9168 | 17254 | 9070 | 15104 |
| 125 | GFRa-2 | 000451 | 0.00000158 | 4048 | 7646 | 3918 | 6748 |
| 126 | Sonic_Hedgehog | Q15465 | 0.00000167 | 1012 | 694 | 981 | 666 |
| 127 | DUS3 | P51452 | 0.00000167 | 6282 | 1696 | 4748 | 1269 |
| 128 | Dynactin_subunit_2 | Q13561 | 0.00000169 | 1041 | 1563 | 2002 | 1412 |
| 129 | PIANP | Q8IYJ0 | 0.00000175 | 3158 | 4886 | 2899 | 4654 |
| 130 | DR6 | D75509 | 0.00000176 | 6413 | 9956 | 6082 | 9157 |
| 131 | FBLN3 | Q12805 | 0.00000177 | 1037 | 2178 | 945 | 1806 |
| 132 | CAMK2D | Q13557 | 0.00000188 | 27132 | 8705 | 27940 | 4562 |
| 133 | LTBP4 | Q8N2S1 | 0.00000194 | 1178 | 2270 | 1127 | 1942 |
| 134 | Fractalkine_CX3CL-1 | P78423 | 0.00000196 | 1555 | 2900 | 1471 | 2718 |
| 135 | FLRT2 | 043155 | 0.00000199 | 13194 | 17283 | 12619 | 17248 |
| 136 | SRCN1 | P12931 | 0.00000206 | 78070 | 26542 | 75765 | 18881 |
| 137 | GRB2_adapter_protein | P62993 | 0.00000212 | 122881 | 73895 | 130648 | 68566 |
| 138 | Thrornbospondin-1 | P07996 | 0.00000214 | 4302 | 1710 | 3957 | 1515 |
| 139 | CD59 | P13987 | 0.00000229 | 1805 | 2652 | 1724 | 2445 |
| 140 | NDP_kinase_B | P22392 | 0.00000229 | 62996 | 29297 | 60071 | 23692 |
| 141 | GFRa-1 | P56159 | 0.00000233 | 338 | 729 | 300 | 599 |
| 142 | 0941_D-dimer | P02671 (P02675/P02679) | 0.00000236 | 7195 | 9631 | 7043 | 9402 |
| 143 | a1-Antichymotrypsin | P01011 | 0.00000244 | 190125 | 217260 | 188203 | 218536 |
| 144 | CATZ | Q9UBR2 | 0.00000254 | 4080 | 7089 | 3845 | 6252 |
| 145 | NSF1C | Q9UNZ2 | 0.00000257 | 13757 | 5127 | 13067 | 3551 |
| 146 | Myostatin | 014793 | 0.00000264 | 4920 | 2781 | 4767 | 2161 |
| 147 | CAMK2A | Q9UQM7 | 0.00000276 | 5484 | 1607 | 5377 | 903 |
| 148 | 1106_AMPK_a2b2g1 | P54646 (O43741/P54619) | 0.00000278 | 22864 | 7390 | 21345 | 5042 |
| 149 | PLXB2 | 015031 | 0.00000289 | 4142 | 6034 | 4065 | 5431 |
| 150 | PDGF-BB | P01127 | 0.00000294 | 62226 | 32971 | 54396 | 28252 |
| 151 | Myokinase_human | P00568 | 0.00000303 | 32589 | 14214 | 31061 | 10424 |
| 152 | IF4G2 | P78344 | 0.00000316 | 29859 | 11532 | 30299 | 8048 |
| 153 | kallikrein_8 | 060259 | 0.00000323 | 6460 | 4735 | 6249 | 4434 |
| 154 | TFPI | P10646 | 0.00000335 | 17825 | 27732 | 17824 | 24907 |
| 155 | CXCL16_soluble | Q9H2A7 | 0.00000353 | 8636 | 14073 | 8508 | 11890 |
| 156 | Cathepsin_B | P07858 | 0.00000382 | 1313 | 3185 | 1298 | 2766 |
| 157 | 0222_C1q | P02745 (P02746/P02747) | 0.00000421 | 27738 | 33862 | 27044 | 32550 |
| 158 | CAMK2B | Q13554 | 0.00000456 | 11206 | 3576 | 10893 | 1920 |
| 159 | IDS | P22304 | 0.00000472 | 5251 | 7282 | 4976 | 6933 |
| 160 | Heparin_cofactor_ll | P05546 | 0.00000477 | 2782 | 2013 | 2783 | 1986 |
| 161 | FSTL3 | 095633 | 0.00000484 | 5891 | 17397 | 5308 | 13552 |
| 162 | RPS6KA3 | P51812 | 0.00000531 | 15455 | 6104 | 14015 | 4804 |
| 163 | SGTA | 043765 | 0.00000538 | 21368 | 7163 | 20584 | 5353 |
| 164 | ILT-2 | Q8NHL6 | 0.00000579 | 3390 | 5021 | 3152 | 5017 |
| 165 | Stanniocalcin-1 | P52823 | 0.00000604 | 1536 | 3923 | 1391 | 2988 |
| 166 | 0652_CK-MB | P12277(P06732) | 0.00000621 | 1206 | 529 | 1084 | 425 |
| 167 | M-CSF_R | P07333 | 0.00000658 | 122 | 218 | 112 | 189 |
| 168 | I-TAC | 014625 | 0.00000709 | 33693 | 68198 | 24817 | 59920 |
| 169 | SREC-I | Q14162 | 0.00000771 | 15143 | 8611 | 14177 | 7882 |
| 170 | MED-1 | Q15648 | 0.00000808 | 46166 | 35616 | 44762 | 34247 |
| 171 | OMD | Q99983 | 0.00000835 | 6784 | 14333 | 6431 | 12642 |
| 172 | MIP-3b | Q99731 | 0.00000849 | 957 | 4608 | 688 | 3413 |
| 173 | DLL1 | 000548 | 0.00000864 | 1645 | 2578 | 1528 | 2222 |
| 174 | IL-2_sRa | P01589 | 0.00000943 | 2488 | 14187 | 634 | 10897 |
| 175 | MMP-7 | P09237 | 0.00000967 | 1916 | 4838 | 1641 | 3617 |
| 176 | siCAM-1 | P05362 | 0.00000984 | 4213 | 7312 | 4412 | 7057 |
| 177 | RAC1 | P63000 | 0.0000101 | 49434 | 21753 | 47664 | 15050 |
| 178 | SCF_sR | P10721 | 0.0000106 | 10566 | 7868 | 10382 | 7857 |
| 179 | Plasminogen | P00747 | 0.0000106 | 3507 | 2744 | 3536 | 2845 |
| 180 | IP-10 | P02778 | 0.000011 | 2891 | 10124 | 2125 | 7905 |
| 181 | BMP10 | 095393 | 0.000012 | 1816 | 3443 | 1672 | 2834 |
| 182 | TFF1 | P04155 | 0.0000129 | 446 | 2422 | 243 | 1386 |
| 183 | PAPP-A | Q13219 | 0.0000132 | 5206 | 18969 | 4289 | 14258 |
| 184 | AN32B | Q92688 | 0.0000136 | 131 | 245 | 129 | 203 |
| 185 | BGH3 | Q15582 | 0.0000136 | 26543 | 40693 | 26954 | 36082 |
| 186 | CD226 | Q15762 | 0.0000139 | 2807 | 1249 | 2062 | 1123 |
| 187 | URB | Q76M96 | 0.0000142 | 1835 | 2927 | 1698 | 2464 |
| 188 | Sphingosine_kinase_1 | Q9NYA1 | 0.0000154 | 18429 | 5492 | 15524 | 3129 |
| 189 | Mcl-1 | Q07820 | 0.0000161 | 900 | 2374 | 808 | 1685 |
| 190 | BAFF_Receptor | Q96RJ3 | 0.0000162 | 3210 | 2167 | 3016 | 1882 |
| 191 | WISP-1 | 095388 | 0.0000171 | 10990 | 7919 | 10578 | 7268 |
| 192 | sCD4 | P01730 | 0.0000219 | 248 | 480 | 232 | 368 |
| 193 | DKK1 | 094907 | 0.0000238 | 35702 | 21314 | 31923 | 18407 |
| 194 | Kallikrein_7 | P49862 | 0.0000241 | 1498 | 969 | 1444 | 775 |
| 195 | IL-16 | Q14005 | 0.000026 | 331 | 1140 | 323 | 729 |
| 196 | HCC-1 | Q16627 | 0.0000261 | 7769 | 11580 | 7744 | 11224 |
| 197 | PDE5A | 076074 | 0.0000263 | 20593 | 8278 | 19990 | 5262 |
| 198 | IL-15_Ra | Q13261 | 0.0000265 | 1007 | 3088 | 948 | 2028 |
| 199 | DLL4 | Q9NR61 | 0.0000265 | 954 | 1254 | 925 | 1193 |
| 200 | JAG1 | P78504 | 0.000027 | 454 | 880 | 423 | 659 |
| 201 | BMPR1A | P36894 | 0.0000282 | 603 | 878 | 572 | 786 |
| 202 | EDA | Q92838 | 0.0000285 | 1087 | 882 | 1063 | 920 |
| 203 | SMOC1 | Q9H4F8 | • 0.0000285 | 2798 | 4539 | 2655 | 3970 |
| 204 | EGF | P01133 | 0.0000288 | 1474 | 893 | 1343 | 811 |
| 205 | SEM5A | Q13591 | 0.0000304 | 4913 | 7795 | 4481 | 7187 |
| 206 | CD39 | P49961 | 0.0000312 | 1861 | 3096 | 1722 | 2608 |
| 207 | IL-10_Rb | Q08334 | 0,0000322 | 1266 | 1750 | 1246 | 1548 |
| 208 | RANK | Q9Y6Q6 | 0.0000332 | 919 | 2267 | 858 | 1597 |
| 209 | EMR2 | Q9UHX3 | 0.0000355 | 488 | 1159 | 439 | 838 |
| 210 | amyloid_precursor_protein | P05067 | 0.0000378 | 57979 | 35364 | 48651 | 30562 |
| 211 | hnRNP_A2_B1 | P22626 | 0.000038 | 11788 | 51483 | 11337 | 28985 |
| 212 | PKB_beta | P31751 | 0.0000393 | 40022 | 18970 | 42966 | 11739 |
| 213 | Cytochrome_P450_3A4 | P08684 | 0.0000406 | 20141 | 10956 | 18841 | 9409 |
| 214 | Neurotrophin-3 | P20783 | 0.0000436 | 103 | 142 | 94 | 129 |
| 215 | JAK2 | 060674 | 0.0000467 | 86246 | 105173 | 94972 | 107732 |
| 216 | METAP1 | P53582 | 0.0000479 | 11864 | 5154 | 11815 | 3528 |
| 217 | UFM1 | P61960 | 0.0000496 | 33274 | 17440 | 34214 | 14129 |
| 218 | EPI | 014944 | 0.0000503 | 2358 | 995 | 1972 | 876 |
| 219 | MMP-12 | P39900 | 0.0000512 | 1529 | 6366 | 1445 | 3905 |
| 220 | Bone_proteoglycan_II | P07585 | 0.0000525 | 4185 | 6278 | 3948 | 5598 |
| 221 | LEAP-1 | P81172 | 0.0000534 | 12002 | 24042 | 10443 | 23780 |
| 222 | annexin_II | P07355 | 0.0000538 | 4244 | 18696 | 3815 | 10033 |
| 223 | Siglec-6 | 043699 | 0.0000659 | 130 | 222 | 120 | 178 |
| 224 | ASGR1 | P07306 | 0.0000736 | 3907 | 11296 | 3804 | 9349 |
| 225 | YES | P07947 | 0.0000742 | 1472 | 9249 | 1364 | 3871 |
| 226 | sICAM-2 | P13598 | 0.0000747 | 1452 | 3676 | 1431 | 2962 |
| 227 | Cyclophilin_F | P30405 | 0.0000749 | 59695 | 24068 | 52592 | 12930 |
| 228 | TFF3 | Q07654 | 0.0000793 | 8836 | 13729 | 8091 | 12314 |
| 229 | Angiostatin | P00747 | 0.0000803 | 23505 | 18922 | 23929 | 19823 |
| 230 | LRIG3 | Q6UXM1 | 0.0000834 | 3315 | 6659 | 3314 | 5063 |
| 231 | Flt-3 | P36888 | 0.0000868 | 1437 | 10433 | 1253 | 4541 |
| 232 | SP-D | P35247 | 0.0000874 | 34906 | 86912 | 34009 | 55092 |
| 233 | ASAH2 | Q9NR71 | 0.0000946 | 2516 | 1587 | 2285 | 1393 |
| 234 | CSK | P41240 | 0.0000953 | 24146 | 11134 | 22396 | 7851 |
| 235 | MP2K4 | P45985 | 0.000102513 | 37781 | 26031 | 37634 | 23121 |
| 236 | NET4 | Q9HB63 | 0.000103807 | 703 | 1107 | 668 | 897 |
| 237 | MIP-5 | Q16663 | 0.000105434 | 4979 | 8304 | 3999 | 6372 |
| 238 | FLRT3 | Q9NZU0 | 0.000112325 | 5401 | 8436 | 5229 | 7111 |
| 239 | HSP 40 | P25685 | 0.000113362 | 1294 | 520 | 1112 | 330 |
| 240 | DAN | P41271 | 0.000114184 | 4024 | 10871 | 3286 | 6792 |
| 241 | CLC7A | Q9BXN2 | 0.000117696 | 425 | 731 | 390 | 588 |
| 242 | Factor_D | P00746 | 0.000122723 | 562 | 723 | 558 | 705 |
| 243 | SLAF6 | Q96DU3 | 0.000128207 | 703 | 4686 | 614 | 2552 |
| 244 | Epithelial_cell_kinase | P29317 | 0.000130162 | 2598 | 6302 | 2457 | 5724 |
| 245 | 0182_Calpain_I | P07384(PD4632) | 0.000136265 | 45722 | 24715 | 44974 | 16307 |
| 246 | Nogo_Receptor | Q9BZR6 | 0.000145431 | 1440 | 2735 | 1283 | 2028 |
| 247 | PDGFRA | P16234 | 0.000148032 | 3516 | 6469 | 3050 | 4786 |
| 248 | C1r | P00736 | 0.000162419 | 148 | 205 | 139 | 183 |
| 249 | PH | P01298 | 0.000163207 | 2475 | 5715 | 2013 | 3923 |
| 250 | NLGNX | Q8NOW4 | 0.000169802 | 884 | 2851 | 823 | 1708 |
| 251 | PKC-G | P05129 | 0.000170371 | 238 | 392 | 228 | 316 |
| 252 | EphB6 | 015197 | 0.000172167 | 1379 | 2773 | 1289 | 2193 |
| 253 | FCN2 | Q15485 | 0.000175439 | 802 | 1148 | 740 | 979 |
| 254 | EFNB2 | P52799 | 0.000117482 | 18212 | 24059 | 17590 | 22567 |
| 255 | IL-23_R | Q5VWK5 | 0.00017957 | 2535 | 21977 | 2142 | 7781 |
| 256 | SIRT2 | Q8IXJ6 | 0.000181991 | 5886 | 17662 | 4956 | 10020 |
| 257 | P-Selectin | P16109 | 0.000182093 | 31758 | 20915 | 27516 | 18423 |
| 258 | RAC3 | P60763 | 0.000183679 | 10416 | 5251 | 10462 | 3402 |
| 259 | LEG9 | 000182 | 0.000190248 | 1738 | 4238 | 1507 | 3118 |
| 260 | MDH1 | P20839 | 0.000200975 | 5606 | 13261 | 5511 | 10092 |
| 261 | CCL28 | Q9NRJ3 | 0.000201074 | 14225 | 9334 | 13774 | 8826 |
| 262 | HRG | P04196 | 0.000204481 | 4104 | 3026 | 4197 | 2825 |
| 263 | BLC | 043927 | 0.000205393 | 1896 | 9063 | 1715 | 6585 |
| 264 | TECK | 015444 | 0.000213908 | 1539 | 3149 | 1352 | 2251 |
| 265 | Protein_C | P04070 | 0.000216456 | 1455 | 1187 | 1482 | 1177 |
| 266 | Caspase-3 | P42574 | 0.000217053 | 63841 | 37407 | 64719 | 26365 |
| 267 | Siglec-3 | P20138 | 0.000219035 | 2248 | 3341 | 2303 | 3028 |
| 268 | Thrombin | P00734 | 0.000225439 | 4229 | 1480 | 2232 | 1096 |
| 269 | Testican-2 | Q92563 | 0.000225736 | 802 | 11507 | 793 | 3746 |
| 270 | Prothrombin | P00734 | 0.000232728 | 118835 | 101692 | 115827 | 100580 |
| 271 | Met | P08581 | 0.000247161 | 6258 | 8647 | 6173 | 7397 |
| 272 | Transferrin | P02787 | 0.000250308 | 176979 | 146196 | 175761 | 158119 |
| 273 | SAP | P02743 | 0.000258713 | 30132 | 22049 | 29878 | 19575 |
| 274 | CD83 | Q01151 | 0.000259179 | 398 | 1179 | 314 | 891 |
| 275 | eIF-4H | Q15056 | 0.000261166 | 98495 | 59880 | 101339 | 46231 |
| 276 | Gelsolin | P06396 | 0.000262882 | 1200 | 801 | 1097 | 685 |
| 277 | IGFBP-3 | P17936 | 0.000263904 | 2137 | 1684 | 2049 | 1645 |
| 278 | MK12 | P53778 | 0.000275489 | 1688 | 871 | 1420 | 666 |
| 279 | CYTF | 076096 | 0.000280349 | 1198 | 8103 | 1127 | 4368 |
| 280 | SECTM1 | Q8WVN6 | 0.000291828 | 1144 | 1578 | 1067 | 1382 |
| 281 | RET | P07948 | 0.000293789 | 1352 | 965 | 1288 | 852 |
| 282 | SHC1 | P29353 | 0.000297374 | 49524 | 28302 | 49957 | 20221 |
| 283 | Trypsin_2 | P07478 | 0.000304996 | 2655 | 8224 | 2459 | 6486 |
| 284 | GDF2 | Q9UK05 | 0.000313339 | 1488 | 733 | 836 | 660 |
| 285 | gp130_soluble | P40189 | 0.000333147 | 6965 | 8755 | 6809 | 8123 |
| 286 | PTP-1C | P29350 | 0.000334392 | 27117 | 12283 | 18350 | 6354 |
| 287 | Aflatoxin_B1 aldehyde_reductase | 043488 | 0.000336157 | 6081 | 2402 | 5117 | 1694 |
| 288 | a1-Antitrypsin | P01009 | 0.000341517 | 600 | 781 | 583 | 731 |
| 289 | Nidogen | P14543 | 0.000354519 | 5675 | 7655 | 5279 | 7193 |
| 290 | DSC2 | Q02487 | 0.000357972 | 2600 | 3553 | 2368 | 3125 |
| 291 | PBEF | P43490 | 0.000360018 | 1770 | 4142 | 1679 | 2461 |
| 292 | ARMEL | Q49AH0 | 0.000362722 | 2963 | 2018 | 2509 | 1937 |
| 293 | resistin | Q9HD89 | 0.000376215 | 1930 | 3251 | 1821 | 2719 |
| 294 | TNR4 | P43489 | 0.000379437 | 567 | 2958 | 415 | 1404 |
| 295 | BAFF | Q9Y275 | 0.000382055 | 971 | 3244 | 855 | 2503 |
| 296 | IFN-g_R1 | P15260 | 0.000383076 | 943 | 1533 | 858 | 1294 |
| 297 | PRKACA | P17612 | 0.000412431 | 55365 | 31021 | 55173 | 21468 |
| 298 | Histone_H1.2 | P16403 | 0.000420845 | 6255 | 17180 | 5654 | 8429 |
| 299 | IL-1_R4 | Q01638 | 0.000452762 | 3042 | 21278 | 2787 | 8185 |
| 300 | UCRP | P05161 | 0.000470284 | 6829 | 23775 | 5964 | 13767 |
| 301 | CSK21 | P68400 | 0.000476701 | 196 | 376 | 181 | 230 |
| 302 | FABP | P05413 | 0.000512089 | 14812 | 32494 | 12494 | 21796 |
| 303 | Spondin-1 | Q9HCB6 | 0.000545428 | 870 | 1156 | 837 | 1055 |
| 304 | CNDP1 | Q96KN2 | 0.000549319 | 5262 | 3946 | 5306 | 3895 |
| 305 | Trefoil_factor_2 | Q03403 | 0.000573786 | 18269 | 26349 | 16612 | 23287 |
| 306 | Myeloperoxidase | P05164 | 0.000592046 | 26517 | 43657 | 23069 | 37229 |
| 307 | HXK2 | P52789 | 0.000601449 | 2126 | 5433 | 2013 | 3172 |
| 308 | CSH | P0DML2(P0DML3) | 0.000605524 | 3839 | 2519 | 3707 | 2101 |
| 309 | PLXC1 | 060486 | 0.000608545 | 662 | 872 | 659 | 813 |
| 310 | ADAMTS-4 | 075173 | 0.000619165 | 94 | 142 | 90 | 108 |
| 311 | SLPI | P03973 | 0.000623518 | 22419 | 28395 | 21865 | 25692 |
| 312 | IL-34 | Q6ZMJ4 | 0.000623743 | 538 | 665 | 503 | 660 |
| 313 | BMP_RII | Q13873 | 0.000642754 | 586 | 859 | 546 | 794 |
| 314 | Apoptosis_regulator_Bcl-W | Q92843 | 0.000645162 | 884 | 541 | 740 | 504 |
| 315 | PFD5 | Q99471 | 0.000684672 | 1462 | 4785 | 1305 | 1987 |
| 316 | HSP_90b | P08238 | 0.000713794 | 67070 | 110003 | 64016 | 90903 |
| 317 | ADAM_9 | Q13443 | 0.000719795 | 1507 | 2216 | 1410 | 1830 |
| 318 | 6Ckine | 000585 | 0.000743981 | 8371 | 13890 | 8136 | 10667 |
| 319 | BCMA | Q02223 | 0.000745571 | 8341 | 6602 | 8238 | 6590 |
| 320 | DAPK2 | Q9UIK4 | 0.000749644 | 6637 | 3794 | 5989 | 2827 |
| 321 | C7 | P10643 | 0.000775785 | 1097 | 1351 | 1060 | 1397 |
| 322 | sLeptin_R | P48357 | 0.00077659 | 2348 | 3790 | 2227 | 2976 |
| 323 | IL-17B | Q9UHF5 | 0.000802905 | 73 | 112 | 69 | 84 |
| 324 | 14-3-3_protein_zeta_delta | P63104 | 0.000808237 | 173257 | 127102 | 183783 | 111440 |
| 325 | PKC-A | P17252 | 0.000825271 | 16084 | 8724 | 13577 | 5743 |
| 326 | CgA | P10645 | 0.000842921 | 4542 | 12449 | 3212 | 7808 |
| 327 | IL-12_RB2 | Q99665 | 0.000852668 | 588 | 1201 | 518 | 794 |
| 328 | BMP-14 | P43026 | 0.000872297 | 2752 | 1628 | 2240 | 1358 |
| 329 | PTN | P21246 | 0.000875108 | 517 | 728 | 457 | 639 |
| 330 | PTHrP | P12272 | 0.000899598 | 541 | 4819 | 538 | 1901 |
| 331 | TXD12 | 095881 | 0.000911475 | 1873 | 2350 | 1739 | 2285 |
| 332 | RS3A | P61247 | 0.00098404 | 442 | 2093 | 372 | 881 |
| 333 | granzyme_A | P12544 | 0.000991485 | 2690 | 5146 | 2714 | 4052 |
| 334 | 0160_HSP_90a_b | P07900(P08238) | 0.001098767 | 17780 | 49289 | 15909 | 22674 |
| 335 | 0520_PKB_a_b_g | P31749 (P31751/Q9Y243) | 0.001113939 | 14614 | 7840 | 14358 | 4781 |
| 336 | Fransgelin-2 | P37802 | 0.001160122 | 13836 | 7661 | 12380 | 5059 |
| 337 | PTP-1B | P18031 | 0.001192604 | 4073 | 2400 | 3960 | 1796 |
| 338 | BSSP4 | Q9GZN4 | 0.001264113 | 1181 | 1718 | 1197 | 1542 |
| 339 | hnRNP_A_B | Q99729 | 0.001266875 | 1921 | 12125 | 1761 | 4597 |
| 340 | SEM6B | Q9H3T3 | 0.001275839 | 6313 | 8372 | 6344 | 7380 |
| 341 | CHK1 | 014757 | 0.001276091 | 299 | 986 | 261 | 422 |
| 342 | PCSK9 | Q8NBP7 | 0.001298983 | 428 | 558 | 415 | 531 |
| 343 | DAF | PD8174 | 0.001313838 | 15424 | 19961 | 14464 | 17881 |
| 344 | TCTP | P13693 | 0.0013524 | 34426 | 20521 | 32455 | 15439 |
| 345 | HGF | P14210 | 0.001368638 | 712 | 1623 | 730 | 1054 |
| 346 | Cathepsin_H | P09668 | 0.001382496 | 1517 | 2495 | 1369 | 2000 |
| 347 | 14-3-3E | P62258 | 0.001428972 | 3686 | 6614 | 3560 | 4252 |
| 348 | RBM39 | Q14498 | 0.001455912 | 1389 | 6450 | 1326 | 2711 |
| 349 | PK3CG | P48736 | 0.001472296 | 2181 | 1409 | 2002 | 1269 |
| 350 | IFN-a_b_R1 | P17181 | 0.001476233 | 3630 | 5311 | 3616 | 4352 |
| 351 | KPCT | Q04759 | 0.001497368 | 4676 | 2217 | 3493 | 1530 |
| 352 | RS3 | P23396 | 0.001524399 | 448 | 1755 | 284 | 675 |
| 353 | Peroxiredoxin-6 | P30041 | 0.001566019 | 621 | 372 | 557 | 281 |
| 354 | IL-1_sRII | P27930 | 000171675 | 10879 | 18407 | 10434 | 13041 |
| 355 | CRDL1 | Q9BU40 | 0.001878485 | 1990 | 2474 | 1844 | 2362 |
| 356 | Lymphotactin | P47992 | 0.00188063 | 233 | 308 | 220 | 264 |
| 357 | Nectin-like_protein_1 | Q8N126 | 0.001881921 | 3338 | 4013 | 3267 | 4017 |
| 358 | Trypsin | P07477 | 0.001890477 | 7004 | 10575 | 7275 | 8607 |
| 359 | GNS | P15586 | 0.001937987 | 3708 | 5820 | 3669 | 4225 |
| 360 | BMP-7 | P18075 | 0.001960934 | 683 | 427 | 540 | 366 |
| 361 | IL-8 | P10145 | 0.001973709 | 26823 | 5954 | 13977 | 1643 |
| 362 | SMAD3 | P84022 | 0.001983072 | 29587 | 17803 | 27822 | 12271 |
| 363 | CSRP3 | P50461 | 0.002010377 | 3315 | 1618 | 2466 | 1451 |
| 364 | Granulysin | P22749 | 0.002084426 | 2293 | 12649 | 2116 | 3788 |
| 365 | PESC | O00541 | 0.002175429 | 188 | 654 | 159 | 281 |
| 366 | HSP_70 | P0DMV8 | 0.002198822 | 11145 | 24644 | 8187 | 14113 |
| 367 | RUXF | P62306 | 0.002217412 | 1795 | 4056 | 1648 | 2267 |
| 368 | Growth_hormone_receptor | P10912 | 0.002228453 | 1451 | 1111 | 1338 | 1070 |
| 369 | IL-1_R_AcP | Q9NPH3 | 0.002308033 | 23839 | 20307 | 24042 | 20747 |
| 370 | SEPR | Q12884 | 0.002329784 | 900 | 731 | 842 | 692 |
| 371 | IMDH2 | P12268 | 0.002391131 | 3130 | 6197 | 2802 | 3629 |
| 372 | Mn SOD | P04179 | 0.002474346 | 34082 | 28765 | 31992 | 28957 |
| 373 | Cathepsin_S | P25774 | 0.00253037 | 1112 | 1707 | 1139 | 1466 |
| 374 | SiG14 | Q08ET2 | 0.002588335 | 10503 | 19449 | 9689 | 20606 |
| 375 | IL-12_Rb1 | P42701 | 0.002621541 | 344 | 473 | 328 | 368 |
| 376 | LCK | P06239 | 0.002639634 | 471 | 1692 | 448 | 676 |
| 377 | Contactin-4 | Q8IWV2 | 0.002648618 | 3605 | 3117 | 3563 | 3100 |
| 378 | LKHA4 | P09960 | 0.00266779 | 7621 | 37743 | 4035 | 4828 |
| 379 | BOC | Q9BWV1 | 0.002683881 | 1335 | 1724 | 1304 | 1578 |
| 380 | COMM07 | Q86VX2 | 0.002710768 | 9311 | 4799 | 7798 | 2906 |
| 381 | RBP | P02753 | 0.002718522 | 206 | 165 | 201 | 170 |
| 382 | BGN | P21810 | 0.002737838 | 15935 | 20412 | 15393 | 18590 |
| 383 | Fibronectin | P02751 | 0.002914316 | 11911 | 16946 | 10996 | 17220 |
| 384 | Ephrin-A2 | 043921 | 0.002938931 | 860 | 1028 | 803 | 985 |
| 385 | GREM1 | 060565 | 0.002990713 | 289 | 625 | 267 | 348 |
| 386 | DRR1 | O95990 | 0.003027104 | 160 | 188 | 154 | 175 |
| 387 | Troponin_T | P45379 | 0.003041302 | 2608 | 7521 | 2391 | 3506 |
| 388 | RS7 | P62081 | 0.003064287 | 1381 | 9254 | 1182 | 2357 |
| 389 | PSA2 | P25787 | 0.003083563 | 477 | 904 | 436 | 579 |
| 390 | SPARCL1 | Q14515 | 0.003137142 | 10300 | 13820 | 10420 | 11392 |
| 391 | SLAF5 | Q9UIB8 | 0.00323362 | 22494 | 17873 | 20105 | 17000 |
| 392 | PDE3A | Q14432 | 0.0324166 | 1861 | 1116 | 1659 | 757 |
| 393 | MCP-4 | Q99616 | 0.003245407 | 290 | 843 | 237 | 365 |
| 394 | ENPP7 | Q6UWV6 | 0.003277521 | 6874 | 16062 | 5597 | 9068 |
| 395 | MIP-1a | P10147 | 0.003305714 | 704 | 1420 | 598 | 1058 |
| 396 | MFGM | Q08431 | 0.003312438 | 961 | 1581 | 892 | 1175 |
| 397 | IGF-II_receptor | P11717 | 0.003393308 | 23367 | 26649 | 23119 | 26402 |
| 398 | sTie-1 | P35590 | 0.003538057 | 6988 | 8517 | 7009 | 8003 |
| 399 | Eotaxin-3 | Q9Y258 | 0.003565977 | 1511 | 2763 | 1267 | 1949 |
| 400 | DC-SIGNR | Q9H2X3 | 0.003567063 | 39794 | 44136 | 39133 | 43786 |
| 401 | 0586_Lymphotoxin_a2_b1 | P01374(Q06643) | 0.003602968 | 1293 | 1912 | 1291 | 1356 |
| 402 | Cofilin-1 | P23528 | 0.003659837 | 11718 | 8453 | 11378 | 6506 |
| 403 | Transketolase | P29401 | 0.00367595 | 82128 | 56936 | 82487 | 43488 |
| 404 | NSE | P09104 | 0 003681778 | 60649 | 37098 | 50482 | 25833 |
| 405 | KI2L4 | Q99706 | 0.003735144 | 854 | 7418 | 651 | 2362 |
| 406 | MIA | Q16674 | 0.003896276 | 2229 | 2803 | 2140 | 2667 |
| 407 | SAA | P0DJI8 | 0.003896354 | 1335 | 9182 | 328 | 1384 |
| 408 | Coagulation_Factor_VII | P08709 | 0.003916835 | 551 | 458 | 544 | 416 |
| 409 | Ficolin-3 | 075636 | 0.003917419 | 349 | 450 | 320 | 434 |
| 410 | SMAD2 | Q15796 | 0.003943018 | 21173 | 10710 | 18480 | 6413 |
| 411 | Karyopherin-a2 | P52292 | 0.004059809 | 442 | 1804 | 428 | 1015 |
| 412 | CSF-1 | P09603 | 0,0040852 | 4136 | 6042 | 3877 | 4640 |
| 413 | SNAA | P54920 | 0.004128854 | 12605 | 7618 | 11407 | 4888 |
| 414 | ATS13 | Q76LX8 | 0.004435453 | 3928 | 3145 | 3826 | 3196 |
| 415 | SHP-2 | Q06124 | 0.004613808 | 37249 | 21858 | 30338 | 12030 |
| 416 | PPase | Q15181 | 0.004673012 | 30475 | 48571 | 30399 | 32965 |
| 417 | Protein_disulfide_isomerase_A3 | P30101 | 0.004780658 | 7525 | 4963 | 6751 | 4030 |
| 418 | 0638_TLR4_MD-2_complex | O00206(Q9Y6Y9) | 0.00492834 | 3885 | 4666 | 3765 | 4272 |
| 419 | a2-Antiplasmin | P08697 | 0.004944612 | 1507 | 1300 | 1442 | 1290 |
| 420 | RGMB | Q6NW40 | 0.004987725 | 2085 | 2524 | 1945 | 2265 |
| 421 | BCL2-like_1_protein | Q07817 | 0 005024294 | 3039 | 1896 | 2900 | 1439 |
| 422 | BDNF | P23560 | 0.005092632 | 1126 | 757 | 1035 | 524 |
| 423 | HDGR2 | Q7Z4V5 | 0.005093058 | 418 | 941 | 380 | 511 |
| 424 | Sorting_nexin_4 | 095219 | 0.005093384 | 20664 | 10582 | 17820 | 6185 |
| 425 | B7-2 | P42081 | 0.00517096 | 1422 | 2364 | 1384 | 1786 |
| 426 | GHC2 | Q9H1K4 | 0.005239079 | 237 | 298 | 224 | 252 |
| 427 | SH21A | 060880 | 0.005243155 | 6103 | 16833 | 4064 | 8884 |
| 428 | PAI-1 | P05121 | 0.00529658 | 2428 | 1719 | 2205 | 1267 |
| 429 | CNTFR_alpha | P26992 | 0.005359993 | 7636 | 6366 | 7514 | 5826 |
| 430 | Granzyme_B | P10144 | 0.005541368 | 112 | 212 | 102 | 133 |
| 431 | 4-1BB | Q07011 | 0.005574188 | 272 | 1263 | 255 | 684 |
| 432 | HMG-1 | P09429 | 0.005675313 | 3765 | 7223 | 3382 | 4693 |
| 433 | RSPO2 | Q6UXX9 | 0.005683838 | 194 | 872 | 177 | 324 |
| 434 | COX-2 | P35354 | 0.005814634 | 454 | 772 | 386 | 562 |
| 435 | bFGF-R | P11362 | 0.005816061 | 653 | 851 | 612 | 729 |
| 436 | Triosephosphate_isomerase | P60174 | 0.006046932 | 24309 | 15441 | 24198 | 9712 |
| 437 | MIP-3a | P78556 | 0.00606173 | 150 | 362 | 134 | 167 |
| 438 | TBP | P20226 | 0.006085849 | 600 | 3133 | 509 | 1058 |
| 439 | MIG | Q07325 | 0.006185856 | 1132 | 3631 | 468 | 2337 |
| 440 | NID2 | Q14112 | 0.006227371 | 2383 | 2959 | 2273 | 2704 |
| 441 | 4EBP2 | Q13542 | 0.006242358 | 157 | 218 | 145 | 176 |
| 442 | eIF-5A-1 | P63241 | 0.006292143 | 38152 | 25532 | 37959 | 18910 |
| 443 | sICAM-3 | P32942 | 0.006411249 | 549 | 942 | 512 | 593 |
| 444 | NCC27 | 000299 | 0.006445978 | 8328 | 4663 | 7968 | 2675 |
| 445 | Ku70 | P12956 | 0.00658031 | 1112 | 5469 | 872 | 1802 |
| 446 | HMGN1 | P05114 | 0.006618388 | 478 | 696 | 443 | 577 |
| 447 | SDF-1 | P48061 | 0.006868329 | 4751 | 5947 | 4692 | 5300 |
| 448 | IGF-I | P05019 | 0.006874876 | 1150 | 957 | 1080 | 972 |
| 449 | VEGF_sR2 | P35968 | 0.006904128 | 6507 | 5726 | 6203 | 5736 |
| 450 | Chymase | P23946 | 0.006997732 | 932 | 4865 | 824 | 1513 |
| 451 | IL-20 | Q9NYY1 | 0.00710398 | 219 | 276 | 220 | 223 |
| 452 | sFRP-3 | Q92765 | 0.007119027 | 1075 | 1310 | 1056 | 1249 |
| 453 | PLCG1 | P19174 | 0.007191642 | 859 | 1771 | 776 | 936 |
| 454 | IL-1_sRI | P14778 | 0.0073365 | 10261 | 11879 | 9965 | 11080 |
| 455 | PIGR | P01833 | 0007478375 | 2385 | 5044 | 2199 | 3465 |
| 456 | H2A3 | Q7L7LO | 0.007498931 | 797 | 1632 | 699 | 918 |
| 457 | B7 | P33681 | 0.00150046 | 599 | 767 | 534 | 672 |
| 458 | CKAP2 | Q8WWK9 | 0.007680158 | 26111 | 18109 | 21408 | 15394 |
| 459 | ARI3A | Q99856 | 0.007833606 | 1773 | 1094 | 1644 | 158 |
| 460 | IL-22BP | Q969J5 | 0.00785667 | 3066 | 6198 | 2779 | 3730 |
| 461 | Factor_I | P05156 | 0.008163122 | 28221 | 31680 | 27976 | 31122 |
| 462 | PAFAH_beta_subunit | P68402 | 0.00819764 | 11104 | 7572 | 10934 | 5894 |
| 463 | c-Jun | P05412 | 0.008241514 | 4036 | 5501 | 3684 | 4527 |
| 464 | MK01 | P28482 | 0.008270633 | 6702 | 3870 | 6141 | 2796 |
| 465 | NPS-PLA2 | P14555 | 0.008307397 | 1673 | 7671 | 1589 | 3142 |
| 466 | UNC5H4 | Q6UXZ4 | 0.008520351 | 4532 | 5537 | 4495 | 5206 |
| 467 | KPCI | P41743 | 0.008628498 | 5500 | 3242 | 5071 | 1862 |
| 468 | Cathepsin_A | P10619 | 0.008823099 | 10636 | 8288 | 10747 | 7613 |
| 469 | CTACK | Q9Y4X3 | 0.008985941 | 705 | 829 | 702 | 824 |
| 470 | IL-10 | P22301 | 0.009076447 | 114 | 151 | 106 | 128 |
| 471 | PDGF_Rb | P09619 | 0.009088795 | 7707 | 10780 | 4954 | 10997 |
| 472 | RSPO3 | Q9BXY4 | 0.009147983 | 1714 | 2129 | 1612 | 2013 |
| 473 | MMP-10 | P09238 | 0.009205119 | 3162 | 4754 | 2854 | 3714 |
| 474 | TCPTP | P17706 | 0.009539443 | 239 | 342 | 227 | 245 |
| 475 | HTRA2 | 043464 | 0.009551527 | 6335 | 13343 | 5696 | 7579 |
| 476 | 0585_Lymphotoxin_a1_b2 | P01374(Q06643) | 0.00958734 | 199 | 333 | 202 | 199 |
| 477 | EG-VEGF | P58294 | 0.009652755 | 136 | 293 | 128 | 159 |
| 478 | Protease_nexin_I | P07093 | 0.009671343 | 1288 | 3398 | 1120 | 1651 |
| 479 | IMB1 | Q14974 | 0.009928414 | 73542 | 50268 | 76858 | 37464 |
| 480 | ANP | P01160 | 0.009936602 | 320 | 494 | 266 | 379 |
| 481 | FGF-12 | P61328 | 0.009959399 | 196 | 416 | 183 | 236 |
| 482 | PSMA | Q04609 | 0.00999171 | 843 | 1914 | 557 | 831 |
| 483 | Angiogenin | P03950 | 0.010126884 | 2519 | 2979 | 2427 | 2891 |
| 484 | EphA1 | P21709 | 0.010126959 | 24486 | 28675 | 25101 | 27443 |
| 485 | Albumin | P02768 | 0.010175617 | 7795 | 6890 | 7859 | 6780 |
| 486 | LDH-H_1 | P07195 | 0.010275101 | 1440 | 3965 | 1313 | 1933 |
| 487 | Thymidine_kinase | P04183 | 0.010403123 | 414 | 6621 | 369 | 1929 |
| 488 | FETUB | Q9UGM5 | 0.01045442 | 4711 | 3721 | 4760 | 3304 |
| 489 | CBX5 | P45973 | 0.010485889 | 301 | 435 | 266 | 309 |
| 490 | VEGF-D | 043915 | 0.010592915 | 759 | 618 | 672 | 583 |
| 491 | Phosphoglycerate_mutase_1 | P18669 | 0.01063914 | 16275 | 3496 | 5488 | 207 |
| 492 | Somatostatin-28 | P61278 | 0.010680994 | 677 | 895 | 612 | 867 |
| 493 | HSP70_protein_8 | P11142 | 0.010727377 | 4224 | 8518 | 3648 | 4745 |
| 494 | Cystatin_M | Q15828 | 0.010738429 | 5797 | 7390 | 5499 | 6518 |
| 495 | TGF-b1 | P01137 | 0.010843233 | 961 | 1448 | 890 | 948 |
| 496 | TAFI | Q96IY4 | 0.011158375 | 8067 | 7086 | 7958 | 7012 |
| 497 | S100A6 | P06703 | 0.011174054 | 4079 | 2840 | 4004 | 2180 |
| 498 | TLR2 | O60603 | 0.011322958 | 865 | 1196 | 803 | 944 |
| 499 | B7-H1 | Q9NZQ7 | 0.011400143 | 1357 | 2284 | 1023 | 1843 |
| 500 | SHPS1 | P78324 | 0.012047092 | 2767 | 5348 | 521 | 5096 |
| 501 | 0500_CDK8_cyclin_C | P49336(P24863) | 0012140445 | 2072 | 1372 | 1896 | 1024 |
| 502 | Coagulation_Factor_V | P12259 | 0.012290098 | 27312 | 31670 | 27355 | 31907 |
| 503 | alpha-1-antichymotrypsin_complex | P01011 | 0.012744812 | 12190 | 18778 | 11661 | 14118 |
| 504 | C2 | P06681 | 0.01282475 | 3379 | 4335 | 3026 | 3545 |
| 505 | PKC-Z | Q05513 | 0.013093847 | 783 | 8070 | 663 | 1649 |
| 506 | NKp44 | 095944 | 0.013416425 | 183 | 216 | 178 | 191 |
| 507 | Ephrin-A5 | P52803 | 0.013537141 | 4334 | 4947 | 4128 | 4862 |
| 508 | elF-5 | P55010 | 0.013787186 | 4802 | 1958 | 2130 | 1280 |
| 509 | PSP | P05451 | 0.01390103 | 484 | 773 | 385 | 552 |
| 510 | Renin | P00797 | 0.013982907 | 688 | 1186 | 562 | 776 |
| 511 | PolyUbiquitin_K63 | P0CG48 | 0.014119809 | 3413 | 5992 | 3256 | 4472 |
| 512 | LYVE1 | Q9Y5Y7 | 0.014208096 | 127 | 158 | 129 | 141 |
| 513 | MCP-2 | P80075 | 0.014584421 | 139 | 169 | 128 | 158 |
| 514 | PDXK | 000764 | 0.014794565 | 3226 | 4989 | 3215 | 3569 |
| 515 | H2B2E | Q16778 | 0.014946278 | 7584 | 17531 | 4962 | 5672 |
| 516 | MOZ | Q92794 | 0.015217425 | 1021 | 1376 | 935 | 1149 |
| 517 | HSP_60 | P10809 | 0 015331497 | 30552 | 48890 | 30669 | 29906 |
| 518 | WFKN2 | Q8TEU8 | 0.015353153 | 4085 | 5597 | 3830 | 4853 |
| 519 | Thrombopoietin_Receptor | P40238 | 0.015501567 | 185 | 133 | 149 | 117 |
| 520 | Dkk-4 | Q9UBT3 | 0.015940084 | 12065 | 9261 | 10634 | 7817 |
| 521 | PPAC | P24666 | 0.016434399 | 8732 | 4453 | 4452 | 3250 |
| 522 | Protein_S | P07225 | 0.016520993 | 3646 | 3269 | 3612 | 3249 |
| 523 | MMP-16 | P51512 | 0.016881359 | 694 | 1404 | 578 | 676 |
| 524 | PIAS4 | Q8N2W9 | 0.017666854 | 667 | 993 | 577 | 702 |
| 525 | Coagulation_Factor_XI | P03951 | 0.017761556 | 1309 | 1165 | 1311 | 1181 |
| 526 | 1116_TAK1-TAB1 | O43318(Q15750) | 0.017791097 | 3191 | 1845 | 2509 | 1229 |
| 527 | GPDA | P21695 | 0.017909898 | 6955 | 3984 | 4853 | 2922 |
| 528 | GPC5 | P78333 | 0.018131409 | 790 | 452 | 607 | 420 |
| 529 | PYY | P10082 | 0.01842469 | 742 | 977 | 655 | 898 |
| 530 | SBDS | Q9Y3A5 | 0.018592764 | 9013 | 5064 | 7163 | 3172 |
| 531 | IL-13_Ra1 | P78552 | 0.018808383 | 3133 | 3983 | 2649 | 3532 |
| 532 | calgranulin_B | P06702 | 0.018818067 | 3308 | 4269 | 3220 | 3553 |
| 533 | Carbonic_Anhydrase_IV | P22748 | 0,01901373 | 3340 | 2962 | 3237 | 2908 |
| 534 | ZAP70 | P43403 | 0.019190832 | 8335 | 10366 | 8120 | 9677 |
| 535 | MAPKAPK3 | Q16644 | 0.019570347 | 1509 | 1038 | 1431 | 736 |
| 536 | NCK1 | P16333 | 0.019612018 | 2183 | 1026 | 1521 | 843 |
| 537 | GPNMB | Q14956 | 0.019841325 | 3872 | 4703 | 3778 | 4286 |
| 538 | DYRK3 | 043781 | 0.020210463 | 1676 | 2198 | 1610 | 1813 |
| 639 | Desmoglein-1 | Q02413 | 0.020322397 | 3062 | 2643 | 2933 | 2388 |
| 540 | PPIE | Q9UNP9 | 0.020424486 | 1537 | 3279 | 1058 | 1181 |
| 541 | PA2G4 | Q9UQ80 | 0.020624369 | 4026 | 2596 | 3625 | 1670 |
| 542 | C34_gp41_HIV_Fragment | Q70626 | 0.020886378 | 565 | 1044 | 537 | 740 |
| 543 | MSP_R | Q04912 | 0.021691718 | 454 | 583 | 423 | 492 |
| 544 | ADAM12 | 043184 | 0.021719882 | 675 | 1242 | 638 | 902 |
| 545 | RAD51 | Q06609 | 0.021881977 | 2951 | 2494 | 2627 | 2331 |
| 546 | HCC-4 | 015467 | 0.021889863 | 24476 | 29273 | 25341 | 27785 |
| 547 | CLC4K | Q9UJ71 | 0.021934002 | 110 | 132 | 100 | 119 |
| 548 | IL-5_Ra | Q01344 | 0.022166829 | 2749 | 3661 | 2420 | 3157 |
| 549 | STRATiFIN | P31947 | 0.022325366 | 931 | 1265 | 881 | 1060 |
| 550 | CHL1 | 000533 | 0.022439023 | 11476 | 10431 | 11365 | 10232 |
| 551 | Neurotrophin-5 | P34130 | 0.022713973 | 111 | 143 | 103 | 120 |
| 552 | 1236_Ferritin | P02794(P02792) | 0.022727263 | 18677 | 31837 | 13178 | 23616 |
| 553 | NACA | Q13765 | 0.023049798 | 3905 | 2395 | 2954 | 1527 |
| 554 | IL22RA1 | Q8N6P7 | 0.023068379 | 404 | 571 | 350 | 429 |
| 555 | DRAK2 | 094768 | 0.023576193 | 5905 | 10255 | 5709 | 6440 |
| 556 | AURKB | Q96GD4 | 0.023838237 | 458 | 584 | 414 | 470 |
| 557 | ING1 | Q9UK53 | 0.02396779 | 8037 | 5135 | 7353 | 3255 |
| 558 | NAGK | Q9UJ70 | 0.023969289 | 1276 | 1942 | 1167 | 1210 |
| 559 | Angiotensinogen | P01019 | 0.024328408 | 4525 | 3990 | 4464 | 3960 |
| 560 | Activin_RIB | P36896 | 0.024387978 | 199 | 853 | 185 | 272 |
| 561 | Hat1 | 014929 | 0.024396057 | 214 | 407 | 183 | 213 |
| 562 | UFC1 | Q9Y3C8 | 0.02447791 | 46742 | 35585 | 46088 | 30638 |
| 563 | SPINT2 | 043291 | 0.024501196 | 919 | 755 | 854 | 700 |
| 564 | JNK2 | P45984 | 0.02450853 | 1914 | 1735 | 1904 | 1807 |
| 565 | ROB02 | Q9HCK4 | 0.024798911 | 2280 | 2672 | 2194 | 2489 |
| 566 | C3b | P01024 | 0.024806423 | 15266 | 38962 | 2283 | 7287 |
| 567 | Cardiotrophin-1 | Q16619 | 0.025822257 | 749 | 604 | 682 | 548 |
| 568 | ICOS | Q9Y6W8 | 0.025919203 | 549 | 972 | 457 | 527 |
| 569 | NEGR1 | Q7Z3B1 | 0.025955694 | 4059 | 3649 | 3885 | 3568 |
| 570 | UBE2N | P61088 | 0.026430692 | 13165 | 8757 | 11827 | 5107 |
| 571 | MAPK14 | Q16539 | 0.026633241 | 5200 | 3557 | 4978 | 2285 |
| 572 | ERAB | Q99714 | 0.027331688 | 1514 | 2688 | 1305 | 1309 |
| 573 | Troponin_I_skeletal_fast_twitch | P48788 | 0.027680155 | 6729 | 11782 | 5809 | 6921 |
| 574 | ARGI1 | P05089 | 0.027987005 | 1832 | 2429 | 1806 | 1987 |
| 575 | MSP | P26927 | 0.028174706 | 3499 | 4318 | 4083 | 4516 |
| 576 | TIG2 | Q99969 | 0.02824644 | 2420 | 2741 | 2365 | 2744 |
| 577 | B7-H2 | 075144 | 0.028323336 | 9134 | 5346 | 5100 | 3393 |
| 578 | BID | P55957 | 0.028444858 | 4718 | 3097 | 4236 | 2484 |
| 579 | FCG2A | P12318 | 0.028466156 | 964 | 2029 | 74 | 878 |
| 580 | C4b | P0C0L4(P0C0L5) | 0.028772719 | 322 | 737 | 191 | 208 |
| 581 | Noggin | Q13253 | 0.028814056 | 2209 | 1699 | 1953 | 1633 |
| 582 | contactin-1 | Q12860 | 0.029107973 | 286 | 242 | 264 | 212 |
| 583 | VEGF_sR3 | P35916 | 0.029572607 | 6240 | 7118 | 6245 | 6629 |
| 584 | FCGR1 | P12314 | 0.029721756 | 230 | 343 | 180 | 230 |
| 585 | LIN7B | Q9HAP6 | 0.02975771 | 329 | 495 | 311 | 316 |
| 586 | TRAIL_R4 | Q9UBN6 | 0.029829654 | 799 | 1142 | 647 | 854 |
| 587 | CDC37 | Q16543 | 0.029920679 | 378 | 707 | 339 | 364 |
| 588 | NRX3B | Q9HDB5 | 0.030227339 | 709 | 843 | 662 | 802 |
| 589 | IGFBP-1 | P08833 | 0.030463489 | 2103 | 2934 | 1819 | 2271 |
| 590 | Survivin | 015392 | 0.030499549 | 412 | 601 | 394 | 462 |
| 591 | Cadherin-5 | P33151 | 0.03110528 | 11734 | 13062 | 11552 | 12576 |
| 592 | CK-MM | P06732 | 0.031351237 | 998 | 645 | 896 | 425 |
| 593 | BCAM | P50895 | 0.031442102 | 1560 | 1793 | 1523 | 1629 |
| 594 | p27Kip1 | P46527 | 0.031536569 | 1007 | 3786 | 753 | 1125 |
| 595 | DBNL | Q9UJU6 | 0.031736481 | 4438 | 3442 | 3614 | 2889 |
| 596 | MATN3 | 015232 | 0.032344613 | 370 | 461 | 341 | 372 |
| 597 | PCNA | P12004 | 0.032763494 | 128 | 157 | 124 | 129 |
| 598 | NMT1 | P30419 | 0.033305568 | 2975 | 1900 | 2239 | 1450 |
| 599 | Elafin | P19957 | 0.033885412 | 10865 | 14658 | 9605 | 12030 |
| 600 | ATS15 | Q8TE58 | 0.034730954 | 235 | 277 | 220 | 239 |
| 601 | TIMP-2 | P16035 | 0.035545467 | 343 | 380 | 318 | 367 |
| 602 | PARK7 | Q99497 | 0.035730463 | 1024 | 2776 | 876 | 1120 |
| 603 | BRF-1 | Q92994 | 0.036041185 | 1580 | 2346 | 1267 | 1806 |
| 604 | PGP9.5 | P09936 | 0.036418003 | 2953 | 2178 | 2732 | 1589 |
| 605 | CYTT | P09228 | 0.036434766 | 16463 | 23655 | 15107 | 18173 |
| 606 | Activin_A | P08476 | 0.03675409 | 5575 | 7605 | 5451 | 5457 |
| 607 | Nr-CAM | Q92823 | 0.036783687 | 12953 | 15383 | 12346 | 14779 |
| 608 | MP2K2 | P36507 | 0.036847254 | 781 | 634 | 743 | 610 |
| 609 | GCP-2 | P80162 | 0.037187957 | 2215 | 3588 | 1570 | 1835 |
| 610 | BASI | P35613 | 0.037933555 | 406 | 528 | 373 | 427 |
| 611 | FGR | PD9769 | 0.038152057 | 1543 | 626 | 994 | 446 |
| 612 | CD97 | P48960 | 0.038591499 | 1543 | 1896 | 1369 | 1727 |
| 613 | IL-3 | P08700 | 0.03926097 | 983 | 1239 | 903 | 1017 |
| 614 | CHST2 | Q9Y4C5 | 0.039836327 | 195 | 251 | 170 | 190 |
| 615 | ASAHL | Q02083 | 0.039916426 | 5390 | 7998 | 4777 | 5559 |
| 616 | EphB4 | P54760 | 0.040589767 | 5264 | 6749 | 4949 | 5537 |
| 617 | Cyclin_B1 | P14635 | 0.041484643 | 243 | 363 | 214 | 279 |
| 618 | 0023_IgA | P01876(P01877) | 0.041716727 | 3471 | 4627 | 3358 | 3768 |
| 619 | CYTN | P01037 | 0.04270225 | 1075 | 1808 | 1001 | 1172 |
| 620 | FCN1 | 000602 | 0.043111936 | 4295 | 5258 | 3986 | 4341 |
| 621 | MMP-2 | P08253 | 0.043503651 | 7079 | 6552 | 7039 | 6692 |
| 622 | 14-3-3_protein_theta | P27348 | 0.044270132 | 1960 | 3068 | 1695 | 2079 |
| 623 | cGMP-stimulated_PDE | 000408 | 0.044529986 | 2739 | 2259 | 2693 | 1955 |
| 624 | MiCB | Q29980 | 0.04562974 | 1522 | 10805 | 1451 | 4316 |
| 625 | Nucleoside_diphosphate_kinase_A | P15531 | 0.045860568 | 854 | 695 | 804 | 543 |
| 626 | Ephrin-A3 | P52797 | 0.046083307 | 1469 | 1124 | 1300 | 1139 |
| 627 | IGFBP-6 | P24592 | 0.046367489 | 457 | 530 | 445 | 494 |
| 628 | PAK6 | Q9NQU5 | 0.047364051 | 369 | 710 | 311 | 337 |
| 629 | KEAP1 | Q14145 | 0.047727433 | 632 | 3411 | 551 | 994 |
| 630 | NG36 | Q96KQ7 | 0.048806117 | 502 | 408 | 486 | 374 |
| 631 | MEK1 | Q02750 | 0.048972054 | 839 | 1293 | 769 | 771 |

With respect to the proteins represented by Nos. 1, 2, 4, 5, 7, 9, 10, 11, 16, 17, 19, 20, 21, 22, 25, 28, 31, 32, 33, 37, 39, 41, 42, 43, 44, 46, 47, 48, 49, 52, 56, 57, 58, 59, 60, 61, 62, 63, 64, 65, 66, 67, 68, 70, 72, 73, 74, 75, 76, 77, 79, 80, 81, 82, 88, 89, 90, 91, 92, 93, 94, 95, 96, 97, 98, 99, 100, 101, 102, 103, 104, 106, 108, 109, 112, 113, 115, 118, 119, 120, 121, 123, 124, 125, 128, 129, 130, 131, 133, 134, 135, 139, 141, 142, 143, 144, 149, 154, 155, 156, 157, 159, 161, 164, 165, 167, 168, 171, 172, 173, 174, 175, 176, 180, 181, 182, 183, 184, 185, 187, 189, 192, 195, 196, 198, 199, 200, 201, 203, 205, 206, 207, 208, 209, 211, 214, 215, 219, 220, 221, 222, 223, 224, 225, 226, 228, 230, 231, 232, 236, 237, 238, 240, 241, 242, 243, 244, 246, 247, 248, 249, 250, 251, 252, 253, 254, 255, 256, 259, 260, 263, 264, 267, 269, 271, 274, 279, 280, 283, 285, 288, 289, 290, 291, 293, 294, 295, 296, 298, 299, 300, 301, 302, 303, 305, 306, 307, 309, 310, 311, 312, 313, 315, 316, 317, 318, 321, 322, 323, 326, 327, 329, 330, 331, 332, 333, 334, 338, 339, 340, 341, 342, 343, 345, 346, 347, 348, 350, 352, 354, 355, 356, 357, 358, 359, 364, 365, 366, 367, 371, 373, 374, 375, 376, 378, 379, 382, 383, 384, 385, 386, 387, 388, 389, 390, 393, 394, 395, 396, 397, 398, 399, 400, 401, 405, 406, 407, 409, 411, 412, 416, 418, 420, 423, 425, 426, 427, 430, 431, 432, 433, 434, 435, 437, 438, 439, 440, 441, 443, 445, 446, 447, 450, 451, 452, 453, 454, 455, 456, 457, 460, 461, 463, 465, 466, 469, 470, 471, 472, 473, 474, 475, 477, 478, 480, 481, 482, 483, 484, 486, 487, 489, 492, 493, 494, 495, 498, 499, 500, 502, 503, 504, 505, 506, 507, 509, 510, 511, 512, 513, 514, 515, 516, 518, 523, 524, 529, 531, 532, 534, 537, 538, 540, 542, 543, 544, 546, 547, 548, 549, 551, 552, 554, 555, 556, 558, 560, 561, 565, 566, 568, 572, 573, 574, 575, 576, 579, 580, 583, 584, 585, 586, 587, 588, 589, 590, 591, 593, 594, 596, 597, 599, 600, 601, 602, 603, 605, 606, 607, 609, 610, 612, 613, 614, 615, 616, 617, 618, 619, 620, 622, 624, 627, 628, 629 and 631 of Table 1, a significant increase was observed in the patient group compared to the carrier group.

Note that, Nectin-like protein 2 (UniProt ID: Q9BY67) represented by No. 62, was identical with protein of TSLCL, which is reported as a gene specifically expressed in leukemia cells taken from ATL patients, in Patent Literature 1.

In contrast, with respect to the proteins represented by Nos. 3, 6, 8, 12, 13, 14, 15, 18, 23, 24, 26, 27, 29, 30, 34, 35, 36, 38, 40, 45, 50, 51, 53, 54, 55, 69, 71, 78, 83, 84, 85, 86, 87, 105, 107, 110, 111, 114, 116, 117, 122, 126, 127, 132, 136, 137, 138, 140, 145, 146, 147, 148, 150, 151, 152, 153, 158, 160, 162, 163, 166, 169, 170, 177, 178, 179, 186, 188, 190, 191, 193, 194, 197, 202, 204, 210, 212, 213, 216, 217, 218, 227, 229, 233, 234, 235, 239, 245, 257, 258, 261, 262, 265, 266, 268, 270, 272, 273, 275, 276, 277, 278, 281, 282, 284, 286, 287, 292, 297, 304, 308, 314, 319, 320, 324, 325, 328, 335, 336, 337, 344, 349, 351, 353, 360, 361, 362, 363, 368, 369, 370, 372, 377, 380, 381, 391, 392, 402, 403, 404, 408, 410, 413, 414, 415, 417, 419, 421, 422, 424, 428, 429, 436, 442, 444 448, 449, 458, 459, 462, 464, 467, 468, 476, 479, 485, 488, 490, 491, 496, 497, 501, 508, 517, 519, 520, 521, 522, 525, 526, 527, 528, 530, 533, 535, 536, 539, 541, 545, 550, 553, 557, 559, 562, 563, 564, 567, 569, 570, 571, 577, 578, 581, 582, 592, 595, 598, 604, 608, 611, 621, 623, 625, 626 and 630 of Table 1, a significant decrease was observed in the patient group compared to the carrier group.

The p values in the increase or decrease in amount of the proteins represented by Nos. 1 to 333 were less than 0.001.

The p values in the increase or decrease in amount of the proteins represented by Nos. 334 to 482 were less than 0.01.

The p values in the increase or decrease in amount of the proteins represented by Nos. 483 to 631 were less than 0.05.

The proteins represented by Nos. 1 and 2 can be particularly useful as markers for discriminating between a carrier group and a patient group because the ranges of the protein amounts in the carrier group are not overlapped with those in the patient group.

**[Table 2]**

| Protein No. | Name of protein | UniProtID | p value | Average value in patient group | Average value in remission group | Median value in patient group | Median value in remission group |
|---|---|---|---|---|---|---|---|
| 1 | eIF-4H | Q15056 | 2.96E-08 | 59880 | 13083 | 46231 | 13652 |
| 2 | Slglec-3 | P20138 | 3.59E-08 | 3341 | 1475 | 3028 | 1483 |
| 3 | HSP_90b | P08238 | 4.55E-08 | 110003 | 30490 | 90903 | 31374 |
| 4 | NXPH1 | P58417 | 5.33E-08 | 3571 | 2321 | 3243 | 2346 |
| 5 | HIV-2_Rev | P18093 | 8.95E-08 | 127 | 84 | 116 | 81 |
| 6 | Ficolin-3 | 075636 | 9.67E-08 | 450 | 200 | 434 | 185 |
| 7 | sL-Selectin | P14151 | 9.98E-08 | 14521 | 3099 | 13032 | 2569 |
| 8 | Cofilin-1 | P23528 | 1.01E-07 | 8453 | 3073 | 6506 | 3055 |
| 9 | Nectin-like_protein_2 | Q9BY67 | 1.26E-07 | 21267 | 3690 | 17315 | 2917 |
| 10 | CD39 | P49961 | 1.43E-07 | 3096 | 1445 | 2608 | 1459 |
| 11 | CD30 | P28908 | 1.62E-07 | 23615 | 2379 | 18909 | 1901 |
| 12 | C3 | P01024 | 1.71E-07 | 58604 | 19848 | 50716 | 16918 |
| 13 | GRB2_adapter_protein | P62993 | 1.77E-07 | 73895 | 21534 | 68566 | 21878 |
| 14 | CD38 | P28907 | 1.79E-07 | 358 | 267 | 339 | 272 |
| 15 | EDAR | Q9UNE0 | 2.18E-07 | 1231 | 466 | 961 | 507 |
| 16 | IGFBP-1 | P08833 | 3.16E-07 | 2934 | 636 | 2271 | 564 |
| 11 | Cyclophilin_A | P62937 | 3.90E-07 | 133620 | 57907 | 125677 | 52657 |
| 18 | 3HAO | P46952 | 3.94E-07 | 5435 | 2202 | 4427 | 1975 |
| 19 | GPC6 | Q9Y625 | 3.96E-07 | 2115 | 1001 | 1724 | 940 |
| 20 | LRP8 | Q14114 | 6.03E-07 | 7609 | 2669 | 6194 | 2840 |
| 21 | LAG-3 | P18627 | 6.23E-07 | 64829 | 15117 | 45759 | 11577 |
| 22 | Thrombopoietin_Receptor | P40238 | 6.87E-07 | 133 | 73 | 117 | 70 |
| 23 | PAI-1 | P05121 | 6.92E-07 | 1719 | 527 | 1267 | 602 |
| 24 | AN32B | Q92688 | 7.36E-07 | 245 | 107 | 203 | 101 |
| 25 | sCD4 | P01730 | 7.77E-07 | 480 | 178 | 368 | 156 |
| 26 | Enterokinase | P98073 | 7.92E-07 | 182 | 88 | 139 | 92 |
| 27 | CONA1 | Q86Y22 | 9.09E-07 | 471 | 132 | 293 | 138 |
| 28 | TIMP-1 | P01033 | 9.17E-07 | 771 | 265 | 614 | 236 |
| 29 | NDP_kinase_B | P22392 | 9.29E-07 | 29297 | 8133 | 23692 | 7606 |
| 30 | TGF-b1 | P01137 | 9.94E-07 | 1448 | 444 | 948 | 435 |
| 31 | LY9 | Q9HBG7 | 1.07E-06 | 17254 | 8651 | 15104 | 8180 |
| 32 | XTP3A | Q9H773 | 1.14E-06 | 111104 | 33384 | 87965 | 31013 |
| 33 | IL-18_Rb | 095256 | 1.24E-06 | 217 | 124 | 199 | 127 |
| 34 | BFI_1 | Q16548 | 1.27E-06 | 947 | 518 | 780 | 523 |
| 35 | NSE | P09104 | 1.37E-06 | 37098 | 10606 | 25833 | 10849 |
| 36 | Testican-1 | Q08629 | 1.40E-06 | 3152 | 1418 | 2543 | 1310 |
| 37 | ENTP3 | 075355 | 1.69E-06 | 953 | 535 | 846 | 529 |
| 38 | MATK | P42679 | 1.72E-06 | 339 | 171 | 282 | 177 |
| 39 | RNF43 | Q68DV7 | 1.73E-06 | 1110 | 728 | 1007 | 749 |
| 40 | Histone_H1.2 | P16403 | 1.83E-06 | 17180 | 1568 | 8429 | 1808 |
| 41 | PG P9.5 | P09936 | 1.85E-06 | 2178 | 834 | 1589 | 748 |
| 42 | P-Selectin | P16109 | 2.05E-06 | 20915 | 10095 | 18423 | 9178 |
| 43 | CSF-1 | P09603 | 2.08E-06 | 6042 | 2321 | 4640 | 2000 |
| 44 | FGF9 | P31371 | 2.13E-06 | 181 | 110 | 160 | 109 |
| 45 | IL-34 | Q6ZMJ4 | 2.23E-06 | 665 | 476 | 660 | 464 |
| 46 | HPG- | P15428 | 2.42E-06 | 1964 | 940 | 1592 | 929 |
| 47 | TIM P-3 | P35625 | 2.52E-06 | 2172 | 692 | 1604 | 607 |
| 48 | Adrenomedullin | P35318 | 2.55E-06 | 2294 | 1324 | 2099 | 1284 |
| 49 | Angiopoietin-4 | Q9Y264 | 2.60E-06 | 341 | 154 | 260 | 150 |
| 50 | SHC1 | P29353 | 2.66E-06 | 28302 | 7342 | 20221 | 6450 |
| 51 | TCCR | Q6UWB1 | 2.99E-06 | 3906 | 1074 | 3121 | 1009 |
| 52 | TCTP | P13693 | 3.10E-06 | 20521 | 5155 | 15439 | 4493 |
| 53 | RPS6KA3 | P51812 | 3.15E-06 | 6104 | 1236 | 4804 | 1342 |
| 54 | MDM2 | Q00987 | 3.27E-06 | 223 | 95 | 175 | 93 |
| 55 | G-CSF-R | Q99062 | 3.32E-06 | 519 | 265 | 406 | 258 |
| 56 | PBEF | P43490 | 3.32E-06 | 4142 | 868 | 2461 | 900 |
| 57 | IL-4 | P05112 | 3.37E-06 | 408 | 217 | 342 | 222 |
| 58 | PKC-A | P17252 | 3.46E-06 | 8724 | 1505 | 5743 | 1640 |
| 59 | DEAD-box_protein_19B | Q9UMR2 | 3.49E-06 | 7070 | 1439 | 4413 | 1557 |
| 60 | Lymphotactin | P47992 | 3.72E-06 | 308 | 178 | 264 | 177 |
| 61 | IFN-lambda_2 | Q8IZJ0 | 3.89E-06 | 142 | 74 | 111 | 77 |
| 62 | PKC-B-II | P05771 | 3.92E-06 | 1744 | 399 | 1034 | 417 |
| 63 | NCK1 | P16333 | 3.96E-06 | 1026 | 493 | 843 | 470 |
| 64 | CHST6 | Q9GZX3 | 3.96E-06 | 224 | 141 | 191 | 142 |
| 65 | ARP19 | P56211 | 4.09E-06 | 833 | 267 | 585 | 254 |
| 66 | IMB1 | Q14974 | 4.10E-06 | 50268 | 14696 | 37464 | 11995 |
| 67 | Alpha_enolase | P06733 | 4.26E-06 | 51192 | 11533 | 28201 | 11010 |
| 68 | Caspase-3 | P42574 | 4.35E-06 | 37407 | 10553 | 26365 | 10791 |
| 69 | TMA | P07202 | 4.44E-06 | 1360 | 823 | 1176 | 808 |
| 70 | UFC1 | Q9Y3C8 | 4.51E-06 | 35585 | 15725 | 30638 | 14277 |
| 71 | Ephrin-A4 | P52798 | 4.51E-06 | 3341 | 1428 | 2746 | 1232 |
| 72 | CD22 | P20273 | 4.53E-06 | 754 | 383 | 611 | 379 |
| 73 | STAB2 | Q8WWQ8 | 4.68E-06 | 731 | 435 | 628 | 430 |
| 74 | PDE4D | Q08499 | 4.73E-D6 | 1184 | 639 | 1048 | 670 |
| 75 | Mammaglobin_2 | 075556 | 4.75E-06 | 12774 | 8105 | 11130 | 1750 |
| 76 | 14-3-3_protein_beta_alpha | P31946 | 4.95E-06 | 38738 | 8225 | 24139 | 7536 |
| 77 | CLC4K | Q9UJ71 | 5.43E-06 | 132 | 86 | 119 | 84 |
| 78 | SHP-2 | Q06124 | 5.67E-06 | 21858 | 4273 | 12030 | 3825 |
| 79 | B7 | P33681 | 5.77E-06 | 767 | 441 | 672 | 441 |
| 80 | eIF-5A-1 | P63241 | 5.84E-06 | 25532 | 7902 | 18910 | 6937 |
| 81 | IL-1_sR9 | Q9NP60 | 5.87E-06 | 772 | 510 | 691 | 520 |
| 82 | HDAC8 | Q9BY41 | 6.22E-06 | 2318 | 1105 | 1754 | 1121 |
| 83 | aldolase_A | P04075 | 7.11E-06 | 89055 | 40465 | 65815 | 38958 |
| 84 | METAP1 | P53582 | 7.41E-06 | 5154 | 797 | 3528 | 740 |
| 85 | HSP_60 | P10809 | 7.51E-06 | 48890 | 11351 | 29906 | 11533 |
| 86 | BARK1 | P25098 | 7.71E-06 | 15311 | 2593 | 9051 | 2509 |
| 87 | PDGF-AA | P04085 | 8.02E-06 | 10151 | 3571 | 8192 | 3666 |
| 88 | Angiopoietin-2 | 015123 | 8.05E-06 | 208 | 116 | 188 | 104 |
| 89 | PPase | Q15181 | 8.12E-06 | 48571 | 16612 | 32965 | 15512 |
| 90 | ATS15 | Q8TE58 | 8.22E-06 | 277 | 170 | 239 | 179 |
| 91 | GFRa-3 | 060609 | 8.40E-06 | 818 | 344 | 591 | 302 |
| 92 | Lamin-B1 | P20700 | 8.81E-06' | 780 | 330 | 566 | 319 |
| 93 | PDGF-BB | P01127 | 8.84E-06 | 32971 | 11714 | 28252 | 9721 |
| 94 | IL-1Ra | P18510 | 8.93E-06 | 10397 | 4299 | 9693 | 4411 |
| 95 | IL-1F6 | Q9UHA7 | 8.96E-06 | 5750 | 839 | 2975 | 779 |
| 96 | Glutamate_carboxypeptidase | Q96KP4 | 9.00E-06 | 929 | 448 | 686 | 432 |
| 97 | AMPM2 | P50579 | 9.12E-06 | 32565 | 6707 | 18381 | 6599 |
| 98 | Transketolase | P29401 | 9.16E-06 | 56936 | 20957 | 43488 | 17581 |
| 99 | GV | P39877 | 9.22E-06 | 302 | 155 | 255 | 150 |
| 100 | FGF-20 | Q9NP95 | 9.47E-06 | 279 | 169 | 250 | 164 |
| 101 | UFM1 | P61960 | 9.90E-06 | 17440 | 5527 | 14129 | 4699 |
| 102 | C3a | P01024 | 1.04E-05 | 1196 | 226 | 746 | 180 |
| 103 | NET4 | Q9H863 | 1.09E-05 | 1107 | 598 | 897 | 634 |
| 104 | Gro- b_g | P19876_P | 1.09E-05 | 874 | 291 | 690 | 288 |
| 105 | 14-3-3_protein_zeta_delta | P63104 | 1.10E-05 | 127102 | 43703 | 111440 | 43314 |
| 106 | EPO-R | P19235 | 1.10E-05 | 223 | 114 | 187 | 116 |
| 107 | TrkA | P04629 | 1.12E-05 | 2249 | 1038 | 1850 | 999 |
| 108 | PRKACA | P17612 | 1.15E-05 | 31021 | 7621 | 21468 | 6580 |
| 109 | amyloid_precursor_protein | P05067 | 1.26E-05 | 35364 | 16014 | 30562 | 14292 |
| 110 | RAC3 | P60763 | 1.26E-05 | 5251 | 1183 | 3402 | 1042 |
| 111 | hnRNP_A2_B1 | P22626 | 1.33E-05 | 51483 | 8555 | 28985 | 8638 |
| 112 | TGF-b3 | P10600 | 1.35E-05 | 434 | 218 | 365 | 227 |
| 113 | FST | P19883 | 1.38E-05 | 591 | 259 | 467 | 240 |
| 114 | DBNL | Q9UJU6 | 1.39E-05 | 3442 | 1179 | 2889 | 1685 |
| 215 | GFRa-1 | P56159 | 1.41E-05 | 729 | 333 | 599 | 297 |
| 116 | OCAD1 | Q9NX40 | 1.42E-05 | 1337 | 693 | 1095 | 690 |
| 117 | DRR1 | 095990 | 1.42E-05 | 188 | 136 | 175 | 141 |
| 118 | Ephrin-B3 | Q15768 | 1.44E-05 | 2004 | 356 | 660 | 358 |
| 119 | HSP.90a_b | P07900_P | 1.47E-05 | 49289 | 5564 | 22674 | 5920 |
| 120 | Sialoadhesin | Q98ZZ2 | 1.48E-05 | 170 | 90 | 135 | 90 |
| 121 | TSLP | Q969D9 | 1.55E-05 | 328 | 146 | 258 | 137 |
| 122 | annexin_VI | P08133 | 1.58E-05 | 1373 | 587 | 923 | 596 |
| 123 | CBG | P08185 | 1.59E-05 | 455 | 234 | 356 | 229 |
| 124 | XPNPEP1 | Q9NQW7 | 1.61E-05 | 25362 | 4856 | 12767 | 4733 |
| 125 | CRK | P46108 | 1.66E-05 | 24824 | 11195 | 20254 | 12563 |
| 226 | Mcl-1 | Q07820 | 1.73E-05 | 2374 | 850 | 1685 | 819 |
| 127 | sICAM-2 | P13598 | 1.74E-05 | 3676 | 1130 | 2962 | 1005 |
| 128 | Carbonic_Anhydrase_X | Q9NS85 | 1.79E-05 | 457 | 351 | 442 | 352 |
| 129 | Thyroglobulin | P01266 | 1.84E-05 | 139 | 88 | 124 | 89 |
| 130 | PCSK7 | Q16549 | 1.94E-05 | 3683 | 1864 | 3483 | 2004 |
| 131 | ERK-1 | P27361 | 2.00E-05 | 34390 | 9384 | 22959 | 9550 |
| 132 | Triosephosphate_isomerase | P60174 | 2.01E-05 | 15441 | 4399 | 9712 | 3785 |
| 133 | MATN3 | 015232 | 2.09E-05 | 461 | 273 | 372 | 274 |
| 134 | MBD4 | 095243 | 2.15E-05 | 345 | 163 | 267 | 163 |
| 135 | CSK21 | P68400 | 2.22E-05 | 376 | 147 | 230 | 140 |
| 136 | FGF-17 | 060258 | 2.35E-05 | 168 | 111 | 153 | 107 |
| 137 | RXFP1 | Q9HBX9 | 2.35E-05 | 236 | 167 | 213 | 166 |
| 138 | TBK1 | Q9UHD2 | 2.36E-05 | 197 | 106 | 154 | 107 |
| 139 | HPV_E7_Type_16 | P03129 | 2.39E-05 | 254 | 120 | 175 | 124 |
| 140 | IL-10 | P22301 | 2.47E-05 | 151 | 87 | 128 | 91 |
| 141 | SMAD3 | P84022 | 2.52E-05 | 17803 | 5179 | 12271 | 4823 |
| 142 | JAM-C | Q9BX67 | 2.52E-05 | 6316 | 2031 | 4563 | 1677 |
| 143 | Bone_proteoglycan_II | P07585 | 2.53E-05 | 6278 | 3593 | 5598 | 3724 |
| 144 | Cathepsin_G | P08311 | 2.57E-05 | 973 | 365 | 626 | 303 |
| 145 | TNF_sR-II | P20333 | 2.60E-05 | 51875 | 18432 | 47932 | 15777 |
| 146 | RAC1 | P63000 | 2.62E-05 | 21753 | 5888 | 15050 | 5017 |
| 147 | PHI | P06744 | 2.63E-05 | 63563 | 24015 | 44995 | 21688 |
| 148 | SMOC1 | Q9H4F8 | 2.77E-05 | 4539 | 2496 | 3970 | 2412 |
| 149 | CSK | P41240 | 2.82E-05 | 11134 | 1923 | 7851 | 1774 |
| 150 | VEGF-C | P49767 | 2.84E-05 | 2166 | 884 | 1633 | 937 |
| 151 | MIF | P14174 | 2.89E-05 | 3241 | 1003 | 2060 | 976 |
| 152 | B7-H2 | 075144 | 2.94E-05 | 5346 | 1634 | 3393 | 1551 |
| 153 | BDNF | P23560 | 2.95E-05 | 757 | 266 | 524 | 254 |
| 154 | AURKB | Q96GD4 | 2.98E-05 | 584 | 363 | 470 | 350 |
| 155 | Transgelin-2 | P37802 | 3.05E-05 | 7661 | 2090 | 5059 | 2121 |
| 156 | ATS1 | Q9UHI8 | 3.18E-05 | 313 | 122 | 232 | 120 |
| 157 | NKp44 | 095944 | 3.18E-05 | 216 | 134 | 191 | 123 |
| 158 | Glutathione_S-transferase_Pi | P09211 | 3.25E-05 | 12831 | 6421 | 9783 | 6163 |
| 159 | CATC | P53634 | 3.29E-05 | 508 | 335 | 447 | 344 |
| 160 | MFRP | Q9BY79 | 3.32E-05 | 1917 | 716 | 1459 | 692 |
| 161 | GHC2 | Q9H1K4 | 3.35E-05 | 298 | 174 | 252 | 172 |
| 162 | YES | P07947 | 3.36E-05 | 9249 | 1010 | 3871 | 992 |
| 163 | GPC2 | Q8N158 | 3.44E-05 | 177 | 88 | 138 | 91 |
| 164 | DLRBL | Q9NP97 | 3.45E-05 | 9687 | 1977 | 6135 | 2121 |
| 165 | EF-1-beta | P24534 | 3.60E-05 | 1024 | 325 | 707 | 310 |
| 166 | CTAP-III | P02775 | 3.71E-05 | 15674 | 6308 | 12758 | 6534 |
| 167 | IL22RA1 | Q8N6P7 | 3.83E-05 | 571 | 269 | 429 | 264 |
| 168 | EPI | 014944 | 4.04E-05 | 995 | 575 | 876 | 566 |
| 169 | Rab_GDP_dissociation_inhibitor_beta | P50395 | 4.05E-05 | 30401 | 11671 | 20375 | 11853 |
| 170 | PCCAM-1 | P16284 | 4.07E-05 | 847 | 441 | 650 | 444 |
| 171 | MMP-17 | Q9ULZ9 | 4.12E-05 | 1505 | 581 | 1004 | 536 |
| 172 | PSA1 | P25786 | 4.19E-05 | 772 | 290 | 536 | 291 |
| 173 | MIP-3b | Q99731 | 4.40E-05 | 4608 | 1264 | 3413 | 1423 |
| 174 | CD226 | Q15762 | 5.01E-05 | 1249 | 738 | 1123 | 794 |
| 175 | c-Jun | P05412 | 5.02E-05 | 5501 | 2672 | 4527 | 2588 |
| 176 | BCL2-like_1_protein | Q07817 | 5.07E-05 | 1896 | 711 | 1439 | 682 |
| 177 | GSK-3 alpha_beta | P49840_P | 5.12E-05 | 10979 | 2332 | 6683 | 2263 |
| 178 | ULBP-3 | Q9BZM4 | 5.12E-05 | 803 | 314 | 573 | 326 |
| 279 | GOT1 | P17174 | 5.26E-05 | 7872 | 4161 | 6144 | 3972 |
| 180 | TSLP_R | Q9HC73 | 5.33E-05 | 1700 | 609 | 1164 | 582 |
| 181 | IL-17D | Q8TAD2 | 5.36E-05 | 336 | 114 | 224 | 117 |
| 182 | CLC18 | Q9P126 | 5.63E-05 | 9118 | 4299 | 8172 | 4159 |
| 183 | IL-17B | Q9UHF5 | 5.63E-05 | 112 | 63 | 84 | 62 |
| 184 | MRCKB | Q9Y5S2 | 5.75E-05 | 1473 | 730 | 1070 | 694 |
| 185 | IL-2_sRg | P31785 | 5.76E-05 | 5307 | 1509 | 3552 | 1299 |
| 186 | SPTA2 | Q 13813 | 5.85E-05 | 146 | 105 | 128 | 103 |
| 187 | Aggrecan | P16112 | 5.95E-05 | 996 | 596 | 886 | 538 |
| 188 | HSP_27 | P04792 | 6.29E-05 | 3465 | 827 | 2040 | 703 |
| 189 | MOZ | Q92794 | 6.40E-05 | 1376 | 702 | 1149 | 643 |
| 190 | PDXK | 000764 | 6.43E-05 | 4989 | 1888 | 3569 | 1833 |
| 191 | C1QBP | Q07021 | 6.43E-05 | 4817 | 3571 | 4492 | 3716 |
| 192 | DHH | 043323 | 6.45E-05 | 1175 | 379 | 820 | 344 |
| 193 | PSA2 | P25787 | 6.50E-05 | 904 | 299 | 579 | 276 |
| 194 | FGF7 | P21781 | 6.57E-05 | 1620 | 468 | 1133 | 421 |
| 195 | PPIB | P23284 | 6.83E-05 | 2552 | 1271 | 2154 | 1162 |
| 196 | Macrophage_scavenger_receptor | P21757 | 6.84E-05 | 809 | 400 | 624 | 379 |
| 197 | CKAP2 | Q8WWK9 | 6.84E-05 | 18109 | 10908 | 15394 | 11424 |
| 198 | LYVE1 | Q9Y5Y7 | 6.86E-05 | 158 | 95 | 141 | 95 |
| 199 | MSP R | Q04912 | 6.88E-05 | 583 | 341 | 492 | 321 |
| 200 | CPNE1 | Q99829 | 6.91E-05 | 2978 | 731 | 1503 | 680 |
| 201 | Clusterin | P10909 | 6.93E-05 | 1021 | 682 | 968 | 681 |
| 202 | IFN-aA | P01563 | 7.04E-05 | 91 | 66 | 84 | 68 |
| 203 | annexin_II | P07355 | 7.06E-05 | 18696 | 4432 | 10033 | 4257 |
| 204 | CD97 | P48960 | 7.10E-05 | 1896 | 1284 | 1727 | 1299 |
| 205 | IMDH2 | P12268 | 7.14E-05 | 6197 | 1956 | 3629 | 1773 |
| 206 | ING1 | Q9UK53 | 7.15E-05 | 5135 | 1212 | 3255 | 972 |
| 207 | IL-1b | P01584 | 7.16E-05 | 1095 | 443 | 780 | 417 |
| 208 | IL-23_R | Q5VWK5 | 7.20E-05 | 21977 | 1145 | 7781 | 1140 |
| 209 | Cystatin_M | Q15828 | 7.23E-05 | 7390 | 4364 | 6518 | 4415 |
| 210 | Vasoactive_Intestinal_Peptide | P01282 | 7.24E-05 | 304 | 128 | 227 | 123 |
| 211 | MK08 | P45983 | 7.43E-05 | 2230 | 452 | 1343 | 454 |
| 212 | H31 | P68431 | 7.47E-05 | 48429 | 20030 | 32732 | 19264 |
| 213 | IF4G2 | P78344 | 7.64E-05 | 11532 | 1994 | 8048 | 1614 |
| 214 | TCPTP | P17706 | 7.72E-05 | 342 | 176 | 245 | 169 |
| 215 | CCL28 | Q9NRJ3 | 7.74E-05 | 9334 | 6421 | 8826 | 6391 |
| 216 | SIRT2 | Q8IXJ6 | 8.00E-05 | 17662 | 4953 | 10020 | 4535 |
| 217 | IL-15_Ra | Q13261 | 8.07E-05 | 3088 | 996 | 2028 | 838 |
| 218 | PAFAH_beta_subunit | P68402 | 8.21E-05 | 7572 | 3185 | 5894 | 2994 |
| 219 | Flt-3 | P36888 | 8.22E-05 | 10432 | 1394 | 4541 | 1524 |
| 220 | Thrombospondin-1 | PD7996 | 8.23E-05 | 1710 | 534 | 1515 | 466 |
| 221 | PSME1 | Q06323 | 8.23E-05 | 1861 | 690 | 1053 | 632 |
| 222 | phosphoglycerate_kinase_1 | P00558 | 8.43E-05 | 11725 | 4548 | 8020 | 4880 |
| 223 | BAD | Q92934 | 8.52E-05 | 10828 | 2798 | 7425 | 2349 |
| 224 | COX-2 | P35354 | 8.58E-05 | 772 | 300 | 562 | 277 |
| 225 | CaMKK_alpha | Q8N5S9 | 8.64E-05 | 441 | 184 | 319 | 167 |
| 226 | STK16 | 075716 | 8.73E-05 | 1680 | 1172 | 1574 | 1137 |
| 227 | KREM2 | Q8NCW0 | 8.90E-05 | 3922 | 1248 | 2578 | 1163 |
| 228 | SUM03 | P55854 | 9.01E-05 | 8300 | 2138 | 5149 | 2084 |
| 229 | PSA6 | P60900 | 9.57E-05 | 1025 | 303 | 702 | 265 |
| 230 | Angiopoietin-1 | Q15389 | 9.68E-05 | 893 | 450 | 737 | 489 |
| 231 | CTACK | Q9Y4X3 | 9.99E-05 | 829 | 540 | 824 | 533 |
| 232 | AK1A1 | P14550 | 1.01E-04 | 9545 | 2862 | 6281 | 2493 |
| 233 | DnaJ_homolog | Q96DA6 | 1.03E-04 | 426 | 177 | 323 | 182 |
| 234 | PTP-1C | P29350 | 1.04E-04 | 12283 | 1707 | 6354 | 1808 |
| 235 | FGF-16 | 043320 | 1.05E-04 | 3417 | 884 | 2295 | 776 |
| 236 | Card iotroph in-1 | Q16619 | 1.06E-04 | 604 | 394 | 548 | 364 |
| 237 | sICAM-3 | P32942 | 1.06E-04 | 942 | 353 | 593 | 350 |
| 238 | IL-6. sRa | P08887 | 1.07E-04 | 24838 | 11859 | 19547 | 11268 |
| 239 | IL-3...Ra | P26951 | 1.08E-04 | 1297 | 452 | 860 | 368 |
| 240 | ALT | P24298 | 1.09E-04 | 5831 | 2523 | 4096 | 2663 |
| 241 | CAMK2D | Q13557 | 1.09E-04 | 8705 | 1861 | 4562 | 1675 |
| 242 | Cadherin-12 | P55289 | 1.10E-04 | 769 | 334 | 592 | 369 |
| 243 | Bcl-2 | P10415 | 1.12E-04 | 1335 | 464 | 918 | 432 |
| 244 | PFD5 | Q99471 | 1.12E-04 | 4785 | 905 | 1987 | 804 |
| 245 | RELT | Q969Z4 | 1.16E-04 | 5248 | 2166 | 4144 | 2010 |
| 246 | OSM | P13725 | 1.18E-04 | 1555 | 1092 | 1531 | 1049 |
| 247 | MEPE | Q9NQ76 | 1.19E-04 | 204 | 109 | 150 | 110 |
| 248 | EP15R | Q9UBC2 | 1.21E-04 | 1311 | 310 | 913 | 277 |
| 249 | eIF-5 | P55010 | 1.22E-04 | 1958 | 743 | 1280 | 690 |
| 250 | Protein_disulfide_isomerase_A3 | P30101 | 1.23E-04 | 4963 | 2664 | 4030 | 2587 |
| 251 | RUXF | P62306 | 1.25E-04 | 4056 | 1127 | 2267 | 1040 |
| 252 | CAMK1D | Q8IU85 | 1.25E-04 | 1690 | 1044 | 1457 | 1050 |
| 253 | CBX5 | P45973 | 1.25E-04 | 435 | 231 | 309 | 222 |
| 254 | Ubiquitin | P62979 | 1.29E-04 | 1678 | 796 | 1284 | 724 |
| 255 | UCRP | P05161 | 1.30E-04 | 23775 | 4394 | 13767 | 3413 |
| 256 | iC3b | P01024 | 1.30E-04 | 27030 | 9848 | 14769 | 9797 |
| 257 | PA2G4 | Q9UQ80 | 1.32E-04 | 2596 | 773 | 1670 | 768 |
| 258 | HEMK2 | Q9Y5N5 | 1.32E-04 | 4709 | 1404 | 3132 | 1153 |
| 259 | NAP-2 | P02775 | 1.35E-04 | 5529 | 2290 | 4331 | 2351 |
| 260 | UBC9 | P63279 | 1.36E-04 | 7343 | 1382 | 3961 | 1435 |
| 261 | AGR2 | 095994 | 1.41E-04 | 610 | 441 | 542 | 432 |
| 262 | CYTF | 076096 | 1.43E-04 | 8103 | 785 | 4368 | 617 |
| 263 | Cyclophilin_F | P30405 | 1.45E-04 | 24068 | 2970 | 12930 | 2616 |
| 264 | PLK-1 | P53350 | 1.46E-04 | 153 | 80 | 119 | 79 |
| 265 | ARI3A | Q99856 | 1.46E-04 | 1094 | 306 | 758 | 255 |
| 266 | 14-3-3_protein_theta | P27348 | 1.47E-04 | 3068 | 896 | 2079 | 845 |
| 267 | MP2K2 | P36507 | 1.49E-04 | 634 | 426 | 610 | 432 |
| 268 | ENA-78 | P42830 | 1.49E-04 | 580 | 246 | 385 | 243 |
| 269 | IL-1_sRII | P27930 | 1.53E-04 | 18407 | 8592 | 13041 | 8103 |
| 270 | HSP70_protein_8 | P11142 | 1.55E-04 | 8518 | 1528 | 4745 | 1542 |
| 271 | SLAF6 | Q96DU3 | 1.58E-04 | 4686 | 756 | 2552 | 727 |
| 272 | PAFAH | Q13093 | 1.59E-04 | 591 | 381 | 581 | 363 |
| 273 | BCAR3 | 075815 . | 1.61E-04 | 1527 | 868 | 1390 | 798 |
| 274 | EGF | P01133 | 1.61E-04 | 893 | 591 | 811 | 556 |
| 275 | UBE2N | P61088 | 1.61E-04 | 8757 | 2473 | 5107 | 2212 |
| 276 | GDF-9 | 060383 | 1.62E-04 | 329 | 158 | 238 | 163 |
| 277 | Nucleoside_diphosphate_kinase_A | P15531 | 1.63E-04 | 695 | 387 | 543 | 342 |
| 278 | ILT-2 | Q8NHL6 | 1.65E-04 | 5021 | 2632 | 5017 | 2401 |
| 279 | HNRPQ | 060506 | 1.67E-04 | 5279 | 1608 | 3119 | 1542 |
| 280 | DRG-1 | Q9NP79 | 1.69E-04 | 27691 | 7688 | 19676 | 6544 |
| 281 | LIN7B | Q9HAP6 | 1.69E-04 | 495 | 193 | 316 | 198 |
| 282 | SNAA | P54920 | 1.75E-04 | 7618 | 2726 | 4888 | 2472 |
| 283 | AIP | 000170 | 1.75E-04 | 5465 | 974 | 3117 | 796 |
| 284 | PDE11 | Q9HCR9 | 1.76E-04 | 1784 | 1119 | 1424 | 1063 |
| 285 | Carbonic_anhydrase_9 | Q 16790 | 1.77E-04 | 611 | 295 | 438 | 307 |
| 286 | SP-D | P35247 | 1.77E-04 | 86912 | 26885 | 55092 | 24995 |
| 287 | SOD | P00441 | 1.83E-04 | 1588 | 461 | 920 | 444 |
| 288 | MEK1 | Q02750 | 1.84E-04 | 1293 | 395 | 771 | 367 |
| 289 | KPCI | P41743 | 1.85E-04 | 3242 | 766 | 1862 | 656 |
| 290 | PIGF | P49763 | 1.87E-04 | 435 | 302 | 413 | 304 |
| 291 | CAMK2B | Q13554 | 1.87E-04 | 3576 | 742 | 1920 | 632 |
| 292 | SBDS | Q9Y3A5 | 1.88E-04 | 5064 | 943 | 3172 | 704 |
| 293 | CFC1 | P0CG37 | 1.89E-04 | 3180 | 1328 | 2424 | 1165 |
| 294 | BAFF Receptor | Q96RJ3 | 1.89E-04 | 2167 | 1485 | 1882 | 1494 |
| 295 | FCGR1 | P12314 | 1.90E-04 | 343 | 157 | 230 | 137 |
| 296 | CD83 | Q01151 | 1.91E-04 | 1179 | 392 | 891 | 396 |
| 297 | Testican-2 | Q92563 | 1.92E-04 | 11507 | 657 | 3746 | 542 |
| 298 | HDGR2 | Q7Z4V5 | 1.93E-04 | 941 | 220 | 511 | 225 |
| 299 | DPP2 | Q9UHL4 | 1.95E-04 | 1929 | 1287 | 1714 | 1206 |
| 300 | ABL2 | P42684 | 1.96E-04 | 2048 | 1081 | 1632 | 917 |
| 301 | Stress-induced-phosphoprotein_1 | P31948 | 2.01E-04 | 8766 | 2910 | 5855 | 2341 |
| 302 | RS3 | P23396 | 2.03E-04 | 1755 | 213 | 675 | 232 |
| 303 | SKP1 | P63208 | 2.03E-04 | 3571 | 1335 | 2550 | 1211 |
| 304 | Plasmin | P00747 | 2.03E-04 | 542 | 311 | 425 | 277 |
| 305 | LAG-1 | Q8NHW4 | 2.04E-04 | 3351 | 1237 | 2575 | 1006 |
| 306 | Midkine | P21741 | 2.07E-04 | 627 | 351 | 485 | 358 |
| 307 | SCGF-alpha | Q9Y240 | 2.07E-04 | 9954 | 3101 | 7496 | 2249 |
| 308 | PGM1 | P36871 | 2.16E-04 | 527 | 281 | 449 | 273 |
| 309 | SCGF-beta | Q9Y240 | 2.19E-04 | 3514 | 1173 | 2575 | 906 |
| 310 | NCC27 | 000299 | 2.23E-04 | 4663 | 955 | 2675 | 907 |
| 311 | UB2L3 | P68036 | 2.24E-04 | 2578 | 693 | 1712 | 590 |
| 312 | STAT1 | P42224 | 2.26E-04 | 9781 | 1942 | 5702 | 1784 |
| 313 | PPIE | Q9UNP9 | 2.27E-04 | 3279 | 507 | 1181 | 544 |
| 314 | SH21A | 060880 | 2.28E-04 | 16833 | 3058 | 8884 | 2713 |
| 315 | AREG | P15514 | 2.31E-04 | 3762 | 1018 | 2548 | 935 |
| 326 | Myeloperoxidase | P05164 | 2.32E-04 | 43657 | 18226 | 37229 | 19275 |
| 317 | RSK-like_protein_kinase | 075582 | 2.32E-04 | 235 | 75 | 157 | 71 |
| 318 | MMEL2 | Q495T6 | 2.32E-04 | 1026 | 618 | 907 | 589 |
| 319 | BMP-14 | P43026 | 2.32E-04 | 1628 | 779 | 1358 | 778 |
| 320 | CAMK2A | Q9UQM7 | 2.36E-04 | 1607 | 350 | 903 | 317 |
| 321 | PF-4 | P02776 | 2.38E-04 | 2358 | 1240 | 1895 | 1305 |
| 322 | IL-12_RB2 | Q99665 | 2.39E-04 | 1201 | 517 | 794 | 551 |
| 323 | LRIG3 | Q6UXM1 | 2.42E-04 | 6659 | 3486 | 5063 | 3755 |
| 324 | b-ECGF | P05230 | 2.42E-04 | 102 | 67 | 95 | 69 |
| 325 | MDHC | P40925 | 2.44E-04 | 32745 | 13421 | 20041 | 13117 |
| 326 | PLPP | Q96GD0 | 2.55E-04 | 5908 | 1387 | 3750 | 1198 |
| 327 | IFN-lambda_1 | Q8IU54 | 2.56E-04 | 406 | 199 | 321 | 183 |
| 328 | H6ST1 | 060243 | 2.56E-04 | 2209 | 1305 | 2094 | 1381 |
| 329 | STXla | Q16623 | 2.60E-04 | 567 | 197 | 324 | 188 |
| 330 | IL-16 | Q14005 | 2.66E-04 | 1140 | 399 | 729 | 374 |
| 331 | ERBB2 | P04626 | 2.70E-04 | 370 | 212 | 295 | 198 |
| 332 | KIF23 | Q02241 | 2.74E-04 | 1408 | 464 | 912 | 413 |
| 333 | Myoglobin | P02144 | 2.75E-04 | 2008 | 943 | 1610 | 924 |
| 334 | M2-PK | P14618 | 2.77E-04 | 31274 | 8626 | 19958 | 8412 |
| 335 | Keratin_18 | P05783 | 2.88E-04 | 874 | 425 | 682 | 381 |
| 336 | PLCG1 | P19174 | 2.97E-04 | 1771 | 499 | 936 | 487 |
| 337 | HMG-1 | P09429 | 2.97E-04 | 7223 | 2409 | 4693 | 2340 |
| 338 | HGF | P14210 | 2.99E-04 | 1623 | 484 | 1054 | 413 |
| 339 | ART | 000253 | 2.99E-04 | 3077 | 1305 | 2321 | 1143 |
| 340 | PAK3 | 075914 | 3.04E-04 | 544 | 422 | 514 | 424 |
| 341 | MAPK14 | Q16539 | 3.13E-04 | 3557 | 1211 | 2285 | 898 |
| 342 | MO2R1 | Q8TD46 | 3.23E-04 | 1967 | 1178 | 1942 | 1108 |
| 343 | HO-2 | P30519 | 3.24E-04 | 726 | 155 | 418 | 161 |
| 344 | ON | Pp9486 | 3.32E-04 | 27995 | 14784 | 22916 | 16860 |
| 345 | Hat1 | 014929 | 3.37E-04 | 407 | 95 | 213 | 96 |
| 346 | LCK | P06239 | 3.38E-04 | 1692 | 200 | 676 | 184 |
| 347 | CHK1 | 014757 | 3.42E-04 | 986 | 212 | 422 | 224 |
| 348 | FAK1 | Q05397 | 3.43E-04 | 281 | 193 | 237 | 187 |
| 349 | Semaphorin_3A | Q14563 | 3.43E-04 | 1019 | 436 | 756 | 427 |
| 350 | MIC-1 | Q99988 | 3.43E-04 | 4137 | 1674 | 3431 | 1197 |
| 351 | NMT1 | P30419 | 3.55E-04 | 1900 | 732 | 1450 | 734 |
| 352 | EPHA3 | P29320 | 3.63E-04 | 157 | 71 | 109 | 70 |
| 353 | CDC37 | Q16543 | 3.65E-04 | 707 | 149 | 364 | 142 |
| 354 | PKB_beta | P31751 | 3.70E-04 | 18970 | 5785 | 11739 | 6014 |
| 355 | Dkk-4 | Q9UBT3 | 3.79E-04 | 9261 | 5803 | 7817 | 6079 |
| 356 | GIIE | Q9NZK7 | 3.79E-04 | 195 | 110 | 155 | 116 |
| 357 | HSP_70 | P0DMV8 | 3.81E-04 | 24644 | 1537 | 14113 | 6000 |
| 358 | R8M39 | Q14498 | 3.88E-04 | 6450 | 716 | 2711 | 653 |
| 359 | Kallikrein_6 | Q92876 | 3.90E-04 | 2075 | 1400 | 1809 | 1391 |
| 360 | TSP2 | P35442 | 3.92E-04 | 76766 | 20845 | 64305 | 13654 |
| 361 | CHIP | Q9UNE7 | 3.92E-04 | 5201 | 826 | 2686 | 715 |
| 362 | WNK3 | Q9BYP7 | 3.97E-04 | 3503 | 614 | 1513 | 568 |
| 363 | C3b | P01024 | 4.04E-04 | 38962 | 2363 | 7287 | 2307 |
| 364 | PESC | 000541 | 4.11E-04 | 654 | 108 | 281 | 111 |
| 365 | TNR4 | P43489 | 4.21E-04 | 2958 | 578 | 1404 | 522 |
| 366 | PKC-G | P05129 | 4.27E-04 | 392 | 248 | 316 | 257 |
| 367 | Moesin | P26038 | 4.27E-04 | 2600 | 744 | 1490 | 691 |
| 368 | GAPDH_liver | P04406 | 4.29E-04 | 82299 | 35464 | 58066 | 32269 |
| 369 | ACE2 | Q9BYF1 | 4.34E-04 | 515 | 258 | 391 | 269 |
| 370 | Osteocalcin | P02818 | 4.49E-04 | 2412 | 1094 | 1626 | 1148 |
| 311 | CD40_ligand_soluble | P29965 | 4.56E-04 | 331 | 151 | 262 | 128 |
| 372 | Tropomyosin_4 | P67936 | 4.58E-04 | 45669 | 12022 | 32924 | 6580 |
| 373 | PKB_a__b_g | P31749_P | 4.64E-04 | 7840 | 2411 | 4781 | 2297 |
| 374 | DKK1 | 094907 | 4.71E-04 | 21314 | 11500 | 18407 | 11828 |
| 375 | FLRT1 | Q9NZU1 | 4.79E-04 | 953 | 515 | 767 | 564 |
| 376 | Lymphotoxin_a2_b1 | P01374_Q | 4.80E-04 | 1912 | 1030 | 1356 | 984 |
| 377 | IL-3 | P08700 | 4.83E-04 | 1239 | 719 | 1017 | 634 |
| 378 | Caspase-10 | Q92851 | 4.86E-04 | 4040 | 2705 | 3447 | 2667 |
| 379 | ANGL4 | Q9BY76 | 4.87E-04 | 3359 | 861 | 2019 | 730 |
| 380 | Cyclin_B1 | P14635 | 4.99E-04 | 363 | 178 | 279 | 178 |
| 381 | DLC8 | P63167 | 5.06E-04 | 394 | 162 | 248 | 161 |
| 382 | Lymphotoxin b_R | P36941 | 5.08E-04 | 7039 | 5286 | 7098 | 4974 |
| 383 | PDE7A | Q13946 | 5.14E-04 | 395 | 221 | 309 | 205 |
| 384 | Notch_1 | P46531 | 5.17E-04 | 28986 | 17956 | 26793 | 16912 |
| 385 | Siglec-6 | 043699 | 5.19E-04 | 222 | 112 | 178 | 103 |
| 386 | MAPKAPK3 | Q16644 | 5.35E-04 | 1038 | 433 | 736 | 411 |
| 387 | RS3A | P61247 | 5.38E-04 | 2093 | 333 | 881 | 264 |
| 388 | PDE1A | P54750 | 5.42E-04 | 1328 | 466 | 886 | 407 |
| 389 | HXK2 | P52789 | 5.55E-04 | 5433 | 1773 | 3172 | 1522 |
| 390 | ERAB | Q99714 | 5.58E-04 | 2688 | 682 | 1309 | 585 |
| 391 | COMMD7 | Q86VX2 | 5.60E-04 | 4799 | 1763 | 2906 | 2068 |
| 392 | sICAM-5 | Q9UMF0 | 5.77E-04 | 800 | 536 | 743 | 556 |
| 393 | Topoisomerase_I | P11387 | 5.93E-04 | 525 | 255 | 332 | 251 |
| 394 | Ubiquitin+1 | P62979 | 5.94E-04 | 5857 | 1389 | 2617 | 1306 |
| 395 | NSF1C | Q9UNZ2 | 5.96E-04 | 5127 | 1516 | 3551 | 1246 |
| 396 | alpha-1-antichymotrypsin_complex | P01011 | 5.99E-04 | 18778 | 7309 | 14118 | 6532 |
| 397 | MPIF-1 | P55773 | 6.16E-04 | 4013 | 2261 | 3557 | 2287 |
| 398 | FGF-5 | P12034 | 6.26E-04 | 602 | 317 | 430 | 307 |
| 399 | SGTA | 043765 | 6.32E-04 | 7163 | 2075 | 5353 | 1603 |
| 400 | ASGR1 | P07306 | 6.42E-04 | 11296 | 4701 | 9349 | 4114 |
| 401 | Esterase_D | P10768 | 6.48E-04 | 19092 | 8907 | 13720 | 8417 |
| 402 | TRAIL_R4 | Q9UBN6 | 6.52E-04 | 1142 | 681 | 854 | 732 |
| 403 | PTP-1B | P18031 | 6.53E-04 | 2400 | 1098 | 1796 | 1080 |
| 404 | HTRA2 | 043464 | 6.54E-04 | 13343 | 3838 | 7579 | 3563 |
| 405 | sFRP-3 | Q92765 | 6.55E-04 | 1310 | 974 | 1249 | 925 |
| 406 | Eotaxin-3 | Q9Y258 | 6.57E-04 | 2763 | 1243 | 1949 | 1224 |
| 407 | Stanniocalcin-1 | P52823 | 6.58E-04 | 3923 | 1944 | 2988 | 2060 |
| 408 | Calpain_I | P07384_P | 6.69E-04 | 24715 | 11135 | 16307 | 9931 |
| 409 | NMGR | P04035 | 6.81E-04 | 220 | 145 | 191 | 137 |
| 410 | Gro-a | PD9341 | 6.87E-04 | 6161 | 2142 | 4494 | 1842 |
| 411 | CLC7A | Q9BXN2 | 6.98E-04 | 731 | 355 | 588 | 297 |
| 412 | C3adesArg | P01024 | 7.08E-04 | 146004 | 88831 | 146867 | 77440 |
| 413 | GM-CSF | P04141 | 7.15E-04 | 349 | 187 | 255 | 182 |
| 414 | SEM5A | Q13591 | 7.36E-04 | 7795 | 4021 | 7187 | 3670 |
| 415 | 14-3-3 | P31946_P | 7.41E-04 | 8254 | 1381 | 4054 | 1184 |
| 416 | SPINT2 | 043291 | 7.52E-04 | 755 | 427 | 700 | 383 |
| 417 | STAT3 | P40763 | 7.67E-04 | 2801 | 714 | 1681 | 587 |
| 418 | IL-1F7 | Q9NZH6 | 7.73E-04 | 2127 | 1398 | 1870 | 1233 |
| 419 | Carbonic_anhydrase_XIII | Q8N1Q1 | 7.83E-04 | 13975 | 4081 | 7746 | 2942 |
| 420 | DYRK3 | 043781 | 7.84E-04 | 2198 | 1324 | 1813 | 1331 |
| 421 | Cytochrome_P450_3A4 | P08684 | 7.86E-04 | 10956 | 6571 | 9409 | 6346 |
| 422 | PDE3A | Q14432 | 8.04E-04 | 1116 | 433 | 757 | 339 |
| 423 | IMDH1 | P20839 | 8.06E-04 | 13261 | 4917 | 10092 | 4835 |
| 424 | GPVI | Q9HCN6 | 8.16E-04 | 11840 | 4466 | 9825 | 3439 |
| 425 | PTHrP | P12272 | 8.18E-04 | 4819 | 497 | 1901 | 441 |
| 426 | IL-7 | P13232 | 8.24E-04 | 99 | 69 | 87 | 66 |
| 427 | Integrin_a1b1 | P56199_P | 8.52E-04 | 7443 | 993 | 2633 | 862 |
| 428 | DUS3 | P51452 | 8.61E-04 | 1696 | 598 | 1269 | 596 |
| 429 | sE-Selectin | P16581 | 8.83E-04 | 70072 | 31853 | 61266 | 29209 |
| 430 | TNF_sR-I | P19438 | 9.00E-04 | 2831 | 1317 | 2512 | 1270 |
| 431 | NAGK | Q9UJ70 | 9.08E-04 | 1942 | 803 | 1210 | 756 |
| 432 | Siglec-7 | Q9Y286 | 9.14E-04 | 1877 | 1069 | 1797 | 1001 |
| 433 | NLGNX | Q8N0W4 | 9.42E-04 | 2851 | 985 | 1708 | 839 |
| 434 | TACI | 014836 | 9.52E-04 | 1417 | 856 | 1164 | 774 |
| 435 | CNTF | P26441 | 9.54E-04 | 120 | 89 | 118 | 84 |
| 436 | M-CSF_R | P07333 | 9.56E-04 | 218 | 111 | 189 | 107 |
| 437 | EphB6 | 015197 | 9.60E-04 | 2773 | 1441 | 2193 | 1413 |
| 438 | Peroxiredoxin-1 | Q06830 | 9.81E-04 | 3767 | 1322 | 2136 | 1129 |
| 439 | ENPP7 | Q6UWV6 | 9.83E-04 | 16062 | 5012 | 9068 | 4893 |
| 440 | Azurocidin | P20160 | 9.89E-04 | 443 | 139 | 279 | 107 |
| 441 | MK01 | P28482 | 1.00E-03 | 3870 | 1379 | 2796 | 1379 |
| 442 | JAG1 | P78504 | 1.04E-03 | 880 | 448 | 659 | 370 |
| 443 | SRCN1 | P12931 | 1.05E-03 | 26542 | 6767 | 18881 | 4197 |
| 444 | hnRNP_A_B | Q99729 | 1.08E-03 | 12125 | 1735 | 4597 | 1657 |
| 445 | RAD51 | Q06609 | 1.10E-03 | 2494 | 1848 | 2331 | 1894 |
| 446 | ALCAM | Q13740 | 1.11E-03 | 21624 | 12230 | 19555 | 10577 |
| 447 | FCN1 | 000602 | 1.13E-03 | 5258 | 2935 | 4341 | 2821 |
| 448 | IL-10_Ra | Q13651 | 1.13E-03 | 1056 | 895 | 1043 | 911 |
| 449 | Tropomyosin_2 | P07951 | 1.15E-03 | 1534 | 1161 | 1439 | 1149 |
| 450 | Granzyme_H | P20718 | 1.16E-03 | 174 | 110 | 142 | 118 |
| 451 | C4b | P0C0L4_P | 1.18E-03 | 737 | 114 | 208 | 106 |
| 452 | IL-18_Ra | Q13478 | 1.18E-03 | 19642 | 9436 | 17346 | 9229 |
| 453 | Granzyme_B | P10144 | 1.20E-03 | 212 | 94 | 133 | 90 |
| 454 | SLAF5 | Q9UIB8 | 1.21E-03 | 17873 | 8768 | 17000 | 7090 |
| 455 | TNF-b | P01374 | 1.21E-03 | 532 | 230 | 357 | 227 |
| 456 | CAD15 | P55291 | 1.22E-03 | 9448 | 5457 | 8022 | 5209 |
| 457 | LKHA4 | P09960 | 1.23E-03 | 37743 | 4208 | 4828 | 1423 |
| 458 | H2B2E | Q16778 | 1.26E-03 | 17531 | 4074 | 5672 | 3695 |
| 459 | SE6L2 | Q6UXD5 | 1.26E-03 | 1618 | 574 | 1022 | 421 |
| 460 | MAPK2 | P49137 | 1.27E-03 | 3772 | 1564 | 2774 | 1353 |
| 461 | EFNB1 | P98172 | 1.29E-03 | 2773 | 1447 | 2569 | 1160 |
| 462 | HSP_40 | P25685 | 1.32E-03 | 520 | 160 | 330 | 128 |
| 463 | NUDC3 | Q8IVD9 | 1.34E-03 | 1867 | 335 | 974 | 323 |
| 464 | LD78-beta | P16619 | 1.34E-03 | 3755 | 2106 | 3165 | 1939 |
| 465 | prostatic_binding_protein | P30086 | 1.35E-03 | 7591 | 3115 | 4769 | 2994 |
| 466 | RS7 | P62081 | 1.36E-03 | 9254 | 653 | 2357 | 611 |
| 467 | AIF1 | P55008 | 1.36E-03 | 4150 | 2397 | 3567 | 2647 |
| 468 | Kallistatin | P29622 | 1.39E-03 | 24304 | 29753 | 24164 | 29686 |
| 469 | GFRa-2 | 000451 | 1.40E-03 | 7646 | 3809 | 6748 | 3149 |
| 470 | Survivin | 015392 | 1.42E-03 | 601 | 310 | 462 | 307 |
| 471 | MICA | Q29983 | 1.43E-03 | 583 | 156 | 495 | 76 |
| 472 | EphB4 | P54760 | 1.43E-03 | 6749 | 3991 | 5537 | 3705 |
| 473 | ICOS | Q9Y6W8 | 1.46E-03 | 972 | 347 | 527 | 304 |
| 474 | Periostin | Q15063 | 1.47E-03 | 2916 | 2043 | 2682 | 2010 |
| 475 | FYN | P06241 | 1.50E-03 | 11344 | 2955 | 7392 | 2450 |
| 416 | BASI | P35613 | 1.52E-03 | 528 | 326 | 427 | 323 |
| 477 | FCN2 | Q 15485 | 1.52E-03 | 1148 | 681 | 979 | 608 |
| 478 | DC-SIGNR | Q9H2X3 | 1.55E-03 | 44136 | 36127 | 43786 | 36712 |
| 479 | RANTES | P13501 | 1.56E-03 | 27994 | 15543 | 22737 | 16877 |
| 480 | SPHK2 | Q9NRA0 | 1.60E-03 | 583 | 322 | 459 | 308 |
| 481 | LDH-H_1 | P07195 | 1.63E-03 | 3965 | 802 | 1933 | 805 |
| 482 | ROBO3 | Q96MS0 | 1.65E-03 | 283 | 201 | 244 | 188 |
| 483 | a1-Antitrypsin | P01009 | 1.66E-03 | 781 | 546 | 731 | 547 |
| 484 | Ferritin | P02794 , P | 1.67E-03 | 31837 | 13854 | 23616 | 12080 |
| 485 | CRISS | P54108 | 1.68E-03 | 372 | 149 | 202 | 161 |
| 486 | GCP-2 | P80162 | 1.71E-03 | 3588 | 1305 | 1835 | 1324 |
| 487 | HPV_E7_Type18 | P06788 | 1.73E-03 | 1990 | 1276 | 1685 | 1164 |
| 488 | Galectin-2 | P05162 | 1.75E-03 | 915 | 527 | 816 | 466 |
| 489 | TIMD3 | Q8TDQO | 1.76E-03 | 18301 | 9012 | 17638 | 8435 |
| 490 | Protease_nexin_I | P07093 | 1.77E-03 | 3398 | 802 | 1651 | 785 |
| 491 | MMP-7 | P09237 | 1.81E-03 | 4838 | 2509 | 3617 | 2311 |
| 492 | IL-1_R4 | Q01638 | 1.82E-03 | 21278 | 5080 | 8185 | 4171 |
| 493 | ZAP70 | P43403 | 1.84E-03 | 10366 | 6944 | 9677 | 6592 |
| 494 | SMAD2 | Q15796 | 1.85E-03 | 10710 | 2376 | 6413 | 1949 |
| 495 | FSTL1 | Q12841 | 1.85E-03 | 38451 | 22593 | 37095 | 20478 |
| 496 | IF4A3 | P38919 | 1.93E-03 | 828 | 315 | 419 | 294 |
| 497 | ERBB4 | Q15303 | 1.96E-03 | 113 | 68 | 93 | 68 |
| 498 | ETHE1 | 095571 | 1.99E-03 | 1672 | 1325 | 1656 | 1269 |
| 499 | DcR3 | 095407 | 1.99E-03 | 467 | 332 | 418 | 330 |
| 500 | B7-2 | P42081 | 2.00E-03 | 2364 | 1258 | 1786 | 1346 |
| 501 | CD48 | P09326 | 2.03E-03 | 1844 | 876 | 1745 | 740 |
| 502 | PIK3CA_PIK3R1 | P42336_P | 2.03E-03 | 2069 | 592 | 1032 | 607 |
| 503 | Granulysin | P22749 | 2.04E-03 | 12649 | 2272 | 3788 | 2388 |
| 504 | Cathepsin_B | P07858 | 2.09E-03 | 3185 | 1847 | 2766 | 1873 |
| 505 | PIAS4 | QSN2W9 | 2.11E-03 | 993 | 518 | 702 | 437 |
| 506 | NACA | Q13765 | 2.13E-03 | 2395 | 971 | 1527 | 902 |
| 507 | FLRT3 | Q9NZU0 | 2.15E-03 | 8436 | 5049 | 7111 | 4875 |
| 508 | Neurotrophin-5 | P34130 | 2.28E-03 | 143 | 94 | 120 | 90 |
| 509 | FCRL3 | Q96P31 | 2.33E-03 | 783 | 322 | 550 | 334 |
| 510 | GP114 | Q8IZF4 | 2.34E-03 | 670 | 310 | 438 | 310 |
| 511 | Cathepsin_H | P09668 | 2.35E-03 | 2495 | 1557 | 2000 | 1560 |
| 512 | a2-Macroglobulin | P01023 | 2.37E-03 | 24438 | 12026 | 14058 | 10984 |
| 513 | VAV | P15498 | 2.40E-03 | 15041 | 3843 | 10974 | 3714 |
| 514 | ATP_synthase_beta_chain | P06576 | 2.41E-03 | 3430 | 939 | 1600 | 848 |
| 515 | MP2K3 | P46734 | 2.44E-03 | 1352 | 660 | 989 | 630 |
| 516 | sLeptin_R | P48357 | 2.45E-03 | 3790 | 1982 | 2976 | 1680 |
| 517 | tPA | P00750 | 2.47E-03 | 779 | 477 | 650 | 477 |
| 518 | CD59 | P13987 | 2.49E-03 | 2652 | 1564 | 2445 | 1349 |
| 519 | Ku70 | P12956 | 2.49E-03 | 5469 | 574 | 1802 | 539 |
| 520 | CSRP3 | P50461 | 2.53E-03 | 1618 | 966 | 1451 | 848 |
| 521 | H2A3 | Q7L7L0 | 2.62E-03 | 1632 | 684 | 918 | 674 |
| 522 | QORL1 | 095825 | 2.67E-03 | 654 | 291 | 401 | 286 |
| 523 | Contactin-5 | 094779 | 2.70E-03 | 388 | 297 | 370 | 312 |
| 524 | Chitotriosidase-1 | Q13231 | 2.73E-03 | 2109 | 1241 | 1826 | 1231 |
| 525 | PKC-D | Q05655 | 2.78E-03 | 6917 | 2479 | 4256 | 2469 |
| 526 | BPI | P17213 | 2.78E-03 | 9449 | 2778 | 7838 | 1264 |
| 527 | PDPK1 | 015530 | 2.80E-03 | 2712 | 503 | 1724 | 453 |
| 528 | GRN | P28799 | 2.83E-03 | 37334 | 23478 | 36614 | 26818 |
| 529 | sCD163 | Q86VB7 | 2.83E-03 | 7606 | 4160 | 7419 | 4233 |
| 530 | GI24 | Q9H7M9 | 2.87E-03 | 1060 | 389 | 637 | 332 |
| 531 | LY86 | 095711 | 2.98E-03 | 6045 | 4008 | 5154 | 3688 |
| 532 | RAP | P30533 | 2.98E-03 | 1031 | 663 | 799 | 635 |
| 533 | STAT6 | P42226 | 2.98E-03 | 1224 | 470 | 768 | 396 |
| 534 | HVEM | Q92956 | 3.03E-03 | 544 | 326 | 461 | 283 |
| 535 | RSPO2 | Q6UXX9 | 3.12E-03 | 872 | 142 | 324 | 144 |
| 536 | Karyopherin-a2 | P52292 | 3.13E-03 | 1804 | 390 | 1015 | 403 |
| 537 | BTK | Q06187 | 3.20E-03 | 6791 | 1573 | 3854 | 1261 |
| 538 | TNFSF15 | 095150 | 3.20E-03 | 7169 | 3053 | 6800 | 2181 |
| 539 | ADAM_9 | Q13443 | 3.33E-03 | 2216 | 1448 | 1830 | 1363 |
| 540 | GNS | P15586 | 3.35E-03 | 5820 | 3403 | 4225 | 2921 |
| 541 | FGFR4 | P22455 | 3.35E-03 | 1433 | 625 | 901 | 603 |
| 542 | MK11 | Q15759 | 3.36E-03 | 653 | 481 | 618 | 455 |
| 543 | PPID | Q08752 | 3.40E-03 | 3899 | 370 | 1938 | 288 |
| 544 | TAK1-TAB1 | 043318_Q | 3.45E-03 | 1845 | 582 | 1229 | 592 |
| 545 | sTie-2 | Q02763 | 3.50E-03 | 1901 | 1206 | 1733 | 1115 |
| 546 | DRAK2 | 094768 | 3.55E-03 | 10255 | 4278 | 6440 | 4235 |
| 547 | ALK-1 | P37023 | 3.67E-03 | 1645 | 689 | 1134 | 666 |
| 548 | Collectin_Kidney_1 | Q9BWP8 | 3.67E-03 | 5886 | 3496 | 4747 | 3258 |
| 549 | CAPG | P40121 | 3.69E-03 | 3656 | 1504 | 1999 | 1497 |
| 550 | LRRT3 | Q86VH5 | 3.72E-03 | 177 | 103 | 141 | 99 |
| 551 | FGF-6 | P10767 | 3.74E-03 | 663 | 144 | 322 | 143 |
| 552 | PDE5A | 076074 | 3.83E-03 | 8278 | 3018 | 5262 | 2105 |
| 553 | TF | P13726 | 3.91E-03 | 1714 | 1094 | 1608 | 1032 |
| 554 | TBP | P20226 | 3.91E-03 | 3133 | 459 | 1058 | 507 |
| 555 | FGF-10 | 015520 | 3.92E-03 | 254 | 105 | 171 | 102 |
| 556 | CTGF | P29279 | 4.04E-03 | 1879 | 1020 | 1309 | 1051 |
| 557 | CDK8__cyclin_C | P49336_P | 4.05E-03 | 1372 | 810 | 1024 | 760 |
| 558 | CD63 | P08962 | 4.08E-03 | 813 | 390 | 548 | 358 |
| 559 | ADAM12 | 043184 | 4.09E-03 | 1242 | 523 | 902 | 494 |
| 560 | fiCkine | 000585 | 4.19E-03 | 13890 | 8313 | 10667 | 7993 |
| 561 | Lymphotoxin_a1_b2 | P01374_Q | 4.20E-03 | 333 | 163 | 199 | 164 |
| 562 | EMR2 | Q9UHX3 | 4.29E-03 | 1159 | 511 | 838 | 396 |
| 563 | JAM-B | P57087 | 4.48E-03 | 7764 | 4661 | 6620 | 4293 |
| 564 | PAK6 | Q9NQU5 | 4.50E-03 | 710 | 217 | 337 | 208 |
| 565 | EG-VEGF | P58294 | 4.55E-03 | 293 | 119 | 159 | 110 |
| 566 | IL-20 | Q9NYY1 | 4.56E-03 | 276 | 191 | 223 | 183 |
| 567 | Met | P08581 | 4.60E-03 | 8647 | 5342 | 7397 | 4852 |
| 568 | VCAM-1 | P19320 | 4.67E-03 | 22797 | 11821 | 21005 | 9359 |
| 569 | VEGF | P15692 | 4.68E-03 | 10202 | 7761 | 10088 | 8168 |
| 570 | kallikrein_13 | Q9UKR3 | 4.81E-03 | 853 | 616 | 733 | 586 |
| 571 | PIANP | Q8IYJ0 | 4.93E-03 | 4886 | 3394 | 4654 | 3394 |
| 572 | SLIK1 | Q96PX8 | 5.00E-03 | 1475 | 867 | 1203 | 808 |
| 573 | IL24 | Q13007 | 5.21E-03 | 336 | 88 | 176 | 88 |
| 574 | Troponin_T | P45379 | 5.24E-03 | 7521 | 2500 | 3506 | 2069 |
| 575 | MCP-4 | Q99616 | 5.28E-03 | 843 | 273 | 365 | 204 |
| 576 | C5a | P01031 | 5.33E-03 | 9090 | 5894 | 8015 | 5625 |
| 511 | PolyUbiquitin_K48 | P0CG47 | 5.37E-03 | 2336 | 1333 | 1722 | 1274 |
| 578 | I-309 | P22362 | 5.51E-03 | 1543 | 1313 | 1550 | 1327 |
| 579 | Chymase | P23946 | 5.57E-03 | 4865 | 812 | 1513 | 814 |
| 580 | FSTL3 | 095633 | 5.60E-03 | 17397 | 9274 | 13552 | 9903 |
| 581 | kallikrein_5 | Q9Y337 | 5.69E-03 | 103 | 77 | 95 | 84 |
| 582 | IL-5_Ra | Q01344 | 5.73E-03 | 3661 | 1838 | 3157 | 1542 |
| 583 | IL-27 | Q8NEV9_O | 5.73E-03 | 567 | 453 | 531 | 477 |
| 584 | S100A4 | P26447 | 5.74E-03 | 5416 | 2364 | 2905 | 2220 |
| 585 | BMP10 | 095393 | 5.95E-03 | 3443 | 2034 | 2834 | 1896 |
| 586 | LEAP-1 | P81172 | 6.10E-03 | 24042 | 11085 | 23780 | 9498 |
| 587 | CK2-A1:B | P68400_P | 6.13E-03 | 2297 | 601 | 1067 | 560 |
| 588 | LYNB | P07948 | 6.15E-03 | 43072 | 17546 | 32330 | 13323 |
| 589 | FGF-12 | P61328 | 6.26E-03 | 416 | 182 | 236 | 183 |
| 590 | Cathepsin_S | P25774 | 6.33E-03 | 1707 | 955 | 1466 | 827 |
| 591 | AMGO2 | Q86SJ2 | 6.36E-03 | 2231 | 1352 | 1856 | 1212 |
| 592 | Sorting_nexin_4 | 095219 | 6.50E-03 | 10582 | 2713 | 6185 | 1571 |
| 593 | RNase_H1 | 060930 | 6.71E-03 | 427 | 173 | 203 | 140 |
| 594 | 4-188 | Q07011 | 6.71E-03 | 1263 | 285 | 684 | 224 |
| 595 | PCI | P05154 | 6.77E-03 | 33122 | 53518 | 31322 | 50794 |
| 596 | Arylsulfatase_A | P15289 | 6.87E-03 | 2074 | 1531 | 1876 | 1540 |
| 597 | NKp46 | 076036 | 6.89E-03 | 3456 | 2168 | 2754 | 2272 |
| 598 | PK3CG | P48736 | 6.91E-03 | 1409 | 960 | 1269 | 967 |
| 599 | CD47 | Q08722 | 6.95E-03 | 740 | 352 | 538 | 327 |
| 600 | IL-12_Rb1 | P42701 | 6.99E-03 | 473 | 285 | 368 | 261 |
| 601 | JNK2 | P45984 | 7.03E-03 | 1735 | 876 | 1807 | 889 |
| 602 | VEGF121 | P15692 | 7.06E-03 | 1001 | 647 | 843 | 602 |
| 603 | RSPO4 | Q2I0M5 | 7.15E-03 | 490 | 293 | 385 | 275 |
| 604 | FGF-18 | 076093 | 7.17E-03 | 123 | 82 | 106 | 80 |
| 605 | a-Synuclein | P37840 | 7.20E-03 | 4858 | 2187 | 3185 | 1886 |
| 606 | IL-18_BPa | 095998 | 7.30E-03 | 12067 | 7298 | 10985 | 7356 |
| 607 | Fibronectin | P02751 | 7.55E-03 | 16946 | 21457 | 17220 | 21675 |
| 608 | IL-5 | P05113 | 7.60E-03 | 192 | 111 | 151 | 117 |
| 609 | IL-7_Ra | P16871 | 7.65E-03 | 246 | 181 | 215 | 170 |
| 610 | CXCL16_soluble | Q9H2A7 | 7.72E-03 | 14073 | 8759 | 11890 | 7781 |
| 611 | TLR4_MD-2_complex | O00206_Q | 7.77E-03 | 4666 | 3311 | 4272 | 3242 |
| 612 | ERBB3 | P21860 | 7.93E-03 | 290 | 207 | 268 | 185 |
| 613 | RAN | P62826 | 8.05E-03 | 3130 | 1430 | 1704 | 1313 |
| 614 | IGFBP-7 | Q16270 | 8.07E-03 | 66232 | 42954 | 67309 | 39232 |
| 615 | NID2 | Q14112 | 8.28E-03 | 2959 | 1921 | 2704 | 1921 |
| 616 | Apo_D | P05090 | 8.42E-03 | 4818 | 3975 | 5010 | 4011 |
| 617 | IL-22BP | Q969J5 | 8.42E-03 | 6198 | 2446 | 3730 | 3180 |
| 618 | Osteopontin | P10451 | 8.58E-03 | 49729 | 22902 | 42709 | 19256 |
| 619 | suPAR | Q03405 | 8.60E-03 | 20841 | 13431 | 18794 | 13931 |
| 620 | IL-10_Rb | Q08334 | 8.66E-03 | 1750 | 1109 | 1548 | 894 |
| 621 | KI2L4 | Q99706 | 8.87E-03 | 7418 | 1443 | 2362 | 988 |
| 622 | TEC | P42680 | 8.88E-03 | 1693 | 509 | 1133 | 464 |
| 623 | PKC-Z | Q05513 | 8.94E-03 | 8070 | 358 | 1649 | 346 |
| 624 | IDE | P14735 | 8.99E-03 | 3001 | 1640 | 1894 | 1519 |
| 625 | IFN-a_b_R1 | P17181 | 9.41E-03 | 5311 | 3832 | 4352 | 3633 |
| 626 | Nidogen | P14543 | 9.43E-03 | 7655 | 4499 | 7193 | 4406 |
| 627 | SREC-I | Q14162 | 9.49E-03 | 8611 | 4615 | 7882 | 3473 |
| 628 | Galectin-4 | P56470 | 9.54E-03 | 479 | 282 | 412 | 231 |
| 629 | C2 | P06681 | 9.68E-03 | 4335 | 2578 | 3545 | 2180 |
| 630 | EMAP-2 | Q 12904 | 9.74E-03 | 2379 | 1706 | 1947 | 1617 |
| 631 | CSH | P0DML2_ | 9.77E-03 | 2519 | 1672 | 2101 | 1497 |
| 632 | DLL4 | Q9NR61 | 9.93E-03 | 1254 | 916 | 1193 | 864 |
| 633 | Ephrin-A3 | P52797 | 9.96E-03 | 1124 | 958 | 1139 | 970 |
| 634 | DAPK2 | Q9UIK4 | 1.01E-02 | 3794 | 2028 | 2827 | 1840 |
| 635 | Aflatoxin_B1_aldehyde_reductase | 043488 | 1.02E-02 | 2402 | 702 | 1694 | 537 |
| 636 | Dynactin_subunit_2 | Q13561 | 1.02E-02 | 1563 | 995 | 1412 | 864 |
| 637 | IL-17_RD | Q8NFM7 | 1.02E-02 | 363 | 234 | 276 | 250 |
| 638 | protein_Z_inhibitor | Q9U K55 | 1.03E-02 | 9241 | 7101 | 8378 | 6850 |
| 639 | IFN-g_R1 | P15260 | 1.04E-02 | 1533 | 927 | 1294 | 751 |
| 640 | Growth_hormone_receptor | P10912 | 1.06E-02 | 1111 | 812 | 1070 | 744 |
| 641 | FBLN3 | Q12805 | 1.07E-02 | 2178 | 1374 | 1806 | 1209 |
| 642 | FN1.4 | P02751 | 1.07E-02 | 106550 | 114894 | 103495 | 114256 |
| 643 | SMAC | Q9NR28 | 1.12E-02 | 5085 | 3678 | 4606 | 3295 |
| 644 | MMP-1 | P03956 | 1.14E-02 | 1328 | 759 | 900 | 720 |
| 645 | Laminin | P25391_P | 1.17E-02 | 2353 | 1671 | 1908 | 1560 |
| 646 | BID | P55957 | 1.19E-02 | 3097 | 1644 | 2484 | 1617 |
| 647 | SARP-2 | Q8N474 | 1.23E-02 | 843 | 635 | 725 | 702 |
| 648 | HHLA2 | Q9UM44 | 1.24E-02 | 1964 | 1349 | 1749 | 1371 |
| 649 | PARK7 | Q99497 | 1.24E-02 | 2776 | 678 | 1120 | 713 |
| 650 | Thymidine_kinase | P04183 | 1.24E-02 | 6621 | 576 | 1929 | 557 |
| 651 | Cytidylate kinase | P30085 | 1.27E-02 | 6009 | 3736 | 5177 | 3199 |
| 652 | LG3BP | QQ$380 | 1.28E-02 | 1790 | 1042 | 1559 | 1057 |
| 653 | HMGN1 | P05114 | 1.30E-02 | 696 | 486 | 577 | 511 |
| 654 | C8 | P07357_P | 1.31E-02 | 2696 | 2134 | 2612 | 2172 |
| 655 | LIF_sR | P42702 | 1.33E-02 | 2074 | 1755 | 2028 | 1702 |
| 656 | C34_gp41 HIV_Fragment | Q70626 | 1.34E-02 | 1044 | 513 | 740 | 581 |
| 657 | Histone_H2A.z | P0C0S5 | 1.34E-02 | 457 | 152 | 250 | 154 |
| 658 | Olfactomedin-4 | Q6UX06 | 1.35E-02 | 178 | 85 | 103 | 70 |
| 659 | Noggin | Q13253 | 1.35E-02 | 1699 | 1237 | 1633 | 1226 |
| 660 | IL-19 | Q9UHD0 | 1.40E-02 | 5037 | 3903 | 5094 | 3797 |
| 661 | Antithrombin_III | P01008 | 1.40E-02 | 79109 | 88300 | 79532 | 90222 |
| 662 | CAMK1 | Q 14012 | 1.40E-02 | 4572 | 3416 | .4192 | 3773 |
| 663 | Proteinase-3 | P24158 | 1.41E-02 | 20677 | 8074 | 15463 | 6704 |
| 664 | Apo_B | P04114 | 1.41E-02 | 13897 | 5483 | 5154 | 5570 |
| 665 | Sphingosine_kinase_1 | Q9NYA1 | 1.43E-02 | 5492 | 1366 | 3129 | 837 |
| 666 | FGF-4 | P08620 | 1.46E-02 | 279 | 84 | 157 | 82 |
| 667 | SPARCL1 | Q 14515 | 1.47E-02 | 13820 | 10588 | 11392 | 10353 |
| 668 | Sonic_Hedgehog | Q15465 | 1.51E-02 | 694 | 1269 | 666 | 1225 |
| 669 | Aminoacylase-1 | Q03154 | 1.52E-02 | 13995 | 4485 | 8002 | 4171 |
| 670 | Troponin_I | P19429 | 1.53E-02 | 531 | 268 | 364 | 255 |
| 671 | NG36 | Q96KQ7 | 1.56E-02 | 408 | 316 | 374 | 317 |
| 672 | FCG3B | 075015 | 1.59E-02 | 3109 | 1876 | 2892 | 1587 |
| 613 | LYN | P07948 | 1.61E-02 | 10289 | 3019 | 6029 | 2235 |
| 674 | LGMN | Q99538 | 1.62E-02 | 4938 | 3745 | 4412 | 3661 |
| 675 | IL-11 | P20809 | 1.66E-02 | 380 | 127 | 230 | 122 |
| 676 | Marapsin | Q9BQR3 | 1.67E-02 | 9720 | 6720 | 8822 | 6070 |
| 677 | AMNLS | Q9BXJ7 | 1.73E-02 | 106 | 70 | 88 | 67 |
| 678 | N-terminal_pro-BNP | P16860 | 1.74E-02 | 15980 | 9783 | 13394 | 9384 |
| 679 | Kallikrein_4 | Q9Y5K2 | 1.75E-02 | 249 | 145 | 187 | 120 |
| 680 | OX40_Ligand | P23510 | 1.81E-02 | 279 | 203 | 236 | 194 |
| 681 | Cystatin_C | P01034 | 1.81E-02 | 3119 | 2139 | 2928 | 2204 |
| 682 | Epithelial_cell_kinase | P29317 | 1.83E-02 | 6302 | 3590 | 5724 | 3508 |
| 683 | IL-2. sRa | P01589 | 1.86E-02 | 14187 | 3821 | 10897 | 1057 |
| 684 | FER | P16591 | 1.87E-02 | 1673 | 350 | 932 | 255 |
| 685 | Ck-b-8-1 | P55773 | 1.91E-02 | 68059 | 45201 | 64163 | 44113 |
| 686 | PACAP-38 | P18509 | 1.95E-02 | 175 | 108 | 130 | 100 |
| 687 | XEDAR | Q9HAV5 | 1.97E-02 | 1142 | 693 | 914 | 593 |
| 688 | b2-Microglobulin | P61759 | 2.01E-02 | 3797 | 2104 | 3400 | 1819 |
| 689 | uPA | P00749 | 2.01E-02 | 2358 | 1525 | 2115 | 1329 |
| 690 | GIB | P94054 | 2.05E-02 | 1649 | 1067 | 1330 | 996 |
| 691 | IL-20_Ra | Q9UHF4 | 2.06E-02 | 214 | 178 | 195 | 175 |
| 692 | BTC | P35070 | 2.08E-02 | 398 | 246 | 295 | 216 |
| 693 | PUR8 | P30566 | 2.09E-02 | 13453 | 6307 | 6808 | 4689 |
| 694 | CBPE | P16870 | 2.09E-02 | 1015 | 634 | 763 | 653 |
| 695 | Coagulation_Factor_Xa | P00742 | 2.11E-02 | 2602 | 1916 | 2419 | 1717 |
| 696 | GDF2 | Q9UK05 | 2.14E-02 | 733 | 613 | 660 | 618 |
| 697 | FAM107B | Q9H098 | 2.15E-02 | 1824 | 1118 | 1761 | 842 |
| 698 | PARC | P55774 | 2.16E-02 | 13696 | 8211 | 10627 | 6989 |
| 699 | NRX1B | P58400 | 2.18E-02 | 392 | 304 | 356 | 310 |
| 700 | KPCT | Q04759 | 2.19E-02 | 2217 | 971 | 1530 | 711 |
| 701 | MMP-13 | P45452 | 2.20E-02 | 579 | 409 | 492 | 429 |
| 702 | C1QR1 | Q9NPY3 | 2.23E-02 | 16555 | 9263 | 15923 | 7598 |
| 703 | Peroxiredoxin-6 | P30041 | 2.24E-02 | 372 | 215 | 281 | 178 |
| 704 | SSRP1 | Q08945 | 2.35E-02 | 1185 | 251 | 447 | 251 |
| 705 | HAI-1 | 043278 | 2.40E-02 | 5472 | 4118 | 5259 | 4155 |
| 706 | LEG9 | 000182 | 2.42E-02 | 4238 | 2311 | 3118 | 1910 |
| 707 | p27Kip1 | P46527 | 2.44E-02 | 3786 | 855 | 1125 | 669 |
| 708 | Layilin | Q6UX15 | 2.47E-02 | 1960 | 1298 | 1819 | 1157 |
| 709 | CLM6 | Q08708 | 2.47E-02 | 8624 | 4427 | 7724 | 3205 |
| 710 | Troponin_I__skeletal__fast_twitch | P48788 | 2.48E-02 | 11782 | 5809 | 6921 | 5298 |
| 711 | SLIK5 | 094991 | 2.51E-02 | 2452 | 2017 | 2366 | 1987 |
| 712 | NovH | P48745 | 2.51E-02 | 793 | 478 | 617 | 419 |
| 713 | NANOG | Q9H9S0 | 2.52E-02 | 96 | 67 | 84 | 55 |
| 714 | FGR | P09769 | 2.65E-02 | 626 | 303 | 446 | 321 |
| 715 | PolyUbiquitin_K63 | P0CG48 | 2.66E-02 | 5992 | 3216 | 4472 | 2654 |
| 716 | Coagulation_Factor_X | P00742 | 2.70E-02 | 3122 | 2296 | 2921 | 2035 |
| 717 | TRAIL_R2 | 014763 | 2.74E-02 | 282 | 189 | 233 | 160 |
| 718 | BGH3 | Q15582 | 2.76E-02 | 40693 | 24378 | 36082 | 18838 |
| 719 | IL-13_Ra1 | P78552 | 2.76E-02 | 3983 | 2544 | 3532 | 2441 |
| 720 | Activin_A | P08476 | 2.77E-02 | 7605 | 4903 | 5457 | 4136 |
| 721 | Fas__soluble | P25445 | 2.78E-02 | 2753 | 1946 | 2594 | 1851 |
| 722 | Tropomyosin_1_alpha_chain | P09493 | 2.78E-02 | 1058 | 717 | 845 | 665 |
| 723 | BGN | P21810 | 2.78E-02 | 20412 | 16499 | 18590 | 16332 |
| 724 | FABP | P05413 | 2.79E-02 | 32494 | 18578 | 21796 | 22317 |
| 725 | Calcineurin_B_a | P63098 | 2.83E-02 | 14630 | 11160 | 11803 | 10800 |
| 726 | CD36_ANTIGEN | P16671 | 2.84E-02 | 11119 | 6660 | 9036 | 6399 |
| 727 | Mesothelin | Q13421 | 2.86E-02 | 362 | 223 | 276 | 214 |
| 728 | Carbonic_anhydrase_VII | P43166 | 2.87E-02 | 321 | 156 | 199 | 156 |
| 129 | CREL1 | Q96HD1 | 2.88E-02 | 3737 | 2643 | 3379 | 2302 |
| 730 | TNF-a | P01375 | 2.92E-02 | 375 | 275 | 331 | 287 |
| 731 | WFKN1 | Q96NZ8 | 2.94E-02 | 181 | 134 | 170 | 124 |
| 732 | Elastase | P08246 | 2.97E-02 | 2231 | 914 | 1438 | 712 |
| 733 | TXD12 | 095881 | 2.99E-02 | 2350 | 1844 | 2285 | 1723 |
| 734 | UBP25 | Q9UHP3 | 2.99E-02 | 1583 | 778 | 975 | 793 |
| 735 | Nogo_Receptor | Q9BZR6 | 3.06E-02 | 2735 | 1606 | 2028 | 1264 |
| 736 | PLXC1 | 060486 | 3.08E-02 | 872 | 626 | 813 | 567 |
| 737 | NKp30 | 014931 | 3.11E-02 | 474 | 305 | 394 | 275 |
| 738 | Lectin_mannose-binding_2 | Q12907 | 3.17E-02 | 20793 | 17331 | 19690 | 17322 |
| 739 | Endothelin-converting_enzyme_1 | P42892 | 3.25E-02 | 9062 | 11034 | 8752 | 11606 |
| 740 | Endoglin | P17813 | 3.27E-02 | 5496 | 3729 | 4994 | 3429 |
| 741 | PDGFRA | P16234 | 3.29E-02 | 6469 | 4279 | 4786 | 4674 |
| 742 | GPDA | P21695 | 3.36E-02 | 3984 | 2250 | 2922 | 2293 |
| 743 | ANK2 | Q01484 | 3.37E-02 | 372 | 298 | 331 | 290 |
| 744 | ASM3A | Q92484 | 3.38E-02 | 5904 | 3491 | 4333 | 2780 |
| 745 | IL-23 | P29460_Q | 3.49E-02 | 6496 | 2549 | 4751 | 1496 |
| 746 | Nectin-like_protein_1 | Q8N126 | 3.52E-02 | 4013 | 3098 | 4017 | 2853 |
| 747 | CO8A1 | P27658 | 3.54E-02 | 1497 | 768 | 756 | 663 |
| 748 | LIMP_II | Q14108 | 3.64E-02 | 346 | 211 | 219 | 191 |
| 749 | Factor D | P00746 | 3.66E-02 | 723 | 618 | 705 | 592 |
| 750 | NADPH-P450_Oxidoreductase | P16435 | 3.70E-02 | 17437 | 6380 | 8332 | 5931 |
| 751 | AFP | P02771 ' | 3.73E-02 | 639 | 444 | 547 | 396 |
| 752 | PGRP-S | 075594 | 3.75E-02 | 1762 | 773 | 1391 | 406 |
| 753 | Calcineurin | Q08209_P | 3.75E-02 | 2479 | 582 | 1021 | 494 |
| 754 | Lipocalin_2 | P80188 | 3.79E-02 | 20416 | 10600 | 15426 | 8474 |
| 755 | OX2G | P41217 | 3.81E-02 | 3644 | 2795 | 3433 | 2538 |
| 756 | AMPK_a2b2g1 | P54546_O | 3.81E-02 | 7390 | 3291 | 5042 | 2120 |
| 757 | Glypican_3 | P51654 | 3.83E-02 | 759 | 604 | 709 | 598 |
| 758 | 14-3-3E | P62258 | 3.85E-02 | 6614 | 4345 | 4252 | 5138 |
| 759 | Prothrombin | P00734 | 3.88E-02 | 101692 | 115113 | 100580 | 112521 |
| 760 | MED-1 | Q15648 | 3.94E-02 | 35616 | 46117 | 34247 | 45516 |
| 761 | DAN | P41271 | 4.06E-02 | 10871 | 5650 | 6792 | 3904 |
| 762 | IGF-I | P05019 | 4.07E-02 | 957 | 739 | 972 | 803 |
| 763 | DAF | P08174 | 4.14E-02 | 19961 | 14235 | 17881 | 12018 |
| 764 | SIG14 | Q08ET2 | 4.19E-02 | 19449 | 8372 | 20606 | 6914 |
| 765 | KEAP1 | Q14145 | 4.19E-02 | 3411 | 550 | 994 | 534 |
| 766 | BNP-32 | P16860 | 4.22E-02 | 347 | 223 | 262 | 226 |
| 767 | gpllbllla | P08514_P | 4.26E-02 | 1808 | 1037 | 1211 | 841 |
| 768 | kallikrein_14 | Q9P0G3 | 4.28E-02 | 11774 | 9747 | 10856 | 9696 |
| 769 | URB | Q76M96 | 4.29E-02 | 2927 | 1882 | 2464 | 1864 |
| 770 | IL-17 | Q16552 | 4.32E-02 | 321 | 191 | 234 | 195 |
| 771 | SDF-1 | P48061 | 4.33E-02 | 5947 | 3701 | 5300 | 2876 |
| 772 | cGMP-stimulated..PDE | 000408 | 4.45E-02 | 2259 | 1834 | 1955 | 1838 |
| 773 | Persephin | 060542 | 4.49E-02 | 182 | 121 | 158 | 105 |
| 774 | Glucagon | P01275 | 4.50E-02 | 2275 | 6302 | 1586 | 6369 |
| 775 | calgranulin B | P06702 | 4.52E-02 | 4269 | 6219 | 3553 | 6005 |
| 776 | FGF23 | Q9GZV9 | 4.54E-02 | 1867 | 710 | 628 | 588 |
| 777 | dopa_decarboxylase | P20711 | 4.58E-02 | 323 | 159 | 209 | 158 |
| 778 | ANP | P01160 | 4.62E-02 | 494 | 303 | 379 | 247 |
| 779 | MMP-2 | P08253 | 4.63E-02 | 6552 | 9196 | 6692 | 8603 |
| 780 | Galectin-8 | 000214 | 4.71E-02 | 356 | 295 | 321 | 287 |
| 181 | Eotaxin | P51671 | 4.73E-02 | 47139 | 63123 | 46050 | 60905 |
| 782 | Cytochrome_c | P99999 | 4.75E-02 | 1368 | 934 | 1078 | 809 |
| 783 | TGF-b2 | P61812 | 4.75E-02 | 279 | 100 | 150 | 91 |
| 784 | MK13 | 015264 | 4.77E-02 | 1296 | 456 | 573 | 432 |
| 185 | DLL1 | O00548 | 4.86E-02 | 2578 | 1946 | 2222 | 1973 |
| 786 | MP2K4 | P45985 | 4.89E-02 | 26031 | 40552 | 23121 | 43510 |
| 787 | IR | P06213 | 4.91E-02 | 11211 | 7984 | 9398 | 7048 |
| 788 | TSH | P01215_P | 4.92E-02 | 1427 | 945 | 1051 | 951 |
| 789 | IL-17E | Q9H293 | 4.94E-02 | 668 | 510 | 607 | 465 |
| 790 | NRP1 | 014786 | 4.95E-02 | 1875 | 1451 | 1874 | 1522 |

With respect to the proteins represented by Nos. 468, 595, 607, 642, 661, 668, 739, 759, 760, 774, 775, 779, 781 and 786 of Table 2, a significant increase was observed in the remission group compared to the patient group.

In contrast, with respect to the proteins represented by Nos. 1 to 467, 469 to 594, 596 to 606, 608 to 641, 643 to 660, 662 to 667, 669 to 738, 740 to 758, 761 to 773, 776 to 778, 780, 782 to 785 and 786 to 790 of Table 2, a significant decrease was observed in the remission group compared to the patient group.

The p values in the increase or decrease in amount of the proteins represented by Nos. 1 to 440 were less than 0.001.

The p values in the increase or decrease in amount of the proteins represented by Nos. 441 to 633 were less than 0.01.

The p values in the increase or decrease in amount of the proteins represented by Nos. 634 to 790 were less than 0.05.

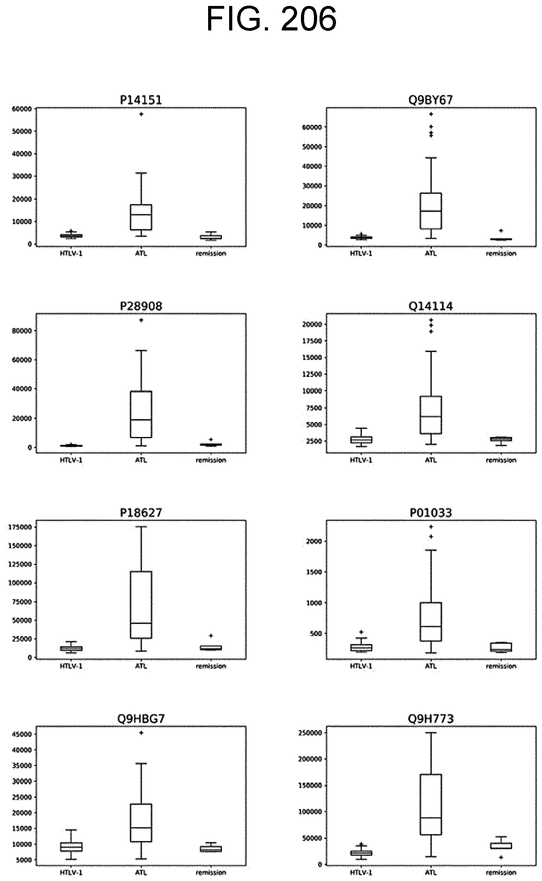

Of the proteins listed in Table 1 and Table 2, with respect to the proteins (represented by Nos. 7, 9, 10, 17, 20, 21, 22, 25, 28, 33, 37, 39, 42, 43, 44, 46, 47, 48, 49, 56, 57, 59, 61, 62, 64, 65, 66, 67, 70, 73, 74, 75, 77, 79, 80, 81, 82, 88, 89, 92, 93, 94, 95, 97, 99, 100, 101, 102, 103, 106, 108, 112, 115, 120, 121, 123, 124, 125, 128, 129, 131, 139, 141, 155, 156, 161, 164, 165, 167, 172, 173, 174, 175, 181, 185, 187, 189, 195, 198, 199, 200, 203, 205, 207, 209, 211, 220, 221, 222, 223, 224, 225, 226, 230, 231, 232, 236, 238, 240, 241, 242, 243, 244, 246, 247, 250, 251, 252, 253, 256, 259, 260, 269, 271, 274, 279, 288, 289, 294, 296, 299, 300, 302, 306, 307, 309, 317, 318, 322, 327, 330, 331, 332, 339, 343, 345, 346, 347, 350, 357, 359, 364, 366, 373, 374, 375, 378, 382, 387, 390, 393, 394, 399, 400, 401, 405, 411, 418, 425, 427, 431, 438, 440, 446, 447, 450, 451, 452, 456, 460, 476, 477, 480, 481, 487, 504, 511, 524, 531, 534, 542, 548, 551, 552, 555, 573, 584, 586, 594, 606, 609 and 629 of Table 1) showing a significant increase in the patient group compared to the carrier group and a significant decrease in the remission group compared to the patient group, or the proteins (represented by Nos. 126, 170, 235, 270 and 621 of Table 1) showing a significant decrease in the patient group compared to the carrier group and a significant increase in the remission group compared to the patient group, the measurement results are shown in Figures 206 to 230.

These proteins can serve as an onset marker for discriminating between a carrier group and a patient group and as a remission marker for discriminating between a patient group and a remission group; and can be a particularly useful marker for ATL diagnosis.

## Claims

1. A diagnostic method for adult T-cell leukemia (ATL), comprising the steps of:
measuring an amount of a marker protein in a blood sample taken from a subject, wherein the marker protein is Coagulation Factor Xa; and
if a measured value is a predetermined reference value or more, determining that when the subject is a carrier of human T-cell leukemia virus type 1 (HTLV-1) or an individual in remission of ATL, the subject suffers from, or is likely to develop, ATL.

2. The diagnostic method according to claim 1, wherein the blood sample is plasma.

3. In vitro use of the protein Coagulation Factor Xa for diagnosing adult T-cell leukemia (ATL).

## Patentansprüche

1. Diagnostisches Verfahren für adulte T-Zell-Leukämie (adult T-cell leukemia; ATL), umfassend die Schritte:
Messen einer Menge eines Markerproteins in einer Blutprobe, die einem Individuum entnommen wurde, wobei das Markerprotein Gerinnungsfaktor Xa ist; und
wenn ein gemessener Wert ein vorbestimmter Referenzwert oder mehr ist, Bestimmen, dass, wenn das Individuum ein Träger von humanem T-Zell-Leukämie-Virus Typ 1 (human T-cell leukemia virus type 1; HTLV-1) oder ein Individuum in Remission von ATL ist, das Individuum an ATL leidet oder wahrscheinlich ATL entwickeln wird.

2. Diagnostisches Verfahren nach Anspruch 1, wobei die Blutprobe Plasma ist.

3. In vitro-Verwendung des Proteins Gerinnungsfaktor Xa zur Diagnose von adulter T-Zell-Leukämie (ATL).

## Revendications

1. Méthode de diagnostic de la leucémie à lymphocytes T de l'adulte (ATL), comprenant les étapes consistant à :
mesurer une quantité d'une protéine marqueur dans un échantillon de sang prélevé sur un sujet, dans laquelle la protéine marqueur est le facteur de coagulation Xa ; et
si une valeur mesurée est une valeur de référence prédéterminée ou plus, déterminer que, lorsque le sujet est porteur du virus de type 1 de la leucémie à lymphocytes T humaine (HTLV-1) ou un individu en rémission d'ATL, le sujet souffre d'ATL, ou est susceptible de développer celle-ci.

2. Méthode de diagnostic selon la revendication 1, dans laquelle l'échantillon de sang est du plasma.

3. Utilisation in vitro de la protéine facteur de coagulation Xa pour le diagnostic de la leucémie à lymphocytes T de l'adulte (ATL).
